# EUROPEAN PATENT SPECIFICATION

(11) **EP 1 268 835 B1**
(45) Date of publication and mention of the grant of the patent: **16.01.2008**
(21) Application number: 01917270.9
(22) Date of filing: 30.03.2001
(51) Int. Cl.: C12N 15/867, C12N 15/12, C07K 14/705, G01N 33/68, A61K 38/17, A61K 31/203, A61K 31/381, A61K 48/00, A61P 25/00

(54) **RETINOIC ACID RECEPTOR BETA-2 AND GENE THERAPY VECTORS FOR THE TREATMENT OF NEUROLOGICAL DISORDERS**
RETINOLSÄURE-REZEPTOR BETA-2 UND GENTHERAPIEVEKTOREN FÜR DIE BEHANDLUNG VON NEUROLOGISCHEN ERKRANKUNGEN
RECEPTEUR DE L'ACIDE RETINOIQUE BETA 2 ET VECTEURS DE THERAPIE GENIQUE POUR LE TRAITEMENT DE TROUBLES NEUROLOGIQUES

(30) Priority: 30.03.2000 WO PCT/GB00/01211; 04.10.2000 GB 0024300
(43) Date of publication of application: 02.01.2003
(73) Proprietor: Oxford Biomedica (UK) Limited, Oxford 0X4 4GA (GB)
(72) Inventor: KINGSMAN, Alan, John, Oxford BioMedica (UK) Ltd, Oxford OX4 4GA (GB); MADEN, M, Biomedical Sciences Division, London, WC2B 5RL (GB); CORCORAN, J, Biomedical Sciences Division, London, WC2B 5RL (GB)
(74) Representative: Harding, Charles Thomas
(86) International application number: PCT/GB2001/001478
(87) International publication number: WO 2001/075135

(56) References cited:
- EP-A- 0 943 684
- WO-A-00/57900
- WO-A-96/23070
- WO-A-97/02030
- WO-A-97/24116
- WO-A-99/21574
- WO-A-99/32646
- US-A- 4 808 630
- WAN H. ET AL: "Overexpression of retinoic acid receptor.beta. in head and neck of squamous cell carcinoma cells increases their sensitivity to retinoid-induced suppression of squamous differentiation by retinoids." CANCER RESEARCH, vol. 59, no. 14, 15 July 1999 (1999-07-15), pages 3518-3526, XP002175703
- CORCORAN J. ET AL.: "Nerve growth factor acts via retinoic acid synthesis to stimulate neurite outgrowth." NATURE NEUROSCIENCE, vol. 2, no. 4, April 1999 (1999-04), pages 307-308, XP000946659 ISSN: 1097-6256 cited in the application
- MADEN M ET AL: "RETINOIC ACID AND DEVELOPMENT OF THE CENTRAL NERVOUS SYSTEM" BIOESSAYS, vol. 14, no. 7, 1992, pages 431-438, XP002149699 ISSN: 0265-9247
- JOHNSON A T ET AL: "IDENTIFICATION OF RETINOIC ACID RECEPTOR BETA SUBTYPE SPECIFIC AGONISTS" JOURNAL OF MEDICINAL CHEMISTRY,US,AMERICAN CHEMICAL SOCIETY. WASHINGTON, vol. 39, no. 26, 20 December 1996 (1996-12-20), pages 5027-5030, XP002065588 ISSN: 0022-2623
- MADEN M ET AL: "Retinoic acid as a chemotactic molecule in neuronal development." INTERNATIONAL JOURNAL OF DEVELOPMENTAL NEUROSCIENCE, vol. 16, no. 5, August 1998 (1998-08), pages 317-322, XP000946687 ISSN: 0736-5748 cited in the application
- HAN, GUANGYANG ET AL: "Enhanced potency of 9-cis versus all-trans-retinoic acid to induce the differentiation of human neuroblastoma cells" DIFFERENTIATION (BERLIN) (1995), VOLUME DATE 1995, 59(1), 61-9, XP000946682
- CORCORAN JONATHAN ET AL: "The role of retinoic acid receptors in neurite outgrowth from different populations of embryonic mouse dorsal root ganglia." JOURNAL OF CELL SCIENCE, vol. 113, no. 14, July 2000 (2000-07), pages 2567-2574, XP000946689 ISSN: 0021-9533
- FALANGA, R. ET AL.: "Cancer procoagulant and tissue factor are differently modulated by all-trans-retinnoic acid in acute pormyelocytic leukemia cells."" BLOOD, vol. 92, no. 1, 1 July 1998 (1998-07-01), pages 143-151,

## Description

### FIELD OF THE INVENTION

The present invention relates to vectors capable of directing the expression of a factor relating to neurite growth.

### BACKGROUND TO THE INVENTION

The human peripheral and central nervous system consists of terminally differentiated cells which are not capable of directing neurite outgrowth or neurite regeneration.

It is desirable to cause neurite development, such as neurite outgrowth and/or neurite regeneration, for example in cases of nervous injuries such as spinal cord injuries or in diseases such as diabetes or neuropathies.

Nerve growth factor (NGF) is known to stimulate certain events such as neurite outgrowth. However, NGF is a relatively large molecule with a correspondingly high molecular weight Moreover, NGF is susceptible to protease mediated degradation. Due to these and other considerations, NGF is difficult to administer. NGF is also relatively expensive to prepare. These are problems associated with the prior art.

### SUMMARY OF THE INVENTION

We have surprisingly found that it is possible to cause neurite development, such as neurite outgrowth and/or neurite regeneration, by using retinoic acid receptor β2 (RARβ2). Moreover, it is surprisingly shown that RARβ2 can be delivered to non-dividing mammalian cells using vectors according to the invention.

### SUMMARY ASPECTS OF THE PRESENT INVENTION

The present invention is based on the surprising finding that it is possible to cause neurite development, such as neurite outgrowth and/or neurite regeneration, by using RARβ2 and that RARβ2 may be introduced into neuronal cells using retroviral vectors based on lentiviral vectors.

Aspects of the present invention utilise this finding. For example it is possible to have a method that causes modulation of neurite development, such as neurite outgrowth and/or neurite regeneration, by using a vector comprising a nucleic acid sequence encoding RARβ2.

### DETAILED ASPECTS OF THE PRESENT INVENTION

In one aspect, the present invention relates to a lentiviral vector comprising a nucleic acid sequence encoding RARβ2.

In another aspect, the present invention relates to the use of a vector as described herein in the preparation of a medicament to cause neurite development.

In another aspect, the present invention relates to the use of a vector as described herein in the preparation of a medicament for the treatment of a neurological disorder.

In another aspect, the present invention relates to a host cell when transduced by a vector as described herein.

In another aspect, the present invention relates to a pharmaceutical composition comprising a vector as described herein in admixture with a pharmaceutically acceptable carrier, diluent or exceipient; wherein the pharmaceutical composition is for use to cause neurite development.

The term 'RARβ2' as used herein may refer to the polypeptide translation product of the RARβ2 gene open reading frame (ORF), that is to say the actual receptor itself, or may refer to the nucleic acid ORF encoding said polypeptide, or may even occasionally refer to the RARβ2 gene itself. It will be apparent to the reader which of these entities, or combination of said entities, is referred to by the term RARβ2 from the particular context in which such term is used.

In the present invention RARβ2 can be termed a pharmaceutically active agent.

Neurites are well known structures which develop from various neuronal cell types. They appear as microscopic branch or comb-like structures or morphological projections from the surface of the cell from which they emanate. Examples of neurite outgrowth are shown in the accompanying figures, and in publications such as those referenced in (Maden 1988-review article), and are well known in the art

The RARβ2 coding sequence (i.e. the RARβ2 gene) is used as described hereinbelow. The RARβ2 gene may be prepared by use of recombinant DNA techniques and/or by synthetic techniques. For example, it may be prepared using the PCR amplified gene fragment prepared as in the Examples section of this document using the primers etc. detailed therein, or it may be prepared according to any other suitable method known in the art

In another aspect, the present invention relates to the use of a vector as described herein in conjunction with an RARβ2 agonist. The agonist may be retinoic acid (RA) and/or CD2019.

Retinoic acid is commercially available. CD2019 is a polycyclic heterocarbyl molecule which is a RARβ2 agonist having the structure as discussed herein and as shown in (Elmazar et al., (1996) Teratology vol. 53 pp158-167).

In a further aspect, the invention relates to a pharmaceutical composition comprising a vector as described herein and optionally an agonist thereof in admixture with a pharmaceutically acceptable carrier, diluent or excipient; wherein the pharmaceutical composition is for use to cause neurite development.

The vector of the present invention may comprise a deleted gag gene from a lentivirus wherein the deletion in gag removes one or more nucleotides downstream of nucleotide 350 of the gag coding sequence. Preferably the deletion extends from nucleotide 350 to at least the C-teminus of the gagpol coding region. More preferably the deletion additionally removes nucleotide 300 of the *gag* coding region and most preferably the deletion retains only the first 150 nucleotides of the *gag* coding region. However even larger deletions of *gag* can also be used, for example the *gag* coding region may contain only the first 109 nucleotides of the *gag* coding region. It may also be possible for the *gag* coding region to contain only the first 2 nucleotides of the *gag* coding region. Preferably, said vector genome is capable of directing the expression of substantially all of the RARβ2 polypeptide.

The vector of the present invention is based on or derived from a lentivirus. The vector of the present invention may be based on or derived from a non-primate lentivirus. In a highly preferred embodiment, the vector of the present invention is based on or derived from a non-primate lentivirus such as equine infectious anaemia virus (EIAV). This is discussed in more detail below.

Additional features of the lentiviral genome are included in the vector genome which are necessary for transduction of the target cell such as reverse transcription and integration. These are, at least, a portion of an LTR containing sequence from the R-region and U5 region, sequences adjacent to the 3' LTR which contain a polypurine tract (PPT) and a 3'LTR from the lentivirus or a hybrid LTR containing sequences from the lentivirus and other elements. Optionally, the retroviral genome may contain accessory genes derived from a retrovirus, such as, but not limited to, a rev gene, a *tat* gene, a vif gene, a *nef* gene, a vpr gene or an *S*2 gene. Additional components may be added such as introns, splice-donor sites, a rev responsive element (RRE), sequences called the cPPT containing the polymerase region (Stetor SR, Rausch JW, Guo MJ, Burnham JP, Boone LR, Waring MJ, Le Grice SF 'Characterization of (+) strand initiation and termination sequences located at the center of the equine infectious anemia virus genome.' Biochemistry. 1999 Mar 23;38(12):3656-67), cloning sites and selectable marker genes.

Moreover, it has been demonstrated (eg. see WO 99/32646) that a lentivirus minimal vector system can be constructed which requires neither S2, Tat, env nor dUTPase for either vector production or for transduction of dividing and non-dividing cells. A lentivirus minimal vector system can also be constructed which requires neither S2, Tat, env, rev nor dUTPase for either vector production or for transduction of dividing and non-dividing cells.

Thus according to another aspect the lentivirus genome from which the vector is derived lacks one or more accessory genes.

The deletion of accessory genes is highly advantageous. Firstly, it permits vectors to be produced without the genes normally associated with disease in lentiviral (e.g. HIV) infections. In particular, *tat* and nef are associated with disease. Secondly, the deletion of accessory genes permits the vector to package more heterologous DNA. Thirdly, genes whose function is unknown, such as *dUTP*ase and S2, may be omitted, thus reducing the risk of causing undesired effects.

In addition, we have shown that the leader sequence of the lentivirus genome is essential for high protein expression.

Therefore in a further aspect the lentivirus genome from which the vector is derived lacks the *tat* gene but includes the leader sequence between the end of the 5' LTR and the ATG of *gag*.

These data further define a minimal essential set of functional components for an optimal lentiviral vector. A vector is provided with maximal genetic capacity and high titre, but without accessory genes that are either of unknown function (S2, *UTPase*), and therefore may present risk, or are analogues of HIV proteins that may be associated with AIDS *(tat, rev).*

It will be appreciated that the present invention provides a retroviral vector derived from a lentivirus genome comprising nucleic acid sequence capable of directing the expression of RARβ2 and (1) comprising a deleted *gag* gene wherein the deletion in gag removes one or more nucleotides downstream of nucleotide 350 of the *gag* coding sequence; (2) wherein one or more accessory genes are absent from the lentivirus genome; (3) wherein the lentivirus genome lacks the *tat* gene but includes the leader sequence between the end of the 5' LTR and the ATG of *gag*; and combinations of (1), (2) and (3). In a preferred embodiment the retroviral vector comprises all of features (1) and (2) and (3).

A "non-primate" vector, as used herein, refers to a vector derived from a virus which does not primarily infect primates, especially humans. Thus, non-primate virus vectors include vectors which infect non-primate mammals, such as dogs, sheep and horses, reptiles, birds and insects.

A lentiviral or lentivirus vector, as used herein, is a vector which comprises at least one component part derived from a lentivirus. Preferably, that component part is involved in the biological mechanisms by which the vector infects cells, expresses genes or is replicated.

The lentivirus may be any member of the family of lentiviridae. Preferably the lentivirus is one which does not naturally infect a primate ('non-primate lentivirus'). Such viruses may include a feline immunodeficiency virus (FIV), a bovine immunodeficiency virus (BIV), a caprine arthritis encephalitis virus (CAEV), a Maedi visna virus (MVV) or an equine infectious anaemia virus (EIAV). Preferably the lentivirus is an EIAV. Equine infectious anaemia virus infects all equidae resulting in plasma viremia and thrombocytopenia (Clabough, et al. 1991. J Virol. 65:6242-51). Virus replication is thought to be controlled by the process of maturation of monocytes into macrophages.

EIAV has the simplest genomic structure of the lentiviruses. In addition to the *gag, pol* and *env* genes EIAV encodes three other genes: *tat, rev,* and *S2. Tat* acts as a transcriptional activator of the viral LTR (Derse and Newbold1993 Virology. 194:530-6; Maury, et al 1994 Virology. 200:632-42.) and Rev regulates and coordinates the expression of viral genes through rev-response elements (RRE) (Martarano et al 1994 J Virol. 68:3102-11.). The mechanisms of action of these two proteins are thought to be broadly similar to the analogous mechanisms in the primate viruses (Martano et al ibid). The function of S2 is unknown. In addition, an EIAV protein, Ttm, has been identified that is encoded by the first exon of *tat* spliced to the *env* coding sequence at the start of the transmembrane protein.

In addition to protease, reverse transcriptase and integrase lentiviruses contain a fourth *pol* gene product which codes for a dUTPase. This may play a role in the ability of these lentiviruses to infect certain non-dividing cell types.

The viral RNA is transcribed from a promoter, which may be of viral or non-viral origin, but which is capable of directing expression in a eukaryotic cell such as a mammalian cell. Optionally an enhancer is added, either upstream of the promoter or downstream. The RNA transcript is terminated at a polyadenylation site which may be the one provided in the lentiviral 3' LTR or a different polyadenylation signal.

A DNA transcription until comprises a promoter and optionally an enhancer capable of directing expression of a retroviral vector genome.

Transcription units as described herein comprise regions of nucleic acid containing sequences capable of being transcribed. Thus, sequences encoding mRNA, tRNA and rRNA are included within this definition. The sequences may be in the sense or antisense orientation with respect to the promoter. Antisense constructs can be used to inhibit the expression of a gene in a cell according to well-known techniques. Nucleic acids may be, for example, ribonucleic acid (RNA) or deoxyribonucleic acid (DNA) or analogues thereof. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements. Nucleic acids may comprise cDNA or genomic DNA (which may contain introns).

In another aspect, the present invention relates to a retroviral vector derived from a lentivirus genome comprising a nucleic acid sequence capable of directing the expression of RARβ2 and comprising a deleted *gag* gene wherein the deletion in *gag* removes one or more nucleotides downstream of nucleotide 350 of the *gag* coding sequence.

In another aspect, the present invention relates to a lentiviral vector as described herein, wherein the deletion extends from nucleotide 350 to at least the C-terminus of the *gag-pol* coding region.

In another aspect, the present invention relates to a lentiviral vector as described herein, wherein the deletion additionally removes nucleotide 300 of the *gag* coding region.

In another aspect, the present invention relates to a lentiviral vector as described herein, wherein the deletion retains the first 150 nucleotides of the *gag* coding region.

In another aspect, the present invention relates to a lentiviral vector as described herein, wherein the deletion retains the first 109 nucleotides of the *gag* coding region.

In another aspect, the present invention relates to a lentiviral vector as described herein, wherein the deletion retains only the first 2 nucleotides of the *gag* coding region.

In another aspect, the present invention relates to a lentiviral vector as described herein, wherein the deletion is of the entire *gag* coding region.

In another aspect, the present invention relates to a retroviral vector derived from a lentivirus genome wherein one or more accessory genes are absent from the lentivirus genome.

In another aspect, the present invention relates to a lentiviral vector as described herein, wherein the accessory genes are selected from *dUTPase, S2, rev* and *tat.*

In another aspect, the present invention relates to a retroviral vector derived from a lentivirus genome such as EIAV wherein the lentivirus genome lacks the *tat* gene but includes the leader sequences between the end of the 5' LTR and the ATG of *gag*.

In another aspect, the present invention relates to a lentiviral vector as described herein, which comprises at least one component from an equine lentivirus.

In another aspect, the present invention relates to a lentiviral vector as described herein, wherein the equine lentivirus is EIAV.

In another aspect, the present invention relates to a lentiviral vector as described herein, wherein the retroviral vector is substantially derived from EIAV.

In another aspect, the present invention relates to a method comprising transfecting or transducing a cell *in vitro* with a lentiviral vector as described herein.

In another aspect, the present invention relates to a delivery system in the form of a vector as described herein.

In another aspect, the present invention relates to a cell transfected or transduced with a vector as described herein.

In another aspect, the present invention relates to use of a vector as described herein.

In another aspect, the invention relates to the use of a vector as described herein in the preparation of a medicament for the delivery of retinoic acid receptor β2 to a cell comprised by the peripheral and central nervous systems.

Expression of RARβ2 in adult spinal cord is shown to stimulate neurite outgrowth and regeneration. Thus, in a preferred aspect, the invention relates to the use of vectors as defined herein in the preparation of a medicament in to methods for stimulation of neurite outgrowth and/or regeneration in mammalian neuronal cells.

As used herein, the term 'adult' is used to mean non-foetal and/or non-embryonic. The term thus includes adults per se, as well as including young such as children and/or pups or other such infants. Thus, the term 'adult' as used herein may be understood to include any 'post-natal' ie. post-birth organism.

The vectors of the present invention may be used in an in vitro differential expression screening method for identifying genes involved in a cellular process which method comprises comparing gene expression in: a first cell of interest; and a second cell of interest which cell comprises altered levels, relative to physiological levels, of a biological molecule due to the introduction into the second cell of a heterologous nucleic acid encoding RARβ2; and identifying gene products whose expression differs.

Optionally, retinoic acid or an analogue thereof may also be present in the cellular environment of one or preferably both cells of interest. This method or a variant thereof may be advantageously applied to comparison of non-dividing neuronal cells with a different sample of the same cells which have been induced to exhibit neurite outgrowth, such as via transduction with a vector delivering RAPβ2, or via other techniques discussed herein.

For ease of reference, these and further aspects of the present invention are now discussed under appropriate section headings. However, the teachings under each section are not necessarily limited to each particular section.

### PREFERABLE ASPECTS

In a preferred aspect, the administration of a nucleic acid construct capable of directing the expression of RARβ2 will be accompanied by the administration of a RARβ2 agonist such as RA, or preferably CD2019 (or a mimetic thereof).

Preferably said agonist will be to some degree selective for the RARβ2 receptor. Preferably said agonist will not significantly affect the RARα receptor. Preferably said agonist will not significantly affect the RARγ receptor. More preferably said agonist will not significantly affect the RARα receptor or the RARγ receptor. Even more preferably, said agonist will exhibit a high degree of selectivity for the RARβ2 receptor.

The vector of the invention may be capable of infecting non-dividing mammalian cells such as neural cells. The vector may be derived from a lentiviral vector (preferably a non-primate lentiviral vector as discussed above), more preferably said vector will be derived from an equine infectious anaemia virus (EIAV). In a highly preferred aspect, said EIAV-derived vector will be a pseudotyped particle, such as VSV-G pseudotyped, or Rabies G pseudotyped.

### ADVANTAGES

The present invention is advantageous because RARβ2, optionally in conjunction with an agonist thereof, can cause modulation of neural cell development.

It is also an advantage of the present invention that administration of NGF to a subject is avoided.

It is also an advantage of the present invention that it enables neurite outgrowth to be promoted in adult neural tissue.

It is also an advantage of the present invention that it enables RARβ2 to be introduced into non-dividing mammalian cells such as neuronal cells.

It is also an advantage of the present invention that the receptor may be delivered to cells whose environment comprises endogenous levels of agonist of the receptor, such as retinoic acid (RA).

### RETINOIDS

Retinoids are a family of molecules derived from vitamin A and include the biologically active metabolite, retinoic acid (RA). The cellular effects of RA are mediated through the action of two classes of receptors, the retinoic acid receptors (RARs) which are activated both by all-*trans*-RA (tRA) and 9-*cis*-RA (9*-cis*-RA)*,* and the retinoid X receptors (RXRs), which are activated only by 9-*cis*-RA (Kastner et al., 1994; Kleiwer et al., 1994). The receptors are of three major subtypes, α, β and γ, of which there are multiple isoforms due to alternative splicing and differential promoter usage (Leid et al.). The RARs mediate gene expression by forming heterodimers with the RXRs, whilst the RXRs can mediate gene expression as homodimers or by forming heterodimers with a variety of orphan receptors (Mangelsdorf & Evans, 1995). Many studies on a variety of embryonic neuronal types have shown that RA can stimulate both neurite number and length (review, Maden, 1998), as, indeed, can the neurotrophins (Campenot, 1977; Lindsay, 1988; Tuttle and Mathew, 1995). The neurotrophins are a family of growth factors that are required for the survival of a variety of neurons of primary sensory neurons in the developing peripheral nervous system (Snider, 1994). One of the earliest genes induced by NGF in PC12 cells is the orphan receptor NGFI-B (NURR1) (Millbrandt, 1989). This suggests that the growth factor and retinoid mediated pathway in developing neurons can interact.

Background teachings on these aspects have been presented by Victor A. McKusick *et al* on hftp://www.ncbi.nim.nih.gov/Omim. The following information has been extracted from that source.

Three retinoic acid receptors, alpha, beta, and gamma, are members of the nuclear receptor superfamily. Retinoic acid was the first morphogen described in vertebrates. The RARA and RARB genes are more homologous to those of the 2 closely related thyroid hormone receptors THRA and THRB, located on chromosomes 17 and 3, respectively, than to any other members of the nuclear receptor family. These observations suggest that the thyroid hormone and retinoic acid receptors evolved by gene, and possibly chromosome, duplications from a common ancestor which itself diverged rather early in evolution from the common ancestor of the steroid receptor group of the family. The RARB gene, formerly symbolized HAP, maps to 3p24 by somatic cell hybridization and in situ hybridization.
Benbrook et al. (1988) showed a predominant distribution in epithelial tissues and therefore used the designation RAR(epsilon). By in situ hybridization, Mattei et al. (1988) assigned the RARB gene to 3p24. Using deletion mapping, de The et al. (1990) identified a 27-bp fragment located 59-bp upstream of the transcriptional start, which confers retinoic acid responsiveness on the herpesvirus thymidine kinase promoter. They found indications that both alpha and beta receptors act through the same DNA sequence. Mattei et al. (1991) assigned the corresponding gene to chromosome 14, band A, in the mouse, and to chromosome 15 in the rat.
Nadeau et al. (1992) confirmed assignment of the mouse homolog to the centromeric portion of chromosome 14.
From a comparison of a hepatitis-B virus (HBV) integration site present in a particular human hepatocellular carcinoma (HCC) with the corresponding unoccupied site in the nontumorous tissue of the same liver, Dejean et al. (1986) found that HBV integration placed the viral sequence next to a liver cell sequence that bears a striking resemblance to both an oncogene, ERBA, and the supposed DNA-binding domain of the human glucocorticoid receptor and estrogen receptor genes.
Dejean et al. (1986) suggested that this gene, usually silent or transcribed at a very low level in normal hepatocytes, becomes inappropriately expressed as a consequence of HBV integration, thus contributing to the cell transformation.
By means of a panel of rodent-human somatic cell hybrid DNAs, Dejean et al. (1986) localized the gene to chromosome 3. Further studies by de The et al. (1987) suggested that the HAP gene product may be a novel ligand-responsive regulatory protein whose inappropriate expression in liver is related to hepatocellular carcinogenesis. Brand et al. (1988) showed that the novel protein called HAP (for HBV-activated protein) is a retinoic acid receptor. They referred to this receptor as the beta type (RARB) and mapped it to 3p25-p21.
Lotan et al. (1995) found that the expression of RARB mRNA is selectively lost in premalignant oral lesions and can be restored by treatment with isotretinoin. Restoration of the expression of RARB mRNA was associated with a clinical response.
RARB, RARG, RXRB, and RXRG are expressed in the striatum. To study the effect of these genes on locomotion, Kreczel et al. (1998) developed single and double knockout mice and analyzed their locomotor skills by open field and rotarod testing. RARB-RXRB, RARB-RXRG, and RXRB-RXRG double null mutant mice, but not the corresponding single null mutants, exhibited reductions in forward locomotion when compared with wildtype littermates. Forty percent of the RARB-RXRB null mutants showed backward locomotion. Rotarod test performance was impaired for RARB, RARB-RXRB, RARB-RXRG, and RXRB-RXRG mice. In contrast, RARA, RARG, RARA-RXRG, and RARG-RXRG null mice showed no defects in locomotion, even though both RARA and RARG are also expressed in the striatum. The morphology, development, and function of skeletal muscle, peripheral nerves, and spinal cord were normal in all single and double null mutants, as were balance reflexes.
These results suggested to Kreczel et al. (1998) that RARB, RXRB, and RXRG are involved specifically in the control of locomotor behaviors, and that heterodimers of RARB with either RXRB or RXRG are the functional receptor units, such that RXRB and RXRG are functionally redundant. Kreczel et al. (1998) studied the expression of D1 and D2 dopamine receptors (D1R and D2R), the most abundant dopamine receptors in the striatum, in these mutant mice. RARB-RXRB, RARB-RXRG, and RXRB-RXRG double null mutants, but not RARB or RXRG single mutants, exhibited 40% and 30% reduction in whole-striatal D1R and D2R transcripts, respectively, when compared with wildtype controls.
The reduction was mostly in the medioventral regions of the striatum, including the shell and core of the nucleus accumbens, and the mediodorsal part of the candate putamen. The reduction was not due to loss of D2R-expressing neurons; no increase in apoptosis was noted. The histology of the striatum was normal.
The characterization of a retinoic acid response element in the D2R promoter by Samad et al. (1997) led Kreczel et al. (1998) to suggest that the reduction in D2R and D2R expression occurs on a transcriptional level The RARB-RXRB, RARB-RXRG, and RXRB-RXRG double null mutants did not exhibit the normal increase in locomotion induced by cocaine, mimicking the phenotype of D1R-null mice.
Taken together, these results indicated to Kreczel et al. (1998) that retinoids are involved in controlling the function of the dopaminergic mesolimbic pathway and suggested that defects in retinoic acid signaling may contribute to neurological disorders.

### AGONISTS

The agonist optionally used in conjunction with a vector of the present invention may be any suitable RARβ2 agonist. Preferably, said agonist of RARβ2 is capable of activating RARβ2 in a transactivation assay.

The agonist may be an organic compound or other chemical. The agonist can be an amino acid sequence or a chemical derivative thereof, or a combination thereof. The agent may even be a nucleotide sequence - which may be a sense sequence or an anti-sense sequence. The agent may even be an antibody.

Typically, the agonist will be an organic compound. Typically the organic compound will comprise two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. For some applications, preferably the agent comprises at least one cyclic group,. The cyclic group may be a polycyclic group, such as a non-fused polycyclic group. For some applications, the agonist comprises at least the one of said cyclic groups linked to another hydrocarbyl group.

### SPECIFIC AGONISTS

An example of a specific agonist is retinoic acid (RA). Both common forms of retinoic acid (either all-trans retinoic acid (tRA), or 9-cis-RA) are agonists of RARβ2.

CD2019 is a RARβ2 agonist having the structure as discussed herein and as shown in (Elmazar et al., (1996) Teratology vol. 53 pp158-167). This and other agonists are also discussed in (Beard and Chandraratna p.194; Johnson *et al*., 1996). The structure of CD2019 is presented as Formula I in the attached figures.

An alternative RARβ2 agonist is presented as Formula II in the attached figures.

The present invention also encompasses the use of mimetics of bioisosteres of the formulae of Formula I and/or Formula II.

Preferably, the agonist useful in conjunction with a vector according to the present invention is selective for RARβ2.

### ASSAY TO DETERMINE RARβ2 AGONISM

Agonists may be identified and/or verified by using an assay to determine RARβ2 agonism.

Hence, an agonist may be identified and used according to the following steps: (i) detemining if a candidate agent is capable of acting as a RARβ2 agonist; (ii) if said candidate agent is capable of acting as a RARβ2 agonist then delivering said agent to a subject and in such an amount to cause neurite development.

### ASSAY

Any one or more of appropriate targets - such as an amino acid sequence and/or nucleotide sequence - may be used for identifying an agent capable of modulating RARβ2 in any of a variety of drug screening techniques. The target employed in such a test may be free in solution, affixed to a solid support, borne on a cell surface, or located intracellularly. The abolition of target activity or the formation of binding complexes between the target and the agent being tested may be measured.

The assay may be a screen, whereby a number of agents are tested. For example; the assay method may be a high through put screen.

Techniques for drug screening may be based on the method described in Geysen, European Patent Application B4/03564, published on September 13, 1984. In summary, large numbers of different small peptide test compounds are synthesized on a solid substrate, such as plastic pins or some other surface. The peptide test compounds are reacted with a suitable target or fragment thereof and washed. Bound entities are then detected - such as by appropriately adapting methods well known in the art. A purified target can also be coated directly onto plates for use in a drug screening techniques. Alternatively, non-neutralising antibodies can be used to capture the peptide and immobilise it on a solid support.

Competitive drug screening assays may also be used in which neutralising antibodies capable of binding a target specifically compete with a test compound for binding to a target.

Another technique for screening provides for high throughput screening (HTS) of agents having suitable binding affinity to the substances and is based upon the method described in detail in WO-A-84/03564.

It is expected that the assay methods will be suitable for both small and large-scale screening of test compounds as well as in quantitative assays.

The assay may be a method of identifying agents that selectively modulate RARβ2.

For example , the assay may utilise cells that display RARβ2 on their surface. These calls may be isolated from a subject possessing such cells. However, preferably, the cells are prepared by transfecting cells so that upon transfect those cells display on their surface RARβ2.

Another example of an assay that may be used is described in WO-A-9849271, which concerns an immortalised human terato-carcinoma CNS neuronal cell line, which is said to have a high level of neuronal differentiation and is useful in detecting compounds which bind to RARβ2.

A vector according to the present invention capable of directing the expression of RARβ2, may be used to produce cell(s) for use in agonist/antagonist assays. For example, the assay may comprise neuronal cell(s), said cells comprising an EIAV-derived vector capable of directing the expression of RARβ2 in said cell(s).

### REPORTERS

A wide variety of reporters may be used in the assay methods (as well as screens) with preferred reporters providing conveniently detectable signals (eg. by spectroscopy). By way of example, a reporter gene may encode an enzyme which catalyses a reaction which alters light absorption properties.

Other protocols include enzyme-linked immunosorbent assay (ELISA), radioimmunoassay (RIA) and fluorescent activated cell sorting (FACS). A two-site, monoclonal-based immunoassay utilising monoclonal antibodies reactive to two non-interfering epitopes may even be used. These and other assays are described, among other places, in Hampton R et al (1990, Serological Methods, A Laboratory Manual, APS Press, St Paul MN) and Maddox DE et al (1983, J Exp Med 15 8:121 1).

Examples of reporter molecules include but are not limited to (galactosidase, invertase, green fluorescent protein, luciferase, chloramphenicol, acetyltransferase, (glucuronidase, exo-glucanase and glucoamylase. Alternatively, radiolabelled or fluorescent tag-labelled nucleotides can be incorporated into nascent transcripts which are then identified when bound to oligonucleotide probes.

By way of further examples, a number of companies such as Pharmacia Biotech (Piscataway, NJ), Promega (Madison, WI), and US Biochemical Corp (Cleveland, OH) supply commercial kits and protocols for assay procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3817837; US-A-3850752; US-A-3939350; US-A-3996345; US-A-4277437; US-A-4275149 and US-A-4366241.

### Differential expression screening techniques

Genes encode gene products, mainly polypeptides but also RNAs, that are involved in a huge variety of cellular processes. The technique of differential expression screening is based on the idea that by comparing expression under two sets of conditions, genes whose expression varies between those two conditions can be identified and their function related back to the differences between those conditions. For example, genes involved in a pathway responsive to mitogens such as plate-derived growth factor (PDGF) can be identified by comparing gene expression in cells exposed to PDGF versus gene expression in cells not exposed to PDGF. Thus the term "differential expression screening" as used herein means comparing gene expression between two cells under different conditions or two different cells under the same or different conditions, with the aim of identifying gene products that differ in their levels of expression between the two cells.

The differences in gene expression may be measured using a variety of techniques. The first main type of technique is based on the measurement of nucleic acids and is termed herein as "genomic or cDNA techniques". A useful review is provided in Kozian and Kirschbaum (1999). The second main type of technique is based on the measurement of cellular protein content and is termed herein as "proteomic techniques".

### Genomic or cDNA techniques

One method well known in the art is subtractive cDNA hybridisation. This technique involves hybridising a population of mRNAs from one cell (e.g. a control cell) with a population of cDNAs made from the mRNA of another cell (e.g. a cell exposed to PDGF). This step will remove all sequences from the cDNA preparation that are common to both cells. The cDNAs derived from mRNAs whose expression is upregulated in the cell exposed to PDGF will not have a corresponding mRNA from the control with which to hybridise and can be isolated. Typically, the cDNAs are also hybridised with mRNA from the same cell to confirm that they represent coding sequences. This procedure is described in detail in WO90/11361 where mRNA from cells from the roots of plants treated with a chemical, N-(amincarbonyl)-2-chlorobenzenesulphonamide, were used to produce a cDNA library that was then hybridised with mRNA from untreated root cells. The procedure identified a number of genes whose expression was upregulated by the chemical.

The polymerase chain reaction (PCR) has led to the development of a number of other methods. RT-PCR differential display was first described by Liang and Pardee (1992). This technique involves the use of oligo-dT primers and random 5' oligonucleotide 10-mers to carry out PCR on reverse-transcribed RNA from different cell populations. PCR is often carried out using a radiolabelled nucleotide so that the products can be visualised after gel electrophoresis and autoradiography. Wilkinson *et al.* (1995) used PCR differential display to identify five mRNAs that are upregulated in strawberry fruit during ripening. A review of differential display RT-PCR (also known as differential display of mRNA) is provided in Zhang et *al.* (1998) and a recent improvement using 'long distance' PCR is described in Zhao *et al.* (1999).

Another technique is termed cDNA library screening. A review of this technique and the other two differential expression screening techniques mentioned above is provided in Maser and Calvet (1995).

Differential display competitive PCR is a fairly recent innovation that has been successfully used to study changes in global gene expression in situations where only a few genes change expression levels, such as exposure of MCF17 cell to oestradiol, and in more complex situations such as neuronal differentiation of human NTERA2 cells (Jorgensen et al., 1999).

A further PCR based technique is representational difference analysis (RDA) - see Kozian and Kirschbaum (1999) for review and references therein. Also reviewed in see Kozian and Kirschbaum (1999) is a technique termed serial analysis of gene expression.

The actual identification of gene products whose expression differs between the two cell populations can be carried out in a number of ways. Subtractive methods will inherently identify gene products whose expression differs since gene products whose expression is the same are eliminated from the sample. Other methods include simply comparing the expression products from one cell with the expression products from another and looking for any differences (with PCR-based techniques, the number of products in each sample can be limited to a reasonable size), optionally with the aid of a computer program. For example using a PCR-based technique a visual comparison of bands present in different lanes allows the identification of bands unique to one lane. These bands can be cut out of the gel and subsequently analysed.

The advent of DNA chip technology, allows comparisons to be conveniently conducted by the use of microarrays (see Kozian and Kirschbaum, 1999 for review and references therein). Typically, arrays are generated using cDNAs (including ESTs), PCR products, cloned DNA and synthetic oligonucleotides that are fixed to a substrate such as nylon filters, glass slides or silicon chips. To determine differences in gene expression, labelled cDNAs or PCR products are hybridised to the array and the hybridisation patterns compared. The use of fluorescently labelled probes allows two different cell populations to be applied simultaneously to one chip and the results measured at different wavelengths A microarray-based differential expression screening technique is described in US-A-5,800,992.

### Proteomic techniques

Proteomics is the study of proteins properties on a large scale to obtain a global, integrated view of disease processes, cellular processes and networks at the protein level. A review of techniques used in proteomics is given in Blackstock and Weir (1999) - see also references provided therein. The methods of the present invention are mainly concerned with expression proteomics, the study of global changes in protein expression in cells using electrophoretic techniques and image analysis to resolve proteins. Whereas nucleic acid analysis emphasises the message, proteomics is more concerned with the product. The two approaches are sometimes complementary since proteomic techniques may be useful in detecting changes in polypeptide levels due to changes in protein stability rather than mRNA levels.

A well known and ubiquitous technique used in the field of proteomics involves measuring the polypeptide content of a cell using 2D polyacrylamide gel electrophoresis (PAGE) and comparing this with the polypeptide content of another cell. The results of electrophoresis are typically a gel visualised with a dye such as silver stain or Coomassie-blue, or an autoradiograph produced from the gel, all with spots corresponding to individual proteins. Fluorescent dyes are also available.

The aim is therefore to identify spots that differ between the two gels/autoradiographs, i.e. missing from one, reduced in intensity or increased in intensity. Thus in the case of proteomics, comparing gene expression simply involves comparing the protein profile from one cell with the protein profile from another. Commercial software packages are available for automated spot detection.

Spots of interest may be excised from gels and the proteins identified using techniques such as matrix-assisted-laser-desorption-ionisation-time-of-flight (MALDI-TOF) mass spectrometry and electrospray.

It may be desirable to perform some measure of prefractionation, such as centrifugation or free-flow electrophoresis to improve the identification of low abundance proteins. Special procedures have also been developed for basic proteins, membrane proteins and other poorly soluble proteins (Rabilloud *et al.,* 1997).

The above discussion provides a description of prior art methods available to the skilled person for performing differential expression screening of two or more cell populations in a general sense. In the method described herein, the levels of an endogenous biological molecule in a cell are altered by the experimenter, so that the levels of gene products that are affected by the molecule become more responsive to cellular perturbations such as signalling events. In other words, the object is to amplify and/or increase the signal to noise ratio of the differential response normally obtained so as to increase the likelihood of detecting gene products whose levels in a cell are low and/or whose expression normally changes by only a small amount.

By way of an example, the transcription factor HIF-1α is responsive to intracellular oxygen levels. Decreases in oxygen levels increase HIF-1α activity and lead to increased transcription from genes comprising a hypoxia responsive element (HRE). If the levels of HIF-1α in the cell are raised artificially, for example by infecting cells with a viral vector that directs expression of HIF-1α, then you would expect to see an increase in the transcriptional response mediated by HIF-1α. Consequently, changes in the expression of genes whose expression is sensitive to the HIF-1α mediated hypoxic response should be greater than in normal cells expressing physiological levels of HIF-1α.

### Biological molecules

The biological molecule can be any compound that is found in cells as a result of anabolic or catabolic processes within a cell or as a result of uptake from the extracellular environment, by whatever means. The term "biological molecule" means that the molecule has activity in a biological sense. Preferably the biological molecule is synthesised within the cell, i.e. is endogenous to that cell, or in the case of multicellular organisms, also within any of the cells of the organism.

Examples of biological molecules will therefore include proteins, nucleic acids, carbohydrates, lipids, steroids, co-factors, prosthetic groups (such as haem), inorganic molecules, ions (such as Ca²⁺), inositides. Where appropriate, precursors, monomeric, oligomeric and polymeric forms, and breakdown products of the above are also included.

Example of polypeptides include enzymes, transcription factors, hormones, structural components of cells and receptors including membrane bound receptors.

Preferably, the biological molecule is known to be involved in the cellular process of interest.

In one embodiment of the invention, the biological molecule is responsive to a signal, which may be an externally applied signal such as an environmental signal, for example redox stress, the binding of an extracellular ligand to a cell surface receptor leading to a cellular response mediated by a signal transduction signal. Alternatively, the signal may be an internally applied signal such as an increase in kinase activity due to falling levels of a cell metabolite.

The levels of the biological molecule may be altered directly or indirectly. Direct alteration may be achieved by, for example, causing cells to take up the molecule by incubating cells in a medium containing higher than physiological levels of the molecule. Other methods include vesicle-mediated delivery and microinjection. In the case of nucleic acids and polypeptides, the level of the biological molecule in the cell may be raised by the introduction of a heterologous nucleic acid into the cell which directs the expression of the nucleic acid or polypeptide.

The term "heterologous nucleic acid" in the present context means that the nucleic acid is not present in its natural context i.e. the cell has been modified so as to contain the nucleic acid which would otherwise not be present in the form in which it is introduced. For example, the nucleic acid may be extrachromosomal. The nucleic acid may also be integrated into the genome by viral transduction or homologous recombination. Nonetheless, part of all of the heterologous nucleic may be identical to a corresponding genomic sequence since the introduction of additional copies of a gene is a convenient means for increasing the levels of expression of that gene.

Indirect means for altering the levels of the biological molecule are numerous and include increasing the levels of an inhibitory or stimulatory molecule using the methods described above. Inhibitory molecules include antisense nucleic acids, ribozyme or an EGS directed against the mRNA encoding the biological molecule, a transdominant negative mutant directed against the biological molecule, transcription factors, enzyme inhibitors, and intracellular antibodies such as scFvs. Stimulatory molecules include enzyme activators, transcriptional activators. Thus cells may be manipulated in a number of ways such that ultimately the levels of the biological molecule are altered. Reduced expression may be achieved by expressing an anti-sense RNA,

The levels of the biological molecule are altered relative to physiological levels. Thus they may be enhanced or reduced. The term "relative to physiological levels" means relative to the concentration or activity of the biological molecule typically present in the cell under normal physiological conditions prior to manipulation of those levels. Thus the intention is that by deliberate means, the activity of the biological molecule is altered above or below that which is found in the cell under a range of normal physiological conditions. "Physiological conditions" includes the conditions normally found *in vivo* and the conditions normally used *in vitro* to culture the cells.

By way of an example, the activity or concentration may be increase or decreased 5-fold, 10-fold, 20-fold, 50-fold or 100-fold compared to the normal physiological activity or concentration found in the cell prior to introducing, for example, the heterologous nucleic acid.

Where, as in a preferred embodiment of the invention, the levels of the biological molecule are altered by the introduction of a heterologous nucleic acid, typically a nucleic acid that directs expression of a polypeptide, the heterologous nucleic acid will comprise a coding sequences operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

Control sequences operably linked to sequences encoding the protein of the invention include promoters/enhancers and other expression regulation signals. These control sequences may be selected to be compatible with the host cell in which the expression vector is designed to be used. The term promoter is well known in the art and encompasses nucleic acid regions ranging in size and complexity from minimal promoters to promoters including upstream elements and enhancers.

The promoter is typically selected from promoters which are functional in mammalian, cells, although promoters functional in prokaryotic cells or other eukaryotic cells may be used where appropriate. Thus, the promoter is typically derived from promoter sequences of viral or eukaryotic genes. For example, it may be a promoter derived from the genome of a cell in which expression is to occur. Eukaryotic promoters, may be promoters that function in a ubiquitous manner (such as promoters of α-actin, β-actin, tubulin) or, alternatively, a tissue-specific manner (such as promoters of the genes for pyruvate kinase). Tissue-specific promoters specific for particular cells may be used. They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may be advantageous for the promoters to be inducible so that the levels of expression from the heterologous nucleic acid can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

Suitable vectors include plasmids, artificial chromosomes and viral vectors. Viral vectors include DNA virus vectors, RNA virus vectors (ie. retroviral vectors), such as lentiviruses, adenoviral vectors, adeno-associated vectors and herpes simplex viral vectors. Vectors/polynucleotides may introduced into suitable host cells using a variety of techniques known in the art, such as transfection, transformation, electroporation, infection with recombinant viral vectors such as retroviruses, herpes simplex viruses and adenoviruses, direct injection of nucleic acids and biolistic transformation. It is particularly preferred to use recombinant viral vector-mediated techniques.

A cell of interest can be any cell, for example a prokaryotic cell, a yeast cell, a plant cell or an animal cell, such as an insect cell or a mammalian cell, including a human cell. In the case of cells from multicellular organism, cells may be primary cells or immortalised cell lines. Although cells are frequently referred to in the singular, in general cells will be part of a cell population.

In the assay method described above, a comparison may be required between gene expression in at least two distinct cells. Typically the first of the two or more cells is termed a reference cell. In a preferred embodiment, the cells to be used in the comparison are substantially identical in all respects. For example they may both be cells of the same cell line or obtained from the same tissue in an organism. One or both of the cells may then be manipulated so that they comprise altered levels, relative to physiological levels, of the biological molecule as described above. In one embodiment, the first cell is unaltered and the second cell altered. This is particularly preferred since it should result in an improved signal to noise ratio. In a highly preferred embodiment, the first cell is unaltered, and the second cell comprises RARβ2 according to the present invention. Preferably, the cells are mammalian neuronal cells.

Nonetheless, it is not necessary that the cells used as the starting point of the investigation be substantially identical. For example, genes involved in disease processes may be investigated using cells from a diseased organism, such as a mammalian patient. These may be compared with cells from a normal organism or similar cells from the same or a different diseased individual. Where cells from a normal organism and a diseased organism are used, generally the normal cells correspond to the first cell of interest and the diseased cells correspond to the second cell of interest. Consequently, at least the diseased cells are modified as described above in so that comprises altered levels of the biological molecule.

One cell may be a cell comprising a mutant gene whereas the other cell may comprise a wild-type version of the same gene.

Another possibility is that the cells are from different tissues or from different stages in development or differentiation, for example as affected by the presence or absence of RARβ2, and/or retinoic acid or derivatives thereof.

The vectors of the present invention may also be used in for identifying genes involved in a cellular process. Essentially one of the cells is manipulated so that the levels within that cell of a biological molecule involved in the cellular process are altered. Preferably, this process is neurite outgrowth and/or neural regeneration as effected by the action of retinoic acid through RARβ2. Typically, this is achieved by the introduction of a heterologous nucleic acid into the cell to direct the expression of a polypeptide such as RARβ2. The polypeptide may be the same as the biological molecule or it may modulate the levels of the biological molecule as described above.

In general, simply modulating the levels of a biological molecule in one of two identical cells and then measuring gene transcription is not the aim of the methods of the present invention since you will be measuring the effect of the biological molecule on gene expression in the cells rather than using the change in the levels of the biological molecule to enhance or reduce the response to an event of interest.

However, where the biological molecule is a gene product, such as a polypeptide, that is produced natural within the cell, altering the levels of the gene product by the introduction of a heterologous nucleic acid may be used to simultaneously both perturb a cellular process and enhance the response to such a perturbation making it easier to identify gene products involved in that cellular process using differential expression techniques. By way of an example, overexpression of HIF-1α not only induces an hypoxic response but amplifies the downstream elements of that response due an enhanced regulatory effect on HIF-1α, mediated transcription.

Nonetheless, two main possibilities arise. Firstly, the two cells are different and have inherently different gene expression patterns. In this situation, alterations in the levels of the biological molecule can be used to enhance those differences. The two cells may be, for example, from different tissues, or from different stages in development or differentiation. The two cells may also be different by virtue of one cell being from diseased tissue and the other cell from normal tissue. Other configurations envisaged are given above.

Secondly, the two cells are the same but one of the cells is stimulated in some manner and the other cell not (or one is stimulated to a greater extent than the other). For example, one cell is incubated in the presence of a growth factor and the other is not. The growth factor is therefore not the biological molecule but is instead a stimuli is designed to perturb gene expression in the cell, the effects of which may be amplified by the biological molecule which in turn is altered in level by the polypeptide expressed from the heterologous nucleic acid..

Genes whose expression is regulated by a signal may be identified by subjecting two distinct cell populations to different levels of a signal, whereby either or both cells have been manipulated so as to after the levels of a biological molecule whose activity is responsive to the signal, and identifying gene products whose expression differs. The term "whose activity is response to the signal" includes biological molecule whose concentration in the cell varies in response to the signal as well as biological molecules whose properties such as enzymatic activity or affinity for another cellular component varies in response to the signal.

Thus returning to our factor example, the cells that are exposed to the factor may have been altered to express increased levels of a transcription factor involved in the signal transduction cascade. Consequently, the effect of the growth factor will be increased downstream of the transcription factor (in either a negative or positive sense) making it easier to identify differential expressed genes whose expression is regulated by the transcription factor and ultimately by the factor. Preferably the factor leads to stimulation of neural regeneration/neurite outgrowth via signalling through RARβ2.

The signal may be either physical, such as redox conditions, CO₂ levels, light or temperature, or chemical such as ligands that bind to receptors on the cell surface and trigger signal transduction pathways (including hormones or cell surface molecules normally attached to other cells), or substrates for enzyme reactions that diffuse into or are transported into the cell.

The first cell is subjected the signal at a first level and the second cell is subjected to the signal at a second level. The first level may simply be the absence of the signal and the second level may be the presence of the signal, or vice-versa. The levels of the signals may be adjusted so as to provide a discernible difference in gene expression but are preferably at physiologically relevant levels.

Knowledge already acquired about genes involved in a disease or other biological process may be used to generate further information about other genes whose expression is altered in a disease or other biological process. To do this, one cell is modified so that the levels of the gene product known to be involved in the disease or other biological process are altered, either directly by the introduction of a heterologous nucleic acid encoding the gene product, or indirectly as described above. Gene expression is then measured in both cells and the results compared to identify gene products whose expression varies.

The two cells may be identical, except for the change in the levels of the gene product known to be involved in the disease or other biological process of interest. The two cells may thus both be normal cells of the same type as a cell type in which the disease or other process manifests itself, or they may both be diseased cells. Alternatively, one cell may be normal and the other diseased. Preferably the diseased cell is the modified cell if only one of the cells is modified.

Differential expression screening methods may be used to identify genes involved in a disease or other process in a two stage procedure. Firstly, gene expression is compared between a first cell of interest, for example a cell from a normal patient, and a second cell of interest, for example corresponding cells from a diseased patient. As discussed above, the first cell and the second cell will be different in some aspect such that they have different expression patterns. This may be because the cells are from different tissues or different individuals (for example a normal patient and a diseased patient) or the cells may be of similar origin but have been treated differently in some respect.

Gene products whose expression diners between the first cell and the second cell are identified. Secondly, a third cell of interest, essentially identical to the first cell is used in a screening procedure where a candidate gene is introduced into the third cell so that levels of the genes are altered (typically raised). Gene expression in this cell is compared with gene expression in the first cell and gene products whose expression differs between the normal cell and the third cell comprising altered levels of the candidate gene are identified. If a gene product whose expression is altered in the second cell also has altered gene expression in the third cell, then the candidate gene is selected for further study. Preferably there is a correlation over two or more gene products, preferably at least four or five gene products to minimise false positives.

Clearly, the methods may advantageously be applied to the differential analysis of non-dividing neuronal cells and a different sample of the same cells which have been induced to regenerate or undergo neurite outgrowth by the methods of the present invention. This differential analysis applies to the discovery and/or validation of candidate molecules, in particular those biological molecules which rie in the signalling pathway between the activation of the RARβ2 receptor and the actual morphological phenotype of neurite outgrowth. This phenomenon of neurite outgrowth/regeneration will be brought about by physiological changes within the cell which are initiated by the activation of RARβ2, and may include changes in gene expresison. Thus, by taking a sample of neuronal cells and introducing RARβ2 as described herein, and allowing retinoic acid to signal through this receptor, and comparing the pattern of gene expression with a sample of such cells which do not contain RARβ2/retinoic acid, key difference(s) in gene expression may be identified. The pathway(s) leading to neurite outgrowth will be switched on in the cells with RARβ2/retinoic acid. By making cDNA from these and from the non-activated cells in parallel, subtractive cDNA libraries may be made in order to isolate differences in gene expression between the two sets of cells. This or other differential screening technique(s), or proteomic techniques such as 2-D electrophoretic mapping, can be used to detect the stimulation and/or repression of particular gene(s) or sets of genes which the different conditions produce. These differentially expressed genes and/or their gene products are each individual candidate factors in the stimulation of neurite outgrowth, and it will be clearly understood that the invention relates also to these. This topic is discussed in more detail below.

### HOST CELLS

The vector of the present invention may be used to introduce a nucleic acid encoding RARβ2 into host cells.

The term "host cell"- in relation to the present invention includes any cell that could comprise the RARβ2-encoding polynucleotide sequence.

Here, polynucleotides may be introduced into prokaryotic cells or eukaryotic cells, for example yeast, insect or mammalian cells.

The lentiviral vector of the present invention may be capable or transfecting/transducing non-dividing mammalian cells. An example of such a vector is a viral vector such as a vector based an or derived from EIAV. This and further examples are discussed at length herein.

Thus, a further embodiment of the present invention provides host cells transformed or transfected with a vector as defined herein. The cells will be chosen to be compatible with the said vector and may for example be prokaryotic (for example bacterial), fungal, yeast or plant cells.

The gram negative bacterium *E*. *coli* is widely used as a host for heterologous gene expression. However, large amounts of heterologous protein tend to accumulate inside the cell. Subsequent purification of the desired protein from the bulk of E. coli intracellular proteins can sometimes be difficult.

In contrast to *E. coli*, bacteria from the genus *Bacillus* are very suitable as heterologous hosts because of their capability to secrete proteins into the culture medium. Other bacteria suitable as hosts are those from the genera Streptomyces and Pseudomonas.

The host may be eukaryotic, such as yeasts or other fungi. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Examples of suitable expression hosts within the scope of the present invention are fungi such as Aspergillus species (such as those described in EP-A-0184438 and EP-A-0284603) and Trichoderma species; bacteria such as Bacillus species (such as those described in EP-A-0134048 and EP-A-0253455), Streptomyces species and Pseudomonas species; and yeasts such as Kluyveromyces species (such as those described in EP-A-0096430 and EP-A-0301670) and Saccharomyces species. By way of example, typical expression hosts may be selected from *Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis and Saccharomyces cerevisiae.*

Polypeptides that are extensively modified may require correct processing to complete their function. In those instances, mammalian cell expression systems (such as HEK-293, CHO, HeLA) are required, and the polypeptides are expressed either intracellularly, on the cell membranes, or secreted in the culture media if preceded by an appropriate leader sequence.

The use of suitable host cells - such as yeast, fungal, plant and mammalian host cells - may provide for post-translational modifications (e.g. myristoylation, glycosylation, truncation, lipidation and tyrosine, serine or threonine phosphorylation) as may be needed to confer optimal biological activity on recombinant expression products of the present invention.

### ORGANISM

The term "organism" in relation to the present invention includes any organism that could comprise the sequence according to the present invention and/or products obtained therefrom. Examples of organisms may include a fungus, yeast or a plant.

The term "transgenic organism" in relation to the present invention includes any organism that comprises the target according to the present invention and/or products obtained.

### TRANSFORMATION OF HOST CELLS/HOST ORGANISMS

As indicated earlier, the host organism can be a prokaryotic or a eukaryotic organism. Examples of suitable prokaryotic hosts include *E*. *coli* and *Bacillus subtilis.* Teachings on the transformation of prokaryotic hosts is well documented in the art, for example see Sambrook et al (Molecular Cloning: A Laboratory Manual, 2nd edition, 1989, Cold Spring Harbor Laboratory Press) and Ausubel et al., Current Protocols in Molecular Biology (1995), John Wiley & Sons, Inc.

If a prokaryotic host is used then the nucleotide sequence may need to be suitably modified before transformation - such as by removal of introns.

In another embodiment the transgenic organism can be a yeast. In this regard, yeast have also been widely used as a vehicle for heterologous gene expression. The species *Saccharomyces cerevisiae* has a long history of industrial use, including its use for heterologous gene expression. Expression of heterologous genes in *Saccharomyces cerevisiae* has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 107-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in *Saccharomyces cerevisiae* and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

Several types of yeast vectors are available, including integrative vectors, which require recombination with the host genome for their maintenance, and autonomously replicating plasmid vectors.

In order to prepare the transgenic Saccharomyces, expression constructs are prepared by inserting the nucleotide sequence of the present invention into a construct designed for expression in yeast. Several types of constructs used for heterologous expression have been developed. The constructs contain a promoter active in yeast fused to the nucleotide sequence of the present invention, usually a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells are selected using various selective markers. Among the markers used for transformation are a number of auxotrophic markers such as LEU2, HlS4 and TRP1, and dominant antibiotic resistance markers such as aminoglycoside antibiotic markers, eg G418.

Another host organism is a plant. The basic principle in the construction of genetically modified plants is to insert genetic information in the plant genome so as to obtain a stable maintenance of the inserted genetic material. Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

Further hosts suitable for the nucleotide sequence of the present invention include higher eukaryotic cells, such as insect cells or vertebrate cells, particularly mammalian cells, including human cells, or nucleated cells from other multicellular organisms. In recent years propagation of vertebrate cells in culture (tissue culture) has become a routine procedure. Examples of useful mammalian host cell lines are epithelial or fibroblastic cell lines such as Chinese hamster ovary (CHO) cells, NIH 3T3 cells, HeLa cells or 293T cells.

The nucleotide sequence may be stably incorporated into host cells or may be transiently expressed using methods known in the art. By way of example, stably transfected mammalian cells may be prepared by transfecting cells with an expression vector having a selectable marker gene, and growing the transfected cells under conditions selective for cells expressing the marker gene. To prepare transient transfectants, mammalian cells are transfected with a reporter gene to monitor transfection efficiency.

To produce such stably or transiently transfected cells, the cells should be transfected with a sufficient amount of the nucleotide sequence The precise amounts of the nucleotide sequence of the present invention may be empirically determined and optimised for a particular cell and assay.

Host cells transformed with the nucleotide sequence may be cultured under conditions suitable for the expression of the encoded protein. The protein produced by a recombinant cell may be displayed on the surface of the cell. If desired, and as will be understood by those of skill in the art, expression vectors containing coding sequences can be designed with signal sequences which direct secretion of the coding sequences through a particular prokaryotic or eukaryotic cell membrane. Other recombinant constructions may join the coding sequence to nucleotide sequence encoding a polypeptide domain which will facilitate purification of soluble proteins (Kroll DJ at al (1993) DNA Cell Biol 12:441-53).

### RECEPTORS

The RARβ2 receptor as discussed herein includes mimetics, homologues, fragments and part or all of the entire gene product Preferably the RARβ2 receptor as discussed herein refers to substantial the entire gene product

In one embodiment, the present invention relates to the use of a vector capable of encoding RARβ2 in the preparation of a medicament in the production of neurite outgrowth. Previously, attempts have been made to produce neurite outgrowth using a number of different techniques. Typically, nerve growth factor (NGF) is used to stimulate neurite outgrowth. However, NGF is a relatively large molecule with a correspondingly high molecular weight, and is susceptible to protease mediated degradation. NGF is also relatively expensive to prepare. Similar approaches to the stimulation of neurite outgrowth have also encountered various difficulties. Moreover, such approaches have centred on the use of stimulatory factors such as growth factors in order to produce such desired phenotype(s). However, it is surprisingly shown herein that the long-feft need for the production of neurite outgrowth, for example in non-dividing calls, may be achieved using the converse approach disclosed herein, ie. the use of receptors to stimulate neurite outgrowth as described and demonstrated in the present invention. This disclosure runs against current thinking in the art, which has been focussed on the use of growth factors to try to elicit neurite outgrowth from non-dividing cells such as terminally differentiated neuronal cells. The surprising finding that receptor(s) may be delivered to such cells to produce neural regeneration/neurite outgrowth is illustrated herein by using RARβ2 as an example of this general approach. Thus, the present invention relates to the vector encoding RARβ2 for use in the production of neurite outgrowth.

### NEUROLOGICAL DISORDERS

Clearly, stimulation of neurite outgrowth according to the present invention will have therapeutic benefit in a number of pathologies. These include, but are not limited to, neurological disorders, for example degenerate neurological disorders such as Parkinson's disease, Alzheimer's syndrome, or related conditions, or neural injury such as spinal cord injury or other such physical condition.

The term neurological disorders as used herein may refer to any injury, whether mechanically (for example by trauma) or chemically induced (for example by neurotoxin(s), or by an regime of treatment having an immunosuppressant effect, whether by design, or as a side-effect), any neural pathology such as caused by viral infection or otherwise, any degenerative disorder, or other nerve tissue related disorder.

Examples of neurological disorders include conditions such as Parkinson's disease, Alzheimer's disease, senility, motor neurone disease, schizophrenia as well as other neural and/or neurodegenerative disorders. Other neural related disorders may include glaucoma or other cause of damage to the optic nerve, Bell's palsy or other forms of localised paralysis, neurally based impotence such as caused by nerve trauma following radical prostatectomy, or other complaints. Other disorders in which the invention may be useful include neuropathological effects of diabetes, AIDS neuropathy, leprosy etc.

The term neurological disorder refers to any disorder of a nervous system, whether the peripheral nervous system or the central nervous system (CNS), whether the sympathetic nervous system, or the parasympathetic nervous system, or whether affecting a subset or superset of different nerve types.

### CODON OPTIMISATION

As used herein, the terms "codon optimised" and "codon optimisation" refer to an improvement in codon usage. By way of example, alterations to the coding sequences for viral components may improve the sequences for codon usage in the mammalian cells or other cells which are to act as the producer cells for retroviral vector particle production. This is referred to as "codon optimisation". Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

Preferably a high titre lentiviral vector is produced using a codon optimised gag and a codon optimised pol or a codon optimised env (see seq. listing and/or WO99/41397).

Preferably a high titre retroviral vector is produced using a modified and/or extended packaging signal.

### PACKAGING SIGNAL

As used herein, the term "packaging signal" or "packaging sequence" refers to sequences located within the retroviral genome which are required for insertion of the viral RNA into the viral capsid or particle. Several retroviral vectors use the minimal packaging signal (also referred to as the psi sequence) needed for encapsidation of the viral genome. By way of example, this minimal packaging signal encompasses bases 212 to 563 of the Mo-MLV genome (Mann et al 1983: Cell 33: 153).

As used herein, the term "extended packaging signal" or "extended packaging sequence" refers to the use of sequences around the psi sequence with further extension into the gag gene. The inclusion of these additional packaging sequences may increase the efficiency of insertion of vector RNA into viral particles.

Preferably a high titre lentiviral vector is produced using a modified packaging signal.

Preferably the lentiviral construct is a based on an EIAV vector genome where all the accessory genes are removed except Rev.

### ACCESSORY GENES

As used herein, the term "accessory genes" refer to a variety of virally encoded accessory proteins capable of modulating various aspects of retroviral replication and infectivity. These proteins are discussed in Coffin et al (ibid) (Chapters 6 and 7). Examples of accessory proteins in lentiviral vectors include but are not limited to tat, rev, nef, vpr, vpu, vif, vpx. An example of a lentiviral vector useful in the present invention is one which has all of the accessory genes removed except rev.

### TRANSCRIPTIONAL CONTROL

The control of proviral transcription remains largely with the noncoding sequences of the viral LTR. The site of transcription initiation is at the boundary between U3 and R in the left hand side LTR and the site of poly (A) addition (termination) is at the boundary between R and U5 in the right hand side LTR. The 3'U3 sequence contains most of the transcriptional control elements of the provirus, which include the promoter and multiple enhancer sequences responsive to cellular and in some cases, viral transcriptional activator proteins.

An LTR present, for example, in a construct of the present invention and as a 3'LTR in the provirus of, for example, a target cell of the invention may be a native LTR or a heterologous regulatable LTR. It may also be a transcriptionally quiescent LTR for use in SIN vector technology.

The term "regulated LTR" also includes an inactive LTR such that the resulting provirus in the target cell can not produce a packagable viral genome (self-inactivating (SIN) vector technology).

Preferably the regulated retroviral vector of the present invention is a self-inactivating (SIN) vector.

### SELF-INACTIVATING (SIN) VECTOR

By way of example, self-inactivating retroviral vectors have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus (Yu et al 1986 Proc Natl Acad Sci 83: 3194-3198; Dougherty and Temin 1987 Proc Natl Acad Sci 84: 1197-1201; Hawley et a/1987 Proc Natl Acad Sci 84: 2406-2410; Yee et al 1987 Proc Natl Acad Sci 91: 9564-9568). However, any promoter(s) internal to the LTRs in such vectors will still be transcriptionally active. This strategy has been employed to eliminate effects of the enhancers and promoters in the viral LTRs on transcription from internally placed genes. Such effects include increased transcription (Jolly et al 1983 Nucleic Acids Res 11: 1855-1872) or suppression of transcription (Emerman and Temin 1984 Cell 39: 449-467). This strategy can also be used to eliminate downstream transcription from the 3' LTR into genomic DNA (Herman and Coffin 1987 Science 236: 845-848). This is of particular concern in human gene therapy where it is of critical importance to prevent the adventitious activation of an endogenous oncogene.

### TARGETED VECTOR

The term "targeted vector" refers to a vector whose ability to infect/transfect/transduce a cell or to be expressed in a host and/or target cell is restricted to certain cell types within the host organism, usually cells having a common or similar phenotype.

Preferably the targeted vector has a pseudotyped envelope gene in order to effectively transduce a specific cell type.

### ENVELOPE (ENV)

If the retroviral component includes an *env* nucleotide sequence, then all or part of that sequence can be optionally replaced with all or part of another *env* nucleotide sequence such as, by way of example, the amphotropic Env protein designated 4070A or the influenza haemagglutinin (HA) or the vesicular stomatitis virus G (VSV-G) protein. Replacement of the *env* gene with a heterologous *env* gene is an example of a technique or strategy called pseudotyping. Examples of pseudotyping may be found in WO-A-98/05759, WO-A-98/05754, WO-A-97/17457, WO-A-96/09400, WO-A-91/00047 and Mebatsion et al 1997 Cell 90, 841-847.

In one preferred aspect, the retroviral vector of the present invention has been pseudotyped. In this regard, pseudotyping can confer one or more advantages. For example, with the lentiviral vectors, the env gene product of the HIV based vectors would restrict these vectors to infecting only cells that express a protein called CD4.

But if the env gene in these vectors has been substituted with env sequences from other RNA viruses, then they may have a broader infectious spectrum (Verma and Somia 1997 Nature 389:239-242). By way of example, workers have pseudotyped an HIV based vector with the glycoprotein from VSV (Verma and Somia 1997 ibid).

In another alternative, the Env protein may be a modified Env protein such as a mutant or engineered Env protein. Modifications may be made or selected to introduce targeting ability or to reduce toxicity or for another purpose (Valsesia-Wittman et al 1996 J Virol 70: 2056-64; Nilson et al 1996 Gene Therapy 3: 280-6; Fielding et al 1998 Blood 9: 1802 and references cited therein).

The term "retroviral vector particle" refers to the packaged retroviral vector, that is preferably capable of binding to and entering target cells. The components of the particle, as already discussed for the vector, may be modified with respect to the wild type retrovirus. For example, the Env proteins in the proteinaceous coat of the particle may be genetically modified in order to alter their targeting specificity or achieve some other desired function.

Preferably, the viral vector preferentially transduces a certain cell type or cell types.

More preferably, the viral vector is a targeted vector, that is it has a tissue tropism which is altered compared to the native virus, so that the vector is targeted to particular cells.
For retroviral vectors, this may be achieved by modifying the Env protein. The Env protein of the retroviral secondary vector needs to be a non-toxic envelope or an envelope which may be produced in non-toxic amounts within the primary target cell, such as for example a MMLV amphotropic envelope or a modified amphotropic envelope. The safety feature in such a case is preferably the deletion of regions or sequence homology between retroviral components.

Preferably the envelope is one which allows transduction of human cells. Examples of suitable *env* genes include, but are not limited to, VSV-G, a MLV amphotropic env such as the 4070A *env,* the RD114 feline leukaemia virus *env* or haemagglutinin (HA) from an influenza virus. The Env protein may be one which is capable of binding to a receptor on a limited number of human cell types and may be an engineered envelope containing targeting moieties. The *env* and *gag-pol* coding sequences are transcribed from a promoter and optionally an enhancer active in the chosen packaging cell line and the transcription unit is terminated by a polyadenylation signal. For example, if the packaging cell is a human cell, a suitable promoter-enhancer combination is that from the human cytomegalovirus major immediate early (hCMV-MIE) gene and a polyadenylation signal from SV40 virus may be used. Other suitable promoters and polyadenylation signals are known in the art.

The packaging cell may be an *in vivo* packaging cell in the body of an individual to be treated or it may be a cell cultured *in vitro* such as a tissue culture cell line. Suitable cell lines include mammalian cells such as murine fibroblast derived cell lines or human cell lines. Preferably the packaging cell line is a human cell line, such as for example: 293 cell line, HEK293, 293-T, TE671, HT1080.

Alternatively, the packaging cell may be a cell derived from the individual to be treated such as a monocyte, macrophage, stem cells, blood cell or fibroblast. The cell may be isolated from an individual and the packaging and vector components administered *ex vivo* followed by re-administration of the autologous packaging cells. Alternatively the packaging and vector components may be administered to the packaging cell *in vivo.* Methods for introducing retroviral packaging and vector components into cells of an individual are known in the art. For example, one approach is to introduce the different DNA sequences that are required to produce a retroviral vector particle e.g. the *env* coding sequence, the *gag-pol* coding sequence and the defective retroviral genome into the cell simultaneously by transient triple transfection (Landau & Littman 1992 J. Virol. 66, 5110; Soneoka et al 1995 Nucleic Acids Res 23:628-633).

In one embodiment the vector configurations of the present invention use as their production system, three transcription units expressing a genome, the *gag-pol* components and an envelope. The envelope expression cassette may include one of a number of envelopes such as VSV-G or various murine retrovirus envelopes such as 4070A.

Conventionally these three cassettes would be expressed from three plasmids transiently transfected into an appropriate cell line such as 293T or from integrated copies in a stable producer cell line. An alternative approach is to use another virus as an expression system for the three cassettes, for example baculovirus or adenovirus. These are both nuclear expression systems. To date the use of a poxvirus to express all of the components of a retroviral or lentiviral vector system has not been described. In particular, given the unusual codon usage of lentiviruses and their requirement for RNA handling systems such as the rev/RRE system it has not been clear whether incorporation of all three cassettes and their subsequent expression in a vector that expresses in the cytoplasm rather than the nucleus is feasible. Until now the possibility remained that key nuclear factors and nuclear RNA handling pathways would be required for expression of the vector components and their function in the gene delivery vehicle. Here we describe such a system and show that lentiviral components can be made in the cytoplasm and that they assemble into functional gene delivery systems. The advantage of this system is the ease with which poxviruses can be handled, the high expression levels and the ability to retain introns in the vector genomes.

According to another aspect therefore there is provided a hybrid viral vector system for *in vivo* gene delivery, which system comprises a primary viral vector which is obtainable from or is based on a poxvirus and a second viral vector which is obtainable from or is based on a lentiviral vector, preferably a non-primate lentiviral vector, more preferably an EIAV.

The secondary vector may be produced from expression of essential genes for retroviral vector production encoded in the DNA of the primary vector. Such genes may include a *gag-pol* from a retrovirus, an env gene from an enveloped virus and a defective retroviral vector containing one or more therapeutic or diagnostic NOI(s). The defective retroviral vector contains in general terms sequences to enable reverse transcription, at least part of a 5' long terminal repeat (LTR), at least part of a 3'LTR and a packaging signal.

If it is desired to render the secondary vector replication defective, that secondary vector may be encoded by a plurality of transcription units, which may be located in a single or in two or more adenoviral or other primary vectors.

In some therapeutic or experimental situations it may be desirable to obviate the need to make EIAV derived from MVA in vitro. MVA-EIAV hybrids are delivered directly into the patient/animal e.g. MVA-EIAV is injected intravenously into the tail vein of a mouse and this recombinant virus infects a variety of murine tissues e.g. lung, spleen etc. Infected cells express transduction competent EIAV containing a therapeutic gene for gene therapy for example. EIAV vector particles bud from these cells and transduce neighbouring cells. The transduced cell then contains an integrated copy of the EIAV vector genome and expresses the therapeutic gene product or other gene product of interest. If expression of the therapeutic gene product is potentially toxic to the host it may be regulated by a specific promoter, e.g. the hypoxic response element (HRE), which will restrict expression to those cells in a hypoxic environment. For gene therapy of lung/trachea epithelium cells e.g to treat cystic fibrosis MVA-EIAV may be given as an aerosol delivered intranasally. Alternatively, macrophages can be transduced in vitro and then reintroduced to create macrophage factories for EIAV-based vectors. Furthermore, because MVA is replication incompetent MVA-EIAV hybrids could also be used to treat immuno-suppressed hosts.

Vaccinia virus, the prototypic member of the orthopox genus within the family poxviridae, was the first virus used for expression of recombinant exogenous proteins (Mackett et al 1982, Paoletti & Panicalli 1982). Vaccinia virus has a large DNA genome of greater than 180 kb and reports indicate that it can accommodate over 25 kb of foreign DNA (Merchlinsky & Moss 1992). Several other strains of poxviruses have been adapted as recombinant expression vectors (for review see Carroll and Moss 1997) e.g. fowlpox (Taylor & Paoletti 1988), canarypox (Taylor et al 1991), swinepox (van der Leek et al 1994) and entomopox (Li et al 1997). Additionally, due to safety concerns, several highly attenuated strains of vaccinia virus have been developed that are compromised in human and other mammalian cells e.g. modified vaccinia virus Ankara (MVA) (Mayr 1978, Sutter 1992), NYVAC (Paoletti et al 1994), vaccinia virus deficient in a DNA replication enzyme (Holzer et al 1997). These may all be used in the present invention.

MVA was derived from a replication competent vaccinia smallpox vaccine strain, Ankara. After >500 passages in chick embryo fibroblast cells the virus isolate was shown to be highly attenuated in a number of animal models including mice that were immune deficient (Mayr et al 1978). The attenuated isolate, MVA, was used to vaccinate over 120,000 people, many of which were immunocompromised (Mahnel 1994) without adverse effects. Studies illustrate that MVA can infect a wide range of mammalian cells but productive infection has only been observed in Hamster kidney cell BHK-21 (Carroll 1997). In all other tested mammalian cell lines early expression, DNA replication and late expression are observed leading to the production of non-infectious immature virus particles (Carroll 1997, Meyer 1991). Virus replication studies show that a minority of mammalian cells can support very low level production of infectious virus i.e. BS-C-1 cells in which 1 infectious MVA particle is produced per cell (Carroll and Moss 1997). Late gene expression usually give rise to >10 fold more protein that those genes under early promoters (Chakrabarti et al 1997, Wyatt et al 1996). In all other attenuated poxvirus strains late gene expression is rarely observed in mammalian cells.

Production of retrovirus vector systems e.g. MLV-HIV and lentivirus vector systems requires the construction of producer lines that express the virus genome and essential structural proteins to make transduction competent virus. Generally, this is a relatively inefficient process which is further complicated when the virus is pseudotyped with toxic envelope proteins such as VSV-G. Expression of a functional genome and the required structural proteins from within a recombinant poxvirus may obviate many of the current inefficient retrovirus and lentivirus vector production technologies. Additional, such recombinant poxviruses may be directly injected into patients to give rise to *in vivo* production of retrovirus or lentivirus.

MVA is a particularly suitable poxvirus for the construction of a pox-retrovirus or pox-lentivirus hybrid due to its non-repticating phenotype and its ability to perform both early and strong late expression for the production of high litre vector preparations.

### HOST/TARGET CELLS

Host and/or target cells transfected or tranduced with a vector of the present invention may be used to express RARβ2 under *in vitro, in vivo* and *ex vivo* conditions.

The term "host cell" and/or "target cell" includes any cell derivable from a suitable organism which a vector is capable of transfecting or transducing. Examples of host and/or target cells can include but are not limited to cells capable of expressing the RARβ2 of the present invention under *in vitro, in vivo* and *ex vivo* conditions. Examples of such cells include but are not limited to neuronal cells, nerve cells, post-mitotically terminally differentiated non-replicating cells such as neurons or combinations thereof.

In a preferred embodiment, the cell is a mammalian cell.

In a highly preferred embodiment, the cell is a human cell.

The term "organism" includes any suitable organism. In a preferred embodiment, the organism is a mammal. In a highly preferred embodiment, the organism is a human.

The present invention also provides a method comprising transforming a host and/or target cell with a vector of the present invention.

The term "transformed cell" means a host cell and/or a target cell having a modified genetic structure. With the present invention, a cell has a modified genetic structure when a vector according to the present invention has been introduced into the cell.

### REGULATION OF EXPRESSION IN VITRO/ VIVO/EX VIVO

The present invention also encompasses the use of vectors for gene therapy whereby the RARβ2 encoding nucleotide sequence(s) is regulated *in vitro*/*in vivolex vivo.*

For example, expression regulation may be accomplished by administering compounds that bind to the RARβ2 encoding nucleotide sequence(s) of the present invention, or control regions associated with the RARβ2 encoding nucleotide sequence of the present invention, or its corresponding RNA transcript to modify the rate of transcription or translation.

### CONTROL SEQUENCES

Control sequences operably linked to sequences encoding RARβ2 include promoters/enhancers and other expression regulation signals. These control sequences may be selected to be compatible with the host cell and/or target cell in which the expression vector is designed to be used. The control sequences may be modified, for example by the addition of further transcriptional regulatory elements to make the level of transcription directed by the control sequences more responsive to transcriptional modulators.

### OPERABLY UNKED

The term "operably linked" means that the components described are in a relationship permitting them to function in their intended manner. A regulatory sequence "operably linked" to a coding sequence is ligated in such a way that expression of the coding sequence is achieved under condition compatible with the control sequences.

Preferably the nucleotide sequence encoding RARβ2 is operably linked to a transcription unit.

The term "transcription unit(s)" as described herein are regions of nucleic acid containing coding sequences and the signals for achieving expression of those coding sequences independently of any other coding sequences. Thus, each transcription unit generally comprises at least a promoter, an optional enhancer and a polyadenylation signal.

### PROMOTERS

The term promoter is well-known in the art and is used in the normal sense of the art, e.g. an RNA polymerase binding site. The term encompasses nucleic acid regions ranging in size and complexity from minimal promoters to promoters including upstream elements and enhancers.

The promoter is typically selected from promoters which are functional in mammalian, cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase).

Preferably the promoter is a constitutive promoter such as CMV.

Preferably the promoters of the present invention are tissue specific.

### TISSUE-SPECIFIC PROMOTERS

The promoters may be tissue-specific promoters. Examples of suitable tissue restricted promoters/enhancers are those which are highly active in tumour cells such as a promoter/enhancer from a *MUC*1 gene, a *CEA* gene or a *5T4* antigen gene. Examples of temporally restricted promoters/enhancers are those which are responsive to ischaemia and/or hypoxia, such as hypoxia response elements or the promoter/enhancer of a *grp*78 or a *grp*94 gene. The alpha fetoprotein (AFP) promoter is also a tumour-specific promoter. One preferred promoter-enhancer combination is a human cytomegalovirus (hCMV) major immediate early (MIE) promoter/enhancer combination.
Preferably the promoters are tissue specific. That is, they are capable of driving transcription of a RARβ2 encoding nucleotide sequence(s) in one tissue while remaining largely "silent" in other tissue types.

The term "tissue specific" means a promoter which is not restricted in activity to a single tissue type but which nevertheless shows selectivity in that they may be active in one group of tissues and less active or silent in another group.

The level of expression of a or the RARβ2 encoding nucleotide sequence(s) under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis). This approach may be used to identify, for example, the smallest region capable of conferring tissue specificity.

A number of tissue specific promoters, described above, may be particularly advantageous in practising the present invention. In most instances, these promoters may be isolated as convenient restriction digestion fragments suitable for cloning in a selected vector. Alternatively, promoter fragments may be isolated using the polymerase chain reaction. Cloning of the amplified fragments may be facilitated by incorporating restriction sites at the 5' end of the primers. Preferably, a tissue-specific promoter used herein is specific for neuronal cells.

### INDUCIBLE PROMOTERS

The promoters may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

### ENHANCER

In addition, any of these promoters may be modified by the addition of further regulatory sequences, for example enhancer sequences. Chimeric promoters may also be used comprising sequence elements from two or more different promoters described above.

The term "enhancer" includes a DNA sequence which binds to other protein components of the transcription initiation complex and thus facilitates the initiation of transcription directed by its associated promoter.

The *in vitro*/ *in vivo*/ *ex vivo* expression of RARβ2 may be used in combination with a protein of interest (POI) or a nucleotide sequence of interest (NOI) encoding same.

### POI AND NOIs

Suitable proteins of interest (POIs) or NOIs encoding same for use in the present invention include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and nbozymes, and derivatives therof (such as with an associated reporter group). When included, the POIs or NOIs encoding same may be typically operatively linked to a suitable promoter, which may be a promoter driving expression of a ribozyme(s), or a different promoter or promoters, such as in one or more specific cell types.

### CYTOKINES

The NOI(s) may encode a POI(s) wherein the POI is a cytokine or a cytokine receptor.

As used herein, the term "cytokines" refers to any varied group of proteins that are released from mammalian cells and act on other cells through specific receptors, said term also including said receptors. The term "cytokine" is often used interchangeably with the term "mediator". Cytokines may elicit from the target cell a variety of responses depending on the cytokine and the target cell. By way of example, cytokines may be important in signalling between cells as inflammatory reactions develop. In the initial stages, cytokines such as IL-1 and IL-6 may be released from calls of the tissue where the inflammatory reaction is occurring. Once lymphocytes and mononuclear cells have started to enter the inflammatory site, they may become activated by antigen and release cytokines of their own such as IL-1, TNF, IL-4 and IFNγ which further enhance cellular migration by their actions on the local endothelium. Other cytokines, such as IL-8, are chemotactic or can activate incoming calls. The term "cytokine" includes but is not limited to factors such as cardiotrophin, EGF, FGF-acidic, FGF-basic, flt3 Ligand, G-CSF, GM-CSF, IFN-γ, IGF-I, IGF-11, IL-1α, IL-1β, IL-2, IL-3, IL-4. IL-5, IL-8, IL-7, IL-8, IL-9, IL-10, IL-11, IL-12, IL-13, IL-15, IL-16, IL-17, IL-18 (IGIF), KGF, UF, M-CSF, Oncostatin M, PDGF-A, PDGF-AB, PDGF-BB, SCF, SCGF, TGF-α, TGF-β₁, TNF-α, TNF-β, TPO and VEGF, as well as their cognate receptors.

### COUPLING

RARβ2 can be coupled to other molecules using standard methods. The amino and carboxyl termini of RARβ2 may be isotopically and nonisotopically labeled with many techniques, for example radiolabeling using conventional techniques (tyrosine residues- chloramine T, iodogen, lactoperoxidase; lysine residues- Bolton-Hunter reagent). These coupling techniques are well known to those skilled in the art The coupling technique is chosen on the basis of the functional groups available on the amino acids including, but not limited to amino, sulfhydral, carboxyl, amide, phenol, and imidazole. Various reagents used to effect these couplings include among others, glutaraldehyde, diazotized benzidine, carbodiimide, and p-benzoquinone.

### CHEMICAL COUPUNG

RARβ2 may be chemically coupled to isotopes, enzymes, carrier proteins, cytotoxic agents, fluorescent molecules, radioactive nucleotides and other compounds for a variety of applications including but not limited to imaging/prognosis, diagnosis and/or therapy.

### IMAGING

The use of labelled RARβ2 with short lived isotopes enables visualization quantitation of RARβ2 binding sites *in vivo* using autoradiographic, or modem radiographic or other membrane binding techniques such as positron emission tomography in order to locate tumours with RARβ2 binding sites. This application provides important diagnostic and/or prognostic research tools.

### CONJUGATES

RARβ2 may be coupled to a scintigraphic radiolabel, a cytotoxic compound or radioisotope, an RARβ2 for converting a non-toxic prodrug into a cytotoxic drug, a compound for activating the immune system in order to target the resulting conjugate to a disease site such as a colon tumour, or a cell-stimulating compound. Such conjugates have a "binding portion", which consists of the RARβ2 of the invention, and a "functional portion", which consists of the radiolabel,

### INDIVIDUAL

As used herein, the term "individual" refers to vertebrates, particularly members of the mammalian species, more in particular, humans.

### TREATMENT

It is to be appreciated that all references herein to treatment include curative, palliative and prophylactic treatment.

### DOSAGE

The dosage of the vector and/or pharmaceutical composition of the present invention will depend on the disease state or condition being treated and other clinical factors such as weight and condition of the individual and the route of administration of the compound. Depending upon the half-life of the RARβ2 in the particular individual, the vector and/or pharmaceutical composition can be administered between several times per day to once a week. It is to be understood that the present invention has application for both human and veterinary use. The methods of the present invention contemplate single as well as multiple administrations, given either simultaneously or over an extended period of time.

Typically, a physician will determine the actual dosage which will be most suitable for an individual subject and it will vary with the age, weight and response of the particular patient and severity of the condition. The dosages below are exemplary of the average case. There can, of course, be individual instances where higher or lower dosage ranges are merited.

In addition or in the alternative the compositions (or component parts thereof) of the present invention may be administered by direct injection. In addition or in the alternative the compositions (or component parts thereof) of the present invention may be administered topically. In addition or in the alternative the compositions (or component parts thereof) of the present invention may be administered by inhalation. In addition or in the alternative the compositions (or component parts thereof) of the present invention may also be administered by one or more of: a mucosal route, for example, as a nasal spray or aerosol for inhalation or as an ingestable solution such as by an oral route, or by a parenteral route where delivery is by an injectable form, such as, for example, by a rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) transdermal, intraperitoneal, intracranial, intracerebroventricular, intracerebral, intravaginal, intrauterine, or parenteral (e.g., intravenous, intraspinal, intracavernosal, subcutaneous, transdermal or intramuscular) route.

By way of further example, the pharmaceutical composition of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

### DISORDERS

The present invention is believed to have a wide therapeutic applicability.

For example, the present invention may be useful in the treatment of the disorders listed in WO-A-98/05635. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the present invention may be useful in the treatment of disorders listed in WO-A-98/07859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of burns, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); antiinflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, the present invention may be useful in the treatment of disorders listed in WO-A-98/09985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

In particular, the present invention may be useful in the treatment of neurological disorders or injuries as discussed herein.

### DELIVERY

The delivery system for use in the present invention is a lentiviral vector.

### VECTORS

As it is well known in the art, a vector is a tool that allows or faciliates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. Optionally, once within the target cell, the vector may then serve to maintain the heterologous DNA within the cell or may act as a unit of DNA replication. Examples of vectors used in recombinant DNA techniques include plasmids, chromosomes, artificial chromosomes or viruses.

The term "vector" includes expression vectors and/or transformation vectors.

The term "expression vector" means a construct capable of *in vivo* or *in vitro*/*ex vivo* expression.

The term "transformation vector" means a construct capable of being transferred from one species to another.

### RETROVIRAL VECTORS

Examples of retroviruses include but are not limited to: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV). A detailed list of retroviruses may be found in Coffin et al ("Retroviruses" 1997 Cold Spring Harbour Laboratory Press Eds: JM Coffin, SM Hughes, HE Varmus pp 758-763).

Vectors for use in accordance with the present invention are lentiviral vectors. Lentiviral vectors are able to deliver genes to non-dividing, terminally differentiated cells.

The term "recombinant retroviral vector" (RRV) refers to a vector with sufficient retroviral genetic information to allow packaging of an RNA genome, in the presence of packaging components, into a viral particle capable of infecting a target cell. Infection of the target cell includes reverse transcription and integration into the target cell genome. The RRV carries non-viral coding sequences which are to be delivered by the vector to the target cell. An RRV is incapable of independent replication to produce infectious retroviral particles within the final target cell. Usually the RRV lacks a functional *gag-pol* and/or *env* gene and/or other genes essential for replication.

Lentiviral genomes can be quite variable. For example there are many quasi-species of HIV-1 which are still functional. This is also the case for EIAV. These variants may be used to enhance particular parts of the transduction process. Examples of HIV-1 variants may be found at http://hiv-web.lanl.gov. Details of EIAV clones may be found at the NCBI database: http://www.ncbi.nlm.nih-gov.

EIAV vectors have been shown to deliver genes very efficiently to a number of neuronal cell types *in vitro* and *in vivo.* Gene expression has been sustained for a number of months *in vivo,* with little or no immunological reaction. Thus, according to the present invention EIAV vectors are a suitable delivery system to direct expression of RARβ2 in the human peripheral and central nervous systems and such systems are discussed in detail herein.

Vector titre may be estimated by infection assays. For example, infections could be carried out with vector preparation in question, and antibody staining for the product of the nucleotide of interest could be used to determine the proportion of productively infected cells, giving an indication of the titre of the vector preparation. For example, antibodies directed against RARβ2 are commercially available and may be advantageously utilised for this purpose according to the manufacturers' instructions. Alternatively, a PCR approach may be used, by amplifying using primers directed at the nucleotide of interest delivered by the vector, such as a nucleotide sequence directing the expression of RARβ2. Primers may advantageously be designed to include or comprise vector sequence(s) in order to ensure that the relevant amplification product has indeed originated from the sequence in question. Other ways in which vector titre may be estimated are known in the art, and are discussed in the Examples section hereinbelow.

### PHARMACEUTICAL COMPOSITIONS

The present invention also provides a pharmaceutical composition comprising a vector of the present invention and a pharmaceutically acceptable carrier, diluent or excipients (including combinations thereof).

The pharmaceutical composition may comprise two components - wherein a first component comprises a vector encoding RARβ2 and a second component which comprises an RARβ2 agonist.

The first and second component may be delivered sequentially, simultaneously or together, and even by different administration routes. The pharmaceutical compositions may be for human or animal usage in human and veterinary medicine and will typically comprise any one or more of a pharmaceutically acceptable diluent, carrier, or excipient Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation -requirements- dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example; an intravenous, intramuscular or subcutaneous route. Alternatively, the formulation may be designed to be delivered by both routes.

Where the vector or nucleic acid sequence is to be delivered mucosally through gastrointestinal mucosa, it should be able to remain stable during transit through the gastrointestinal tract; for example, it should be resistant to. proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### PHARMACEUTICAL COMBINATIONS

The vector of the present invention may be administered with one or more other pharmaceutically active substances. By way of example, the present invention covers the simultaneous, or sequential treatments with a vector according to the present invention and one or more steroids, analgesics, antivirals or other pharmaceutically active substance(s).

It will be understood that these regimes include the administration of the substances sequentially, simultaneously or together.

### EXAMPLES

The present invention will now be described, by way of example only, in which reference will be made to the following figures:
Figure 1 (which is Figure 1 referred to in Example 1) shows a photograph.
Figure 2, (which is Figure 2 referred to in Example 1) shows barcharts and a photograph.
Figure 3, (which is Figure 1 referred to in Example 2) shows a photograph.
Figure 4, (which is Figure 2 referred to in Example 2) shows a photograph.
Figure 5, (which is Figure 3 referred to in Example 2) shows a photograph.
Figure 6, (which is Figure 4 referred to in Example 2) shows a photograph.
Figure 7, (which is Figure 5 referred to in Example 2) shows a photograph.
Figure 8, (which is Figure 6 referred to in Example 2) shows a barchart.

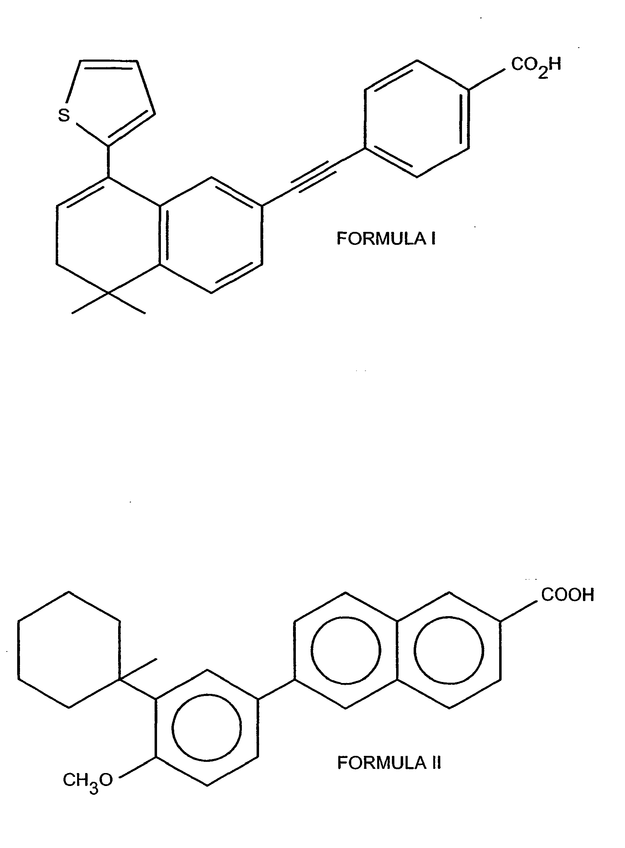

Figure 9, (which is Figure 1 referred to in Example 3) shows a photograph.
Figure 10, (which is Figure 2 referred to in Example 3) shows a photograph.
Figure 11, (which is Figure 3 referred to in Example 3) shows a photograph.
Figure 12, (which is Figure 4 referred to in Example 3) shows a photograph.
Figure 13, (which is Figure 5 referred to in Example 3) shows a photograph.
Figure 14, (which is Figure 6 referred to in Example 3) shows a photograph.
Figure 15, (which is Figure 7 referred to in Example 3) shows barcharts.
Figure 16, (which is Figure 8 referred to in Example 3) shows a photograph.
Figure 17 shows chemical formulae.
Figure 18 shows photomicrographs.

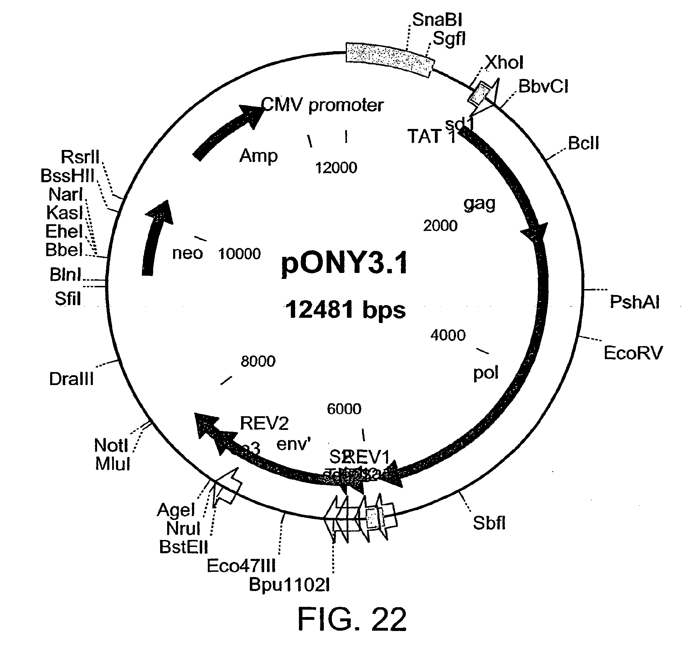

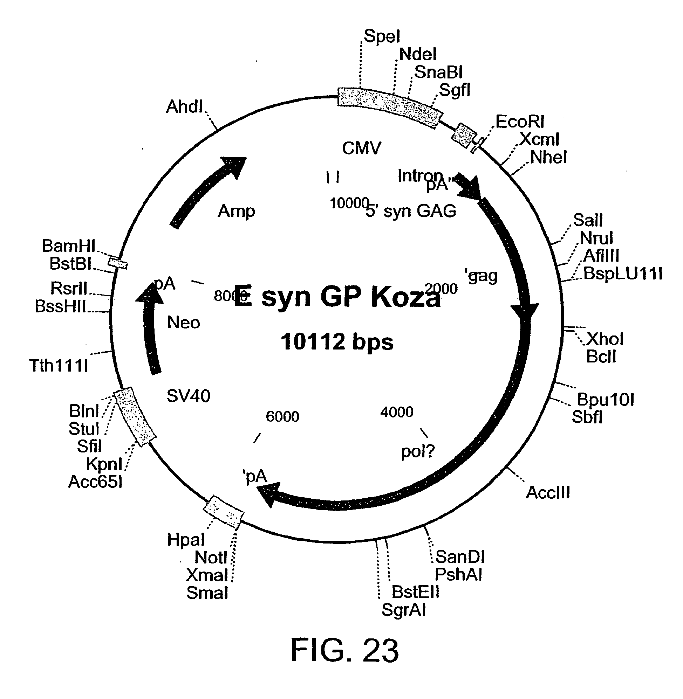

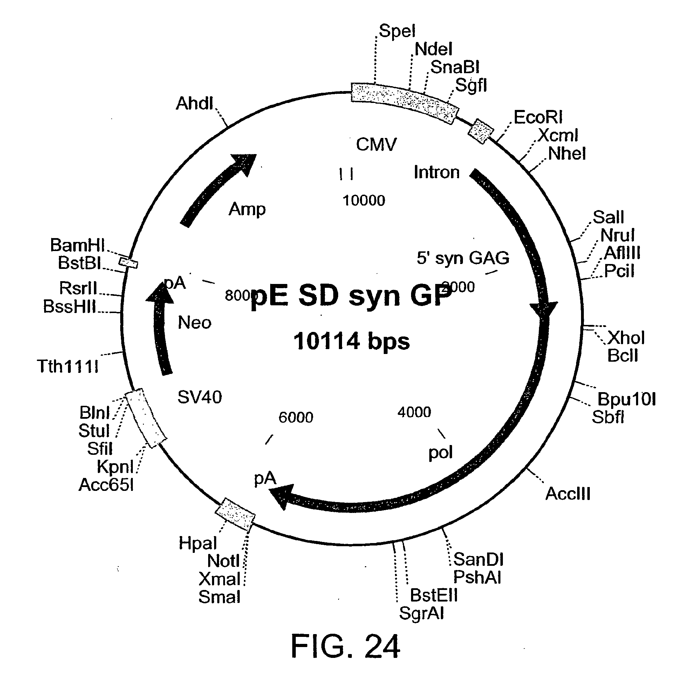

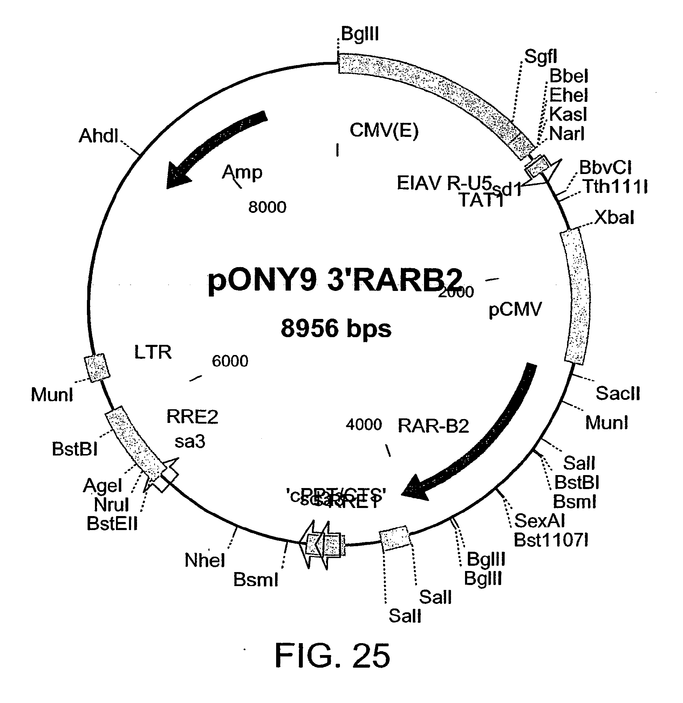

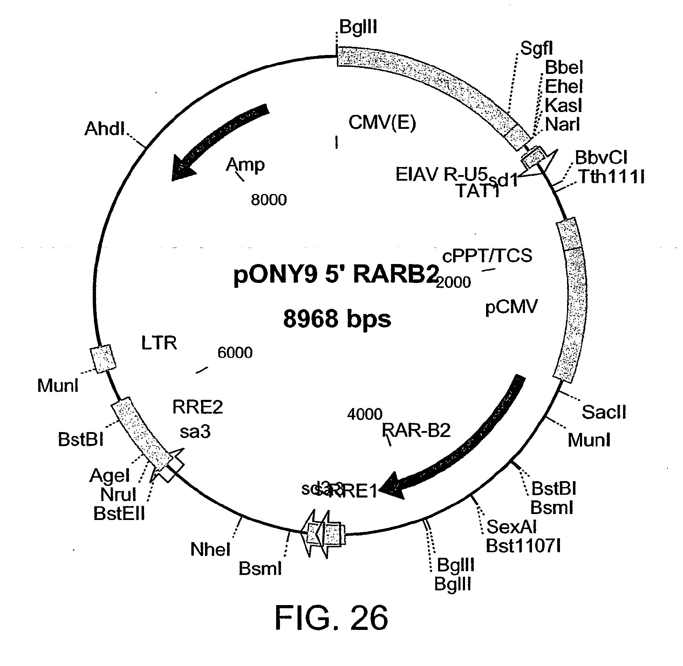

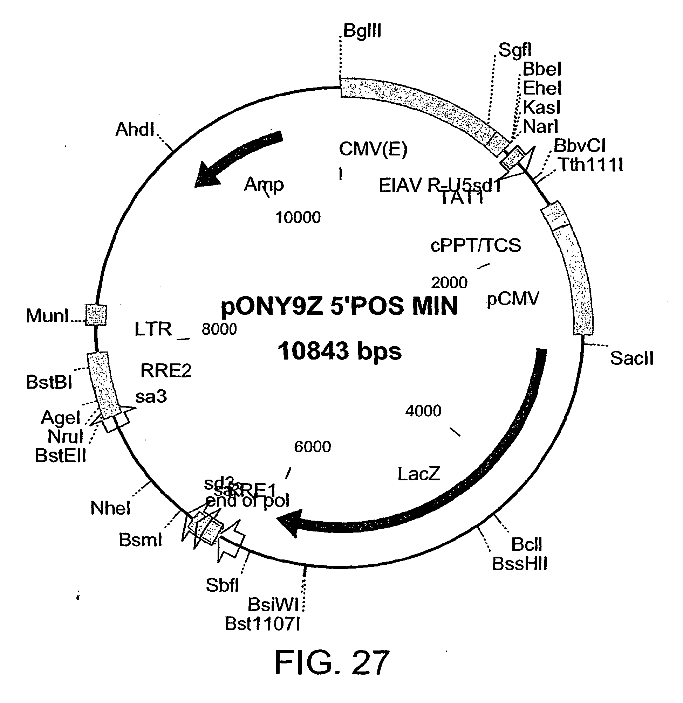

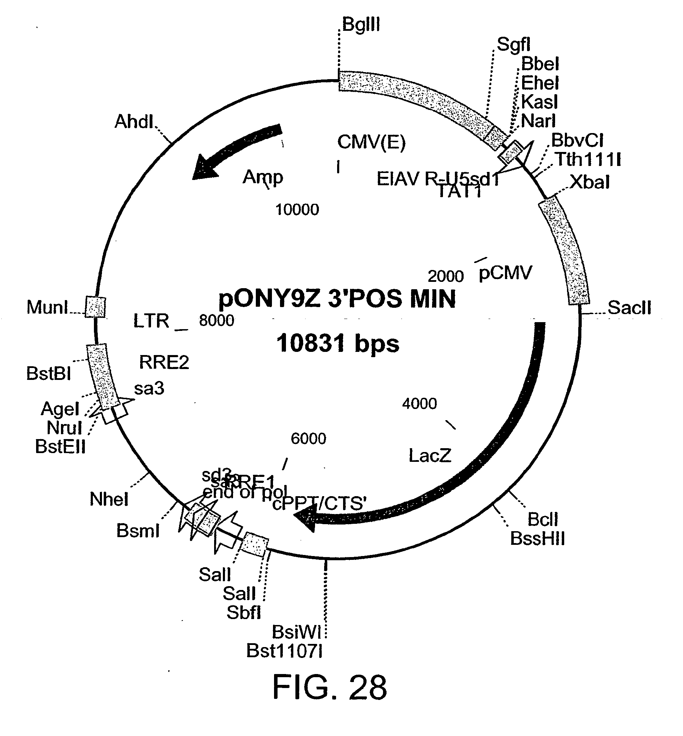

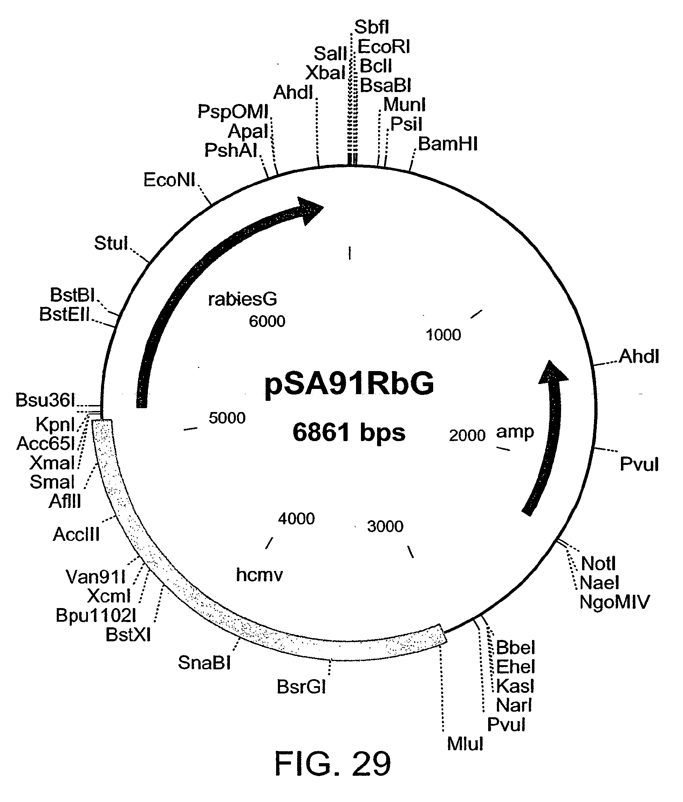

Figures 19-29 show plasmid constructs.
Figure 30 shows sequence.
Figure 31 shows a plasmid construct.
Figure 32 shows sequence.
Figure 33 shows a plasmid construct.
Figure 34 shows sequence.
Figure 35 shows a plasmid construct.
Figures 36-40 shows sequences.
Figure 41 shows a plasmid construct.
Figure 42 shows sequence.
Figure 43 shows a plasmid construct.
Figure 44 shows sequence.
Figure 45 shows a plasmid construct.
Figure 46 shows sequence.
Figure 47 shows a plasmid construct.
Figure 48 shows sequence.
Figure 49 shows a plasmid construct.
Figure 50 shows two photomicrographs.
Figure 51 shows three photomicrographs.

The figures are described more fully in the following example sections.

### Plasmid construction

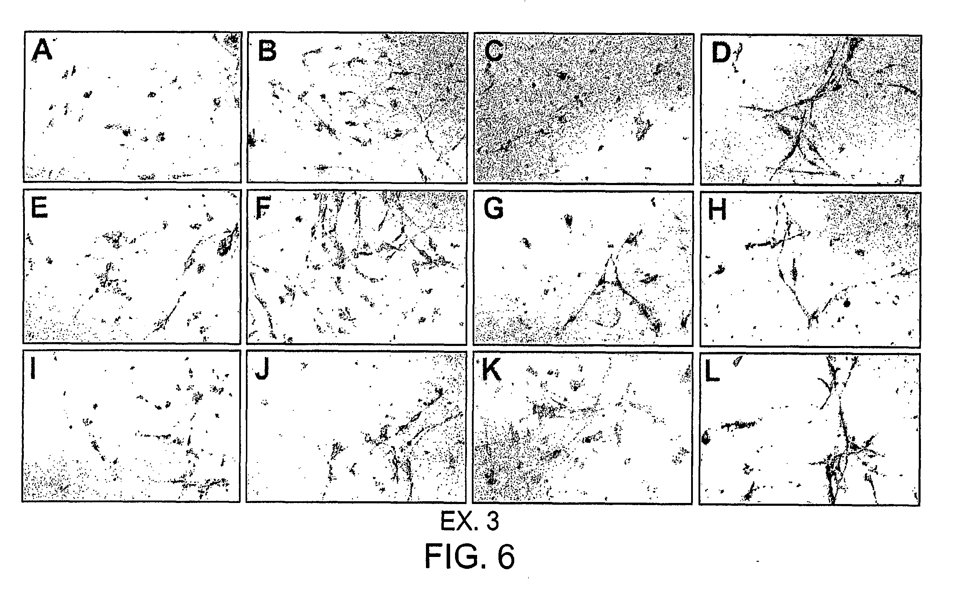

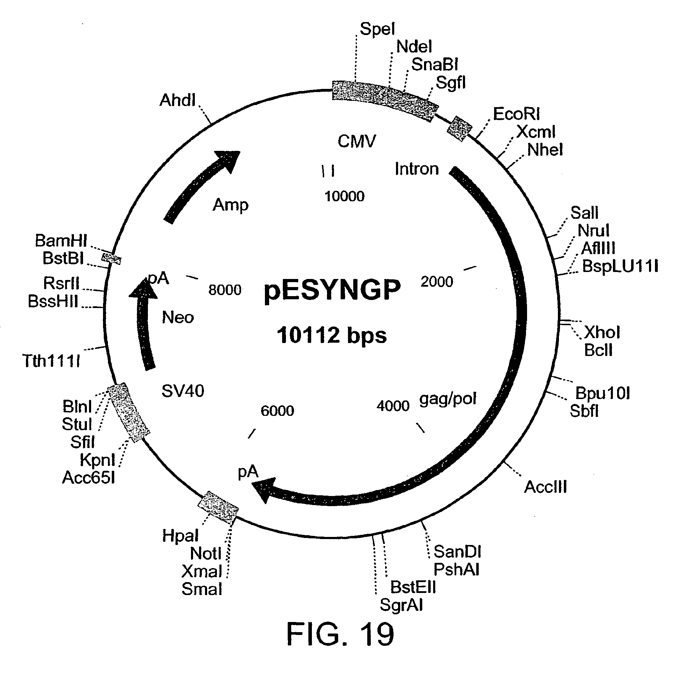

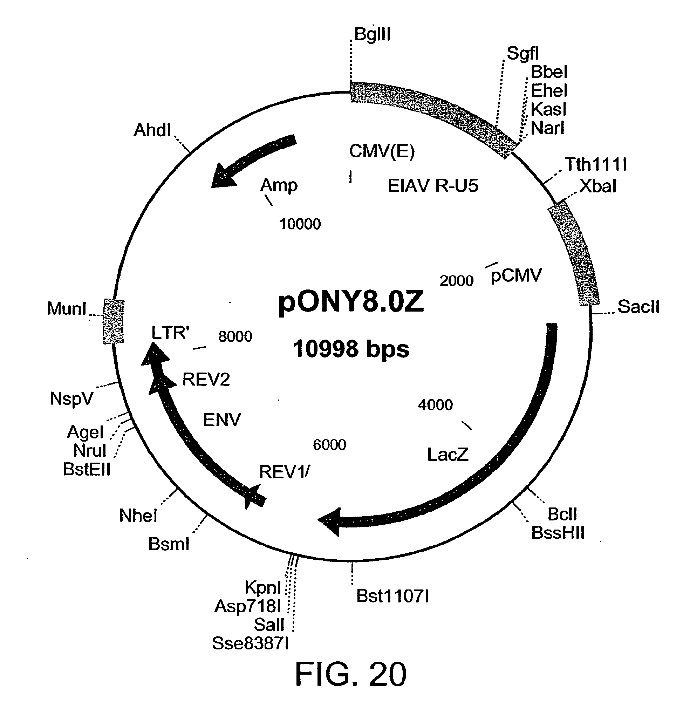

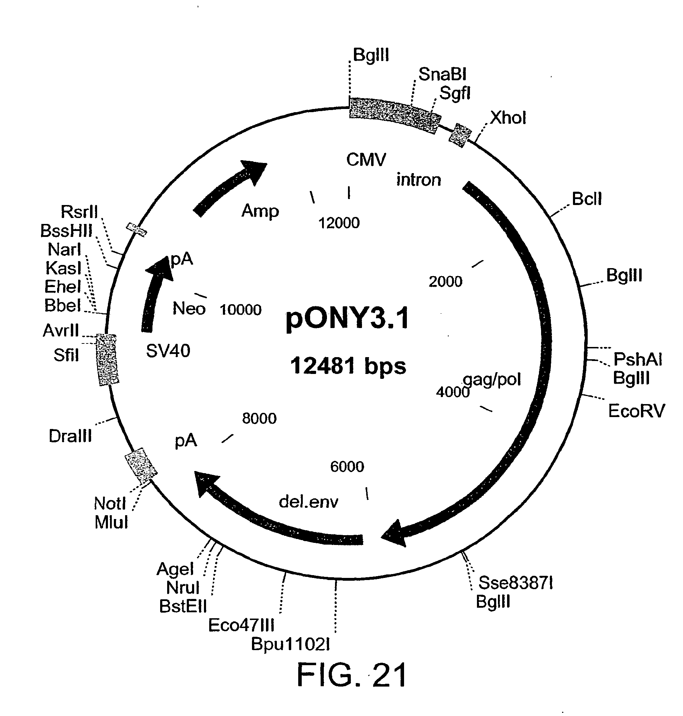

Numerous plasmids/constructs used in the following Examples (including pRV67, pRabG/pSA91RbG and others), are described in WO99/61639; pONY3.1 is described in WO 99/32646 (eg. see example 9, fig. 6) and elsewhere; pSP72 is a standard Promega cloning vector, Genbank Acc.No.X65332; sources of other materials are as indicated or described herein, for example see figures 19-21 and/or the accompanying sequence listing.

The plasmid used to express EIAV REV is pE syn REV which is a pCIneo based plasmid (Promega) which is made by introducing the EcoRI to SalI fragment from a synthetic EIAV REV plasmid into the polylinker region of the pCIneo using the same sites. The synthetic EIAV REV plasmid made by Operon contains a codon-optimised EIAV REV open reading frame flanked by EcoRI and SalI. The sequence of this fragment is shown in the sequence listing as codon optimised EIAV REV.

ESDSYNGP is made from pESYNGP by exchange of the 306bp EcoRI-Nhel fragment, from just upstream of the start codon for gag/pol to approximately 300 base pairs inside the gag/pol ORF with a 308bp EcoRI-Nhel fragment derived by digestion of a PCR made using pESYNGP as template and using the following primers: SD FOR [GGCTAGAGAATTCCAGGTAAGATGGGCGATCCCCTCACCTGG] and SD REV [TTGGGTACTCCTCGCTAGGTTC]. This manipulation replaces the Kozak concensus sequence upstream of the ATG in pESYNGP with the splice donor found in EIAV. The sequence between the EcoRI site and the ATG of gag/pol is thus CAGGTAAG.

The codon-optimised EIAV gag/pol ORF is synthesised by Operon Technologies Inc., Alameda and supplied in a proprietary plasmid backbone, GeneOp. The complete fragment synthesised includes sequences flanking the EIAV gag/pol ORF: tctagaGAATTCGCCACC**ATG- EIAV gag/pol- UGA**ACCCGGGgcggccgc. The ATG start and UGA stop codons are shown in bold. Xbal and Notl sites are in lower case. These are used to transfer the gag/pol ORF from GeneOp into pClneo (Promega) using the Nhel and Notl sites in the latter.

pONY8.0Z construction: pONY8.0Z is derived from pONY4.0Z by introducing mutations which (1) prevent expression of TAT by an 83nt deletion in the exon 2 of tat (2) prevent S2 ORF expression by a 51 nt deletion (3) prevent REV expression by deletion of a single base within exon 1 of rev and (4) prevent expression of the N-terminal portion of gag by insertion of T in ATG start codons, thereby changing the sequence to ATTG from ATG. With respect to the wild type EIAV sequence Acc. No. U01866 these correspond to deletion of nt 5234-5316 inclusive, nt 5346-5396 inclusive and nt 5538. The insertion of T residues is after nt 526 and 543.

### COMPARATIVE EXAMPLE 1: STIMULATION OF NEURITE OUTGROWTH

Nerve growth factor acts via retinoic acid synthesis to stimulate neurite outgrowth in the peripheral nervous system.

Nerve growth factor (NGF) stimulates neurite outgrowth from cultured adult dorsal root ganglia (DRG)1. The vitamin A derivative retinoic acid (RA) also induces neurite outgrowth from various embryonic sources, including DRG2,3 Are such similarities in effects of NGF and RA because they are both components of the same genetic cascade leading to neurite outgrowth? RA up-regulates low-and high-affinity NGF receptors3,4 and induces the transcription of NGF itself5, suggesting that RA may be upstream of NGF in the cascade. However, here we show the converse, namely, that NGF is upstream of RA. We show that when adult mouse DRG are cultured in the presence of NGF and a compound that inhibits enzymes involved in RA synthesis, neurite outgrowth does not occur. Conversely, when RA is added along with a blocking antibody to NGF, neurite outgrowth occurs as normal. We further show that NGF induces transcription of both the retinoic acid-synthesizing enzyme RALDH-2 and the retinoic acid receptor-β as well as detectable release of synthesized RA. We propose that RA is required for adult DRG neurite regeneration and that NGF acts upstream of RA to induce its synthesis.

Cellular effects of RA are mediated by binding to nuclear receptors that are ligand activated transcription factors. There are two classes of receptors, retinoic acid receptors (RARs) and retinoid X receptors (RXRs), with three subtypes of each: α, β and γ6,7 In addition, there are multiple isoforms of each subtype due to alternative splicing and differential promoter usage. RAR receptors mediate gene expression by forming heterodimers with the RXRs, whereas RXRs can mediate gene expression either as homodimers or by forming heterodimers with orphan receptors such as LXR8 An additional mechanistic association between NGF and RA pathways is suggested by the findings that the nuclear receptor NGFIB heterodimerizes with the RXRs8 and that NGFIB is rapidly induced in PC12 cells by the administration of NGF9.

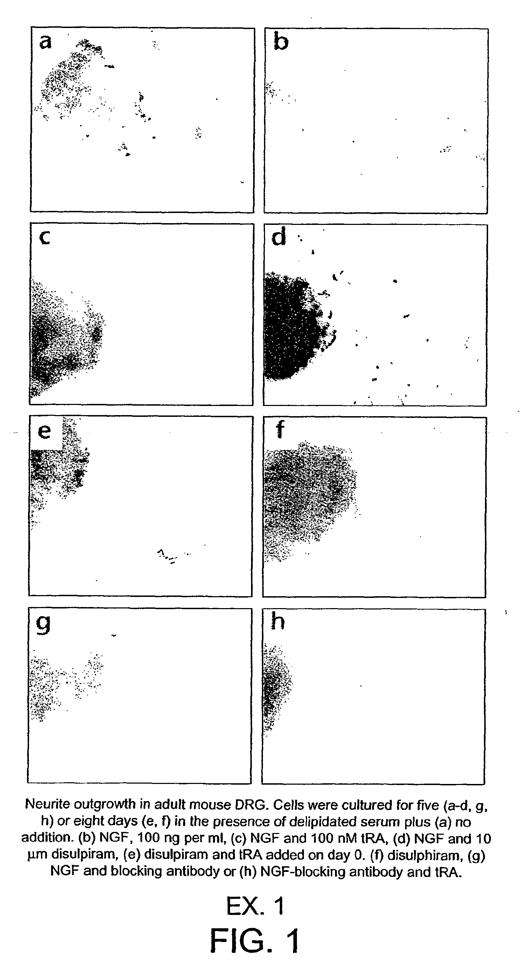

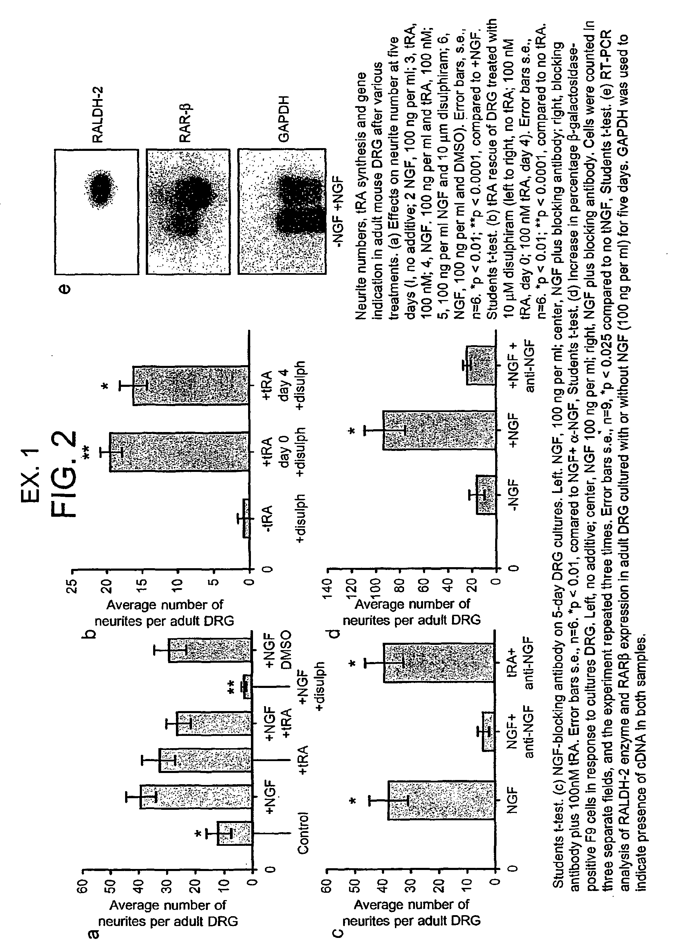

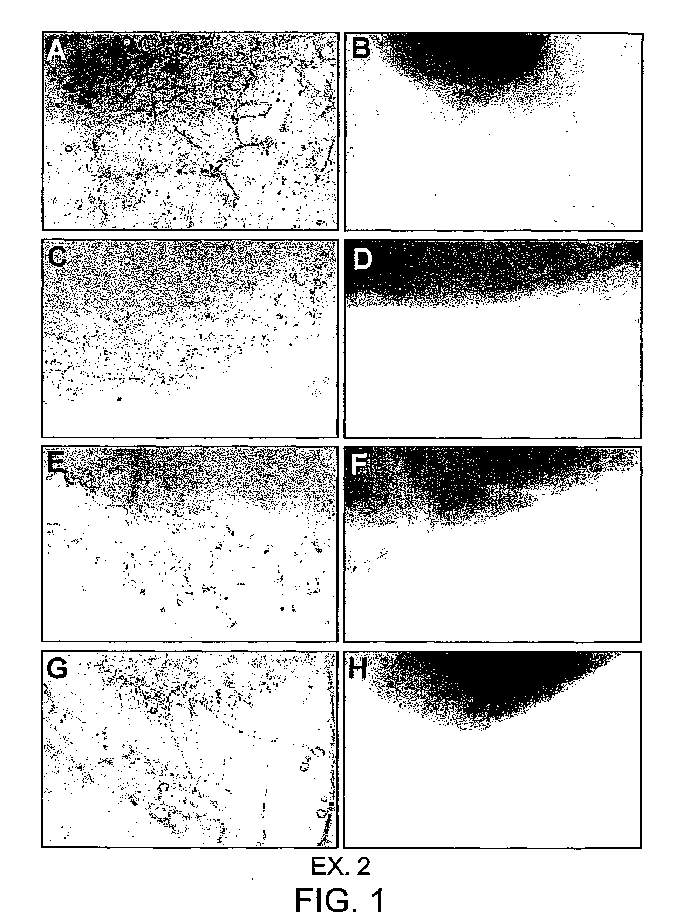

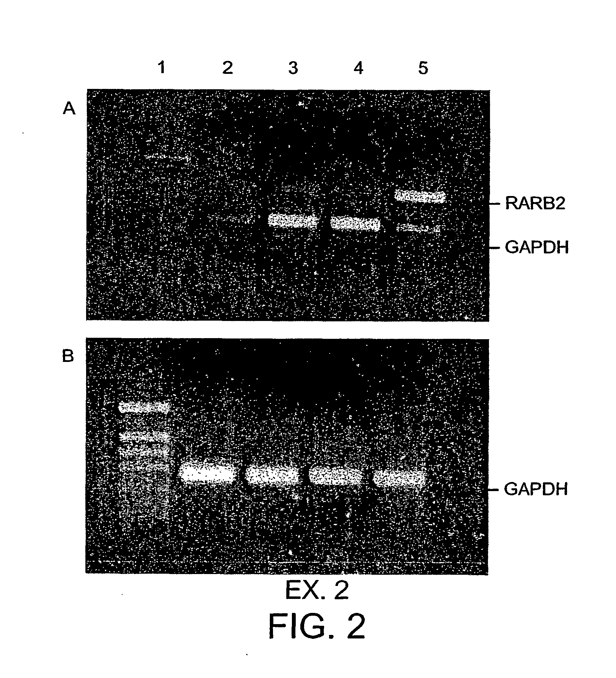

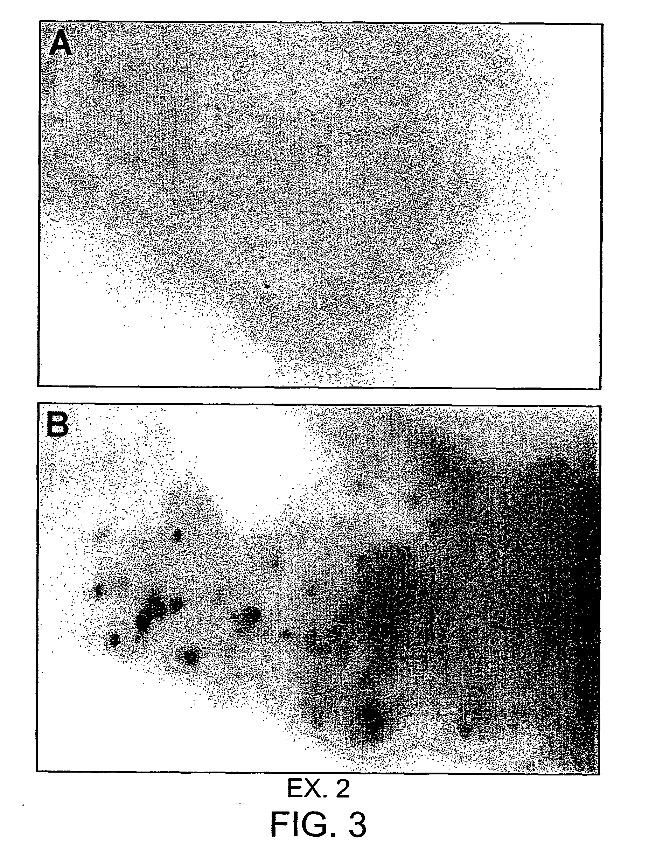

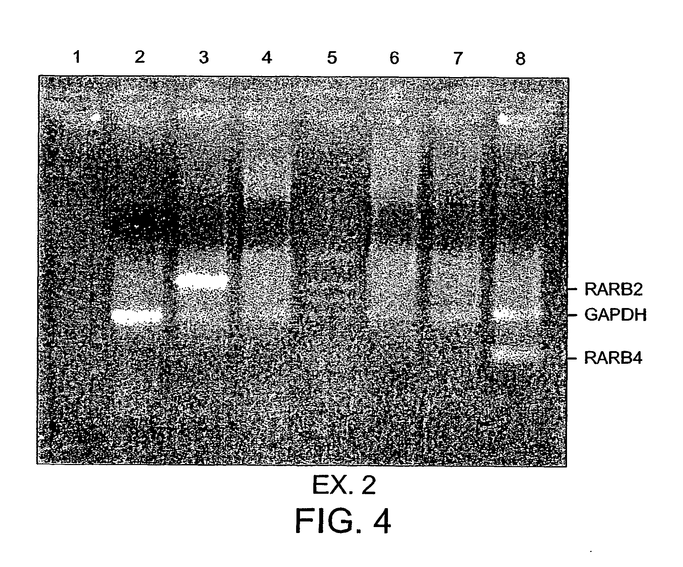

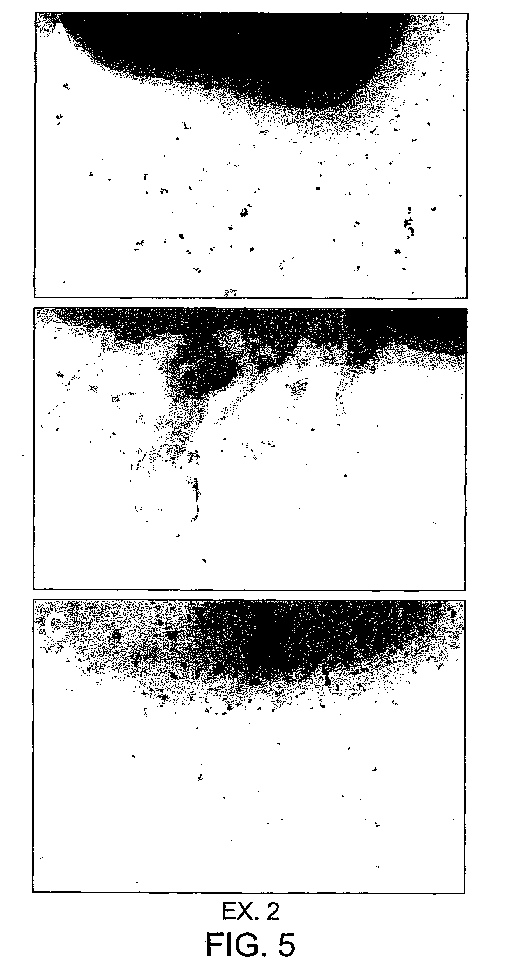

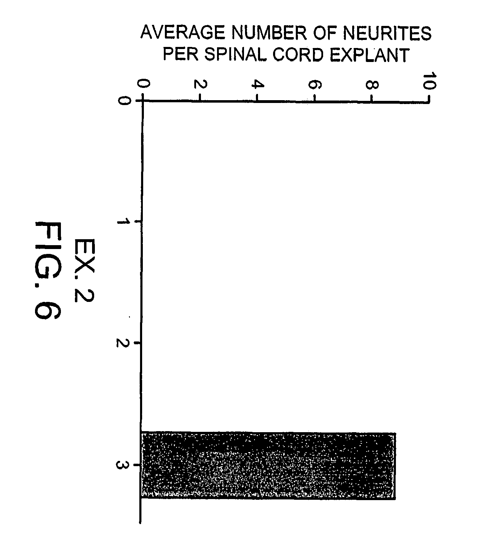

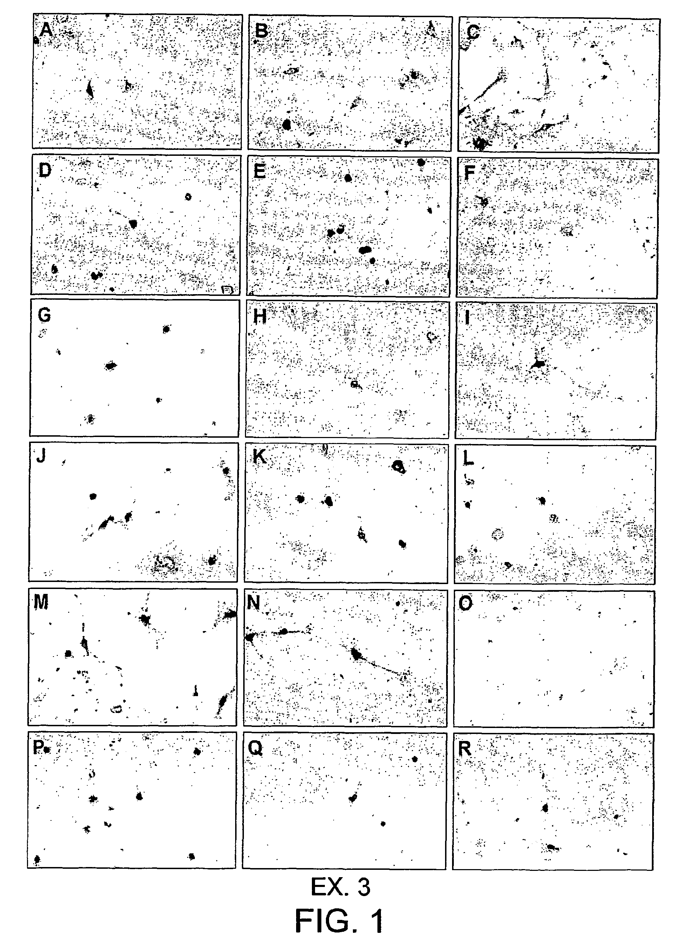

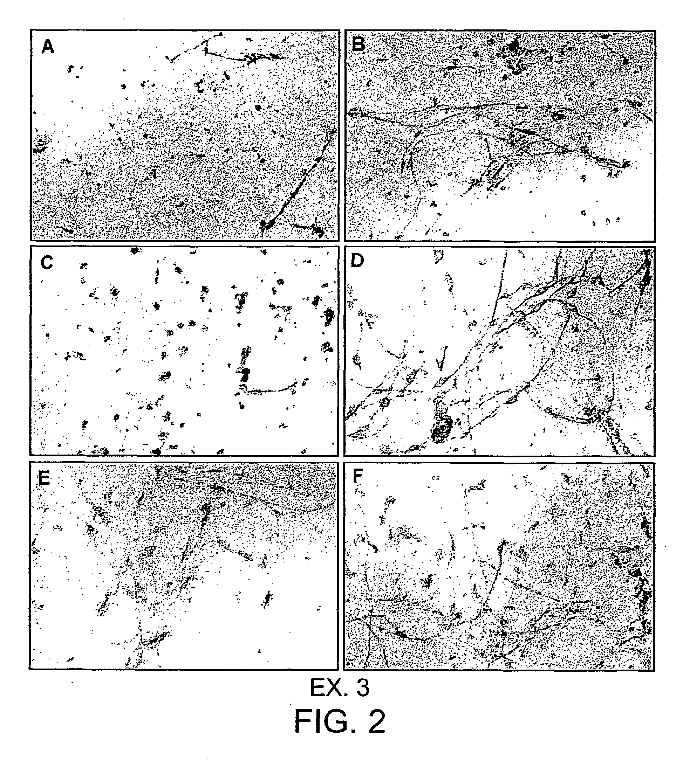

Although there is clearly a role for RA in the stimulation of neurite outgrowth from embryonic DRG2,3, it is not yet known if the same occurs in the adult DRG. To test this, we cultured adult mouse DRG in the presence of NGF (100 ng per ml), or RA (100 nM) in delipidated serum for five days. In both cases neurite outgrowth occurred (**Fig. 1b**). Little or no neurite outgrowth occurred in adult DRG cultured in only delipidated serum (**Fig. 1a**). Differences in number of neurites were significant (**Fig. 2a; 1,** 2 and 3). It is important to note that the number of neurites extended from RA- or NGF-treated adult DRG, although significantly greater than the number extended from untreated DRG, was smaller than the number obtained using embryonic tissue. When RA was added together with NGF, there was no additive effect of the two treatments (**Fig. 1c**), and no significant difference was seen between RA, NGF or RA plus NGF groups (**Fig. 2a**; 2, 3 and 4). Although it may be that both NGF and RA are at individual saturating concentrations, the lack of synergy may also imply that NGF and RA act through the same pathway in order to cause neurite outgrowth. One could imagine either RA inducing the production of NGF5, or NGF inducing the production of RA by stimulating a RA-synthesizing enzyme.

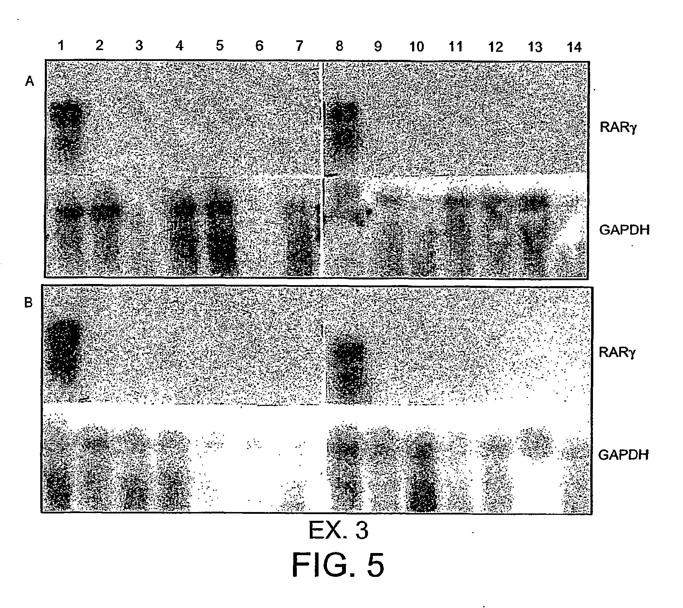

To test which of these hypotheses is most likely, we cultured adult DRG in the presence of NGF and 10 µM disulphiram, a compound which blocks the conversion of retinaldehyde to RA by inhibiting the enzyme aldehyde dehydrogenase10. If RA acts to stimulate NGF production then disulphiram should have no effect on NGF-stimulated neurite outgrowth, whereas if NGF induces RA synthesis then disulphiram should inhibit outgrowth. As shown in Fig. 1d, addition of 10 µM disulphiram along with NGF completely abolished NGF-induced neurite outgrowth (significant difference; **Fig. 2a,** 2 and 5), whereas addition of DMSO (vehicle for disulphiram) and NGF did not affect neurite outgrowth (**Fig. 2a,** 2 and 6). To confirm that disulphiram did not affect cell survival within the explants, we performed two types of rescue. In both cases, explants were cultured for eight days in medium supplemented with disulphiram. In the first rescue, 100 nM RA was added to the explants from the beginning of the experiment; in the second, RA was added on day 4. In both cases, significantly greater neurite outgrowth occurred compared to cultures grown in medium supplemented with disulphiram alone (**Figs. 1e**, **f** and **2b**). These experiments also confirm the specificity of disulphiram for the RA synthesis pathway, as RA can rescue the cellular response.

Inhibition of the inductive effect of NGF but not of RA by disulphiram suggests that NGF may precede RA in the cascade leading to neurite outgrowth. To test this, we used a blocking antibody against NGF In the presence of NGF and the blocking antibody, virtually no neurite outgrowth occurred (**Fig. 1g;** compare to DRG cultured in the presence of NGF alone, **Fig. 1b).** On the other hand, DRG cultured in the presence of the NGF-blocking antibody and 100 nM RA (**Fig. 1h)** showed neurite outgrowth equivalent to that obtained with NGF alone (**Figs. 1b** and **2c**).

If NGF is upstream of RA, it should induce synthesis of RA after addition to DRG cultures. To test this prediction, we used an F9 reporter cell line that responds specifically to the presence of RA due to transfection with 1.8 kb of the mouse RARβ2 gene promoter containing a retinoic acid response element (RARE) linked to the *lacZ* gene (Sonneveld, E., van den Brink, C. E., van der Leede, B. J., Maden, M. & van der Saag, P. T. (1999) Embryonal carcinoma cell line stably transfected with mRARb2-lacZ: sensitive system for measuring levels of active retinoids. Exp.Cell.Res. vol. 250 pp284-297). In the presence of RA, activated cells can be detected after β-galactosidase histochemical staining. We first eliminated the possibility that NGF itself activates F9 cells by growing them in the presence of NGF (100 ng per ml), whereupon there was no labeling of the F9 cells above background. We then cultured adult DRG in delipidated serum for five days under three different conditions: in the absence of NGF, in the presence of NGF or in the presence of both NGF and the NGF-blocking antibody. Cultured DRG were then sonicated and placed on the F9 reporter cells. NGF-treated DRG homogenates produced a clear RA signal relative to untreated DRG (**Fig. 2d**). This activation was prevented when the DRG were cultured with blocking antibody in addition to NGF (**Fig. 2d**).

We next considered which retinoic acid synthesizing enzymes might be induced by NGF. Retinol is converted by a two-step oxidative process to an aldehyde, retinal, which is then oxidized to retinoic acid (for review, see ref. 11). It has been shown that retinaldehyde dehydrogenase type 2 (RALDH-2) is expressed in the developing nervous system, including the DRG12. Using RT-PCR, we found strong induction of RALDH-2 by NGF in cultured adult DRG as well (**Fig. 2e**). Lastly, we also found up-regulation of the RARβ receptor in NGF-stimulated cultures (**Fig. 2e**), a phenomenon shown to be involved in neurite outgrowth13.

Our results show that RA can stimulate neurite outgrowth from an adult neural tissue, the DRG. NGF similarly stimulates neurite outgrowth from this tissue, and we have demonstrated that it does so by inducing RA synthesis via an enzyme, RALDH-2. In the presence of either a NGF-blocking antibody or an inhibitor of RA synthesis, then NGF fails to act. Thus the most likely sequence of events in the induction of neurite outgrowth by NGF is: NGFRALDH-2RARARβ neurite outgrowth. We have not yet determined if NGF is directly responsible for inducing RALDH-2, or if some intermediary protein is required for this process. However, as NGFIB is one of the earliest genes induced by NGF9 and its product can heterodimerize with the RXRs8, the NGFIB/RXR heterodimer may be responsible for activating the RALDH-2 gene. Neurotrophins classically have been considered as potential agents for induction of nerve regeneration14 and treatment of neurodegenerative diseases15, but a major problem for their use is lack of effective modes of delivery to the site of the injury. Because RA is required for the regenerative response and it is downstream of NGF, then the problem of delivery to the lesion could be overcome, as RA is a low-molecular-weight lipophilic compound that can be administered orally. Thus, RA may be of clinical use in neurology.

### FIGURES FOR COMPARATIVE EXAMPLE 1

**Fig. 1.** Neurite outgrowth in adult mouse DRG cultured for five (**a-d, g, h**) or eight days **(e, f)** in the presence of delipidated serum plus: (a) no addition; (b) NGF, 100 ng per ml; (c) NGF and 100 nM tRA; (d) NGF and 10 M disulphiram; (e) disulphiram and tRA added on day 0; (f) disulphiram; (g) NGF and blocking antibody (h) NGF-blocking antibody and tRA.

**Fig. 2. (a-c)** Neurites produced by adult DRG cultured in cellogen. (a) Effects of NGF, RA and disulphiram at five days (**1**, no additive; **2**, NGF, 100 ng per ml; **3**, RA, 100 nM; **4**, NGF, 100 ng per ml and RA, 100 nM; **5**, 100 ng/ml NGF and 10 M disulphiram; **6**, NGF, 100 ng per ml and DMSO). Error bars, s.e.; *n* = 6, all groups. Differences between NGF-treated (2) and other groups: **p* < 0.01; ***p* < 0.0001; Student's *t*-test. (b) RA rescue of DRG treated with 10 M disulphiram (**left to right:** no RA; 100 nM RA, day 0; 100 nM RA, day 4) Error bars, s.e.; *n* = 6, all groups. Differences from RA-absent cultures: **p* < 0.01, ***p* < 0.0001; Student's *t*-test. (c) Effect of NGF-blocking antibody on 5-day DRG cultures. Left, NGF, 100 ng per ml; center, NGF plus blocking antibody; right, blocking antibody plus 100 nM RA; *n* = 4. Differences from NGF plus blocking antibody: **p* < 0.01, ***p* < 0.0001, Student's *t-*test. **(d)** Increase in percentage β-gal-positive F9 cells in response to DRG cultured with or without NGF. Left, no additive; center, NGF, 100 ng per ml; right, NGF with blocking antibody. Differences in percentage β-gal-positive cells from that produced by NGF-treated DRG ; **p* < 0.025, Student's *t*-test; for each group, *n =* 9. **(e)** RT-PCR analysis of RALDH-2 enzyme and RARβ expression in adult DRG cultured with or without NGF (100 ng per ml) for five days. GAPDH was used to indicate presence of cDNA in both samples. Use of F9 reporter cells in studying RA distribution in chick embryo has been described16.

### COMPARATIVE EXAMPLE 2: INDUCTION OF NEURITE DEVELOPMENT IN ADULT NEURAL TISSUE

It is surprisingly shown herein that retinoic acid receptor-β2 induces neurite outgrowth in the adult mouse spinal cord.

Retinoic acid has been shown to be required for neurite outgrowth. We have recently demonstrated that the mechanism of it's action in peripheral nerve regeneration is by activating the retinoic acid receptor _{β2}. The adult central nervous system cannot regenerate. Therefore, we have investigated if regenerative failure in the adult spinal cord is related to the expression of retinoic acid receptor _{β2}.

**Results:** We report here that in embryonic mouse spinal cord which can regenerate RAR_{β2} is up-regulated at concentrations which maximally stimulate neurite outgrowth. In contrast in the adult mouse spinal cord, RAR_{β2} is not detected nor is it induced by RA and no neurites are extended *in vitro.* When the adult cord is transfected with RAR_{β2} neurite regeneration can be induced. There is no neurite outgrowth when the cord is transfected with another isoform of RAR_{β,} RAR_{β4}. This shows the importance of receptor specificity in neurite regeneration.

**Conclusion:** These data suggest that the loss in regenerative potential of the adult CNS is due in part to the loss of expression of RAR_{β2} and that it is intrinsic to the neuron itself. We suggest that gene therapy with RAR_{β2} may result in functional recovery of the injured spinal cord.

**Background** The induction of axonal regeneration in the adult central nervous system (CNS) is a major goal in neurobiology. The failure of CNS axons to regenerate under normal circumstances has been attributed to one or a combination of causes: the low abundance of neurotrophic factors; the absence of growth-promoting molecules; the presence of growth-inhibiting molecules. Thus attempts to restimulate axon growth in the CNS have centered on these three possible. When peripheral nerve grafts were used to provide a permissive environment then spinal cord and medulla neurons extended axons up to 30mm in the adult rat¹. A similar strategy combined with fibroblast growth factor application resulted in the partial restoration of hind limb function². Neutralisation of neurite growth inhibitors present in myelin with antibodies permitted longer extension of axons than in control young rats³ and led to the recovery of specific reflex and locomotor functions after spinal cord injury⁴. A combination of neurotrophin-3 and these antibodies was successful in inducing long distance regeneration of corticospinal tract (CST) axons⁵. A suspension of olfactory ensheathing cells was also effective in returning locomotor function to the lesioned CST of rats⁶. If neurotrophins act simply to keep axotomised neurons alive⁷ then in these methodologies for inducing regeneration it is the environment surrounding the axons which is the focus of attention rather than the intrinsic capabilities of the neuron itself. However, at least part of the regenerative loss of the CNS is intrinsic to the neuron itself(4 refs). This suggests that the identification of genes that are not expressed in the non regenerating adult CNS but are in the developiong CNS which can regenerate neurites may lead to new stratagies of treatment of spinal cord injuries by gene therapy.

We show here that one such gene is RARβ2 which is activated by retinoic acid (RA) the biologically active metabolite of vitamin A. RA is present in various tissues of the developing embryo and adult animal, especially the nervous system⁸⁻¹³. In its absence, developing neurons of the CNS do not extend neurites into the periphery^{14,15}. Conversely, when applied to cultured neurons, RA induces both a greater number and longer neurites ¹⁶ as well as being capable of dictating their direction of growth¹⁷. RA acts at the level of gene transcription because it is a ligand for two classes of nuclear transcription factors, the retinoic acid receptors (RARs) and the retinoid X receptors (RXRs)^{18,19}. There are three members of each class of retinoid receptor a, b and g as well as several isoforms of each member and this diversity may be responsible for the pleitropic effects of RA on cells.

We have been studying the molecular mechanisms of action of RA on neurons and have concluded that one of these retinoic acid receptors, RAR_{β2} is the crucial transducer of the RA signal in neurons as it is up-regulated in situations where RA stimulates neurite outgrowth²⁰. We hypothesised therefore that the absence or below threshold level of this nuclear receptor in the adult spinal cord may contribute to the failure of this tissue to regenerate axonal projections.

### Results and discussion

### Effect of RA on embryonic mouse spinal cord in vitro.

We began by confirming that the mouse embryonic spinal cord will respond to RA by extending neurites as do other areas of the embryonic CNS^{12,17, 21-23} and that this behaviour involves an up-regulation of RARβ2. E13.5 spinal cord was dissected from mouse embryos placed in a cellogen matrix and cultured in 10 % delipidated serum. All*-trans-*RA was added at 3 different concentrations (10⁻⁸M, 10⁻⁷M, 10⁻⁶M) and after 5 days the explants were stained with a neurofilament antibody and examined for the presence of neurites. There was an increasing number of neurites emerging from the cultured cord with increasing concentrations of RA with the maximal effect at 10⁻⁶M (Fig. 1C, E, G). Even in the absence of RA the embryonic cord extended neurites (Fig. 1A) presumably because of the high endogenous content of RA and its precursor retinol^{9,13}. Indeed, when the endogenous synthesis of RA is inhibited with disulphiram then no neurites are extended²⁴. To demonstrate that the induction of neurites involved the up-regulation of RARβ2, RT-PCR was performed on cultures after 5 days in the same range of RA treatments. This revealed that RAR_{β2} is normally expressed in embryonic spinal cord at all concentrations of RA used (Fig. 2A, lanes 1-5) and that it is strongly up-regulated after 1 x 10⁻⁶ M RA treatment (Fig. 2A, lane 5), the same concentration which gives maximal neurite outgrowth.

### Lack of effect of RA on adult mouse spinal cord in vitro.

We next performed an identical series of experiments using 10 month old adult spinal cord rather than the embryonic cord. In contrast to the embryonic cord, RA had no effect on neurite outgrowth at any concentration tested and like the untreated controls, these RA treated adult cords failed to extend any neurites at all (Fig. 1B, D, F, H). Examining the involvement of RAR_{β2} by RT-PCR revealed that control adult spinal cord had little or no detectable endogenous levels of this receptor (Fig. 2B, lane 1) and that there was no change in its level in response to RA treatment at any concentration (Fig. 2, lanes 2-5), unlike the embryonic cord.

### Induction of neurites in adult spinal cord

We therefore hypothesised that it was the lack of RAR_{β2} expression which may be responsible for the completely inert behaviour of the adult spinal cord. Our previous observations that adult DRG which do respond to RA by extending neurites also up-regulate RAR_{β2}²⁴ demonstrates that the same behaviour is elicited by embryonic and the appropriate adult neurons and reinforces the differences in regulative behaviour between PNS and CNS neurons. To test our hypothesis we used a defective herpes simplex virus type 1 (HSV-1) vector to transfect pieces of adult (10 months) mouse spinal cord.

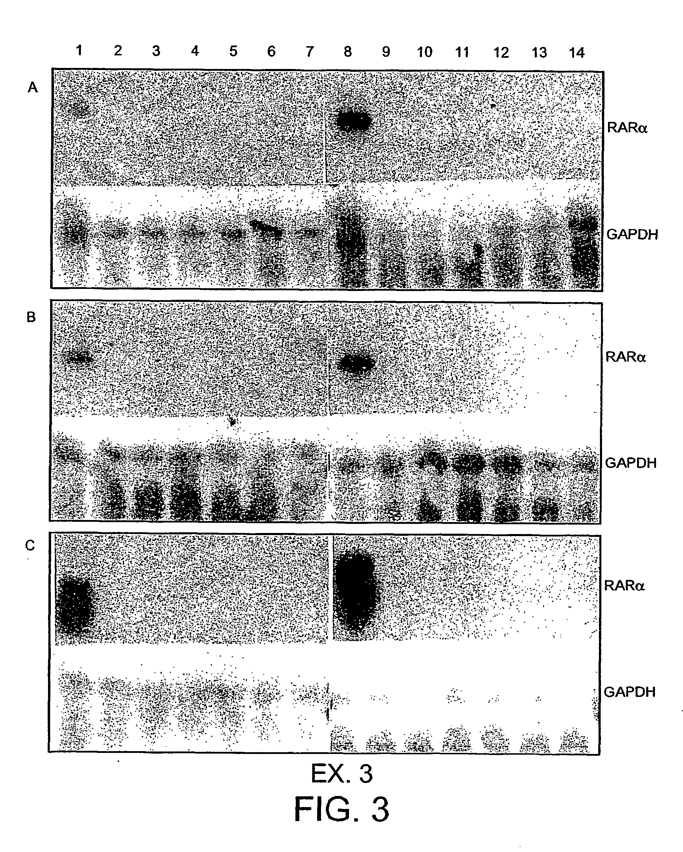

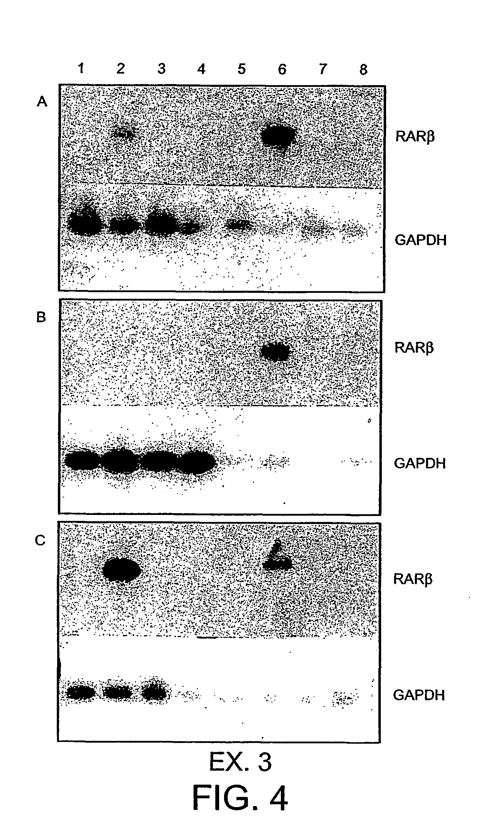

Three different transfections were performed, two of which served as controls. Firstly, just the vector containing lacZ (pHSVlacZ); secondly the vector containing RAR_{β2} (pHSVRAR_{β2}) ; thirdly the vector containing another isoform of the RARb gene, RAR_{β4} (pHSVRAR_{β4}). The latter served as a very precise control for transfection since we do not detect the RAR_{β4} isoform after RA treatment of neurons in our previous experiments²⁰ hence it is not involved in neurite outgrowth. We first ensured that the transfections were successful and that the relevant receptor isoform was expressed in the cultured cord. Pieces of spinal cord were transfected overnight with the appropriate construct and analysed either three or four days later. The pHSVlacZ treated cords showed a significant amount of transfection had taken place as judged by b-galactosidase staining of the adult cord (Fig. 3B). RT-PCR demonstrated that transfection with the RAR_{β2} vector resulted in the expression of RAR_{β2} (Fig. 4, lane 3) but not RARβ4 (Fig. 4, lane 4) and transfection with the RAR_{β4} vector resulted in the expression of RAR_{β4} (Fig. 4, lane 8) but not RARβ2 (Fig. 4, lane 7). In the non transfected cord neither RAR_{β2} or RAR_{β4} were detected (Fig. 4, lanes 2 and 6).

The effects of these transfections on neurite outgrowth were clear-cut. Transfection with the pHSVlacZ failed to change the behaviour of the cultured adult cord which remained completely un-responsive in terms of neurite outgrowth (Fig. 5A, 12/12 transfections). Similarly, the transfections with pHSVRAR_{β4} produced no response in the cultured cord which remained inert (Fig. 5C, 12/12 transfections). However, transfections with the pHSVRAR_{β2} isoform clearly produced a different behaviour and many neurites appeared in the cultures (Fig. 5B, 8/12 transfections). The number of neurites produced in the pHSVRAR_{β2} cord varivaried between 3 and 23. In the pHSVlacZ transfections there was considerable variability in the number of lacZ-positive cells per explant. This suggests that the variability in neurite number may be due to variability in number of cells transfected.

These results provide strong support for our hypothesis that the RAR_{β2} isoform plays a key role in the induction of neurite outgrowth in response to RA and that this may be a crucial component which fails to be up-regulated in the injured adult CNS. Our hypothesis is based upon several experiments involving either regenerating or non-regenerating neuronal tissues and their response to RA. Thus the embryonic mouse spinal cord, the embryonic mouse DRG and the adult mouse DRG all respond to RA by up-regulating RAR_{β2} and extending neurites. In contrast, the adult mouse spinal cord fails to up-regulate RAR_{β2} and fails to extend neurites. Furthermore, NGF stimulates neurite outgrowth and acts by up-regulating RAR_{β2} ²⁴ and neurite outgrowth from embryonic mouse DRG can be stimulated by a RAR_{β} agonist²⁰.

These results reveal that when the genome of the neuron itself is manipulated then regeneration can be reawakened. This is in contrast to the recent inductions of neurite outgrowth *in vivo* which have concentrated on the inhibitory factors present in the CNS environment¹⁻⁵. During development the loss of regenerative capacity of the spinal cord correlates with the appearance of myelin associated neurite growth inhibitory molecules and some of these are thought to be produced by the oligodendrocytes²⁵. Either the regeneration of neurites we see in our cultures and that seen in cultures where the environment has been manipulated are two different mechanism of neurite regeneration or they are related processes. Support for the latter view is provided by the fact that CNS neurons themselves have been shown to be involved in myelination²⁶. Therefore it is tempting to speculate that the presence of RAR_{β2} in neurons during development may regulate genes involved in myelination, and that this process is recapitulated by transfection of the RAR_{β2} gene in the adult CNS.

None of the neurites we observed in the RAR_{β2} transfected cord elongated over an appreciable distance. This suggests that elongation of the neurite may require the expression of a different set of genes. Evidence that this may be true is shown from regeneration of axons in the adult PNS, where a transition from arborizing to elongating growth depends upon a transcriptional dependent switch²⁷. Alternatively the failure of elongation of neurites in our cultures may be due to the fact that there is likely to be a loss of expression of RAR_{β2} over time due to the transient nature of the transfections and that this does not allow enough time for elongation to occur.

Nonetheless we propose that these preliminary data support a role of RAR_{β2} in the regeneration of neurites in the adult CNS and that gene therapy with this transcript in combination with other treatments may one day lead to functional recovery of the injured spinal cord.

### Methods

**Cultures**. Spinal cord was dissected from either E13.5 or 10 month old mice and cut into transverse pieces of about 5 mm. These were cultured in cellogen matrix (ICN flow), prepared by mixing 1 volume of 7.5 % sodium bicarbonate, 1 volume of 10x MEM (Gibco) and 8 parts cellogen (ICN flow). The pH was adjusted to 7.5 by dropwise addition of 5M NaOH. Explants were fed every two days. The media consisted of DMEM-F12 with glutamine (Gibco), 6 % glucose, GMS-A (Gibco) 10% delipidated serum and all*-trans-*RA (stock solution, 1 x 10⁻⁵ M, Sigma). On the fifth day they were fixed in 4 % paraformaldyde and stained with the neurofilament antibody, NF200 (Sigma).

**RT-PCR analysis.** RNA was extracted (trizol, Gibco) and cDNA prepared by the use of a Pharmacia kit as described in the manufactures instructions. The primers used were from GAPDH, RAR_{β2} and RAR_{β4}. PCR was carried out for 25 cycles for embryonic spinal cord and 40 cycles for adult spinal cord. Amplification was carried out as follows, denaturation for 30 s at 95 °C, annealing for 30 s at 55 °C and extension for 1 min at 72 °C. One fifth of the resultant product was then run on a gel.

**Transfections.** Virus stocks were prepared and *B* galactosidase staining carried out as described in ref²⁸. The titres used were: pHSV RAR_{β2}, 5 x 10⁻⁴ ip/ul, pHSVRAR_{β4},, 4 x 10⁻⁴ ip/ ul, pHSVlacz 5 x 10⁻⁴ ip/ul .

### FIGURES FOR COMPARATIVE EXAMPLE 2:

Fig.1. Comparison of the effect of retinoic acid on neurite outgrowth on cultured E13.5 (A, C, E, G) and 10 month old adult spinal cord (B, D, F, H). Pieces of spinal cord were cultured in cellogen in the presence of 10 % delipidated serum and RA for a period of five days. The medium was changed every two days. **A, B,** no RA; **C, D,** 1 x 10⁻⁸ MRA; **E, F,** 1x10⁻⁷ M RA; **G, H,** 1x10⁻⁶ M RA.

Fig.2. Expression of RAR_{β2} in E13.5 and 10 month old adult spinal cord. Pieces of spinal cord were cultured in the presence of various concentrations of RA for a period of five days after which time RT-PCR analysis of RAR_{β2} was performed. A. E13.5 (lanes 2-5) and B. 10 month old adult spinal cord (lanes 2-5). Lanes: 1. bluescript/HPA II size markers, 2. no RA, 3. 1 x 10⁻⁸ M RA, 4. 1 x 10⁻⁷ M RA, 5. 1 x 10⁻⁶ M RA. The presence of GAPDH was used to indicate equal amounts of cDNA in the samples.

Fig.3. Transfection of adult spinal cord with pHSVlacZ. Cultured 10 month old adult spinal cord was transfected with 5 x 10⁻⁴ ipu/ul pHSVlacZ overnight and analysed for *B* galactosidase staining 3 days later. A. non-transfected adult spinal cord. B. adult spinal transfected with pHSVlacZ.

Fig.4. Transfection of adult spinal cord with either pHSVRAR_{β2} or pHSVRAR_{β4}. Adult spinal cord was cultured in cellogen and transfected either with 5 x 10⁻⁴ ipu/ul of pHSVRAR_{β2} or 4 x 10⁻⁴ ipu/ul pHSVRAR_{β4} overnight. RT-PCR analysis four days after transfection, of RAR_{β2} (lanes 2-4) and RAR_{β4} (lanes 6-8) expression in adult spinal cord transfected with Lanes 2, 6 no virus , 3, 7 pHSVRAR_{β2}, 4, 8, pHSVRAR_{β4}. The presence of GAPDH was used to indicate equal amounts of cDNA in the samples. Lanes 1,2 bluescript/HPA II size markers.

Fig.5. Effect of either pHSVlacZ, pHSVRAR_{β2} or pHSVRAR_{β4} transfection in cultured adult spinal cord on neurite outgrowth. Ten month old spinal cord was cultured in cellogen and transfected with either 5 x 10⁻⁴ ipu/ul, pHSVlacZ, 5 x 10⁻⁴ ipu/ul, pHSVRAR_{β2} or 4 x 10⁻⁴ ipu/ul pHSVRAR_{β4} overnight, and analysed for neurite staining with NF200 4 days after transfection. Cultured spinal cord transfected with A. pHSVlacZ, B. pHSVRAR_{β2}, C. pHSVRAR_{β4}.

Fig. 6 is a barchart of the average number of neurites per spinal cord explant.

### COMPARATIVE EXAMPLE 3: NEURITE OUTGROWTH FROM MOUSE DORSAL ROOT GANGLION NEURONES

This Example demonstrates stimulation of neurite outgrowth by retinoic acid/RARβ in the peripheral nervous system. The role of retinoic acid receptors in neurite outgrowth from different populations of embryonic mouse dorsal root ganglia.

Dorsal root ganglion (DRG) neurons can be categorised into at least three types based upon their neurotrophin requirement for survival. We have analysed the expression of the retinoic acid receptors (RARs) and the retinoid X receptors (RXRs) in NGF, NT-3 and BDNF dependent neurons isolated from embryonic day 13.5 mouse DRG. We show that each population of neurons expressed each of the three RXRs, α, β and γ. However, whilst the NGF and NT-3 dependent neurons expressed each of the RARs α, β and γ the BDNF dependent neurons only expressed RARα and β. When retinoic acid was added to each of the neuronal classes only the NGF and NT-3 dependent neurons responded by extending neurites, and this response involved the up-regulation of RARβ2. This specificity was confirmed by the use of receptor selective agonists as only a RARβ selective compound stimulated neurite outgrowth. These results suggest a role for RA acting via RARβ2 in the outgrowth of neurites.

### Introduction

The neurotrophins are a family of growth factors that are required for the survival of a variety of primary sensory neurons in the developing peripheral nervous system (Snider, 1994). The family includes nerve growth factor (NGF) (Levi-Montalcini, 1987) neurotrophin-3 (NT-3) (Maisonpierre et al., 1990) and brain-derived neurotrophic factor (BDNF) (Barde et al., 1982). They are synthesised in the target fields innervated by peripheral neurons and are thought to be transported by a retrograde mechanism from the target field to support the survival of the developing neurons. The neurotrophins act through receptor tyrosine kinases designated Trk. NGF specifically activates TrkA; BDNF activates TrkB and NT-3 activates TrkC (reviewed in Snider, 1994). Analysis of the phenotypes resulting from loss of function experiments of the neurotrophins and the receptor tyrosine kinases have revealed that the dorsal root ganglia (DRG) neurons can be classified into at least three types. Neurons that require NGF for their survival mediate nocioceptive (pain) and thermal receptive functions. In the periphery the axons terminate in the superficial layers of the skin and innervate the superficial laminae of the spinal cord (Crowley et al., 1994; Smeyne et al., 1994). Proprioceptive neurons (sense of position of the limbs in space), which are much larger than NGF type neurons, project into the periphery to the primary endings of muscle spindles and extend a collateral branch to the motor pools in the spinal cord are dependent upon NT-3 for their survival (Ernfors et al., 1994; Farinas et al., 1994; Klein et al., 1994). BDNF neurons are small to medium sized and may include some classes of the mechanoreceptors (Klein et al., 1993; Jones et al., 1994).

In addition to growth factors being involved in the survival of neurons, retinoids can also carry out the same role. Retinoids are a family of molecules derived from vitamin A and include the biologically active metabolite, retinoic acid (RA). The cellular effects of RA are mediated through the action of two classes of receptors, the retinoic acid receptors (RARs) which are activated both by all*-trans-*RA (*t*RA) and 9*-cis-*RA (9-*cis*-RA), and the retinoid X receptors (RXRs), which are activated only by 9*-cis-*RA (Kastner et al., 1994; Kleiwer et al., 1994). The receptors are of three major subtypes, α, β and γ, of which there are multiple isoforms due to alternative splicing and differential promoter usage (Leid et al. 1992). The RARs mediate gene expression by forming heterodimers with the RXRs, whilst the RXRs can mediate gene expression as homodimers or by forming heterodimers with a variety of orphan receptors (Mangelsdorf & Evans, 1995). Interestingly, one of the earliest genes induced by NGF in PC12 cells is the orphan receptor NGFI-B (NURR1) (Millbrandt, 1989). This suggests that the growth factor and retinoid mediated pathway in developing neurons can interact. This interaction may be critical for the survival of the neuron because RA has been shown to be involved in the survival and differentiation of neurons (Wuarin and Sidell, 1991; Quinn and De Boni, 1991). Furthermore, many studies on a variety of embryonic neuronal types have shown that RA can stimulate both neurite number and length (reviewed in Maden, 1998) as indeed, can the neurotrophins (Campenot, 1977; Lindsay, 1988; Tuttle and Mathew, 1995). Recently we have shown that RA is critical for neurite regeneration in adult DRG and that it's synthesis may be regulated by NGF (Corcoran and Maden, 1999).

In the work described here, we use E13.5 mouse DRG to investigate further the nature of the interaction between the retinoid mediated pathway and the growth factor pathway by asking which of the RARs and RXRs are expressed in neurons that are dependent upon different neurotrophins for their survival. We show that a different retinoid receptor profile is indeed expressed in NGF, NT-3 and BDNF neurons and that this profile is altered after an RA treatment which induces neurite outgrowth. Specifically, RARβ2 is up-regulated in NGF and NT-3 neurons but not in BDNF type neurons. This result was confirmed by the use of receptor selective agonists, as only the RARβ agonist will substitute for RA in inducing neurite outgrowth. These results suggest a role for RA acting via RARβ2 in the outgrowth of neurites.

### Materials and Methods

**DRG cultures.** DRG were obtained from E13.5 mice, freed of non-ganglionic tissue and collected in ice-cold calcium magnesium free phosphate buffered saline. To prepare dissociated cell suspensions the ganglia were treated with 0.05 % trypsin for 15 minutes at 15 °C. The reaction was stopped by the addition of 1% serum and single cells obtained by trituration with a 23 G needle. The cells were then spun at 1000 g for ten minutes and resuspended in media. They were plated out at a density of approximately of 25000 cells/cm² in wells that had been precoated with 100µg/ml poly D lysine for 2 hrs. The cultures were fed every 2 days.

Culture media consisted of DMEM-F12 with glutamine (Gibco), 6 % glucose, ITS (Gibco). The growth factors used were either 50ng/ml NGF (7s, Promega) 50ng/ml NT3 (Promega) or 50ng/ml BDNF (Promega). Retinoids were used at a concentration of 1 x 10⁻⁷ M. All-trans-retinoic acid was obtained from Sigma and the receptor agonists were synthesised by CIRD Galderma: CD366 activates RARα, CD2019 activates RARβ, CD437 activates RARγ and CD2809 activates all of the RXRs.

**RT-PCR analysis.** RNA was extracted (trizol, Gibco) and cDNA prepared by the use of a Pharmacia kit as described in the manufacturer's instructions. The primers used were from mouse RARs, RXRs and GAPDH. In order to identify which RAR/RXR receptors were involved neurite outgrowth semi quantitative PCR was used. Amplification was carried out in the linear range for each RAR and RXR and their levels of expression were compared to GAPDH. For the RXRs 28 cycles were performed, while 25 cycles were used for RARα and RARγ and 22 cycles for RARβ and GAPDH. Amplification was carried out as follows, denaturation for 30 s at 95 °C, annealing for 30 s at 55 °C and extension for 1 min at 72 °C. One fifth of the resultant product was then run on a gel and blotted. This was then probed with the appropriate RAR, RXR or GAPDH for normalisation.

**In situ Hybridisation:** Cells were washed once with PBS and fixed in 4% PFA for 30 mins. They were then washed twice for 5 mins in PBS-0.05% Tween (PBT). Hybridisation was carried out at 55 °C overnight. The buffer consisted of 0.1M Tris-Cl, pH9.5, 0.05M MgCl₂, 0.1M NaCl and 0.1% Tween-20. The cells were then washed sequentially for 15 min. at 65°C in 50 % hybridisation buffer, 50% 2x SSC, 100% 2x SSC, and finally in 0.2% SSC. They were then washed at RT for 5 minutes each in 75% 0.2x SSC, 25% PBT, 50% 0.2x SSC, 50% PBT, 25% 0.2x SSC, 75% PBT, and 100% PBT. The cells were blocked in 2% sheep serum in PBT for 1 hr and incubated with anti DIG antibody overnight at 4°C. The cells were then washed 8 times in PBT for 2 hrs. Colour was developed by using NBT/BCIP according to the manufacturer's instructions (Boehringer Mannheim).

**Immunohistochemistry and measurement of neurite length:** Cells were washed once with PBS and fixed in 4% PFA for 30 mins. They were then washed twice for five minutes in PBS-0.05% Tween (PBT). They were then incubated in primary antibody NF200 (sigma) at 4°C overnight and washed 8 times for 2 hrs in PBT.

Secondary antibody was then applied for 2 hrs at RT, and the cells again washed 8 times for 2 hrs in PBT. They were then incubated for 5 mins. in PBS containing 0.5 mg/ml DAB and 6% H₂O₂. Neurite length was measured by using NIH image software. The experiments were repeated three times and three random fields were taken for each experiment for analysis. On average there were 40 neurons in each field and the longest neurite branch was measured for a given neuron.

### Results

### Expression of receptors by in situ hybridisation.

We first examined the expression of the RARs and the RXRs in primary cultures of E13.5 mouse DRG by in situ hybridisation. Dissociated DRG neurons were cultured in serum free medium either in the presence of NGF, NT-3 or BDNF for a period of five days. In the absence of neurotrophins the cells died. We found that all three types of neurons expressed RXRα (Fig. 1 D, J, P), RXRβ (Fig. 1 E, K, Q) and RXRγ (Fig. 1 F, L, R). In contrast, the RARs showed a differential expression between the three types of neurons. Whilst the NGF and NT-3 dependent neurons expressed RARα (Fig. 1 A, G), RARβ (Fig. 1 B, H) and RARγ (Fig. 1 C, I), the BDNF dependent neurons only expressed RARα (Fig. 1 M) and RARβ (Fig. 1 N). RARγ was not detectable by in situ hybridisation in the BDNF dependent cultures (Fig. 1 O).

### Effect of RA on neurite outgrowth

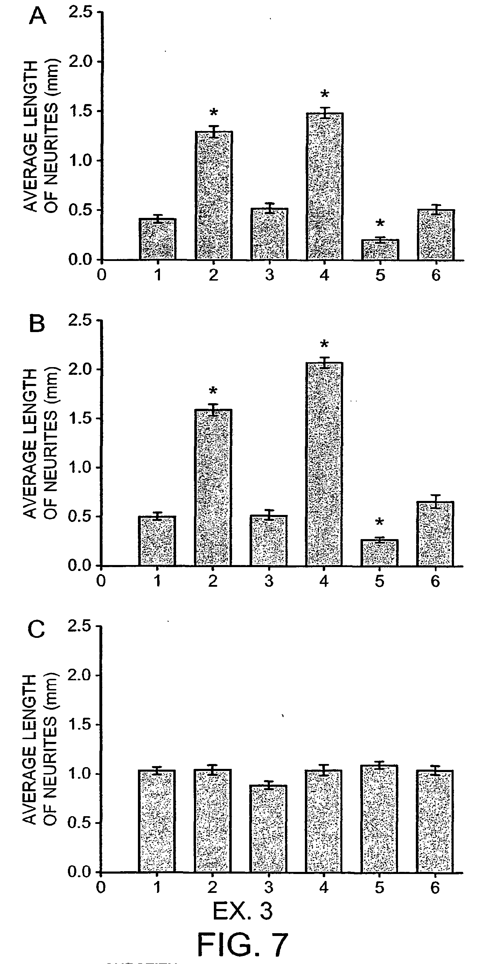

In order to eliminate any trophic effect of RA on the different populations of neurons we grew the neurons in serum free medium plus the relevant neurotrophin for a period of two days before adding 1 x 10-⁷M RA to the cultures for 3 days. Control cultures had no RA added and were maintained in neurotrophin only. There was no significant difference in the numbers of neurons cultured in the presence or absence of RA. This suggests that the effect of RA was on neurite outgrowth and not due to the selective survival of subsets of neurons under the different culture conditions used. In order to analyse neurite outgrowth the cultures were fixed after five days and stained with the monoclonal antibody NF200. Neurite length was measured by NIH image software. The experiment was repeated three times. In total approximately 120 neurons were counted in each experiment and the longest neurite was measured from each neuron from which an average neurite length was taken for each treatment. In the absence of RA the BDNF dependent neurons (Fig. 2 E and Fig.7 C column 1) grew neurites whilst the NGF (Fig. 2 A) and NT-3 dependent neurons (Fig. 2 C) showed limited neurite outgrowth (Fig. 7 A and B column 1). In contrast, when RA was added to the medium there was a dramatic increase in the length and number of neurites in the NGF (Fig. 2 B) and NT-3 dependent neurons (Fig. 2 D) and this difference was found to be significant when the length of the neurites were compared (Fig 7 A and B, columns 1 and 2). In contrast RA had no affect on neurite outgrowth of the BDNF dependent neurons (Fig. 2 F and fig. 7 C columns 1 and 2).

### Expression of receptors and response to RA by RT-PCR

In order to identify which of the receptors are involved in neurite outgrowth semiquantitative PCR was carried using primers against the RXRs and the individual RAR isoforms as described in the materials and methods. There was no difference in the expression of the RXRs in each of the three types of neurons cultured with or without RA. In contrast, there were variations in the RAR receptor profiles. Each of the three types of neurons expressed RARα₁ (Fig. 3 A, B, C, lane 1) which was strongly up-regulated in response to RA in the NGF (Fig. 3 A, lane 8) and NT-3 (Fig. 3 B, lane 8) dependent neurons and only slightly up-regulated in the BDNF dependent neurons (Fig. 3 C, lane 8). It is clear from Fig. 3 that only the RARα₁ isoform is readily detectable in these DRG neurons although on over-exposure of the blots the NT-3 dependent neurons expressed the RARα₅ and RARα₇ isoforms and the BDNF dependent neurons expressed the RARα₆ and RARα₇ isoforms.

Of the four possible RARβ isoforms only the RARβ₂ isoform was detected in all three types of neuron. This isoform was strongly up-regulated by RA in the NGF (Fig. 4 A, lane 6) and NT-3 dependent neurons (Fig. 4 B, lane 6) but not in the BDNF dependent neurons (Fig. 4 C, lane 6) as compared to the non-stimulated cultures (Fig. 4 A, B, C, lane 2)..

Of the seven RARγ isoforms only RARγ₁ isoform was detected in the neuronal cultures and then only in the NGF (Fig. 5 A, lane 1 and 8) and NT-3 dependent neurons (Fig. 5 B, lane 1 and 8). No RARγ₁ was detected by RT-PCR in the BDNF dependent neurons.

### Receptor selective analogues and neurite outgrowth

The above data suggested that the up-regulation of either RARα₁ or RARβ₂ may be responsible for the increase of neurite outgrowth observed in the NGF and NT-3 dependent neurons (Fig. 2 B, D). It is more likely to be the RARβ₂ isoform since this receptor is not upregulated in the BDNF dependent neurons and there is no increase in neurite outgrowth when these are stimulated with RA (Fig. 2 F) whereas the RARα₁ isoform is up-regulated despite a lack of neurite response to RA. In order to distinguish between these two receptors we used receptor selective synthetic retinoids which have been developed specifically to activate individual receptors. CD366 activates RARα, CD2019 activates RARβ, CD437 activates RARγ and CD2809 activates all of the RXRs.

In the presence of the RARα agonist there was no significant increase in neurite outgrowth in any neuronal population (Fig. 6 A, E, I and Fig 7 A B and C columns 1 and 3). In contrast, the RARβ agonist significantly increased neurite outgrowth in the NGF and NT-3 dependent neurons compared to non treated neurons (Fig. 6 B, F and Fig 7 A B columns 1 and 4), but did not effect neurite outgrowth in the BDNF dependent neurons (Fig. 6 J and Fig. 7 C columns 1 and 4). When the different neuronal populations were cultured in the presence of the RARγ agonist there was significant decrease in neurite outgrowth in the NGF and NT-3 dependent neurons (Fig. 6 C, G and Fig. 7 A B columns 1 and 5) whereas neurite outgrowth still occurred in the BDNF dependent neurons (Fig. 6 K and Fig. 7 C columns 1 and 5). There was no significant effect on neurite outgrowth in any of the neuronal populations when they were cultured in the presence of the RXR agonist (Fig. 6 D, H, L and Fig. 7 A B and C columns 1 and 6).

Therefore, in agreement with our RT-PCR data RARβ₂ is required for neurite outgrowth. Furthermore, RARγ can inhibit neurite outgrowth.

### Interrelationships between RARs

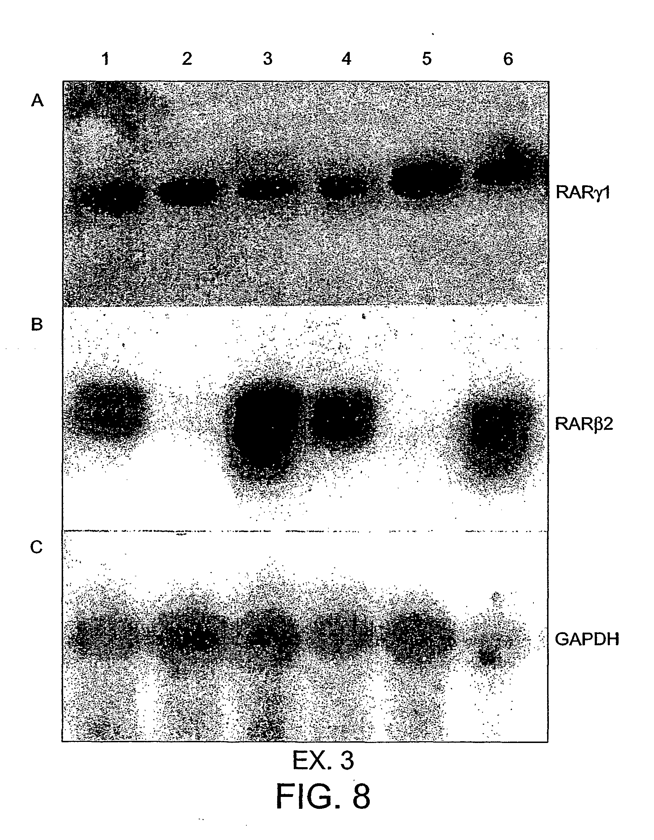

Finally, we attempted to investigate whether there were any regulative interactions between the receptors RARβ2 and RARγ1 since these have opposite affects on neurite outgrowth. In order to examine this we cultured NGF and NT-3 dependent neurons in serum free medium in the presence of either the RARγ agonist or the RARβ agonist and looked at the levels of receptor expression by semi quantitative RT-PCR 24 hrs. later. The RARβ agonist up-regulated the expression of RARβ2 in both the NGF and NT-3 dependent neurons (Fig. 8 B, lanes 3 and 6) compared to non-stimulated cultures (Fig.8 lanes 1 and 4) but did not affect the expression of RARγ₁ (Fig. 7 A, lanes 3 and 6). However, in the presence of the RARγ agonist, RARβ2 is reduced in both the NGF and NT-3 dependent neurons (Fig. 8 B, lanes 2 and 5) compared to non-stimulated cultures (Fig. 8 B, lanes 1 and 4). The RARγ agonist had no effect on the level of RARγ1 (Fig. 8 A, lanes 2 and 5). Thus RARγ1 can regulate the expression of RARβ2.

### DISCUSSION OF COMPARATIVE EXAMPLE 3

Our results show that each of the three dorsal root ganglia neuronal populations we have isolated (NGF, NT-3 and BDNF dependent) express both a common set and a unique set of retinoid receptors. With regard to the RXRs they each express RXRα, RXRβ and RXRγ and none of these were found to be directly involved in neurite outgrowth. In contrast, the neurons expressed different RARs depending on the neurotrophin used to select them. The major RAR isoforms that were common to each population were RARα₁ and RARβ₂. In addition, the NGF and NT-3 populations expressed RARγ₁ which was not expressed in the BDNF population at the time point analysed.

Only the NGF and NT-3 dependent neurons responded to RA by extending neurites whereas the BDNF dependent neurons produced neurites irrespective of the presence or absence of RA. In parallel there was a change in the RAR profile after RA addition. RARα₁ and RARβ₂ were strongly up-regulated in the NGF and NT-3 dependent neurons whereas in the BDNF dependent neurons only the RARα₁ was upregulated. This suggested that RARβ₂ was required for the induction of neurite outgrowth and to confirm this observation we used receptor selective agonists.

The development of receptor selective agonists has provided an extremely valuable tool to begin to examine the role of individual receptors in any particular biological process. We showed here that only the RARβ agonist, CD2019, mimiced the effect of RA by inducing neurite outgrowth in NGF and NT-3 neurons thus confirming our RT-PCR results.

We also observed that the RARγ agonist caused a decrease in neurite outgrowth of the NGF and NT-3 dependent neurons. In an attempt to show whether this was associated with the RARβ₂ expression we examined whether the RAR agonists had any effect on receptor expression. The RARβ agonist upregulated the expression of RARβ₂ but had no effect on the expression of RARγ₁. In contrast whilst the RARγ agonist had no effect on the expression of RARγ₁ it did down-regulate the level of RARβ₂ expression, this phenomenon may also be a prelude to neurite outgrowth. This suggests that the RARβ transcript can be regulated by RARγ/RXR heterodimers. The lack of increase in neurite outgrowth in response to RA of the BDNF dependent neurons also suggests that in this type of neuron that RARβ may be regulated differently to RARβ in the NGF and the NT-3 dependent neurons at the embryonic stage studied.

Our results suggest that it is the activation of the RAR pathway that is responsible for neurite outgrowth, since a RXR agonist which activates RXR/orphan receptors had no effect on neurite outgrowth whereas the RARβ agonist which activates RAR/RXR heterodimers increased the amount of neurite outgrowth. This suggest that if NGF acts via the RXR/orphan receptor pathway by utilising for example NGFI-B then it is not, in these embryonic stages, directly responsible for neurite outgrowth, rather it may be required for neuron survival. Interestingly in the adult the contrast seems to be true. NGF is not required for neuron survival but it is required for neurite outgrowth (Lindsay, 1988). Thus there may be different mechanisms for neurite outgrowth in developing and adult regenerating neurites. However, there is a absolute requirement for RA in neurite outgrowth during development. In the vitamin A deficient quail the neural tube fails to extend neurites into the periphery (Maden et al. 1996; Maden et al., 1998).

The differential response of these neurons to RA and the receptor agonists may have some significance for embryonic and adult tissues which require retinoids for their development and/or survival. In order to activate different RAR/RXR and RXR/orphan receptor combinations there may be different retinoids present in the tissues. Some support for this view is provided by the fact that there are numerous RA generating enzymes which show localised expression during development (McCaffery et al., 1992; Drager & McCaffery, 1995; Godbout et al., 1996; Neiderreither et al., 1997; Ang & Duester, 1997) and each of these enzymes could make different retinoids. Several novel retinoids have so far been discovered, for example 5, 6-epoxyretinoic acid, which is found in the intestine (McCormick et al., 1978), 4*-oxo*retinol which is the biologically active metabolite that is responsible for the differentiation of murine embryonic F9 cells (Achkar et al., 1996) and 14- hydroxy-4, 14-retroretinol which is found in B lymphocytes (Buck et al., 1991).

Therefore, the embryo may be able to regulate the amount of neurite outgrowth by synthesising different retinoids. By activating RARβ₂/RXR heterodimers neurite outgrowth could occur whereas by activating RARγ/RXR heterodimers neurite outgrowth could be stopped. In addition the amount of neurite outgrowth could be regulated by the amount of retinoic acid. For example in the developing mouse spinal cord there are high concentrations of retinoids in the brachial and lumbar enlargements (McCaffery & Drager, 1994). This may be an intrinsic requirement for innervation of the extremities of the limb where the neurites have to travel large distances to reach their final targets, whereas in the thoracic region where the concentration of retinoids are lower such extensive neurite outgrowth would not be required.

### Figure Legends for Comparative Example 3

Figure 1. Expression of the RARs and RXRs by E13.5 mouse embryo DRG neurons cultured either in the presence of NGF, NT-3 or BDNF. In situ hybridisation of: **A-F,** NGF neurons; **G-L,** NT-3 neurons; **M-R,** BDNF neurons. Expression of : **A, G, M,** RARα; **B, H, N,** RARβ; **C, I, O,** RARγ; **D, J, P.** RXRα; **E, K,,** RXRβ; **F, L, R,** RXRγ.

Figure 2. Effect of RA on neurite outgrowth from DRG neurons. DRG neurons were cultured either in the presence of NGF, NT-3 or BDNF for a period of two days at which point 1 x 10⁻⁷ M all-*trans*-RA was added. They were then examined for neurite outgrowth after a total of five days with NF200 antibody. **A,** NGF; **B,** NGF + 1 X 10⁻⁷ M RA; **C**, NT-3; **D**, NT-3 + 1 X 10⁻⁷; **E,** BDNF; **F,** BDNF + 1 X 10⁻⁷ M RA.

Figure 3. Expression of RARα isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARα isoforms were then assayed by RT-PCR. Controls had no RA added. A, control NGF neurons lanes 1-7; NGF neurons + 1 X 10⁻⁷ M RA lanes 8-14. B, control NT-3 neurons lanes 1-7; NT-3 neurons + 1 X 10⁻⁷ M RA lanes 8-14. C, control BDNF neurons lanes 1-7; BDNF neurons + 1 X 10-⁷ M RA lanes 8-14. Lanes: 1 & 8, RARα1; 2 & 9, RARα2; 3 & 10, RARα3; 4 & 11, RARα4; 5 & 12, RARα5; 6 & 13, RARα6; 7 & 14, RARα7.

Figure 4. Expression of RARβ isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARβ isoforms were then assayed by RT-PCR. Controls had no RA added. **A**, control NGF neurons lanes 1-4; NGF neurons + 1 X 10⁻⁷ M RA lanes 5-8. **B**, control NT-3 neurons lanes 1-4; NT-3 neurons + 1 X 10⁻⁷ M RA lanes 5-8. **C**, control BDNF neurons lanes 1-4; BDNF neurons + 1 X 10⁻⁷ M RA lanes 5-8. Lanes: 1 & 5, RARβ1; 2 & 6, RARβ2; 3 & 7, RARβ3; 4 & 8, RARβ4.

Figure 5. Expression of RARγ isoforms in DRG neurons cultured either in the absence or presence of RA. DRG neurons were cultured in the presence of either NGF, NT-3 or BDNF for a period of two days, 1 X 10⁻⁷ M RA was then added and the presence of the RARγ isoforms were then assayed by RT-PCR. Controls had no RA added. **A**, control NGF neurons lanes 1-7; NGF neurons + 1 X 10⁻⁷ M RA lanes 8-14. **B**, control NT-3 neurons lanes 1-7; NT-3 neurons + 1 X 10⁻⁷ M RA lanes 8-14. Lanes: 1 & 8, RARγ1; 2 & 9, RARγ2; 3 & 10, RARγ3; 4 & 11, RARγ4; 5 & 12, RARγ5; 6 & 13, RARγ6; 7 & 14, RARγ7.

Figure 6. Effect of RAR and RXR agonists on neurite outgrowth from DRG neurons. DRG neurons were cultured either in the presence of NGF , NT-3 or BDNF for a period of two days at which point either 1 x 10⁻⁷M of either CD366 (RARα agonist), CD2019 (RARβ agonist), CD437 (RARγ agonist) or CD2809 (pan-RXR agonist) were added to the cultures. Cultures were then stained for neurite outgrowth at five days with the NF200 antibody. **A-D**, NGF type neurons; **E-H**, NT-3 type neurons; **I-L**, BDNF type neurons. Agonists: RARα **A, E, I**; RARβ **B, F, J**; RARγ **C, G, K**; RXR **D**, **H, L.**

Figure 7. Effect of retinoid agonists on the length of neurites from **A**. NGF type neurons; **B**. NT-3 type neurons, C. BDNF type neurons. Columns 1. no agonist, 2. RA, 3. RARα, 4. RARβ, 5. RARγ, 6. RXR. Error bars s.e.m., n = 50. *p < 0.01.

Figure 8. Effect of a RARγ or RARβ agonist on the expression of RARγ1 and RARβ2 expression in DRG neurons cultured in the presence of NGF or NT-3. DRG neurons were cultured in the presence of serum free medium. After two days 1 x 10⁻⁷M RARγ or RARβ agonist were then added to the cultures for a period of 24 hrs. RT-PCR analysis of **A**, RARγ1; **B**, RARβ2 expression in NGF (lanes, 1-3) and NT-3 (lanes, 4-6) type neurons. Lanes: 1,4, no agonist; 2,5, RARγ agonist; 3, 6, RARβ agonist

### COMPARATIVE EXAMPLE 4: GENE TRANSFER TO NON-DIVIDING CELLS.

This example demonstrates gene transfer to dorsal root ganglion, ie. gene transfer to non-dividing neuronal cells, by the equine infectious anaemia virus vector, pONY8Z.

The EIAV vector, pONY8Z, is made by transient co-transfection of 293T human embryonic kidney cells with either pONY8Z plasmid, pONY3.1 and an envelope expression plasmid, pRV67 (which encodes the vesicular stomatitis virus protein G, VSV-G) using the calcium phosphate precipitation method.

The pONY8.0Z plasmid is a variant of pONY4.0Z (see sequence listing) but it does not express any EIAV sequence. The first two ATG sequences in *gag* are changed to ATTG and the accessory genes *Tat, S2* and *Rev* are either deleted or stop codons inserted in their open reading frames. The env start codon is also removed.

Twenty four hours before transfection the 293T cells are seeded at 3.6 x 10⁶ cells per 10cm dish in 10ml of DMEM supplemented with glutamine, non-essential aminoacids and 10% foetal calf serum. Transfections are carried out in the late afternoon and the cells are incubated overnight prior to replacement of the medium with 6ml of fresh media supplemented with sodium butyrate (5mM). After 7 hours the medium is collected and 6ml of fresh unsupplemented media added to the cells. The collected medium is cleared by low speed centrifugation and then filtered through 0.4micron filters.

Vector particles are then concentrated by low speed centrifugation (6,000g, JLA10.500 rotor) overnight at 4°C and then the supernatant is poured off, leaving the pellet in the bottom of the tube. The following morning the remaining tissue culture fluid is harvested, cleared and filtered. It is then placed on top of the pellet previously collected and overnight centrifugation repeated. After this the supernatant is decanted and excess fluid is drained. Then the pellet is resuspended in phosphate-buffered saline to 1/1000 of the volume of starting supernatant. Aliquots are then stored at -80°C.

Dorsal root ganglia are prepared for transduction with pONY8Z vector. Adult rats (eight months old) are sacrificed, and the dorsal root ganglia (DRG) removed. This is accomplished by first dissecting away the spinal cord, and then removing the DRG from the bony crevices on the inner surface of the vertebrae (ie. from crevices in the intra-vertebral space or canal which carries the spinal cord). The whole explanted ganglia are then placed in a cellogen matrix medium, said medium as described in Examples 1 and 2 above.

Transduction is carried out by injecting 3ul of the 1000x concentrated vector particles, produced as described above, into the DRG explant. This is accomplished using a fine chromatography needle.

pONY8Z carries a β-galactosidase gene, expression of which is driven by the CMV promoter. Transduction is assessed by X-gal staining, said staining as described in (Lim, F., Hartley, D., Starr, P., Song, S., Lang, P., Yu, L., Wang, Y.M. & Geller, A.I. Use of defective herpes-derived plasmid vectors. Meth.Mol. Biol. 62, 223-232 (1997)).

Thus, expression of nucleic acid sequences using EIAV vectors according to the present invention is demonstrated.

The vectors may also be produced according to the invention using different proteins capable of pseudotyping EIAV, such as Rabies G or variants of Rabies G (WO99/61639) (using pRabG plasmid or a variant such as pSA91 RbG plasmid) or VSV-G (using pRV67 plasmid) as described above. The method of production is as described above, except that a VSV-G expression plasmid is replaced with an expression plasmid for Rabies G protein.

DRGs are prepared and infected as described above.

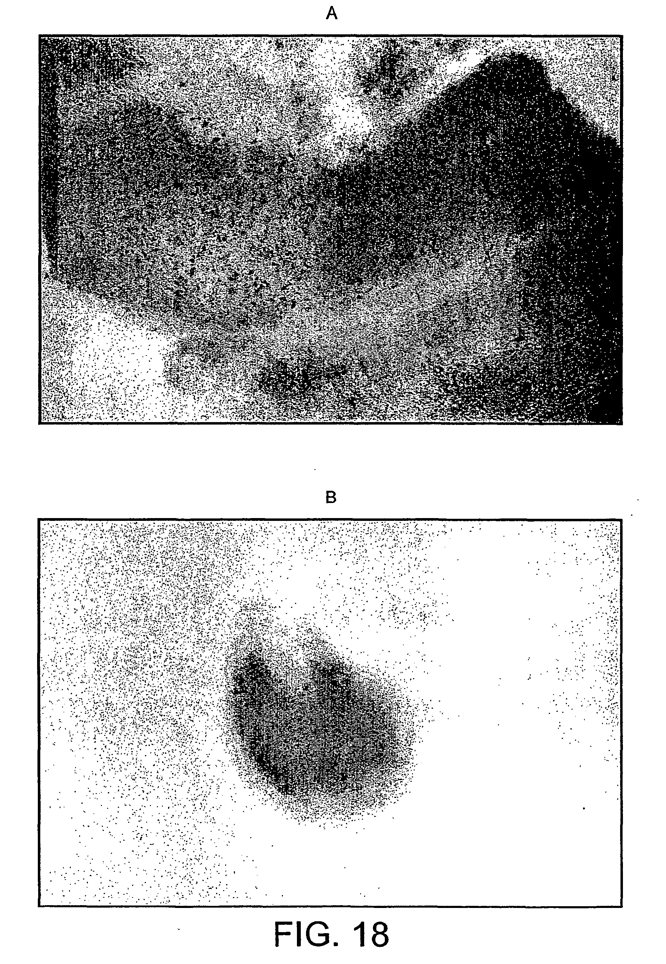

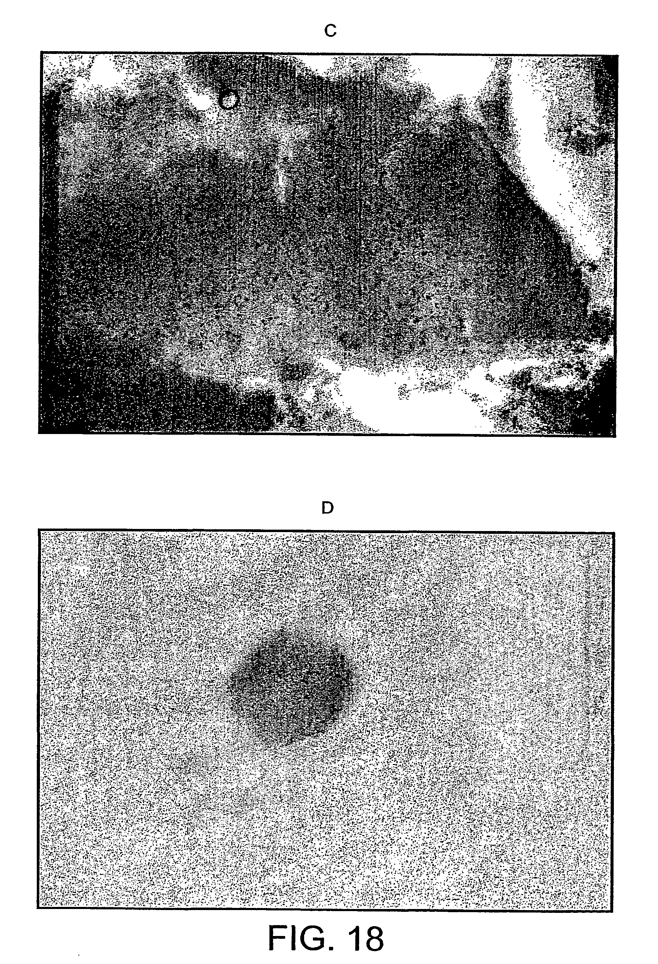

X-gal staining is performed as described above (Lim, F., Hartley, D., Starr, P., Song, S., Lang, P., Yu, L., Wang, Y.M. & Geller, A.I. Use of defective herpes-derived plasmid vectors. Meth.Mol. Biol. 62, 223-232 (1997)). Typical results are shown in Figure 18, which shows four photomicrographs.

### FIGURE FOR COMPARATIVE EXAMPLE 4:

Figure 18 shows four photomicrographs. These photomicrographs demonstrate expression of β-galactosidase in spinal cord (panels A and C) or DRG (panels B and D) explants transduced with pONY8z vector particles pseudotyped with VSV-G (panels A and B) or Rabies G (panels C and D) protein. Briefly, spinal cord (panels A and C) and dorsal root ganglia (DRG-panels B and D) were obtained from eight month old adult rats, placed in a cellogen matrix and treated as described above, being injected with 3ul of virus comprising the lacZ gene and cultured in DMEM/F12 medium with 5% Foetal Calf Serum (FCS). After 5 days, they were stained for lacZ expression.

Thus, it is demonstrated the vectors of the present invention are capable of producing expression of a nucleic acid of interest in non-dividing cells. Further, it is clearly demonstrated that expression of vector sequences according to the present invention may be produced in non-dividing adult neuronal cells.

### EXAMPLE 5: PRODUCTION OF EIAV VECTOR GENOME EXPRESSING RARβ2

A fragment of DNA encoding the retinoic acid receptor β2 is amplified by the polymerase chain reaction from a suitable template for RARβ2 such as cDNA produced from Trizol-prepared RNA as described in Example 2, or alternatively any nucleic acid molecule comprising RARβ2 such as described in Accession Number NM_000965. The oligonucleotide primers used are:
**RARβ2 FWD: 5'** CAG TAC ccg.cgg GCC ACC ATG TTT GAC TGT ATG GAT GTT CTG 3'
**RARβ2 REV: 5'** CAG TAC ctg cag.ATC ATT GCA CGA GTG GTG ACT GAC T 3'

The oligonucleotide primers contain *Sac*II and *Pst*l recognition sites respectively in order to facilitate cloning into the EIAV vector genome. In addition a Kozak sequence (GCCACC) is introduced upstream of the ATG initiation codon of the RARβ2 gene in RARβ2 FWD to improve the efficiency of translational initiation and the termination codon and context (in RARβ2 REV) is changed to UGAA which has been shown to be the most efficient termination signal in eukaryotes.

The resultant 1,378 bp PCR product encoding RARβ2 is digested with *Sac*II and *Pst*I and ligated into the EIAV vector genomes, pONY9Z 5'POS MIN or pONY9Z 3'POS MIN prepared for ligation by digestion with Sacll and *Sbf*I. These enzymes cut the DNA on either side of the LacZ reporter gene. Vector plasmids pONY9Z 5'POS MIN or pONY9Z 3'POS MIN are derivatives of pONY8Z constructed as described in the following paragraphs.

### Construction of pONY9Z 5'POS MIN and pONY9Z 3'POS MIN.

The presence of a sequence termed the central polypurine tract (cPPT-see Stetor et al. Biochemistry. 1999 Mar 23;38(12):3656-6) may improve the efficiency of gene delivery to non-dividing cells. This *cis*-acting element is located in the polymerase coding region element and can be obtained as a functional element by using PCR amplification using any plasmid which contains the EIAV polymerase coding region (for example pONY3.1) as follows. The PCR product includes the central polypurine tract and the central termination sequence (CTS). The oligonucleotide primers used in the PCR reaction are:
EIAV cPPT POS : CAGGTTAT*TCTAGA***GTCG**ACGCTCTCATTACTTGTAAC
EIAV cPPT NEG: CGAATGCGT*TCTAGA***GTCGAC**CATGTTCACCAGGGATTTTG

Recognition sequences for *Xba*l and *Sal*l are in italic and bold respectively and facilitate insertion into the pONY8Z backbone.

Before insertion of the cPPT/CTS PCR product prepared as described above, pONY8Z is modified to remove the CTS which already is present the pONY8Z vector. This is achieved by subcloning the Sa*l*I to *Sca*l fragment encompassing the CTS and RRE region from pONY8Z into pSP72, prepared for ligation by digestion with Sa*l*I and *Eco*RV. The CTS region is then removed by digestion with *Kpn*l and *Ppu*Ml, the overhanging ends 'blunted' by T4 DNA polymerase treatment and then the ends religated. The modified EIAV vector fragment is then excised using Sa*l*I and *Nh*el and ligated into pONY8Z prepared for ligation by digestion with the same enzymes. This new EIAV vector is termed pONY8Z del CTS.

pONY8Z del CTS has unique *Xba*l and *Sal*I sites which are located immediately upstream and downstream of the CMV-LacZ unit, respectively. The cPPT/CTS PCR product is digested with either of these enzymes and then ligated into pONY8Z del CTS prepared for ligation by digestion with either *Xbal* or Sall. Ligation into these sites results in plasmids with the cPPT/CTS element in either the positive or negative senses. Clones in which the cPPT/CTS is in the positive sense (functionally active) at either the 5' or 3'-position are termed pONY9Z 5'POS and pONY9Z 3'POS, respectively.

The safety profile of the EIAV vector can be improved by arranging for the integrated vector to have functionally inactive LTR's. Such vectors are termed SIN (Self Inactivating Vectors). In this way the only transcription events associated with the presence of the vector following transduction are those from the internal promoter. In the pONY8 and pONY9 series of vectors the internal promoter is CMV however other promoters, such as tissue specific promoters, can be used. The SIN configuration is created by making a deletion in the U3 region of the 3'LTR using PCR-based techniques as follows. The template for amplification is pONY8Z, and the primers used for amplification are:
MIN FOR: CACCTAGCAGGCGTGACCGGTGG
MIN REV: CCTACCAATTGTATAAAACCCCTCATAAAAACCCCAC

The forward primer binds just 5' of a unique *Nsp*V site in pONY8Z and the reverse primer binds to the 5' end of the U3 region and has *Mun*l site (in bold) at the 5'end. Thus the PCR product includes sequences corresponding to the second exon of EIAV REV and extends through the 3'polypurine tract to include the 5' 26 nucleotides of U3. The PCR product is digested with *Nsp*V and *Mun*I and ligated into either pONY9Z 5'POS or pONY9Z 3'POS prepared for ligation using the same enzymes. The sequence of the resulting plasmid is confirmed by sequence analysis and the plasmids termed pONY9Z 5'POS MIN or pONY9Z 3'POS MIN.

### Production of pONY9-RARB2 vector preparations

Vector preparations are made by transient co-transfection of 293T human embryonic kidney cells with either pONY9 5'-RARβ2 or pONY9 3'-RARβ2 plasmid, pONY3.1 and an envelope expression plasmid such as pRV67 (which encodes VSV-G), pRabG or derivatives of pRabG (Rabies virus G protein) or expression plasmids encoding other proteins capable of pseudotyping EIAV.

Alternatively the pE SYN GP cassette, which encodes the EIAV gag/pol protein but which is optimised for expression in human cells by altering the codon usage, can be used instead of pONY3.1. This cassette can be expressed in any conventional eukaryotic gene expression vector. Alternatively, pESDSYNGP which has a splice donor in the leader can be used. When transfections are carried out in this way, higher yields are obtained if a fourth plasmid encoding EIAV REV is also included in the transfection.

Transfections, harvesting and concentration of the vector particles are carried out as described above for pONY8Z.

### Assessement of pONY9-RARβ2 vector preparations

pONY9 RARβ2 assessment of titre is made by measuring properties of the vector preparation: the reverse transcriptase (RT) activity and the incorporation of vector RNA into particles can be used together or independently in order to estimate vector titre. These are then related to those of other vector preparations, for example pONY8Z, of known biological titre, giving an estimate of the titre of the vector preparation being tested.

The amount of RT activity is assessed by performance enhanced reverse transcriptase assay (PERT). This assay has been previously described by Lovatt et al., (1999), J. Virological Methods, 82, 185-200 using brome mosaic virus RNA as template for the RT. In this Example, MS2 bacteriophage RNA is used instead of the brome mosaic virus RNA described therein. Briefly this assay works as follows: RT activity is released from the vector particles present in the preparation by mild detergent treatment and used to synthesise cDNA to RNA from MS2 bacteriophage. The MS2 RNA and primer are present in excess therefore the amount of cDNA synthesised is proportional to the amount of RT activity released. The MS2 cDNA is then quantitated by PCR methods using an ABI PRISM 7700 Sequence Detector. The value obtained enables comparison with the standard pONY8Z vector preparation, and hence titre to be assessed. The details of the assay are as follows:

The PERT assay uses real time quantitative RT-PCR technology to detect a specific PCR product from MS2 RNA and the retroviral reverse transcriptase present in the viral particles (in this case EIAV RT). Briefly, the viral particles are disrupted by mixing 1:1 volumes of viral vector stocks and disruption buffer (40mM Tris-HCI pH7.5, 50mM KCI, 20mM DTT and 0.2% NP-40). Serial dilutions of the disrupted particles are carried out prior to adding them to the RT-PCR TaqMan reaction mix (Perkin-Elmer). The reaction mix contains 1/10th volume of disrupted viral particles, 300nM PERT forward primer, 300nM PERT reverse primer, 150nM PERT probe, 80mg/ml MS2 RNA. The RT-PCR conditions are as follows: 48oC for 30min; 95oC for 10min; then 40 cyles of, 95oC for 15sec and 60oC for 1min. The data is analysed using the TaqMan software (Perkin-Elmer).

PERT pimers are derived using the primer/probe prediction programs on the TaqMan using MS2RNA Acc. No. J02467 as input;
NEGATIVE SENSE PRIMER, FOR REVERSE TRANSCRIPTASE STEP = 5'-CACAGGTCAAACCTCCTAGGAATG
PLUS SENSE PRIMER = 5' TCCTGCTCAACTTCCTGTCGA
PROBE = 5' FAM-CGAGACGCTACCATGGCTA-(TAMRA)p3'

The use of MS2 RNA as template in the F-PERT assay is as described in Arnold et al (BioTechniques, (1998), vol25, 98-106).

### Assessment of titre via measurement of packaged vector RNA

Detection of the packaging signal is accomplished by monitoring the incorporation of vector RNA into particles which is quantified using the packaging signal assay as follows. The RNA content of the viral preparations is estimated by RT-PCR comparing to a pONY8G vector preparation of known biological titre (see above). Vector RNA is isolated from the vector stocks using a Qiagen RNA isolation kit (Qiagen) and then DNAse treated using RNAse free DNAse (Ambion). Serial dilutions of the RNA are used as template in the RT-PCR reaction. Two TaqMan (Perkin-Elmer) reaction mixes are prepared, +RT and -RT, containing 1/10th volume of RNA template and the specific forward and reverse primers and probe. The RT-PCR conditions are as follows: Hold, 48oC for 30min; hold, 95oC for 10min; forty cycles, 95oC for 15sec and 60oC for 1 min. The data was analysed using the TaqMan software (Perkin-Elmer).
NEGATIVE SENSE PRIMER, FOR REVERSE TRANSCRIPTASE STEP = 5'-accagtagttaatttctgagacccttgta
PLUS SENSE PRIMER = 5' ATTGGGAGACCCTTTGACATT
PROBE = 5' FAM-CACCTTCTCTAACTTCTTGAGCGCCTTGCT-(TAMRA)p3'
(This set of primers detects vector genome, but not wild type gag/pol.)

The biological titre of the vector is related to the amount of vector RNA packaged in virions and can be assessed using quantitative RT-PCR using the ABI PRISM 7700 Sequence Detector. The primers and probe for the reaction bind to the packaging signal region of the vector.

Thus, it is demonstrated that vector particles for the delivery of RARβ2 may be produced according to the invention.

### COMPARATIVE EXAMPLE 6: TRANSFER OF GENETIC MATERIAL TO NON-DIVIDING CELLS.

This example demonstrates gene transfer to dorsal root ganglion (DRG), i.e., gene transfer to non-dividing neuronal cells, by the equine infectious anaemia virus vector, pONY8.0Z.

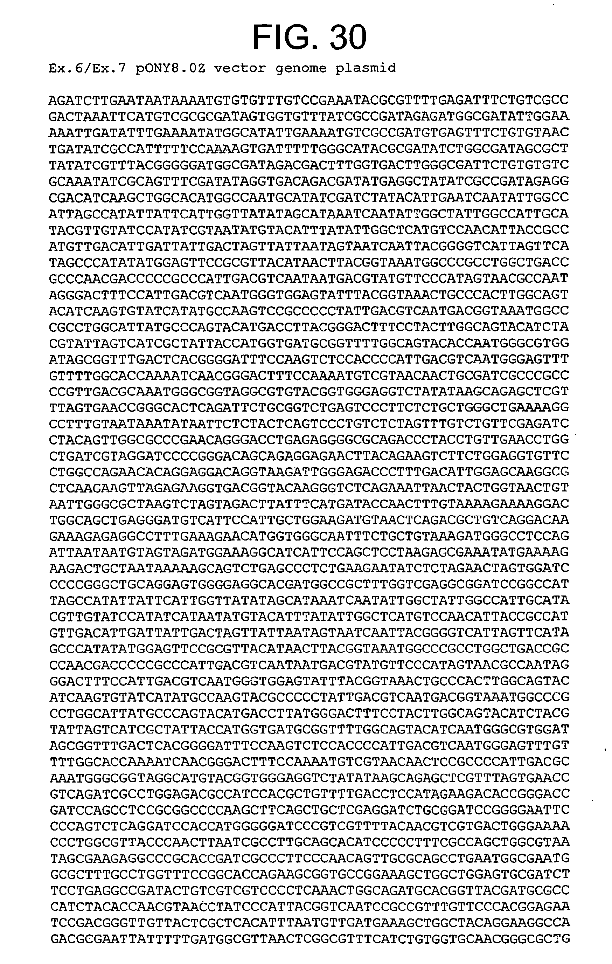

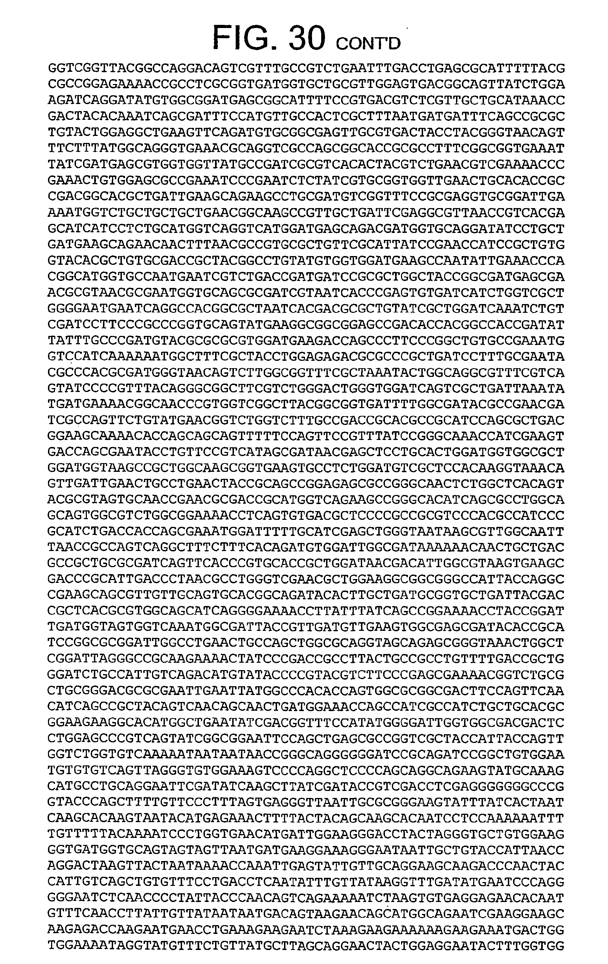

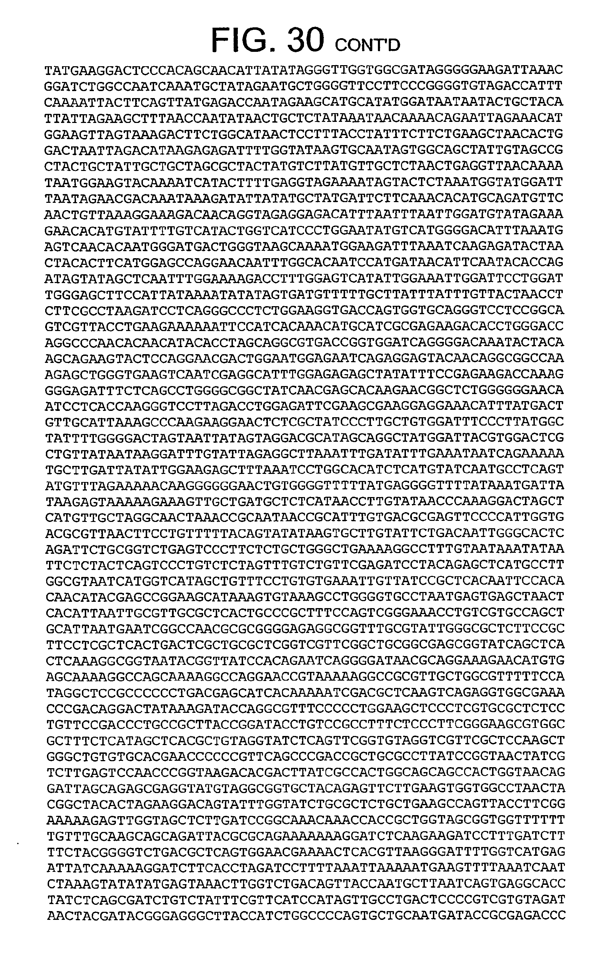

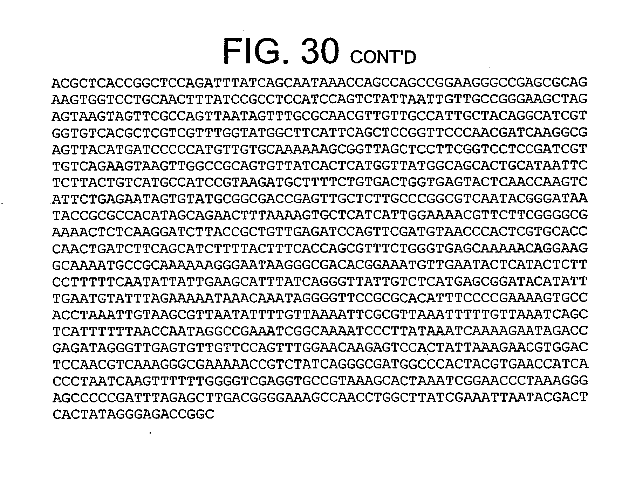

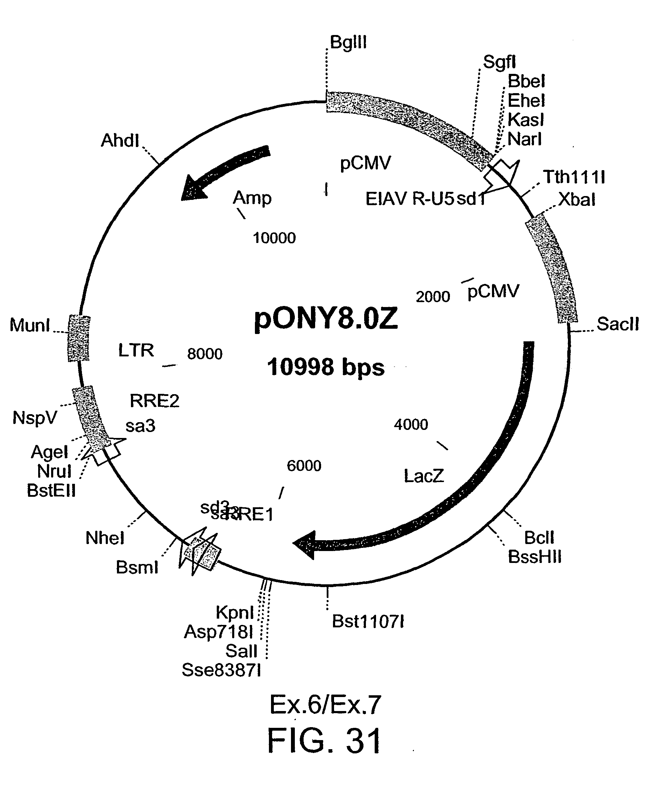

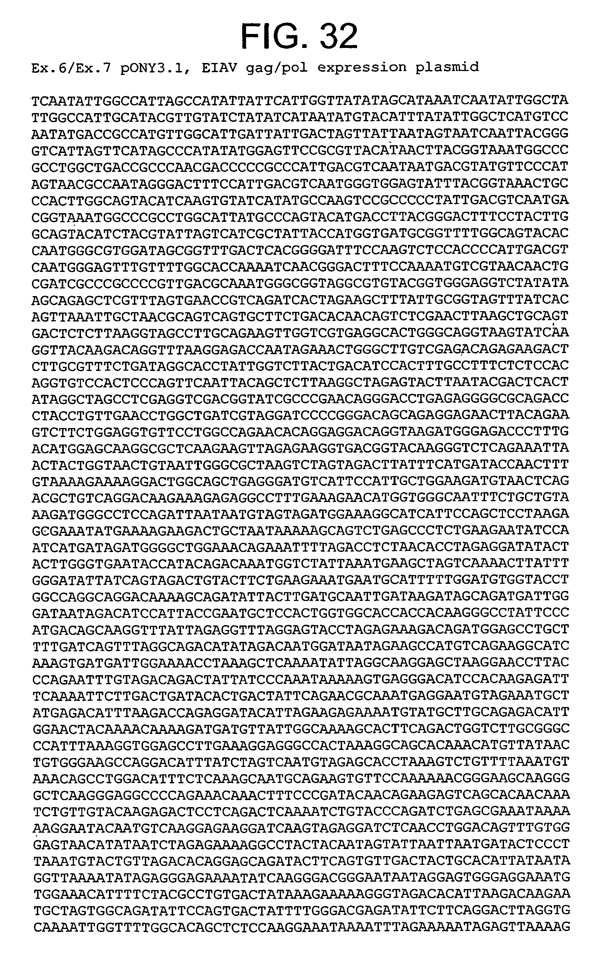

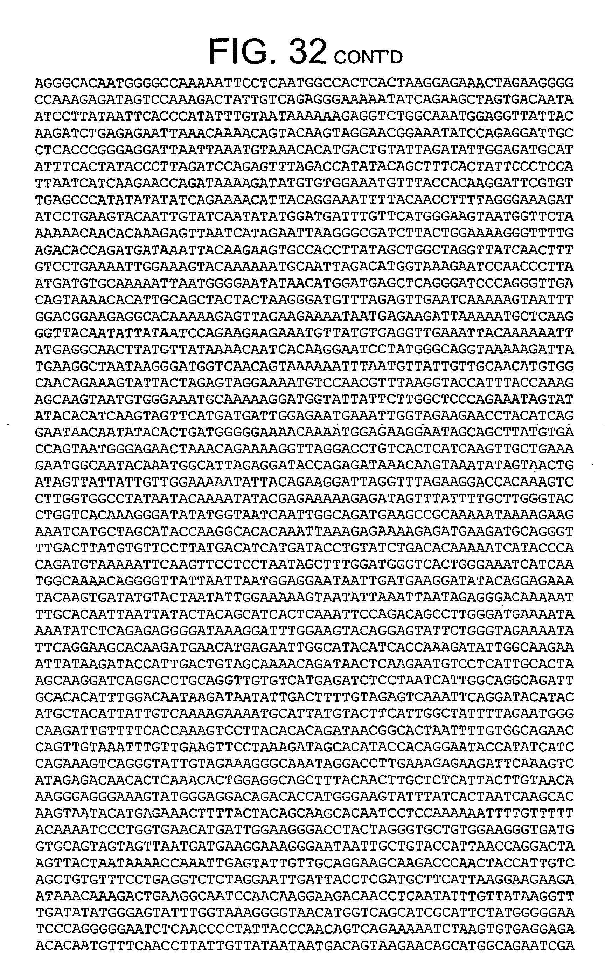

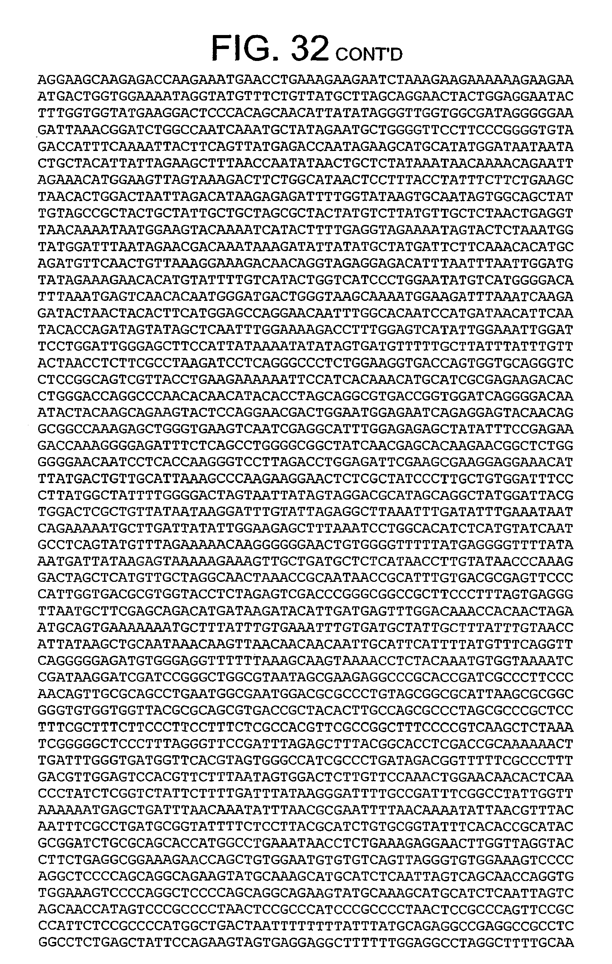

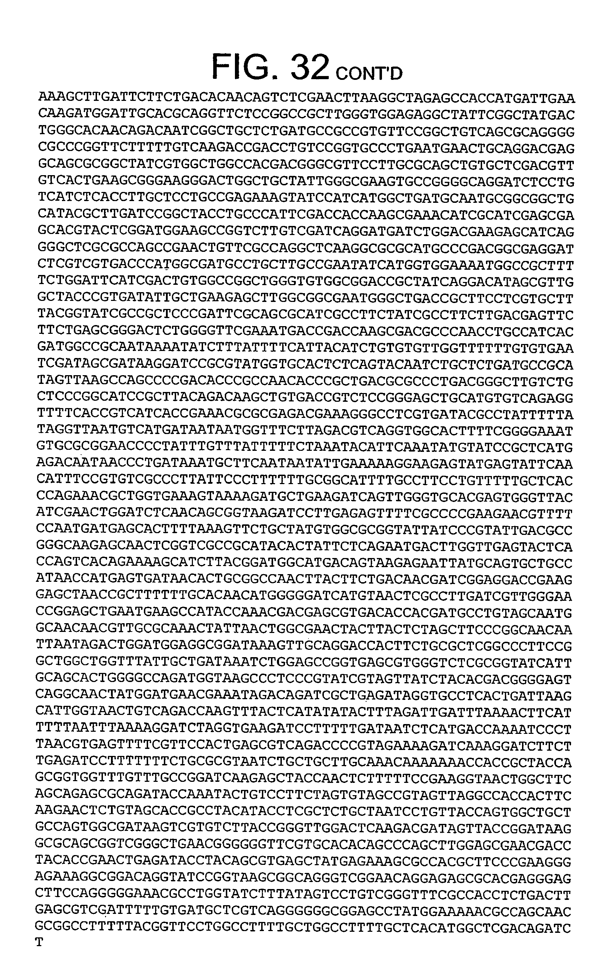

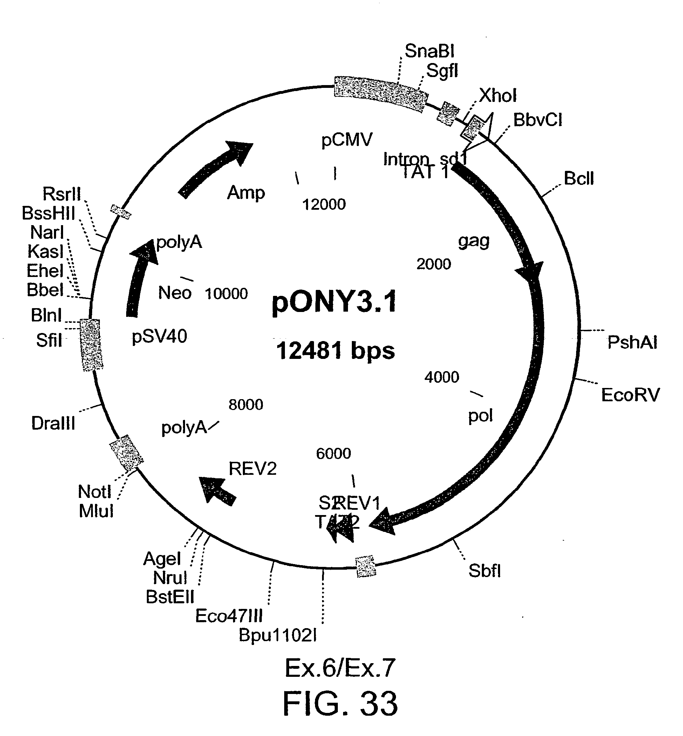

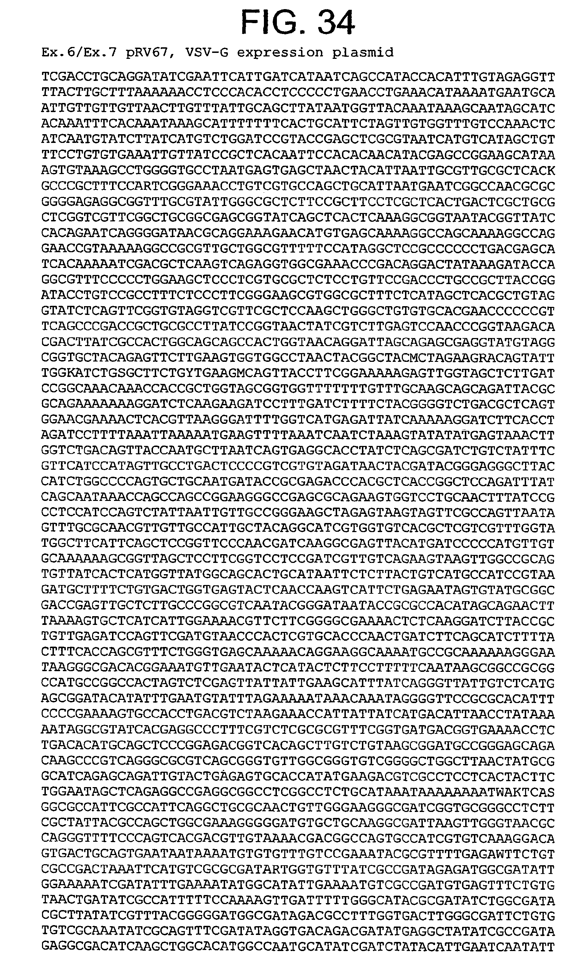

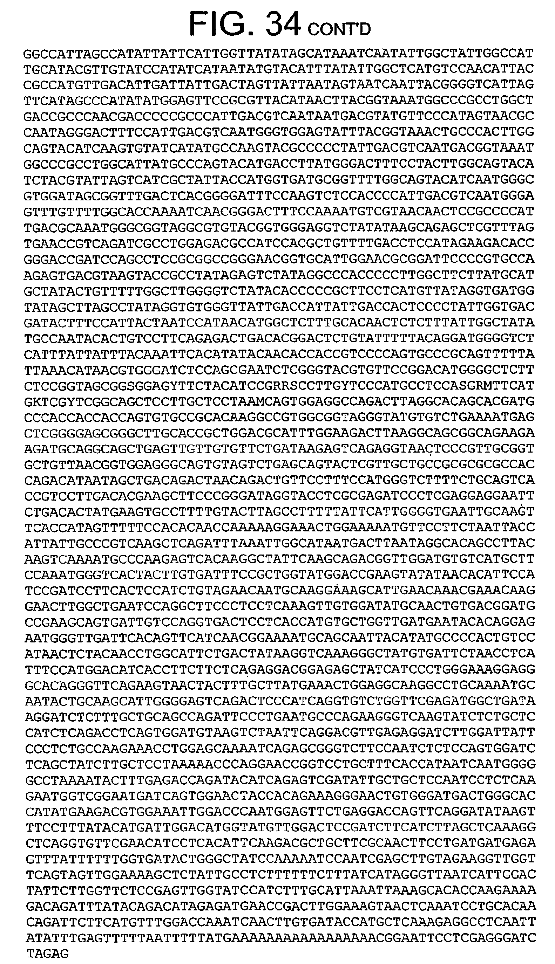

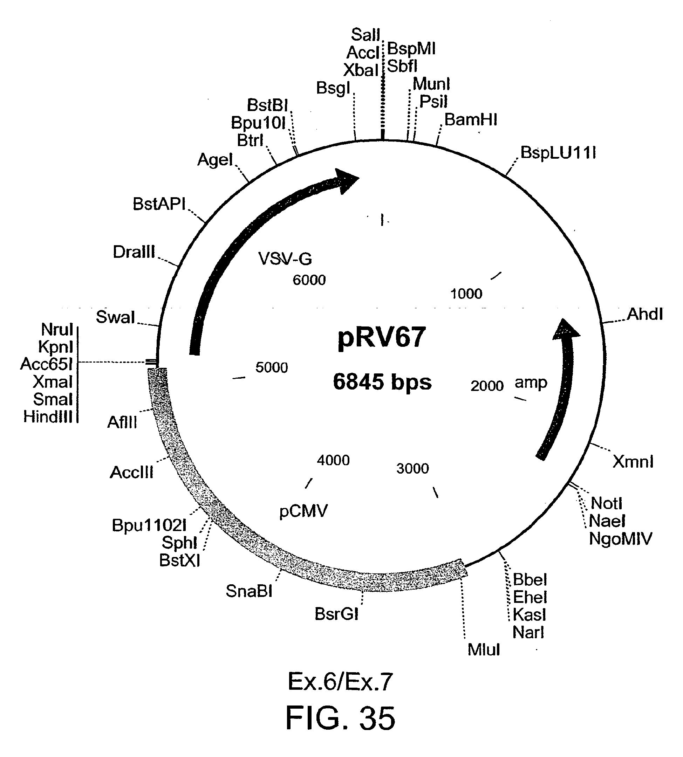

The EIAV vector, pONY8.0Z is made by transient co-transfection of HEK 293T human embryonic kidney cells with pONY8.0Z vector genome plasmid (Figure 30 and 31), pONY3.1 (Figure 32 and 33) (WO99/32646) and an envelope expression plasmid, pRV67 (Figure 34 and 35) (WO99/61639)(which encodes the vesicular stomatitis virus protein G, VSV-G) using the calcium phosphate precipitation method, as described below.

Vectors may also be produced according to the invention using different proteins capable of pseudotyping EIAV, such as Rabies G or variants of Rabies G (WO99/61639) (using pRabG plasmid or a variant such as pSA91RbG plasmid) or VSV-G (using pRV67 plasmid) as described above. The method of production is as described above, except that a VSV-G expression plasmid is replaced with an expression plasmid for Rabies G protein.

pONY8.0Z is derived from pONY4.0Z (WO/9932646) by introducing mutation(s) as follows:
Mutation(s) are introduced which prevent expression of tat. In the present Example, this is accomplished by an 83nt deletion in the exon 2 of tat.
Mutation(s) are introduced which prevent S2 ORF expression. In the present Example, this is accomplished by a 51 nt deletion.
Mutation(s) are introduced which prevent REV expression. In the present Example, this is accomplished by deletion of a single base within exon 1 of rev.
Mutation(s) are introduced which prevent expression of the N-terminal portion of gag. In the present Example, this is accomplished by insertion of T in ATG start codons, thereby changing the sequence to ATTG from ATG. With respect to the wild type EIAV sequence Acc. No. U01866 these correspond to deletion of nt 5234-5316 inclusive, nt 5346-5396 inclusive and nt 5538. The insertion of T residues is after nt 526 and 543.

The method of vector production by calcium phosphate-mediated transfection is as follows. Twenty four hours before transfection the HEK 293T cells are seeded at 3.6 x 10⁶ cells per 10cm dish in 10ml of DMEM supplemented with glutamine, non-essential aminoacids and 10% foetal calf serum. Transfections are carried out in the late afternoon and the cells are incubated overnight prior to replacement of the medium with 6ml of fresh media supplemented with sodium butyrate (5mM). After 7 hours the medium is collected and 6ml of fresh unsupplemented media added to the cells. The collected medium is cleared by low speed centrifugation and then filtered through 0.45 micron pore-size filters.

Vector particles are then concentrated by low speed centrifugation (6,000g, JLA10.500 rotor) overnight at 4°C and then the supernatant is poured off, leaving the pellet in the bottom of the tube. The following morning the remaining tissue culture fluid is harvested, cleared and filtered. It is then placed on top of the pellet previously collected and overnight centrifugation repeated. After this the supernatant is decanted and excess fluid is drained. Then the pellet is resuspended in phosphate-buffered saline to 1/1000 of the volume of starting supernatant. Aliquots are then stored at -80°C.

Dorsal root ganglia are prepared for transduction with pONY8.0Z vector. Adult rats (eight months old) are sacrificed, and the DRG removed. This is accomplished by first dissecting away the spinal cord, and then removing the DRG from the bony crevices on the inner surface of the vertebrae (i.e., from crevices in the intra-vertebral space or canal which carries the spinal cord). The whole explanted ganglia are then placed in a cellogen matrix medium, said medium as described in Examples 1 and 2 above. Sections of the spinal cord were also cultured in cellogen matrix medium in DMEM/F12 medium with 5% Foetal Calf Serum (FCS).

The ability of pONY8.0Z to transduce either the spinal cord or DRG explants was assessed by injecting 3µl of the 1000x concentrated pONY8.0Z vector particles, produced as described above, into the explants. This was accomplished using a fine chromatography needle. After 5 days, they were stained for β-galactosidase expression. pONY8.0Z carries a β-galactosidase gene, expression of which is driven by the CMV promoter. Therefore transduction is easily assessed by X-gal staining, said staining as described in (Lim, F., Hartley, D., Starr, P., Song, S., Lang, P., Yu, L., Wang, Y.M. & Geller, A.I. Use of defective herpes-derived plasmid vectors. Meth.Mol. Biol. 62, 223-232 (1997)).

Thus, expression of nucleic acid sequences using EIAV vectors according to the present invention is demonstrated. Furthermore, it is demonstrated the vectors of the present invention are capable of producing expression of a nucleic acid of interest in non-dividing cells. Further, it is clearly demonstrated that expression of vector sequences according to the present invention may be produced in non-dividing adult neuronal cells.

### EXAMPLE 7: CONSTRUCTION AND MANUFACTURE OF EIAV VECTOR GENOME EXPRESSING RETINOIC ACID RECPTOR β2 (RARβ2)

A fragment of DNA encoding the retinoic acid receptor β2 is amplified by the polymerase chain reaction from a suitable template for RARβ2 such as cDNA produced from Trizol-prepared RNA as described in Example 2, or alternatively any nucleic acid molecule comprising RARβ2, such as described in Genbank Acc. No. S56660. Two versions were made: one in which a wild type RAR-β2 sequence was constructed and one in which it was preceded by the 'FLAG' epitope tag (Immunex Corporation). The FLAG sequence allows easy identification RARβ2 expression. The oligonucleotide primers used were:
**EX7 RARβ2 FWD:**
   5'ACTGccg.cgg GCC ACC ATG TTT GAC TGT ATG GAT GTT CTG TC**3'**
**EX7 RARβ2 FLAG FWD:**
   **5'** ACTGccg.cgg GCC ACC ATG GACTACAAGGACGACGATGACAAG TTT GAC TGT ATG GAT GTT CTG TC**3'**
**EX7 RARβ2 REV:**
   **5'**ACTGGCGGCCGCTCACTGCAGCAGTGGTG**3'**

The oligonucleotide forward (FWD) and reverse (REV) primers contain *Sac*II and *Not*l recognition sites, respectively, in order to facilitate cloning into the EIAV vector genome. In addition a Kozak sequence (GCCACC) was introduced upstream of the ATG initiation codon of the RARβ2 or FLAG RARβ2 gene in the forward (FWD) primers to improve the efficiency of translational initiation.

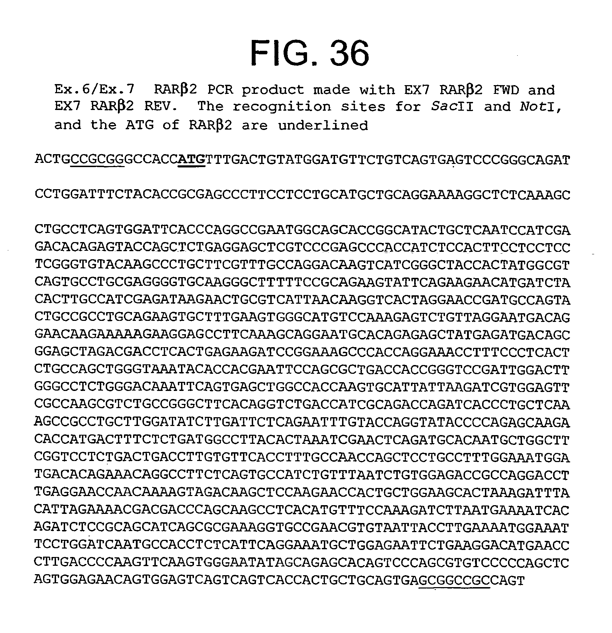

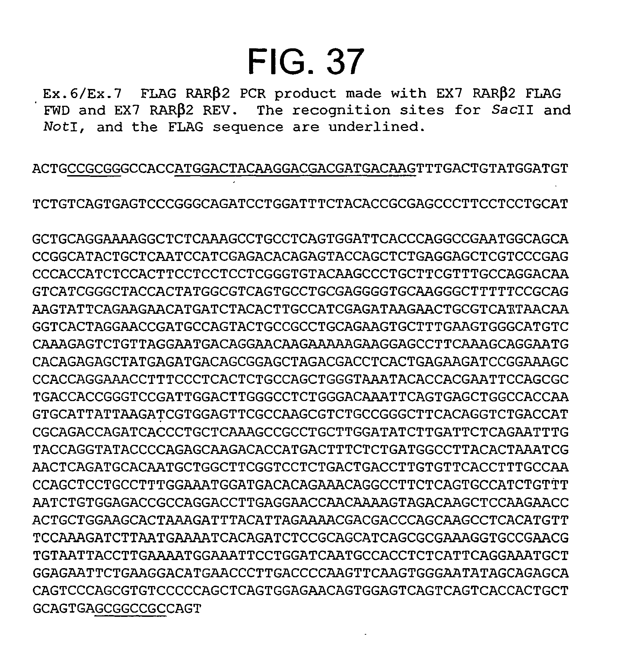

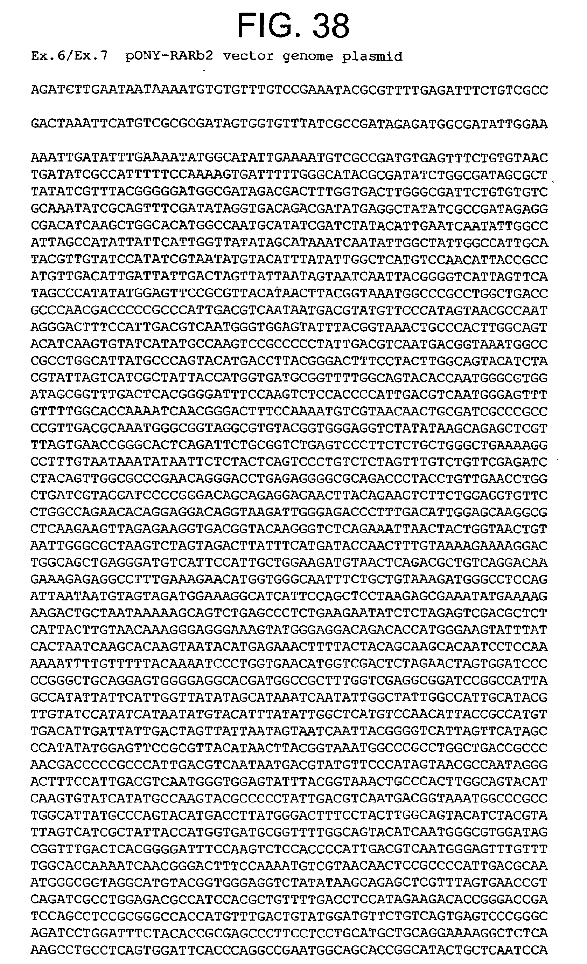

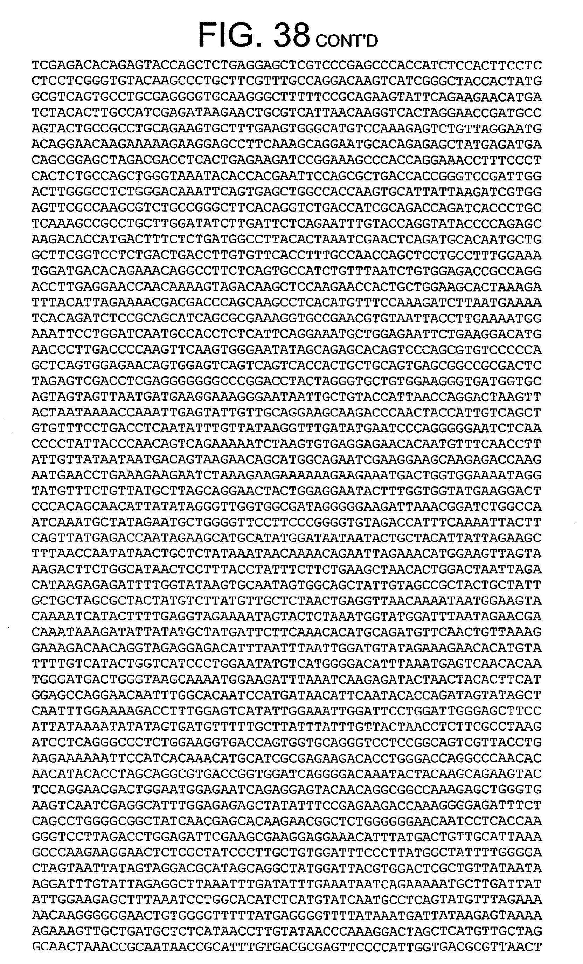

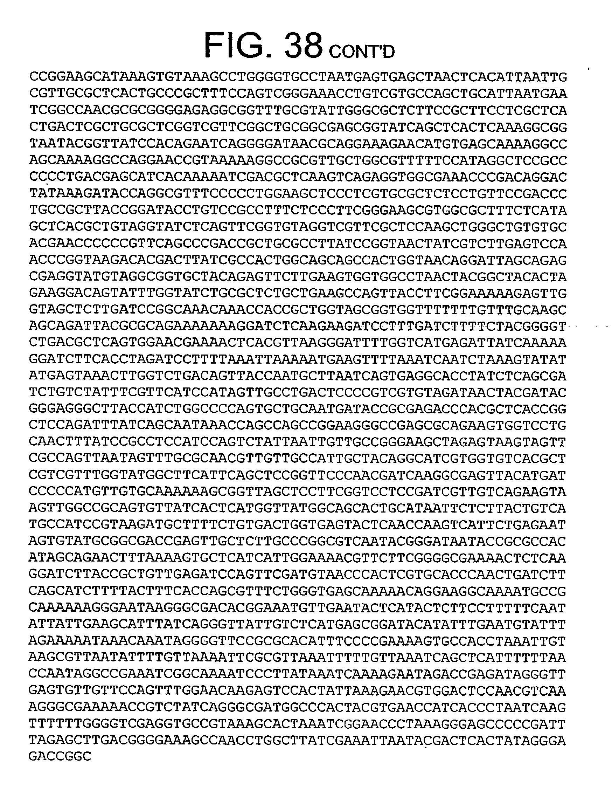

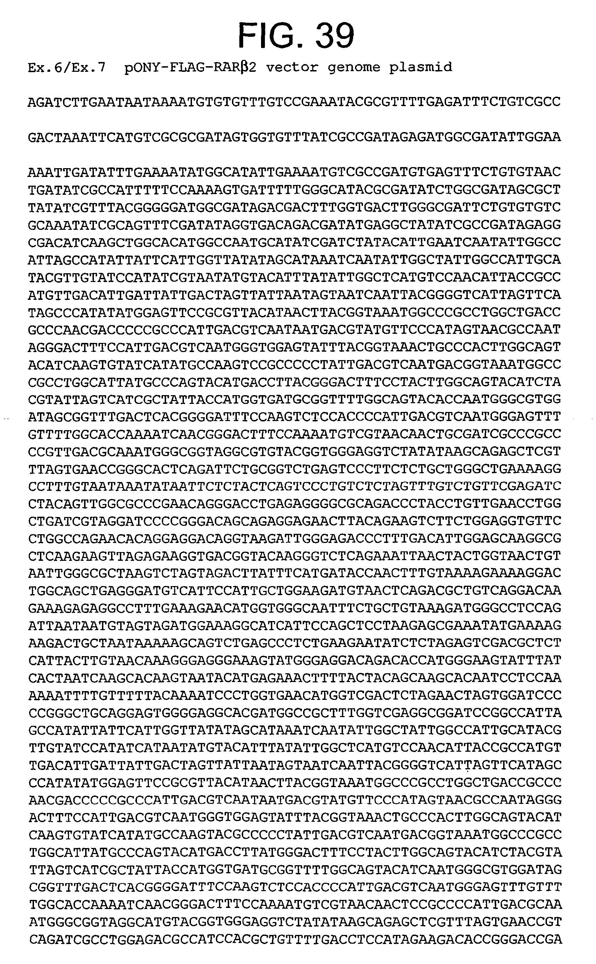

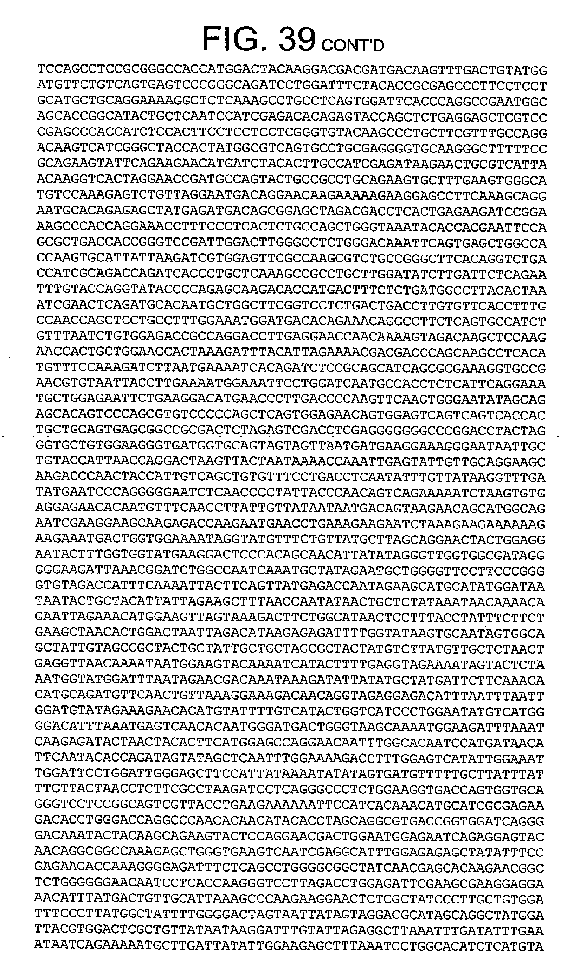

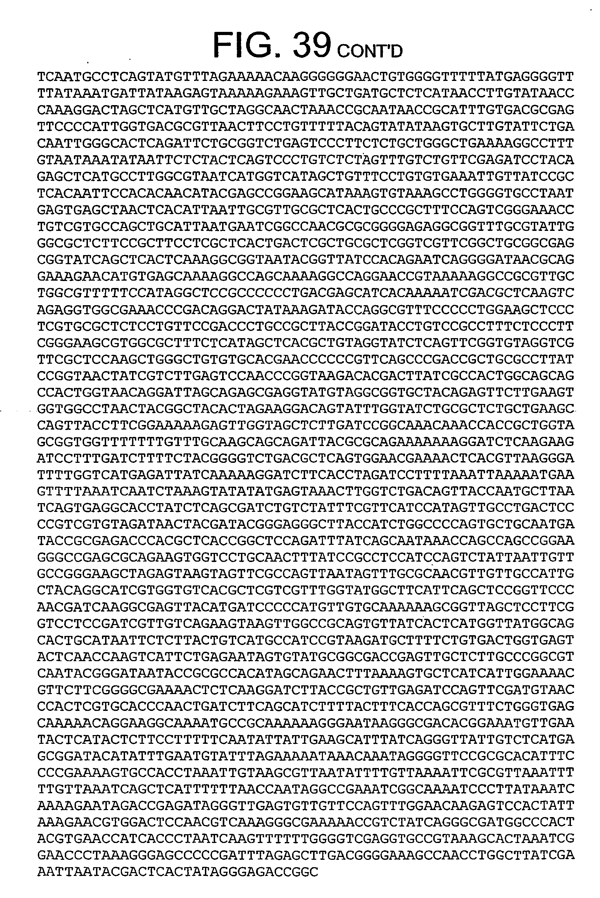

The resultant PCR products encoding RARβ2 or FLAG RAR-β2 (Figure 36 and 37) are digested with *Sac*ll and *Not*I and ligated into the EIAV vector genome, pONY8G 5'cPPT POS delCTS prepared for ligation by digestion with *Sac*ll and *Not*I. These enzymes cut the DNA on either side of the enhanced green fluorescent protein (eGFP) reporter gene. The resulting EIAV vector carrying the RARβ2 of FLAG RAR-β2 insert are termed pONY-RARβ2 and pONY-FLAG-RARβ2, respectively (Figure 38 and 39). Vector genome plasmid pONY8G 5'cPPT POS delCTS is a derivative of pONY8.0Z constructed as described in the following paragraphs.

### Construction of pONY8G 5'cPPT POS del CTS

The presence of a sequence termed the central polypurine tract and central termination sequence (cPPT-see Stetor et al. Biochemistry. 1999 Mar 23;38(12):3656-6) improves the efficiency of gene delivery to non-dividing cells (WO 99/55892). This cis-acting element is located in the polymerase coding region element and can be obtained as a functional element by using PCR amplification using any plasmid which contains the EIAV polymerase coding region (for example pONY3.1) as follows. The PCR product includes the central polypurine tract and the central termination sequence (CTS). The oligonucleotide primers used in the PCR reaction are:
EX7 EIAV cPPT POS:
   CAGGTTATTCTAGAGTCGACGCTCTCATTACTTGTAAC
EX7 EIAV cPPT NEG:
   CGAATGCGTTCTAGAGTCGACCATGTTCACCAGGGATTTTG

Recognition sequences for *Xba*l are shown in italic and use of this enzyme facilitates insertion of the PCR product into the pONY8G backbone.

Before insertion of the cPPT/CTS PCR product prepared as described above, the vector backbone is modified to remove the CTS which is already present due the presence of some EIAV *pol* sequences downstream of the reporter gene. This is achieved by subcloning the Sa/l to *Sca*l fragment encompassing the CTS and RRE region from pONY8.0Z into pSP72 (Genbank Acc.No.X65332), prepared for ligation by digestion with *Sa*/l and *Eco*RV. The CTS region is then removed by digestion with *Kpn*l and *Ppu*MI, the overhanging ends 'blunted' by T4 DNA polymerase treatment and then the ends religated. The modified EIAV vector fragment is then excised using Sa*l*I and *Nhe*l and ligated into pONY8G prepared for ligation by digestion with the same enzymes. This new EIAV vector is termed pONY8G delCTS. pONY8G is derived from pONY8.0Z by exchange of the LacZ reporter gene for the enhanced green fluorescent protein (GFP) gene. This is done by transferring the *Sac*II-*Kpn*I fragment corresponding to the GFP gene and flanking sequences from pONY2.13GFP (WO 99/32646) into pONY8.0Z cut with the same enzymes.

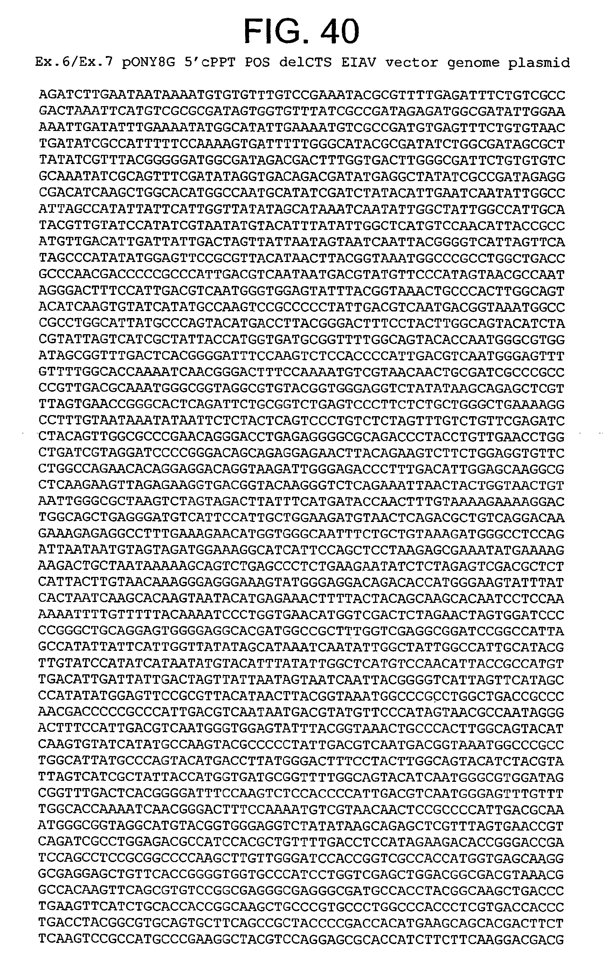

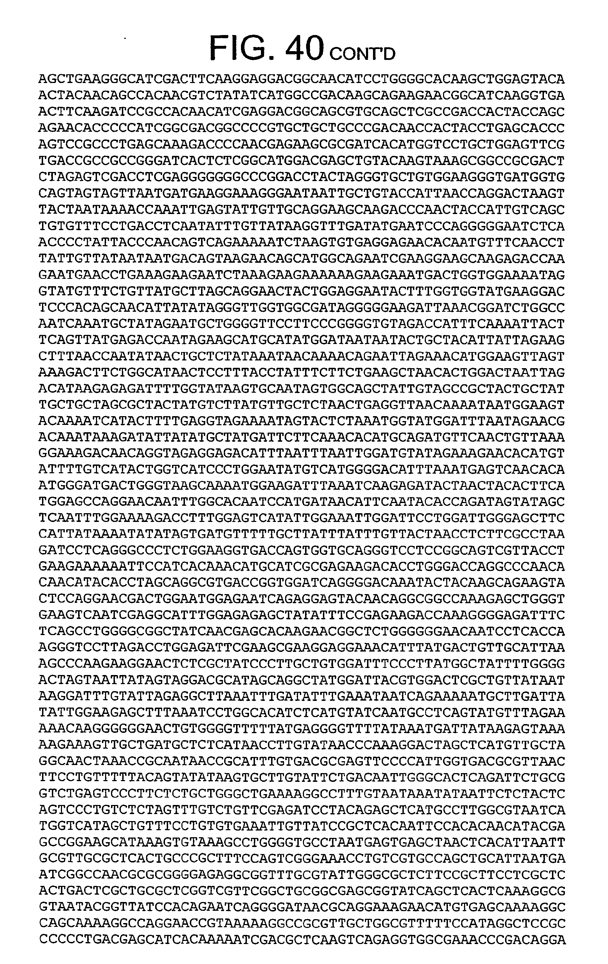

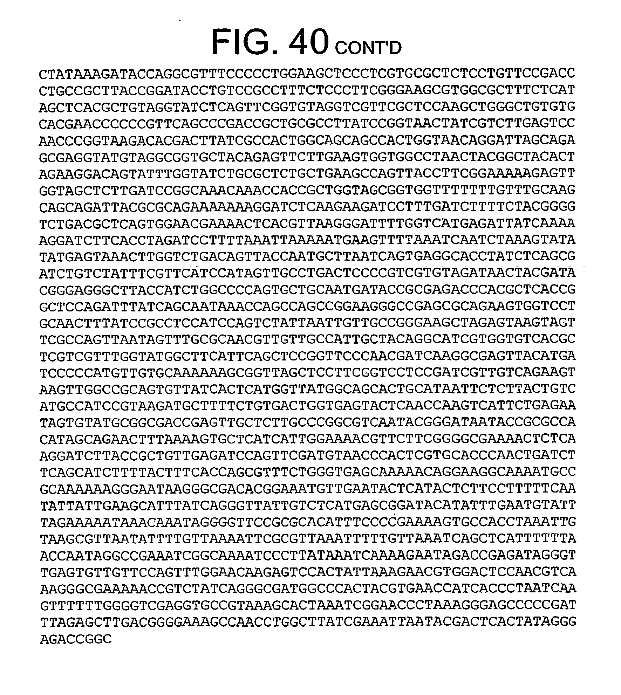

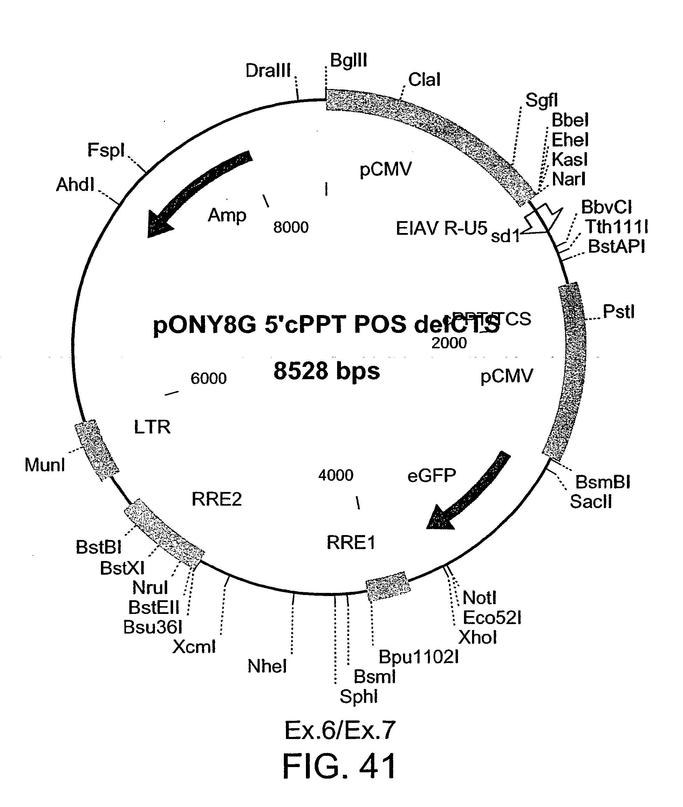

pONY8G delCTS has two *Xbal* sites which are located immediately upstream and downstream of the CMV-LacZ unit, respectively. The cPPT/CTS PCR product is digested with *Xbal* and then ligated into pONY8G delCTS prepared for ligation by partial digestion with either *Xba*l. Ligation into these sites results in plasmids with the cPPT/CTS element in either the positive or negative senses. A clone in which the cPPT/CTS is in the positive sense (functionally active) and located to the 5'-side of the internal CMVpromoter was selected and termed pONY8G 5'cPPT POS delCTS (Figure 40 and 41).

### Production of pONY-RARβ2 vector preparations

Vector preparations are made by transient co-transfection of HEK 293T, human embryonic kidney cells as described above (see Example 6) for pONY8.0Z, except that the vector genome plasmid was the pONY-RARβ2 vector genome plasmid. Vectors are made using either envelope expression plasmid pRV67 (which encodes VSV-G), pRabG or derivatives of pRabG (which encode Rabies virus G protein). However, expression plasmids encoding other proteins capable of pseudotyping EIAV may equally be used.

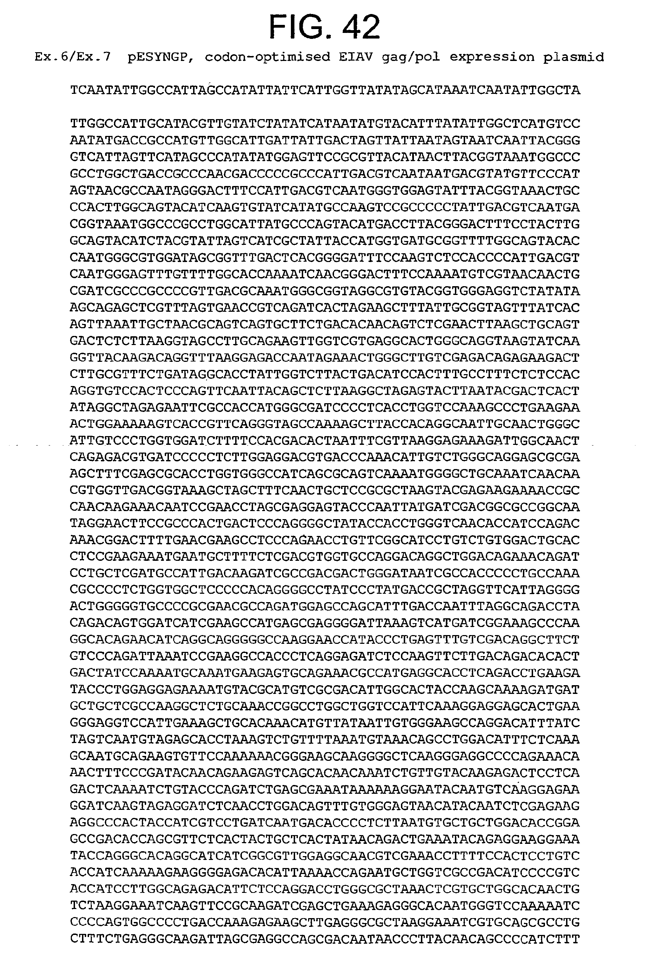

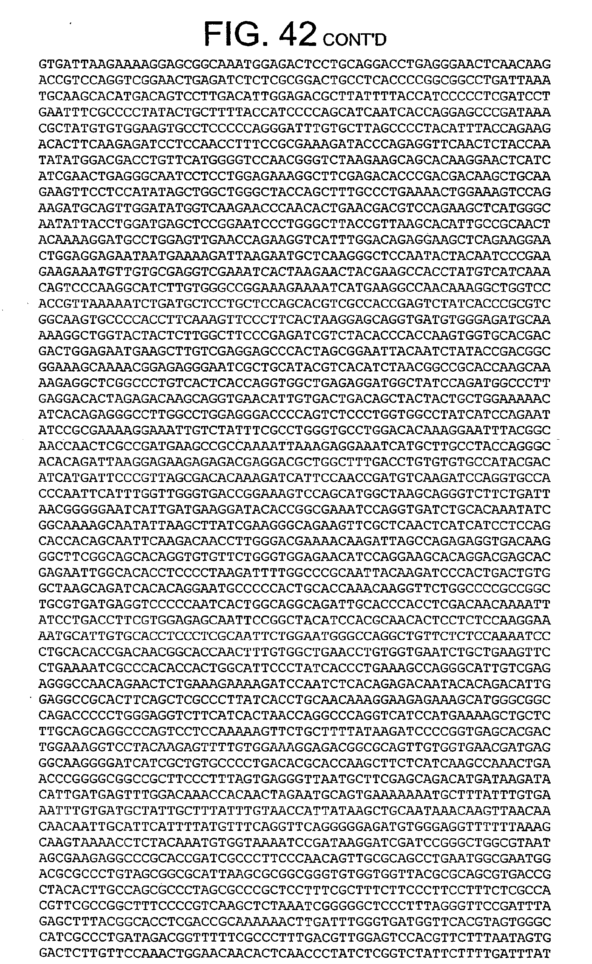

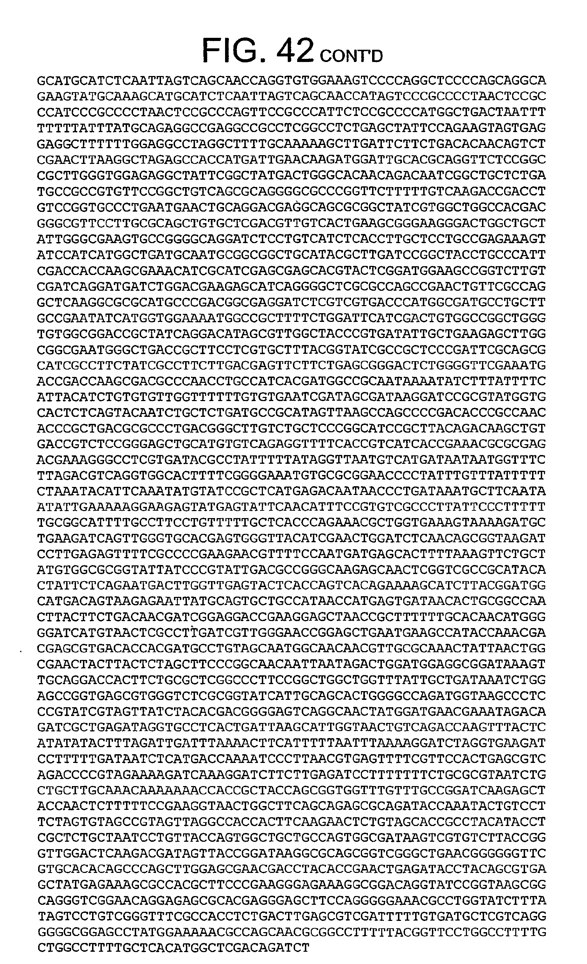

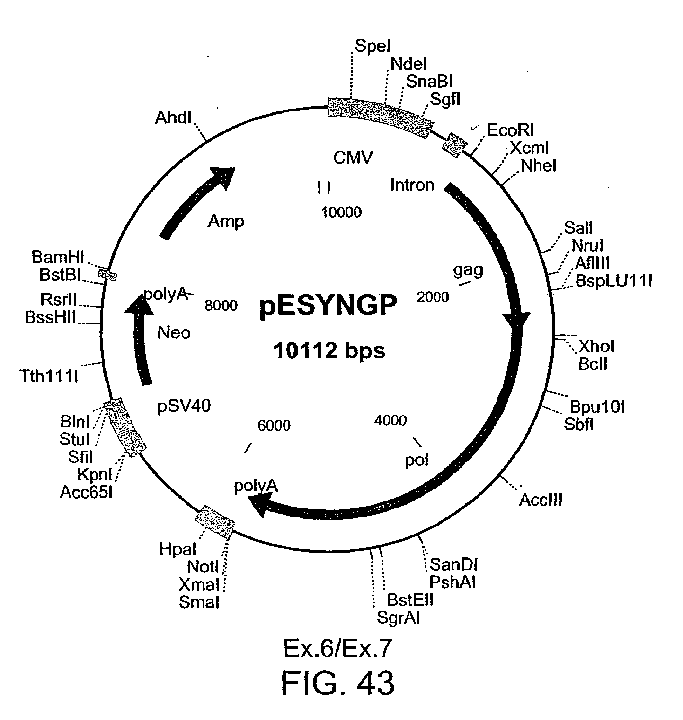

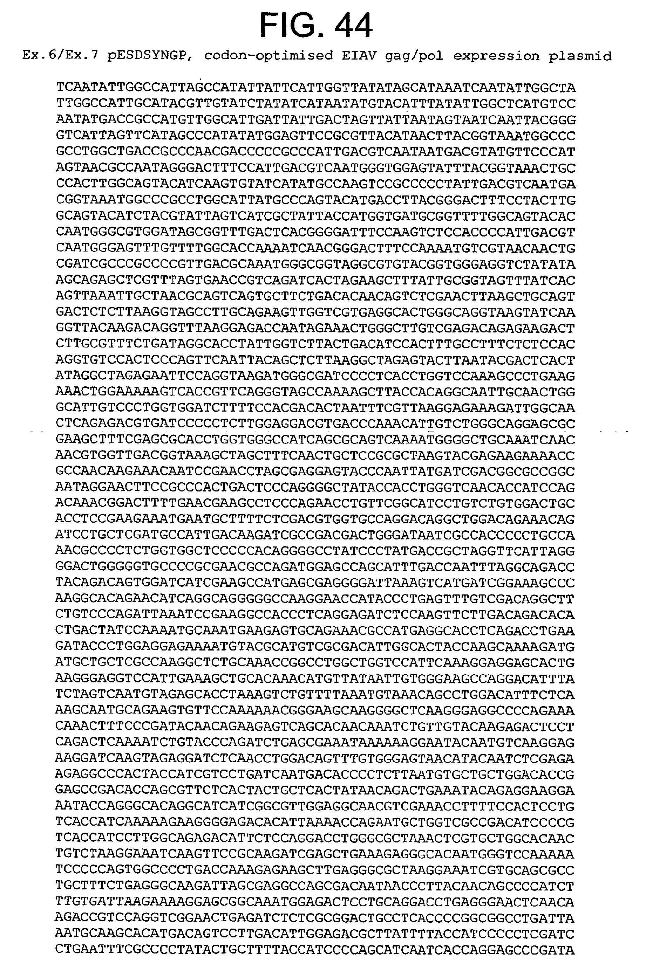

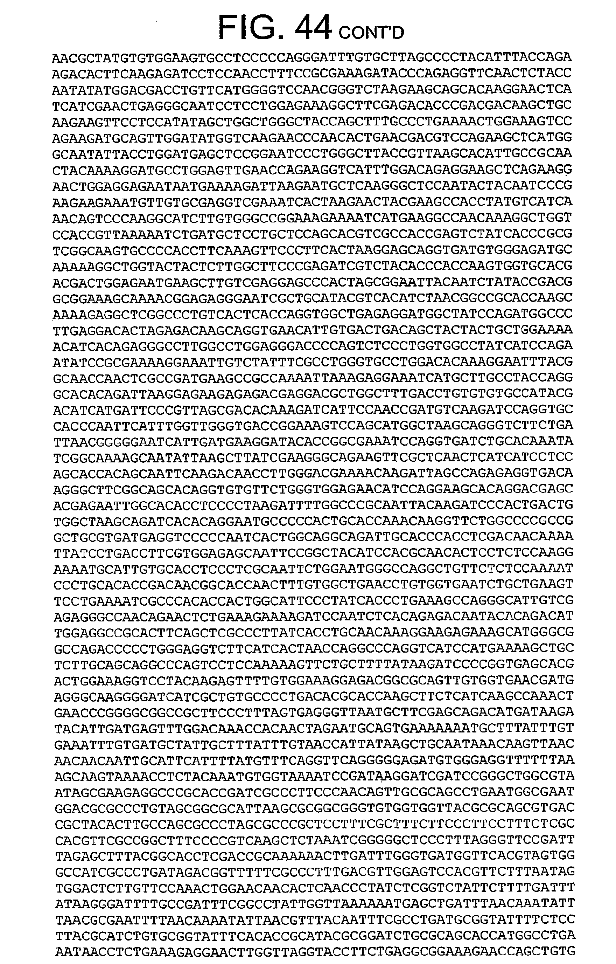

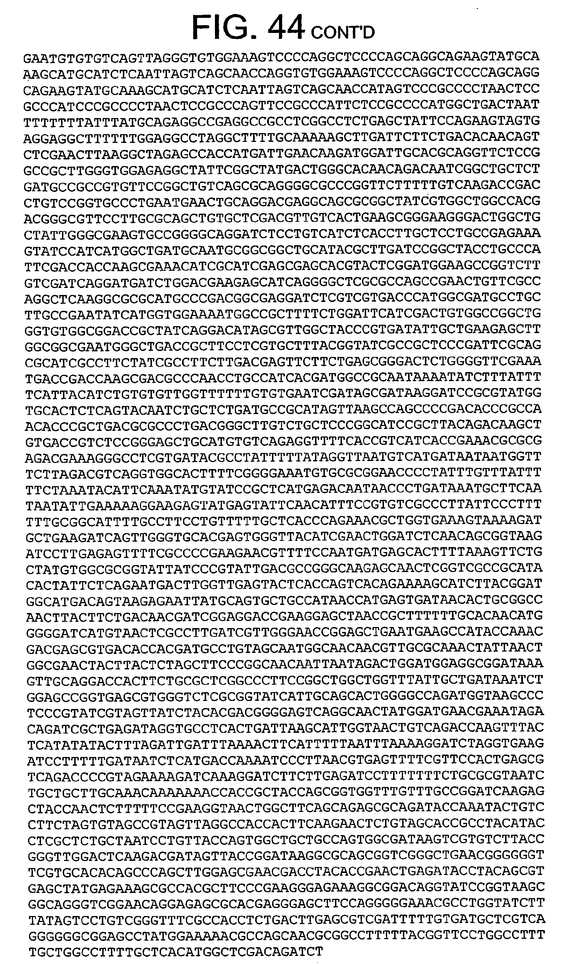

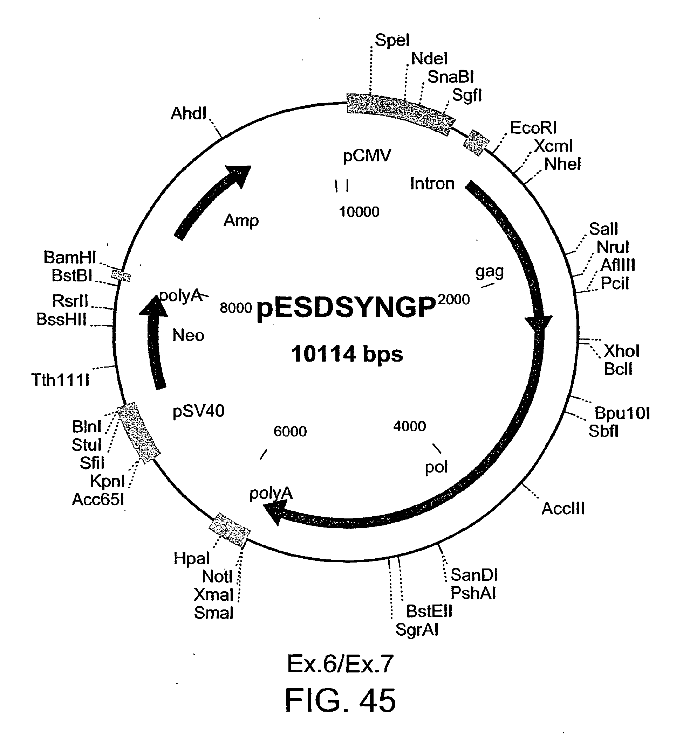

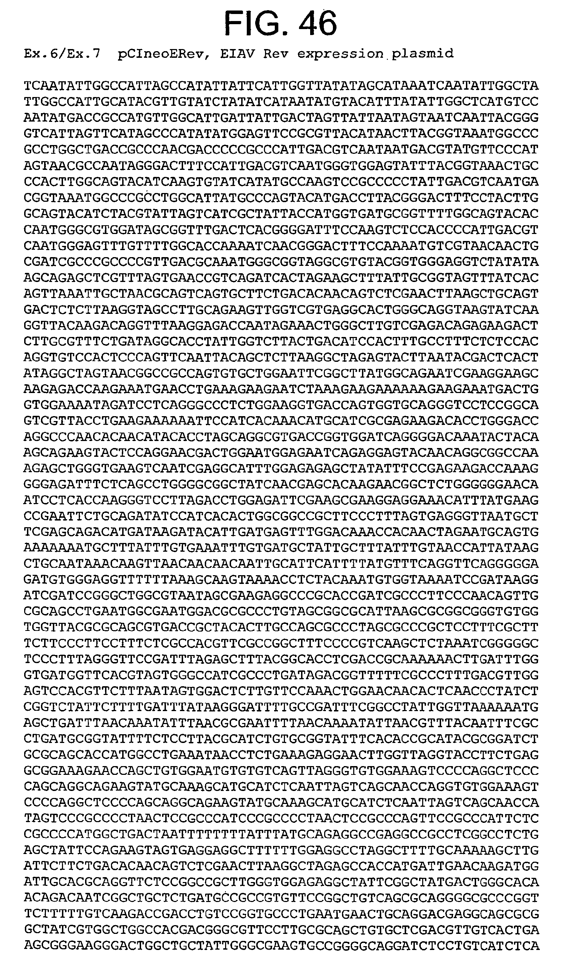

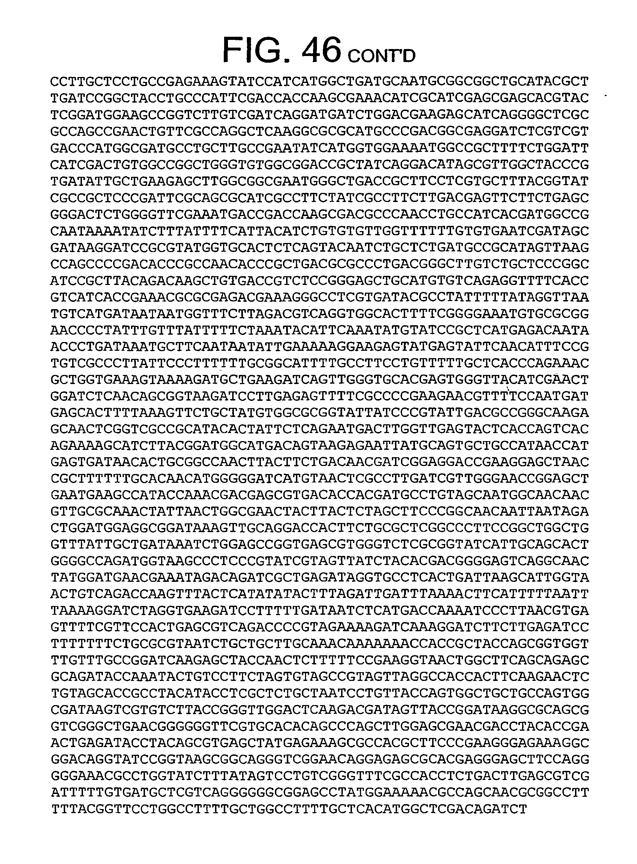

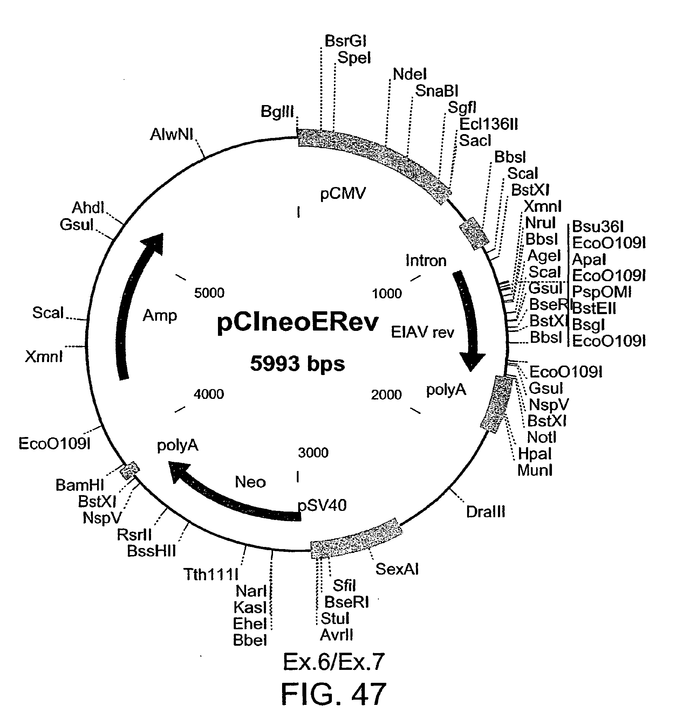

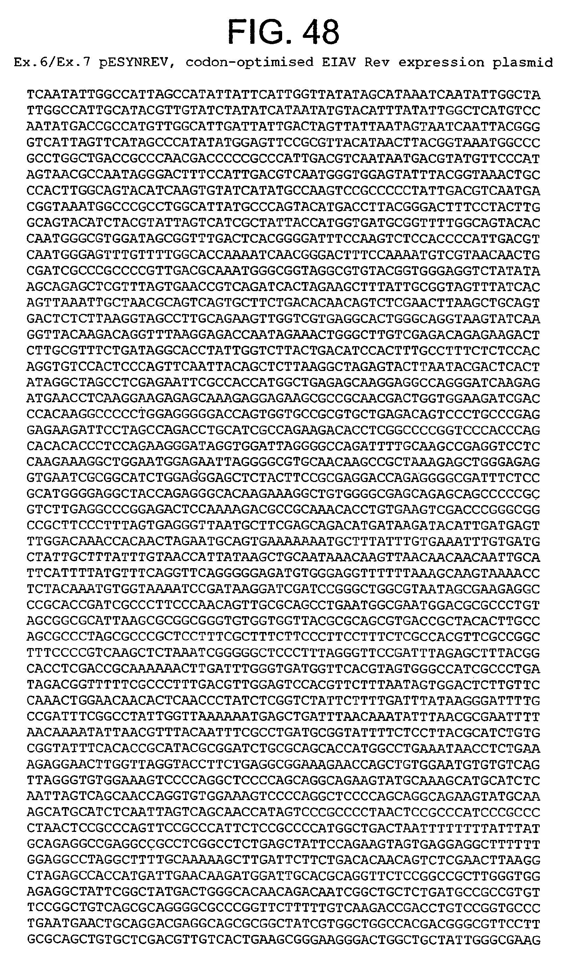

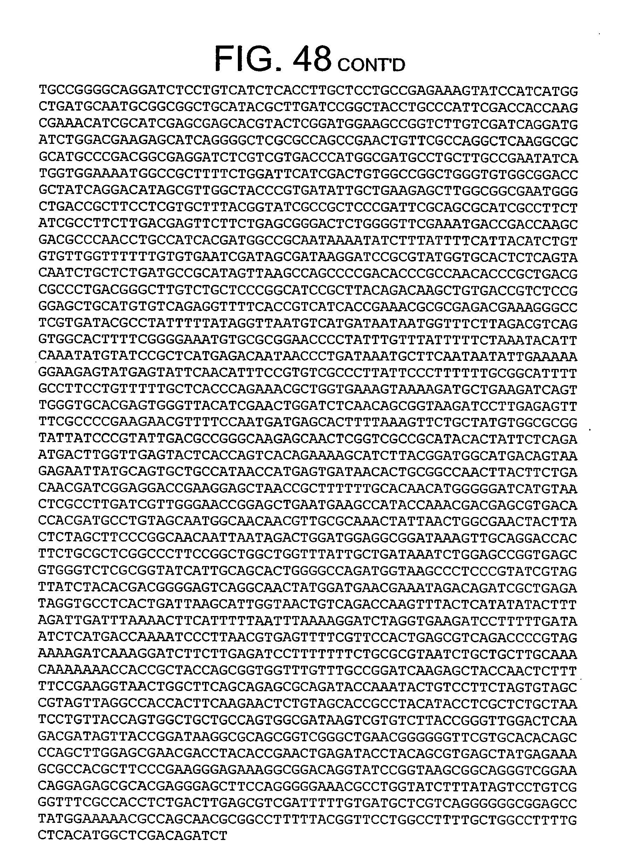

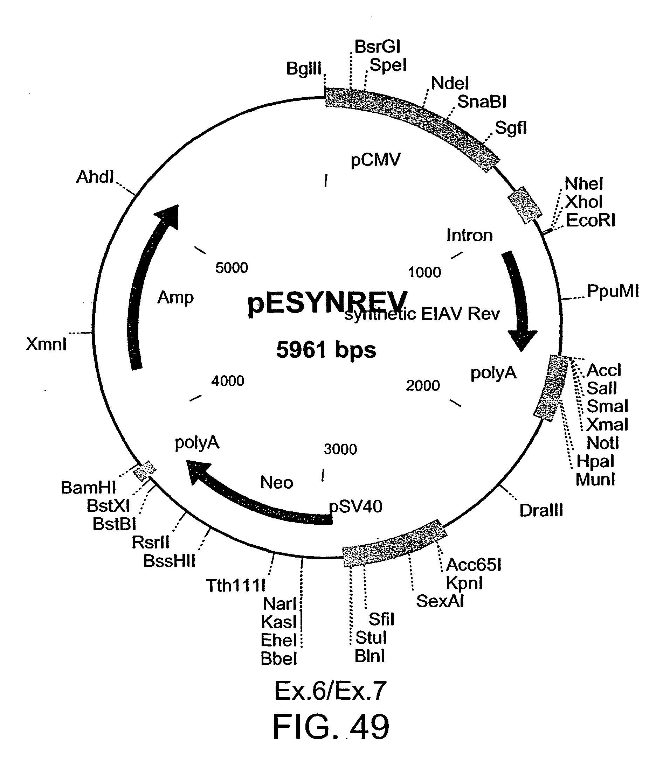

Vector may be made using Gag/Pol expression plasmids other than pONY3.1. For example, they can be made using the pESYNGP plasmid (Figure 42 and 43), in which the sequence of gag/pol gene is altered to optimise expression in human cells. This process is termed 'codon-optimisation' (Kotsopoulou et al., (2000) J. Virol. 74, 4839-4852 and GB0009760.2). pESYNGP is made by transferring a *Xba*I*-Not*I fragment from a plasmid containing a codon-optimised EIAV gag/pol ORF into pCIneo (Promega). The gene is synthesised by Operon Technologies Inc., Alameda, CA and supplied in a proprietary plasmid backbone, GeneOp. The complete fragment transferred includes sequences flanking the EIAV gag/pol ORF: tctagaGAATTCGCCACC**ATG- EIAV gag/pol- UGA**ACCCGGGgcggccgc. The ATG start and UGA stop codons are shown in bold and the recognition sequences for *Xbal* and *Not*I sites in lower case. Alternatively, pESDSYNGP (Figure 44 and 45) which has a splice donor in the leader can be used. pESDSYNGP was made from pESYNGP by exchange of the 306bp *Eco*RI-*Nhe*I fragment, which runs from just upstream of the start codon for gag/pol to approximately 300 base pairs inside the gag/pol ORF with a 308bp *Eco*RI*-Nhe*l fragment derived by digestion of a PCR product made using pESYNGP as template and using the following primers: EX7 SD FOR [GGCTAGAGAATTCCAGGTAAGATGGGCGATCCCCTCACCTGG] and EX7 SD REV [TTGGGTACTCCTCGCTAGGTTC]. This manipulation replaces the Kozak concensus sequence upstream of the ATG in pESYNGP with the splice donor found in EIAV. The sequence between the *E*coRI site and the ATG of gag/pol is thus CAGGTAAG. When transfections are carried out to make vector preparations using codon-optimised Gag/Pol expression plasmids higher yields of vector are obtained if a fourth plasmid encoding EIAV REV is also included in the transfection. Plasmids suitable for expression of EIAV Rev protein are pCIneoERev (WO 99/32646)(Figure 46 and 47) and pESYNREV(GB0009760.2) (Figure 48 and 49).

pESYNREV which is a pCIneo-based plasmid (Promega) which is made by introducing the *Eco*RI to *Sal*I fragment from a synthetic EIAV REV plasmid, made by Operon Technologies Alameda, CA, and contains a codon-optimised EIAV REV open reading frame flanked by *Eco*RI and Sa/l recognition sequences.

Transfections, harvesting and concentration of the vector particles are carried out as described above for pONY8.0Z.

### Assessement of the titre of pONY-RARβ2 vector preparations

The pONY-RARβ2 vector preparations lack β-galasctosidase or GFP markers which are commonly used to assess titre. However, titre of a vector preparation can be assessed, relative to a preparation of pONY8.0Z or pONY8G vector of known biological titre, by comparison of the levels of vector RNA incorporated into particles. This validity of this measurement method is dependent on equivalent efficiencies of vector entry, reverse transcription and integration for the vectors being compared. Similar methodology can be used to assess titre by direct measurement of integrated vector DNA in chromosomes of target cells. In this approach a direct measurement of biological titre is made.

### A) Assessment of titre via measurement of packaged vector RNA

The biological titre of the vector is related to the amount of vector RNA packaged in virions and can be assessed using quantitative RT-PCR using the ABI PRISM 7700 Sequence Detector. Any sequence within the vector genomic RNA can be used as a target however it should be unique to the vector component of the production system. A convenient target is the packaging signal. Vector RNA is isolated from the vector stocks using a Qiagen RNA isolation kit (Qiagen) and then DNAse treated using RNAse free DNAse (Ambion). Serial dilutions of the RNA are used as template in the RT-PCR reaction. Two TaqMan (Perkin-Elmer) reaction mixes are prepared, plus-RT and minus-RT, containing 1/10th volume of RNA template and the specific forward and reverse primers and probe. The minus-RT reaction is used to assess the efficiency of DNAse treatment. RT-PCR is carried out on an ABI PRISM 7700 Sequence Detector using conditions as follows: Hold, 48°C for 30min; hold, 95°C for 10min; forty cycles, 95°C for 15sec and 60°C for 1min. The data is analysed using the TaqMan software (Perkin-Elmer).
EX7 NEGATIVE SENSE PRIMER, FOR REVERSE TRANSCRIPTASE STEP = 5'-accagtagttaatttctgagacccttgta
EX7 PLUS SENSE PRIMER = 5' ATTGGGAGACCCTTTGACATT
EX7 PROBE = 5' FAM-CACCTTCTCTAACTTCTTGAGCGCCTTGCT-(TAMRA)p3'
(This set of primers detects vector genome, but not wild type gag/pol.)

### A) Assessment of titre via measurement of integrated vector DNA

A similar approach to the above is used except that DNA is prepared from cells transduced with pONY-RARβ2 or pONY-FLAG-RARβ2, or the vector standard, using a Qiagen QIAamp DNA Mini Kit and the RT step is omitted.

Thus, it is demonstrated that vector particles for the delivery of RARβ2 may be produced according to the invention.

### COMPARATIVE EXAMPLE 8: GENE TRANSFER TO ADULT NERVOUS TISSUE

The vectors of the present invention may be introduced into a subject by direct administration. In this example, it is demonstrated how nucleic acid expression constructs of the present invention can transduced into adult non-dividing neural cells using psudotyped EIAV-derived vectors as described above. It is further demonstrated that robust gene expression mediated by the vectors of the present invention is observed *in vivo* following gene transfer as disclosed herein.

### Intraspinal injection of EIAV vectors

Lentiviral vector is used to facilitate direct *in vivo* gene transfer, and to express the reporter gene β-galactosidase in rodent spinal cord cells. A system comprising a stereotaxic frame and an automatic micropump allows the localised injection of viral stock solution into the rat spinal cord without inducing any significant damage (Azzouz et al., 2000 Hum Mol Genet vol 9 pp803-11). In this Example, this is accomplished as follows:
Rats are anesthetized with an intraperitoneal injection of mixture solution of Hypnorm and Hypnovel (Wood et al., 1994 Gene Therapy vol 1 pp283-291). Animals are placed in a stereotax and their spinal cords are immobilized using a spinal adapter (Stoelting Co., IL, USA). EIAV vector is injected into the lumbar spinal cord following laminectomy.

To assess transduction efficiency of EIAV vectors into the spinal cord, 2 months old Albino rats are injected with 1 ul EIAVLacZ pseudotyped with VSV-G envelope (n = 3) (6 x 10⁸ T.U./ml) at one site. Injections, controlled by an infusion pump (World Precision Instruments Inc., Sarasota, USA), are at 0.1 ul/min through a 10 ul Hamilton syringe fitted with a 33 gauge needle. Following injection, the needle is left in place for 5 minutes before being retrieved. Three weeks following virus injection, animals are perfused transcardially with 4% paraformaldehyde. The lumbar spinal cord is dissected out and histological analysis is performed.

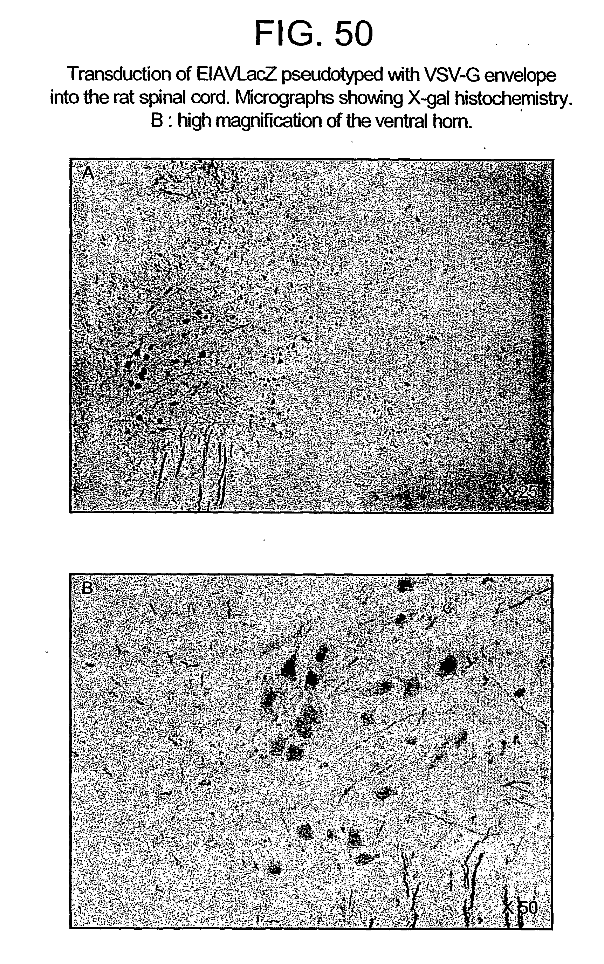

Intraspinal injection of the lentiviral vector is associated with only a mild degree of inflammation, with no significant cell damage. All subjects tolerated the surgery and vector injections with no detectable complications. Subjects continue to move normally in the cage post-injection, indicating the absence of functional deterioration following intraspinal injection of the viral vector. Both histochemistry (x-gal staining) and immunofluorescence reveal robust reporter gene expression within VSV-G injected spinal cord (Fig. 50).

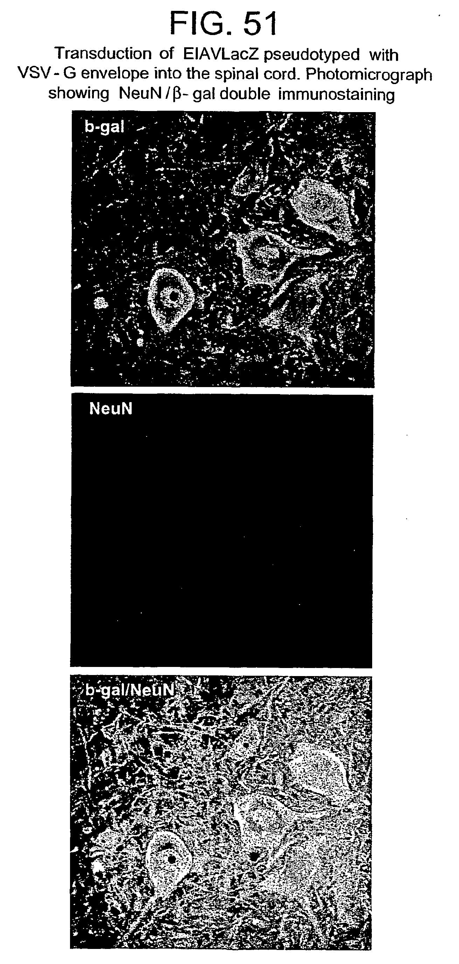

Transverse sections of the spinal cord reveal high transduction efficiency of the VSV-G pseudotyped vector. To demonstrate the phenotype of these cells, sections are double-labelled with antibodies to NeuN and to β-gal. At least about 90 % of the transduced cells are double-labeled with NeuN in VSV-G pseudotyped vector injected sections (Fig. 51).

### EIAV injection into the rat lumbar DRG

The protocol described above is adapted for direct injection of EIAV based vector in the DRG. Briefly, DRG (levels L4/L5) are surgically exposed by dissecting the musculus multifidus and the musculus longissimus lumborum and by removing the processus accessorius and parts of the processus transversus. EIAV vector coding for the reporter gene β-gal (∼2-5 x 10⁹ TU/ml) is injected directly into the DRG. Rats receive 1 ul of the viral vector solution per ganglion. All injections are carried out using a stereotaxic frame and a Hamilton syringe with 34-gauge needle. The solution is slowly infused at the speed of approximately 0.1 ul/min. To confirm the transduction of sensory neurons by EIAV vector, histology and immunohistology using β-gal antibodies (Affiniti) are performed at 2, 4 and 8 weeks.

### Figures for Comparative Example 8

**Figure 50.** Transduction of EIAVLacZ pseudotyped with VSV-G envelope into the rat spinal cord. Micrographs showing X-gal histochemistry. B : high magnification of the ventral horn.

**Figure 51.** Transduction of EIAVLacZ pseudotyped with VSV-G envelope into the spinal cord. Photomicrograph showing NeuN/β-gal double immunostaining. β-gal staining shows as green in the left-hand panel (marked 'β-gal'), NeuN staining shows as red in the middle panel, and both red and green channels are shown in the β-gal/NeuN double stain in the right hand panel.

### SUMMARY

The Examples demonstrate that RARβ2 thereof can be used to cause neurite development.

In particular, we show *inter alia* the use of retinoids to stimulate neurite regeneration in peripheral nerves by activation of RARβ2.

The delivery and expression of nucleic acid sequences into non-dividing neuronal cells is demonstrated using retroviral vectors, in particular using EIAV pseudotyped with Rabies-G or V SV-G proteins. It is the latter subject-matter, i.e. a lenti-viral vector comprising RARβ2 to which the present invention specifically pestains, as defined in the claims.

We show that viral vectors can be produced for delivery of nucleic acid sequences encoding RARβ2 into cells.

Thus, neurite outgrowth and/or neurite regeneration are brought about via the vectors of the present invention delivering nucleic acid sequences encoding RARβ2 to non-dividing cells of the mammalian nervous system.

When peripheral nerves are damaged some regeneration can occur unlike nerves of the central nervous system which show no regeneration. However regeneration of peripheral nerves is limited particularly when there is traumatic nerve injury where there is a loss of nerve tissue such that a gap is created which the regenerating neurite cannot grow across. This delay in nerve regeneration can lead to muscle atrophy and lead to permanent disability.

In response to peripheral nerve injury neurotrophins are produced. These are a family of growth factors that are required for the survival of a variety of neurons. The family includes nerve growth factor (NGF) neurotrophin-3 (NT-3) and brain-derived neurotrophic factor (BDNF). It was hoped that neurotrophins could be used in the treatment of PNS injuries. However the results have not been encouraging. Two major problems have been encountered, firstly the problem of delivery to the injury, and secondly since different neurons need different neurotrophins a cocktail of them as to be administered in order for all the nerves to regenerate. We have investigated how neurotrophins stimulate neurite regeneration.

We have found that the vitamin A derivative all-trans-retinoic acid (tRA) like NGF induces neurite outgrowth from various embryonic sources, including PNS. Cellular effects of tRA are mediated by binding to nuclear receptors that are ligand activated transcription factors. There are two classes of receptors, retinoic acid receptors (RARs) and retinoid X receptors (RXRs), with three subtypes of each: α, β and γ. RAR receptors mediate gene expression by forming heterodimers with the RXRs, whereas RXRs can mediate gene expression either as homodimers or by forming heterodimers with orphan receptors.

We have found that only RARβ2 is required for neurite outgrowth of all types of neurons we have cultured. Furthermore when adult mouse DRG are cultured in the presence of NGF and an inhibitor of tRA synthesis, neurite outgrowth does not occur. Conversely, when tRA is added along with a blocking antibody to NGF, neurite outgrowth occurs as normal. We have also shown that NGF induces transcription of both the tRA-synthesizing enzyme RALDH-2 and the RARβ2 as well as a detectable release of synthesized tRA.

We propose that the stimulation of RARβ2 is an intrinsic requirement for the regeneration of neurites in the peripheral nervous system and that crucially this is downstream of the neurotrophins. Therefore in regard to the peripheral nervous system we want to administer retinoids that can activate the RARβ2 receptor in order for neurite regeneration to occur.

We have extended our observations to the CNS. We have found that the embryonic spinal cord expresses RARβ2 and that the amount of its expression correlates with the amount of neurite outgrowth. In contrast the adult spinal cord does not express RARβ2 nor can it regenerate neurites. In work leading to the presently claimed invention we have shown that by transfecting RARβ2 by use of a defective herpes simplex virus type 1 (HSV-1) vector into cultured adult spinal cord we have transformed the normally inert spinal cord into one which can extend neurites. We propose that gene therapy of injured spinal cord with RARβ2 will lead to functional recovery.

The use of retinoid to treat peripheral nervous system (PNS) injuries would have at least three major advantages over the use of neurotrophins. Firstly retinoids unlike neurotrophins are small lipophilic molecules which can be easily administered to the site of injury therefore regeneration should occur at.a much quicker, rate than can be achieved with neurotrophins, this should lead to a reduction in muscle atrophy and consequent paralysis. Secondly since the stimulation of RARβ2 is crucial to the regeneration of all neurons we have tested only one type of retinoid need be taken circumventing the need to administer a cocktail of neurotrophins. Thirdly retinoids are relatively easy to synthesise unlike neurotrophins.

Gene therapy with RARβ2 to treat CNS and/or PNS injuries should lead to functional recovery and therefore the prevention of paralysis.

Increasing RARβ2 levels according to the present invention preferably stimulates other neural repair mechanisms such as peripheral repair.

PNS and CNS injuries occur all over the world unfortunately it is unlikely that the incidence of such injuries will decrease. World wide a 1000 people per million of the population a year suffer spinal cord injury, ten times this number suffer some sort of PNS injury.

In addition there are three other areas where retinoids would be of use. In leprosy diabetes and AIDS neuropathy occurs (the neurites die) this is equivalent to PNS injury. In both leprosy and diabetes it has been shown that there is a loss of NGF in the skin of both types of patients leading to the loss of pain sensation and inflammation which can lead to ulcer formation. In AIDS patients sensory neuropathy is one of the most common effects of HIV infection, already NGF as been used to treat this condition.

We propose that gene therapy with RARβ2 can be used to treat CNS injuries and/or PNS injuries.

In summation, our results indicate a role for RA acting via RARβ2 in the outgrowth of neurites from certain classes of neurons.

The present invention relates specifically to compositions for the treatment of neurodegenerative disease in which expression of the retinoic acid receptor RARβ2 is ensured in affected cells or tissues. This is achieved by subject-matter defined as a composition comprising a LV-RARβ2 optionally in combination with an antagonist. The invention may also be seen as medicaments comprising these agents in medication for the treatment of peripheral nervous injuries and spinal cord regeneration e.g. in cases of paraplegia.

It may be possible to substitute some or all of the RARβ2 agonist of the present invention with an inhibitor of an antagonist of RARβ2.

### REFERENCES TO RETINOIC ACID SECTION

1. Benbrook, D.; Lernhardt, E.; Pfahl, M. : A new retinoic acid receptor identified from a hepatocellular carcinoma. Nature 333: 669-672, 1988.
2. Brand, N.; Petkovich, M.; Krust, A.; Chambon, P.; de The, H.; Marchio, A.; Tiollais, P.; Dejean, A. : Identification of a second human retinoic acid receptor. Nature 332: 850-853, 1988.
3. Dejean, A.; Bougueleret, L.; Grzeschik, K.-H.; Tiollais, P. : Hepatitis B virus DNA integration in a sequence homologous to v-erb-A and steroid receptor genes in a hepatocellular carcinoma. Nature 322: 70-72, 1986.
4. de The, H.; del Mar Vivanco-Ruiz, M.; Tiollais, P.; Stunnenberg, H.; Dejean, A.:Identification of a retinoic acid responsive element in the retinoic acid receptor beta gene. Nature 343: 177-180, 1990.
5. de The, H.; Marchio, A.; Tiollais, P.; Dejean, A. : A novel steroid thyroid hormone receptor-related gene inappropriately expressed in human hepatocellular carcinoma. Nature 330: 667-670, 1987.
6. Kreczel, W.; Ghyselinck, N.; Samad, T. A.; Dupe, V.; Kastner, P.; Borrelli, E.; Chambon, P. : Impaired locomotion and dopamine signaling in retinoid receptor mutant mice. Science 279: 863-867, 1998.
7. Lotan, R.; Xu, X.-C.; Lippman, S. M.; Ro, J. Y.; Lee, J. S.; Lee, J. J.; Hong, W. K. : Suppression of retinoic acid receptor-beta in premalignant oral lesions and its up-regulation by isotretinoin. New Eng. J. Med. 332: 1405-1410, 1995.
8. Mattei, M.-G.; de The, H.; Mattei, J.-F.; Marchio, A.; Tiollais, P.; Dejean, A. : Assignment of the human hap retinoic acid receptor RAR-beta gene to the p24 band of chromosome 3. Hum. Genet. 80: 189-190, 1988.
9. Mattei, M.-G.; Riviere, M.; Krust, A.; Ingvarsson, S.; Vennstrom, B.; Islam, M. Q.; Levan, G.; Kautner, P.; Zelent, A.; Chambon, P.; Szpirer, J.; Szpirer, C. : Chromosomal assignment of retinoic acid receptor (RAR) genes in the human, mouse, and rat genomes. Genomics 10: 1061-1069, 1991.
10. Nadeau, J. H.; Compton, J. G.; Giguere, V.; Rossant, J.; Varmuza, S. : Close linkage of retinoic acid receptor genes with homeobox- and keratin-encoding genes on paralogous segments of mouse chromosomes 11 and 15. Mammalian Genome 3: 202-208, 1992.
11. Samad, A.; Kreczel, W.; Chambon, P.; Borrelli, E. : Regulation of dopaminergic pathways by retinoids: activation of the D2 receptor promoter by members of the retinoic acid receptor-retinoid X receptor family. Proc. Nat. Acad. Sci. 94: 14349-14354, 1997.

### References to Example 1

1. Lindsay, R. J. Neurosci. 8, 2394-2405 (1988).
2. Quinn, S. D. P. & De Boni, U. In Vitro Cell. Dev. Biol. 27A, 55-62 (1991).
3. Haskell, B. E., Stach, R. W., Werrbach-Perez, K. & Perez-Polo, J. R. Cell Tissue Res. 247, 67-73 (1987).
4. Rodriguez-Tebar, A. & Rohrer, H. Development 112, 813-820 (1991).
5. Wion, D., Houlgatte, R., Barbot, N., Barrand, P., Dicou, E. & Brachet, P. Biochem. Biophys. Res. Comm. 149, 510-514 (1987).
6. Kastner, P., Chambon, P. & Leid, M. in Vitamin A in Health and Disease (ed. Blomhoff, R.) 189-238 (Dekker, New York, 1994).
7. Kliewer, S. A., Umesono, K., Evans, R. M. & Mangelsdorf, D. J. in Vitamin A in Health and Disease (ed. Blomhoff, R.) 239-255. (Dekker, New York, 1994)
8. Mangelsdorf, D. J. & Evans, R. M. Cell 83, 841-850 (1995).
9. Millbrandt, J. Neuron 1, 183-188 (1988).
10. McCaffery, P., Lee, M.-O., Wagner, M. A., Sladek, N. E. & Drager, U. Development 115, 371-382 (1992).
11. Duester, G. Biochemistry 35, 12221-12227 (1996).
12. Drager, U. C. & McCaffery, P. in Enzymology and Molecular Biology of Carbonyl Metabolism Vol. 5 (eds. Weiner, H. et al.) 185-192 (Plenum, New York, 1995).
13. Plum, L. A. & Clagett-Dame, M. Dev. Dynam. 205, 52-63 (1996).
14. Schnell, L., Schneider, R., Kolbeck, R., Barde, Y.-A. & Schwab, M. E. Nature 367, 170-173 (1994).
15. Schatzl, H. M. Trends Neurosci. 18, 463-464 (1995).
16. Maden, M., Sonneveld, E., van der Saag, P. T. & Gale, E. Development 125, 4133-4144 (1998).

### REFERENCES TO EXAMPLE 2

1. David, S. & Agayo, A.J. Axonal elongation into peripheral nervous system "bridges" after central nervous system injury in adult rats. Science 214, 931-933 (1981).
2. Cheng, H., Cao, Y. & Olsen, L. Spinal cord repair in adult paraplegic rats: partial restoration of hind limb function. Science 273, 510-513 (1996).
3. Schwab, M.E. Nerve fibre regeneration after traumatic lesions of the CNS; progress and problems. Phil. Trans. R. Soc. Lond. B 331, 303-306 (1991).
4. Bregman, B.S. et al. Recovery from spinal cord injury mediated by antibodies to neurite growth inhibitors. Nature 378, 498-501 (1995).
5. Schnell, L. et al. Neurotrophin-3 enhances sprouting of corticospinal tract during development and after adult spinal cord lesion. Nature 367, 170- 173 (1994).
6. Li, Y. et al. Repair of adult rat corticospinal tract by transplants of olfactory ensheathing cells. Science 277, 2000-2002 (1997).
7. Kobayashi, N.R. et al. BDNF and NT4/5 prevent atrophy of rat rubrospinal neurons after cervical axotomy, stimulate GAP-43 and Ta1-tubulin mRNA expression, and promote axonal regeneration.J. Neurosci. 17, 9583-9595 (1997).
8. Wagner, M. et al. Regional differences in retinoid release from embryonic neural tissue detected by an in vitro reporter assay. Development 116, 55-66 (1992).
9. Horton, C. & Maden, M. Endogenous distribution of retinoids during normal development and teratogenesis in the mouse embryo.Dev. Dynam. 202, 312-323 (1995).
10. McCaffery, P. & Drager, U.C. Hot spots of retinoic acid synthesis in the developing spinal cord. Proc. Natl. Acad. Sci. USA 91, 71947197 (1994).
11. McCaffery, P. & Drager, U.C. Retinoic acid synthesising enzymes in the embryopnic and adult vertebrate. In Enzymology and Molecular Biology of Carbonyl Metabolism 5 (H. Weiner et al. eds) pp173-183. Plenum Press, New York (1995).
12. Yamamoto, M. et al. Influence of the choroid plexus on cerebellar development: analysis of retinoic acid synthesis. Dev. Brain Res. 93, 182-190 (1996).
13. Maden, M. et al. The distribution of endogenous retinoic acid in the chick embryo: implications for developmental mechanisms. Development 125, 4133-4144 (1998).
14. Maden, M. et al. Vitamin A-deficient quail embryos have half a hindbrain and other neural defects. Current Biol. 6, 417-426 (1996).
15. Maden, M. et al. The role of vitamin A in the development of the central nervous system. J. Nutr 128, 471S-475S (1998).
16. Maden, M. Retinoids in neural development. In Handbook of Experimental Pharmacology. (H. Nau & W.S. Blaner eds.) Springer-Verlag, Heidelberg (1998) in press.
17. Maden, M. et al. Retinoic acid as a chemotactic molecule in neuronal development. Int. J. Devl. Neurosci. 16, 317322 (1998).
18. Kastner, P et al. Role of nuclear retinoic acid receptors in the regulation of gene expression. In Vitamin A in Health and Disease. (R. Blomhoff ed.) pp189-238. Marcel Dekker Inc., New York (1994).
19. Kliewer, S.A. et al. The retinoid X receptors: modulators of multiple hormonal signalling pathways. InVitamin A in Health and Disease. (R. Blomhoff ed.) pp239- 255. Marcel Dekker Inc., New York (1994).
20. Corcoran, J and Maden M. (1999). Nerve growth factor acts via retinoic acid synthesis to stimulate neurite outgrowth. Nat. Neuroscience 2, 307-308.
21. Quinn, S.D.P. & De Boni, U. Enhanced neuronal regeneration by retinoic acid of murine dorsal root ganglia and of fetal murine and human spinal cord in vitro. In Vitro Cell. Dev. Biol. 27A, 55-62 (1991).
22. Wuarin, L. & Sidell, N. Differential susceptibilities of spinal cord neurons to retinoic acid-induced survuval and differentiation. Dev. Biol. 144, 429-435 (1991).
23. Ved, H.S. & Pieringer, R.A. Regulation of neuroanl differentiation by retinoic acid alone and in cooperation with thyroid hormone or hydrocortisone. Dev. Neurosci. 15, 49-53 (1993).
24. Corcoran, J. & Maden, M. Nerve growth factor acts via retinoic acid synthesis to stimulate neurite outgrowth. Nature Neurosci. in press (1999).
25. Caroni, P. & Schwab, M.E. Codistribution of neurite growth inhibitors and oligodendrocytes in rat CNS: appearance follows nerve fiber growth and precedes myelination. Dev. Biol. 136, 287-295. (1989).
26. Notterpek, L.M. & Rome, L.H. Functional evidence for the role of axolemma in CNS myelination. Neuron 13, 473-485. (1994).
27. Smith, D.S. & Skene, J.H.P. A transcription-dependent switch controls competence of adult neurons for distinct modes of axon growth. J Neurosci. 17, 646-658 (1997).
28. Lim, F., Hartley, D., Starr, P., Song, S., Lang, P., Yu, L., Wang, Y.M. & Geller, A.I. Use of defective herpes-derived plasmid vectors. Meth.Mol. Biol. 62, 223-232 (1997).

### REFERENCES TO EXAMPLE 3

Achkar, C. C., Dergiuni, F., Blumberg, B., Langston, A., Levin, A.A., Speck, J., Evans, R.M., Bolado, J., Nakanishi, K., Buck, J. and Gudas, L.J. (1996). 4-oxoretinol, a new natural ligand and transactivator of the retinoic acid receptors. Proc.Natl.Acad.Sci. USA 93, 4879-4884.
Ang, H.L. and Duester, G. (1997). Initiation of retinoid signalling in primitive streak mouse embryos: spatiotemporal expression patterns of receptors and metabolic enzymes for ligand synthesis. Dev. Dynam. 208, 536-543.
Barde, Y. -A., Edgar, D. and Thoenen, H. (1982). Purification of a new neurotrophic factor from mammalian brain. EMBO J. 1, 549-553.
Buck, J., Derguini, F., Levi, E., Nakanishi, K. and Hammerling, U. (1991). Intracellular signalling by 14-hydroxy-4,14-retro-retinol.Science 254, 1654-1656.
Campenot, R.B. (1977). Local control of neurite development by nerve growth factor. Proc.Natl.Acad.Sci. USA 74, 4516-4519.
Crowley, C., Spencer, S.D., Nishimura, M.C., Chen, K.S., Pitts-Meek, S., Armanini, M.P., Ling, L.H., McMahon, S.B., Shelton, D.L, Levinson, A.D. and Phillips, H.S. (1994). Mice lacking nerve growth factor display perinatal loss of sensory and sympathetic neurons yet develop basal forebrain cholinergic neurons. Cell 76, 1001-1012.
Corcoran, J and Maden M. (1999). Nerve growth factor acts via retinoic acid synthesis to stimulate neurite outgrowth. Nat. Neuroscience 2, 307-308.
Drager, U.C. and McCafery, P. (1995). Retinoic acid synthesis in the developing spinal cord. In Enzymology and Molecular Biology of Carbonyl Metabolism. 5 (ed. H. Weiner et al.) 185-192 (Plenum Press, New York.
Ernfors, P, Lee, K, Kucera. J. and Jaenisch, R. (1994). Lack of Neurotrophin-3 leads to deficiencies in the peripheral nervous system and loss of limb proprioceptive afferents. Cell 77, 503-512.
Farinas, I., Jones, K.R., Backus, C., Wang, X-Y. and Reichardt, L.F. (1994). Severe sensory and sympathetic deficits in mice lacking neurotrophin-3. Nature 369, 658-661.
Godbout, R., Packer, M., Poppema, S. & Dabbath, L. (1996). Localization of cytosolic aldehyde dehydrogenase in the developing chick retina: in situ hydridisation and immunohistochemical analyses.Dev. Dynam. 205, 319-331.
Jones, K.R, Farlinas, I, Backus , C. and Reichardt, L.F. (1994). Targeted disruption of the BDNF gene perturbs brain and sensory neuron development but not motor neuron development. Cell 76, 989-999.
Klein, R., Silos-Santiago, I., Smeyne, R.J., Lira, S.A., Brambilla, R., Bryant, S., Zhang, L., Snider, W.D. and Barbacid. M. (1994). Disruption of the neurotrophin-3 receptor gene trkC eliminates 1a muscle afferents and results in abnormal movements. Nature 368, 249-251.
Klein, R., Smeyne, R.J., Wurst, W., Long, L.K., Auerbach, B.A., Joyner, A.L. and Barbacid. M. (1993). Targeted disruption of the trkB neurotrophin receptor gene results in nervous system lesions and neonatal death. Cell 75, 113-122.
Kastner, P., Chambon, P. and Leid, M. (1994). Role of nuclear retinoic acid receptors in the regulation of gene expression. In Vitamin A in Health and Disease. (R. Blomhoff ed.) pp 189-238. Marcel Dekker Inc., New York.
Kliewer, S.A., Umesono, K., Evans, R.M. and Mangelsdorf, D.J. (1994). The retinoid X receptors: modulators of multiple hormonal signalling pathways. In Vitamin A in Health and Disease. (R. Blomhoff ed.) pp 239-255. Marcel Dekker Inc., New York.
Leid, M., Kastner, P. and Chambon, P. (1992).Multiplicity generates diversity in the retinoic signalling pathways. Trends Biol. Sci. 17,427-433
Levi-Montanlcini, R. The nerve growth factor: Thirty five years later. Science 237, 1154-1164 (1987).
Lindsay, R. (1998). Nerve growth factors (NGF, BDNF) enhance axonal regeneration but are not required for survival of adult sensory neurons. J. Neurosci. 8, 2394-2405.
Maden, M., Gale, E., Kostetskii, I. and Zile, M.(1996). Vitamin A-deficient quail embryos have half a hindbrain and other neural defects. Current Biol. 6, 417-426.
Maden, M. Retinoids in neural development. In Handbook of Experimental Pharmacology. (H. Nau & W.S. Blaner eds.) Springer-Verlag, Heidelberg (1998) in press.
Maden, M., Gale, E. and Zile, E. (1998). The role of vitamin A in the development of the central nervous system. J. Nutr. 128, 471 S-475 S.
Maden, M., Sonneveld, E., van der Saag, P.T. and Gale, E. (1998). The distribution of endogenous retinoic acid in the chick embryo: implications for developmental mechanisms. Development 125 in press.
Mangelsdorf, D.J. and Evans, R.M. (1995). The RXR heterodimers and orphan receptors. Cell 83, 841-850.
Maisonpierre. P.C., Belluscio, L., Squinto, S., Ip, N.Y., Furth, M.E., Lindsay, R.M. and Yancopoulos, G.D. (1990). Neurotrophin-3: a neurotrophic factor related to NGF and BDNF. Science 247, 1446-1451.
McCaffery, P. and Drager, U.C. (1994). Hot spots of retinoic acid synthesis in the developing spinal cord. Proc. Natl. Acad. Sci. USA 91, 7194-7197.
McCaffery, P., Lee, M.-O., Wagner, M.A., Sladek, N.E. & Drager, U. (1992). Asymmetrical retinoic acid synthesis in the dorsoventral axis of the retina. Development 115,371-382.
McCormick, A. M., Napoli, J. L., Schnoes, H. K. & Deluca, H. F. (1978). Isolation and identification of 5,6-epoxyretinoic acid: a biologically active metabolite of retinoic acid. Biochemistry 17, 4084-4090.
Millbrandt, J. (1989). Nerve growth factor induces a gene homologous to the glucocorticoid receptor gene. Neuron 1, 183-188.
Niederreither, K., McCaffery, P., Drager, U.C., Chambon, P. and Dolle, P. (1997). Restricted expression and retinoic acid-induced downregulation of the retinaldehyde dehydrogenase type 2 (RALDH-2) gene during mouse development. Mech of dev 62, 67-68
Quinn, S.D.P and De Boni, U. (1991). Enhanced neuronal regeneration by retinoic acid of murine dorsal root ganglia and of fetal murine and human spinal cord in vitro. In Vitro Cell. Dev. Biol. 27A, 55-62.
Smeyne, R.J., Klein, R., Schnapp, A., Long, L.K., Bryant, S., Lewin, A, Lira, S.A. and Barbacid, M. (1994). Severe sensory and sympathetic neuropathies in mice carrying a disrupted trk/NGF receptor gene. Nature 368, 246-249.
Snider, W.D. (1994). Functions of the neurotrophins during nervous system development: what the knockouts are teaching us. Cell 77, 627-638.
Tuttle R.& Mathew, W.D (1995). Neurotrophins affect the pattern of DRG neurite growth in a bioassay that presents a choice of CNS and PNS substrates. Development 121, 1301-1309.
Wuarin, L. & Sidell, N. (1991). Differential susceptibilities of spinal cord neurons to retinoic acid-induced survival and differentiation. Dev. Biol. 144, 429-435.

### References to Example 4:

Lim, F., Hartley, D., Starr, P., Song, S., Lang, P., Yu, L., Wang, Y.M. & Geller, A.I. Use of defective herpes-derived plasmid vectors. Meth.MoL Biol. 62, 223-232 (1997).
World Intellectual Property Organization publication number WO 98/17817 (IMPROVED RETROVIRAL VECTORS).
World Intellectual Property Organization publication number WO 98/17816 LENTIVIRAL VECTORS (Tradsducing non-dividing cells).
World Intellectual Property Organization publication number WO 99/61639 DELIVERY SYSTEM (Pseudotyping with Rabies G).

### SEQUENCES

RARalpha reverse primer: 535 tgtagctctctgagcactc 517
RARalphal forward strand primer 648 tacgccttcttctttcccc 666
RARalpha2 forward strand primer 376 cttttataaccagaaccgggc 396
RARalpha3 forward strand primer 111 caagtagaagccaggaaagtc 131
RARalpha4 forward strand primer 3 ctaagaagacccacacttctg 23
RARalpha5 forward strand primer 30 aagtgaggtgaaaactggg 48
RARalpha6 forward strand primer 42 ttcacagcctggcataac 59
RARalpha6 forward strand primer 24 gagaaggaagtgagccatc 42
RARbeta reverse strand primer 508 tctctgtgcattcctgctttg 488
RARbeta 1 forward strand primer 180 tggacacatgactcactacc 199
RARbeta 2 forward strand primer 598 atgttctgtcagtgagtccc 617
RARbeta 3 forward strand primer 457 gcatgtcagaggacaactg 475
RARbeta 4 forward strand primer 21 agcctggaaaatgccatc 38
RARGamma reverse strand primer 481 ttacagcttccttggacatgcc 460
RARGamma1 forward strand primer 119 agatgctgagccctagcttc 138
RARGamma2 forward strand primer 73 ttactacgcagagccactgg 92
RARGamma3 forward strand primer 169 ggaagatggaagagggaac 187
RARGamma4 forward strand primer 230 caaatttactgggggttgg 248
RARGamma5 forward strand primer 18 ggctggattttggattgaag 37
RARGamma6 forward strand primer 329 ttctgtcctctcactaccttgg 350
RARGamma7 forward strand primer 85 cattaccgcgagtcactaac 104

### GAPDH

forward primer 37 cgtagacaaaatggtgaagg 56
reverse primer 333 gactccacgacatactcagc 314

### RALDHII

forward primer 1190 gcttcttcattgacccac 1208
reverse primer 1539 cttcaccgtcaggtctttac 1519

### RXRalpha

forward primer 745 gcaaggaccggaatgagaac 764
reverse primer 994 tctaggggcagctcagaaaag 974

### RXRbeta

forward primer 1910 agaataaaggggtagtgaagg 1930
reverse primer 2176 catcaatgtccccacttg 2159

### RXRgamma

forward primer 713 tgccagtagtagccacgaag 732
reverse primer 966 tgagcagttcattccaccc 948
**RARβ2 FWD:**
   **5'** CAG TAC ccg.cgg GCC ACC ATG TTT GAC TGT ATG GAT GTT CTG **3'**
**RARβ2 REV:**
   **5'** CAG TAC ctg cag.ATC ATT GCA CGA GTG GTG ACT GAC T **3'**
EIAV cPPT POS:
   CAGGTTAT*TCTAGA***GTCGAC**GCTCTCATTACTTGTAAC
EIAV cPPT NEG:
   CGAATGCGT*TCTAGA***GTCGAC**CATGTTCACCAGGGATTTTG
MIN FOR:
   CACCTAGCAGGCGTGACCGGTGG
MIN REV:
   CCTAC**CAATTG**TATAAAACCCCTCATAAAAACCCCAC

### pONY8.0Z

### pONY3.1

PERT NEGATIVE SENSE PRIMER, FOR REVERSE TRANSCRIPTASE STEP
   5'-CACAGGTCAAACCTCCTAGGAATG
PERT PLUS SENSE PRIMER
   5'TCCTGCTCAACTTCCTGTCGA
PERT PROBE
   5' FAM-CGAGACGCTACCATGGCTA-(TAMRA)p3'
Packaging Signal assay:
   NEGATIVE SENSE PRIMER, FOR REVERSE TRANSCRIPTASE STEP
   5'-accagtagttaatttctgagacccttgta
Packaging Signal assay:PLUS SENSE PRIMER
   5' ATTGGGAGACCCTTTGACATT
Packaging Signal assay:PROBE
   5' FAM-CACCTTCTCTAACTTCTTGAGCGCCTTGCT-(TAMRA)p3'

### pEsynGP

### pESDSYNGP

### SEQ ID No. - pONY4.0Z

### codon optimised EIAV REV

SD FOR
   GGCTAGAGAATTCCAGGTAAGATGGGCGATCCCCTCACCTGG
SD REV
   TTGGGTACTCCTCGCTAGGTTC
ElAV gag/pol ORF
   tctagaGAATTCGCCACC**ATG- EIAV gag/pol- UGA**ACCCGGGgcggccgc
**EX7 RARβ2 FWD:**
   **5'**ACTGccg.cgg GCC ACC ATG TTT GAC TGT ATG GAT GTT CTG TC**3'**
**EX7 RARβ2 FLAG FWD:**
   **5'** ACTGccg.cgg GCC ACC ATG GACTACAAGGACGACGATGACAAG TTT GAC TGT ATG GAT GTT CTG TC**3'**
**EX7 RARβ2 REV:**
   **5'** ACTGGCGGCCGCTCACTGCAGCAGTGGTG**3'**
EX7 EIAV cPPT POS:
   CAGGTTATTCTAGAGTCGACGCTCTCATTACTTGTAAC
EX7 EIA V cPPT NEG:
   CGAATGCGTTCTAGAGTCGACCATGTTCACCAGGGATTTTG
EX7 EIAV gag/pol ORF: tctagaGAATTCGCCACC**ATG- EIAV gag/pol-UGA**ACCCGGGgcggccgc
EX7 SD FOR
   GGCTAGAGAATTCCAGGTAAGATGGGCGATCCCCTCACCTGG
EX7 SD REV
   TTGGGTACTCCTCGCTAGGTTC
EX7 NEGATIVE SENSE PRIMER, FOR REVERSE TRANSCRIPTASE STEP
   5'-accagtagttaatttctgagacccttgta
EX7 PLUS SENSE PRIMER
   5' ATTGGGAGACCCTTTGACATT
EX7 PROBE
   5' FAM-CACCTTCTCTAACTTCTTGAGCGCCTTGCT-(TAMRA)p3'

Further sequences are as disclosed in the figures, such as figures 30-49, with reference to the Examples, particularly Examples 6 and 7.

## Claims

1. A lentiviral vector comprising a nucleic acid sequence encoding retinoic acid receptor β2 (RARβ2).

2. A vector according to claim 1, which comprises at least one component from an equine lentivirus.

3. A vector according to claim 2, wherein the equine lentivirus is EIAV.

4. A vector according to claim 3 wherein the retroviral vector is derived from EIAV.

5. A vector according to any preceding claim, comprising a deleted *gag* gene wherein the deletion in *gag* removes one or more nucleotides downstream of nucleotide 350 of the *gag* coding sequence.

6. A vector according to claim 5 wherein the deletion extends from nucleotide 350 to at least the C-terminus of the *gag-pol* coding region.

7. A vector according to claim 5 or claim 6 wherein the deletion additionally removes nucleotide 300 of the *gag* coding region.

8. A vector according to claim 5 wherein the deletion retains the first 150 nucleotides of the *gag* coding region.

9. A vector according to claim 5 wherein the deletion retains the first 109 nucleotides of the *gag* coding region.

10. A vector according to claim 5 wherein the deletion retains only the first 2 nucleotides of the *gag* coding region.

11. A vector according to any preceding claim, derived from a lentivirus genome wherein one or more accessory genes are absent from the lentivirus genome.

12. A vector according to claim 11 wherein the accessory genes are selected from *dUTPase, S2, rev* and *tat.*

13. A vector according to any preceding claim, derived from a lentivirus gemome which lacks the *tat* gene but includes the leader sequences between the end of the 5' LTR and the ATG of *gag.*

14. A delivery system in the form of a vector according to any preceding claim.

15. A method comprising transfecting or tranducing a cell *in vitro* with a vector according to any one of claims 1 to 13.

16. A cell transfected or transduced with a vector according to any one of claims 1 to 13.

17. Use of a vector according to any one of claims 1 to 13, in the preparation of a medicament to cause neurite development, outgrowth and/or regeneration.

18. Use of a vector according to any one of claims 1 to 13, in the preparation of a medicament for the treatment of a neurological disorder.

19. Use of a vector according to any one of claims 1 to 13, in the preparation of a medicament for the delivery of retinoic acid receptor β2 to a cell comprised by the peripheral or central nervous systems.

20. The use according to claim 19, for the delivery of retinoic acid receptor β2 to a mammalian neuronal cell.

21. The use according to claim 19, for the delivery of retinoic acid receptor β2 to an adult mammalian spinal cord cell.

22. The use according to any of claims 17 to 21, wherein the vector is used in conjunction with RARβ2 agonist.

23. The use according to claim 22, wherein the RARβ2 agonist is retinoic acid (RA) and/or CD2019.

24. A pharmaceutical composition comprising a vector according to any one of claims I to 13 in admixture with a pharmaceutically acceptable carrier, diluent or excipent.

25. A pharmaceutical composition according to claim 24 which also comprises an RARβ2 agonist for sequential or simultaneous administration.

26. A pharmaceutical composition according to claim 25, wherein the RARβ2 agonist is retinoic acid (RA) and/or CD2019.

27. A pharmaceutical composition according to any of claims 24 to 26, for use to cause neurite development, outgrowth and/or regeneration.

## Patentansprüche

1. Lentivirusvektor, umfassend eine Nukleinsäuresequenz, die Retinsäurerezeptor β2 (RARβ2) codiert.

2. Vektor nach Anspruch 1, welcher wenigstens eine Komponente eines Pferde-Lentivirus umfaßt.

3. Vektor nach Anspruch 2, wobei der Pferde-Lentivirus EIAV ist.

4. Vektor nach Anspruch 3, wobei der retrovirale Vektor von EIAV abgeleitet ist.

5. Vektor nach einem der vorangegangenen Ansprüche, welcher ein deletiertes *gag*-Gen umfaßt, wobei die Deletion in *gag* ein oder mehrere Nukleotide abstromig von Nukleotid 350 der *gag* codierenden Sequenz entfernt.

6. Vektor nach Anspruch 5, wobei die Deletion sich von Nukleotid 350 wenigstens bis zum C-Terminus der *gag-pol* codierenden Region erstreckt.

7. Vektor nach Anspruch 5 oder Anspruch 6, wobei die Deletion zusätzlich Nukleotid 300 der *gag* codierenden Region entfernt.

8. Vektor nach Anspruch 5, wobei die Deletion die ersten 150 Nukleotide der *gag* codierenden Region beibehält.

9. Vektor nach Anspruch 5, wobei die Deletion die ersten 109 Nukleotide der *gag* codierenden Region beibehält.

10. Vektor nach Anspruch 5, wobei die Deletion nur die ersten 2 Nukleotide der *gag* codierenden Region beibehält.

11. Vektor nach einem der vorangegangenen Ansprüche, abgeleitet von einem Lentivirus-Genom, wobei ein oder mehrere akzessorische Gene in dem Lentivirus-Genom fehlen.

12. Vektor nach Anspruch 11, wobei die akzessorischen Gene unter *dUTPase, S2, rev* und *tat* ausgewählt sind.

13. Vektor nach einem der vorangegangenen Ansprüche, abgeleitet von einem Lentivirus-Genom, dem das *tat*-Gen fehlt, welches jedoch die Leitsequenzen zwischen dem Ende der 5'-LTR und dem ATG von *gag* beinhaltet.

14. Zuführungssystem in Form eines Vektors nach einem der vorangegangenen Ansprüche.

15. Verfahren, welches das Transfizieren oder Transduzieren einer Zelle *in vitro* mit einem Vektor nach einem der Ansprüche 1 bis 13 umfaßt.

16. Zelle, transfiziert oder transduziert mit einem Vektor nach einem der Ansprüche 1 bis 13.

17. Verwendung eines Vektors nach einem der Ansprüche 1 bis 13 bei der Herstellung eines Medikaments zur Herbeiführung von Neuritenentwicklung, -wachstum und/oder -regeneration.

18. Verwendung eines Vektors nach einem der Ansprüche 1 bis 13 bei der Herstellung eines Medikaments zur Behandlung einer neurologischen Störung.

19. Verwendung eines Vektors nach einem der Ansprüche 1 bis 13 bei der Herstellung eines Medikaments für die Zuführung von Retinsäurerezeptor β2 zu einer Zelle, die im peripheren oder zentralen Nervensystem enthalten ist.

20. Verwendung nach Anspruch 19 für die Zuführung von Retinsäurerezeptor β2 zu einer neuronalen Säugerzelle.

21. Verwendung nach Anspruch 19 für die Zuführung von Retinsäurerezeptor β2 zu einer adulten Rückenmarkszelle eines Säugers.

22. Verwendung nach einem der Ansprüche 17 bis 21, wobei der Vektor zusammen mit einem RARβ2-Agonisten verwendet wird.

23. Verwendung nach Anspruch 22, wobei der RARβ2-Agonist Retinsäure (RA) und/oder CD2019 ist.

24. Pharmazeutische Zusammensetzung, welche einen Vektor nach einem der Ansprüche 1 bis 13 im Gemisch mit einem pharmazeutisch verträglichen Träger, Verdünnungsmittel oder Hilfsstoff umfaßt.

25. Pharmazeutische Zusammensetzung nach Anspruch 24, welche auch einen RARβ2-Agonisten für die sequentielle oder gleichzeitige Verabreichung umfaßt.

26. Pharmazeutische Zusammensetzung nach Anspruch 25, wobei der RARβ2-Agonist Retinsäure (RA) und/oder CD2019 ist.

27. Pharmazeutische Zusammensetzung nach einem der Ansprüche 24 bis 26 für die Verwendung zum Herbeiführen von Neuritenentwicklung, -wachstum und/oder -regeneration.

## Revendications

1. Vecteur lentiviral comprenant une séquence d'acide nucléique codant pour le récepteur d'acide rétinoïque β2 (RARβ2).

2. Vecteur suivant la revendication 1, qui comprend au moins un constituant provenant d'un lentivirus équin.

3. Vecteur suivant la revendication 2, dans lequel le lentivirus équin est le EIAV.

4. Vecteur suivant la revendication 3, dans lequel le vecteur rétroviral est dérivé du EIAV.

5. Vecteur suivant l'une quelconque des revendications précédentes, comprenant un gène *gag* présentant une délétion, dans lequel la délétion dans *gag* élimine un ou plusieurs nucléotides en aval du nucléotide 350 de la séquence codante *gag.*

6. Vecteur suivant la revendication 5, dans lequel la délétion s'étend du nucléotide 350 à au moins l'extrémité C-terminale de la région codante *gag-pol.*

7. Vecteur suivant la revendication 5 ou la revendication 6, dans lequel la délétion élimine en outre le nucléotide 300 de la région codante *gag.*

8. Vecteur suivant la revendication 5, dans lequel la délétion conserve les 150 premiers nucléotides de la région codante *gag.*

9. Vecteur suivant la revendication 5, dans lequel la délétion conserve les 109 premiers nucléotides de la région codante *gag.*

10. Vecteur suivant la revendication 5, dans lequel la délétion conserve seulement les deux premiers nucléotides de la région codante *gag.*

11. Vecteur suivant l'une quelconque des revendications précédentes, dérivé d'un génome de lentivirus dans lequel un ou plusieurs gènes accessoires sont absents du génome du lentivirus.

12. Vecteur suivant la revendication 11, dans lequel les gènes accessoires sont choisis entre les gènes *dUTPase, S2, rev* et *tat.*

13. Vecteur suivant l'une quelconque des revendications précédentes, dérivé d'un génome de lentivirus qui est dépourvu du gène *tat* mais qui comprend les séquence leaders entre l'extrémité de la LTR 5' et le ATG de *gag.*

14. Système d'administration sous forme d'un vecteur suivant l'une quelconque des revendications précédentes.

15. Procédé comprenant la transfection ou la transduction d'une cellule *in vitro* avec un vecteur suivant l'une quelconque des revendications 1 à 13.

16. Cellule transfectée ou transduite avec un vecteur suivant l'une quelconque des revendications 1 à 13.

17. Utilisation suivant d'un vecteur suivant l'une quelconque des revendications 1 à 13, dans la préparation d'un médicament destiné à provoquer le développement, l'extension et/ou la régénération d'un neurite.

18. Utilisation d'un vecteur suivant l'une quelconque des revendications 1 à 13, dans la préparation d'un médicament destiné au traitement d'un trouble neurologique.

19. Utilisation d'un vecteur suivant l'une quelconque des revendications 1 à 13, dans la préparation d'un médicament destiné à délivrer un récepteur d'acide rétinoïque β2 à une cellule faisant partie du système nerveux périphérique ou du système nerveux central.

20. Utilisation suivant la revendication 19, pour délivrer le récepteur d'acide rétinoïque β2 à une cellule neuronale de mammifère.

21. Utilisation suivant la revendication 19, pour délivrer le récepteur d'acide rétinoïque β2 à une cellule de la moelle épinière d'un mammifère adulte.

22. Utilisation suivant l'une quelconque des revendications 17 à 21, dans laquelle le vecteur est utilisé conjointement avec un agoniste de RARβ2.

23. Utilisation suivant la revendication 22, dans laquelle l'agoniste de RARβ2 est l'acide rétinoïque (RA) et/ou le CD2019.

24. Composition pharmaceutique comprenant un vecteur suivant l'une quelconque des revendications 1 à 13, en mélange avec un support, diluant ou excipient pharmaceutiquement acceptable.

25. Composition pharmaceutique suivant la revendication 24, qui comprend également un agoniste de RARβ2 pour une administration séquentielle ou simultanée.

26. Composition pharmaceutique suivant la revendication 25, dans laquelle l'agoniste de RARβ2 est l'acide rétinoïque (RA) et/ou le CD2019.

27. Composition pharmaceutique suivant l'une quelconque des revendications 24 à 26, destinée à être utilisée pour provoquer le développement, l'extension et/ou la régénération d'un neurite.


### SEQUENCE LISTING

<110> Oxford Biomedica (UK) Limited
   Kingsman, Alan J
   Maden, Malcolm
   Corcoran, Jonathan PT
<120> Factor
<130> P009156WOCTH
<140> PCT/GB01/01478
   <141> 2001-03-30
<150> PCT/GB00/01211
   <151> 2000-03-30
<150> GB 0024300.6
   <151> 2000-10-04
<160> 73
<170> PatentIn Ver. 2.1
<210> 1
   <211> 9
   <212> PRT
   <213> Human immunodeficiency virus type 1
<400> 1
   Arg Lys Lys Arg Arg Gln Arg Arg Arg
    1               5
<210> 2
   <211> 15
   <212> PRT
   <213> Human immunodeficiency virus type 1
<400> 2
   Cys Gly Arg Lys Lys Arg Arg Gln Arg Arg Arg Pro Pro Gln Cys
    1               5                  10                  15
<210> 3
   <211> 16
   <212> PRT
   <213> Drosophila sp.
<400> 3
   Arg Gln Ile Lys Ile Trp Phe Gln Asn Arg Arg Met Lys Trp Lys Lys
    1               5                  10                  15
<210> 4
   <211> 10998
   <212> DNA
   <213> Artificial Sequence
<220>
   <223> Description of Artificial Sequence: pONY8.OZ vector genome plasmid
<400> 4
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagaa ctagtggatc 1800
   ccccgggctg caggagtggg gaggcacgat ggccgctttg gtcgaggcgg atccggccat 1860
   tagccatatt attcattggt tatatagcat aaatcaatat tggctattgg ccattgcata 1920
   cgttgtatcc atatcataat atgtacattt atattggctc atgtccaaca ttaccgccat 1980
   gttgacattg attattgact agttattaat agtaatcaat tacggggtca ttagttcata 2040
   gcccatatat ggagttccgc gttacataac ttacggtaaa tggcccgcct ggctgaccgc 2100
   ccaacgaccc ccgcccattg acgtcaataa tgacgtatgt tcccatagta acgccaatag 2160
   ggactttcca ttgacgtcaa tgggtggagt atttacggta aactgcccac ttggcagtac 2220
   atcaagtgta tcatatgcca agtacgcccc ctattgacgt caatgacggt aaatggcccg 2280
   cctggcatta tgcccagtac atgaccttat gggactttcc tacttggcag tacatctacg 2340
   tattagtcat cgctattacc atggtgatgc ggttttggca gtacatcaat gggcgtggat 2400
   agcggtttga ctcacgggga tttccaagtc tccaccccat tgacgtcaat gggagtttgt 2460
   tttggcacca aaatcaacgg gactttccaa aatgtcgtaa caactccgcc ccattgacgc 2520
   aaatgggcgg taggcatgta cggtgggagg tctatataag cagagctcgt ttagtgaacc 2580
   gtcagatcgc ctggagacgc catccacgct gttttgacct ccatagaaga caccgggacc 2640
   gatccagcct ccgcggcccc aagcttcagc tgctcgagga tctgcggatc cggggaattc 2700
   cccagtctca ggatccacca tgggggatcc cgtcgtttta caacgtcgtg actgggaaaa 2760
   ccctggcgtt acccaactta atcgccttgc agcacatccc cctttcgcca gctggcgtaa 2820
   tagcgaagag gcccgcaccg atcgcccttc ccaacagttg cgcagcctga atggcgaatg 2880
   gcgctttgcc tggtttccgg caccagaagc ggtgccggaa agctggctgg agtgcgatct 2940
   tcctgaggcc gatactgtcg tcgtcccctc aaactggcag atgcacggtt acgatgcgcc 3000
   catctacacc aacgtaacct atcccattac ggtcaatccg ccgtttgttc ccacggagaa 3060
   tccgacgggt tgttactcgc tcacatttaa tgttgatgaa agctggctac aggaaggcca 3120
   gacgcgaatt atttttgatg gcgttaactc ggcgtttcat ctgtggtgca acgggcgctg 3180
   ggtcggttac ggccaggaca gtcgtttgcc gtctgaattt gacctgagcg catttttacg 3240
   cgccggagaa aaccgcctcg cggtgatggt gctgcgttgg agtgacggca gttatctgga 3300
   agatcaggat atgtggcgga tgagcggcat tttccgtgac gtctcgttgc tgcataaacc 3360
   gactacacaa atcagcgatt tccatgttgc cactcgcttt aatgatgatt tcagccgcgc 3420
   tgtactggag gctgaagttc agatgtgcgg cgagttgcgt gactacctac gggtaacagt 3480
   ttctttatgg cagggtgaaa cgcaggtcgc cagcggcacc gcgcctttcg gcggtgaaat 3540
   tatcgatgag cgtggtggtt atgccgatcg cgtcacacta cgtctgaacg tcgaaaaccc 3600
   gaaactgtgg agcgccgaaa tcccgaatct ctatcgtgcg gtggttgaac tgcacaccgc 3660
   cgacggcacg ctgattgaag cagaagcctg cgatgtcggt ttccgcgagg tgcggattga 3720
   aaatggtctg ctgctgctga acggcaagcc gttgctgatt cgaggcgtta accgtcacga 3780
   gcatcatcct ctgcatggtc aggtcatgga tgagcagacg atggtgcagg atatcctgct 3840
   gatgaagcag aacaacttta acgccgtgcg ctgttcgcat tatccgaacc atccgctgtg 3900
   gtacacgctg tgcgaccgct acggcctgta tgtggtggat gaagccaata ttgaaaccca 3960
   cggcatggtg ccaatgaatc gtctgaccga tgatccgcgc tggctaccgg cgatgagcga 4020
   acgcgtaacg cgaatggtgc agcgcgatcg taatcacccg agtgtgatca tctggtcgct 4080
   ggggaatgaa tcaggccacg gcgctaatca cgacgcgctg tatcgctgga tcaaatctgt 4140
   cgatccttcc cgcccggtgc agtatgaagg cggcggagcc gacaccacgg ccaccgatat 4200
   tatttgcccg atgtacgcgc gcgtggatga agaccagccc ttcccggctg tgccgaaatg 4260
   gtccatcaaa aaatggcttt cgctacctgg agagacgcgc ccgctgatcc tttgcgaata 4320
   cgcccacgcg atgggtaaca gtcttggcgg tttcgctaaa tactggcagg cgtttcgtca 4380
   gtatccccgt ttacagggcg gcttcgtctg ggactgggtg gatcagtcgc tgattaaata 4440
   tgatgaaaac ggcaacccgt ggtcggctta cggcggtgat tttggcgata cgccgaacga 4500
   tcgccagttc tgtatgaacg gtctggtctt tgccgaccgc acgccgcatc cagcgctgac 4560
   ggaagcaaaa caccagcagc agtttttcca gttccgttta tccgggcaaa ccatcgaagt 4620
   gaccagcgaa tacctgttcc gtcatagcga taacgagctc ctgcactgga tggtggcgct 4680
   ggatggtaag ccgctggcaa gcggtgaagt gcctctggat gtcgctccac aaggtaaaca 4740
   gttgattgaa ctgcctgaac taccgcagcc ggagagcgcc gggcaactct ggctcacagt 4800
   acgcgtagtg caaccgaacg cgaccgcatg gtcagaagcc gggcacatca gcgcctggca 4860
   gcagtggcgt ctggcggaaa acctcagtgt gacgctcccc gccgcgtccc acgccatccc 4920
   gcatctgacc accagcgaaa tggatttttg catcgagctg ggtaataagc gttggcaatt 4980
   taaccgccag tcaggctttc tttcacagat gtggattggc gataaaaaac aactgctgac 5040
   gccgctgcgc gatcagttca cccgtgcacc gctggataac gacattggcg taagtgaagc 5100
   gacccgcatt gaccctaacg cctgggtcga acgctggaag gcggcgggcc attaccaggc 5160
   cgaagcagcg ttgttgcagt gcacggcaga tacacttgct gatgcggtgc tgattacgac 5220
   cgctcacgcg tggcagcatc aggggaaaac cttatttatc agccggaaaa cctaccggat 5280
   tgatggtagt ggtcaaatgg cgattaccgt tgatgttgaa gtggcgagcg atacaccgca 5340
   tccggcgcgg attggcctga actgccagct ggcgcaggta gcagagcggg taaactggct 5400
   cggattaggg ccgcaagaaa actatcccga ccgccttact gccgcctgtt ttgaccgctg 5460
   ggatctgcca ttgtcagaca tgtatacccc gtacgtcttc ccgagcgaaa acggtctgcg 5520
   ctgcgggacg cgcgaattga attatggccc acaccagtgg cgcggcgact tccagttcaa 5580
   catcagccgc tacagtcaac agcaactgat ggaaaccagc catcgccatc tgctgcacgc 5640
   ggaagaaggc acatggctga atatcgacgg tttccatatg gggattggtg gcgacgactc 5700
   ctggagcccg tcagtatcgg cggaattcca gctgagcgcc ggtcgctacc attaccagtt 5760
   ggtctggtgt caaaaataat aataaccggg caggggggat ccgcagatcc ggctgtggaa 5820
   tgtgtgtcag ttagggtgtg gaaagtcccc aggctcccca gcaggcagaa gtatgcaaag 5880
   catgcctgca ggaattcgat atcaagctta tcgataccgt cgacctcgag ggggggcccg 5940
   gtacccagct tttgttccct ttagtgaggg ttaattgcgc gggaagtatt tatcactaat 6000
   caagcacaag taatacatga gaaactttta ctacagcaag cacaatcctc caaaaaattt 6060
   tgtttttaca aaatccctgg tgaacatgat tggaagggac ctactagggt gctgtggaag 6120
   ggtgatggtg cagtagtagt taatgatgaa ggaaagggaa taattgctgt accattaacc 6180
   aggactaagt tactaataaa accaaattga gtattgttgc aggaagcaag acccaactac 6240
   cattgtcagc tgtgtttcct gacctcaata tttgttataa ggtttgatat gaatcccagg 6300
   gggaatctca acccctatta cccaacagtc agaaaaatct aagtgtgagg agaacacaat 6360
   gtttcaacct tattgttata ataatgacag taagaacagc atggcagaat cgaaggaagc 6420
   aagagaccaa gaatgaacct gaaagaagaa tctaaagaag aaaaaagaag aaatgactgg 6480
   tggaaaatag gtatgtttct gttatgctta gcaggaacta ctggaggaat actttggtgg 6540
   tatgaaggac tcccacagca acattatata gggttggtgg cgataggggg aagattaaac 6600
   ggatctggcc aatcaaatgc tatagaatgc tggggttcct tcccggggtg tagaccattt 6660
   caaaattact tcagttatga gaccaataga agcatgcata tggataataa tactgctaca 6720
   ttattagaag ctttaaccaa tataactgct ctataaataa caaaacagaa ttagaaacat 6780
   ggaagttagt aaagacttct ggcataactc ctttacctat ttcttctgaa gctaacactg 6840
   gactaattag acataagaga gattttggta taagtgcaat agtggcagct attgtagccg 6900
   ctactgctat tgctgctagc gctactatgt cttatgttgc tctaactgag gttaacaaaa 6960
   taatggaagt acaaaatcat acttttgagg tagaaaatag tactctaaat ggtatggatt 7020
   taatagaacg acaaataaag atattatatg ctatgattct tcaaacacat gcagatgttc 7080
   aactgttaaa ggaaagacaa caggtagagg agacatttaa tttaattgga tgtatagaaa 7140
   gaacacatgt attttgtcat actggtcatc cctggaatat gtcatgggga catttaaatg 7200
   agtcaacaca atgggatgac tgggtaagca aaatggaaga tttaaatcaa gagatactaa 7260
   ctacacttca tggagccagg aacaatttgg cacaatccat gataacattc aatacaccag 7320
   atagtatagc tcaatttgga aaagaccttt ggagtcatat tggaaattgg attcctggat 7380
   tgggagcttc cattataaaa tatatagtga tgtttttgct tatttatttg ttactaacct 7440
   cttcgcctaa gatcctcagg gccctctgga aggtgaccag tggtgcaggg tcctccggca 7500
   gtcgttacct gaagaaaaaa ttccatcaca aacatgcatc gcgagaagac acctgggacc 7560
   aggcccaaca caacatacac ctagcaggcg tgaccggtgg atcaggggac aaatactaca 7620
   agcagaagta ctccaggaac gactggaatg gagaatcaga ggagtacaac aggcggccaa 7680
   agagctgggt gaagtcaatc gaggcatttg gagagagcta tatttccgag aagaccaaag 7740
   gggagatttc tcagcctggg gcggctatca acgagcacaa gaacggctct ggggggaaca 7800
   atcctcacca agggtcctta gacctggaga ttcgaagcga aggaggaaac atttatgact 7860
   gttgcattaa agcccaagaa ggaactctcg ctatcccttg ctgtggattt cccttatggc 7920
   tattttgggg actagtaatt atagtaggac gcatagcagg ctatggatta cgtggactcg 7980
   ctgttataat aaggatttgt attagaggct taaatttgat atttgaaata atcagaaaaa 8040
   tgcttgatta tattggaaga gctttaaatc ctggcacatc tcatgtatca atgcctcagt 8100
   atgtttagaa aaacaagggg ggaactgtgg ggtttttatg aggggtttta taaatgatta 8160
   taagagtaaa aagaaagttg ctgatgctct cataaccttg tataacccaa aggactagct 8220
   catgttgcta ggcaactaaa ccgcaataac cgcatttgtg acgcgagttc cccattggtg 8280
   acgcgttaac ttcctgtttt tacagtatat aagtgcttgt attctgacaa ttgggcactc 8340
   agattctgcg gtctgagtcc cttctctgct gggctgaaaa ggcctttgta ataaatataa 8400
   ttctctactc agtccctgtc tctagtttgt ctgttcgaga tcctacagag ctcatgcctt 8460
   ggcgtaatca tggtcatagc tgtttcctgt gtgaaattgt tatccgctca caattccaca 8520
   caacatacga gccggaagca taaagtgtaa agcctggggt gcctaatgag tgagctaact 8580
   cacattaatt gcgttgcgct cactgcccgc tttccagtcg ggaaacctgt cgtgccagct 8640
   gcattaatga atcggccaac gcgcggggag aggcggtttg cgtattgggc gctcttccgc 8700
   ttcctcgctc actgactcgc tgcgctcggt cgttcggctg cggcgagcgg tatcagctca 8760
   ctcaaaggcg gtaatacggt tatccacaga atcaggggat aacgcaggaa agaacatgtg 8820
   agcaaaaggc cagcaaaagg ccaggaaccg taaaaaggcc gcgttgctgg cgtttttcca 8880
   taggctccgc ccccctgacg agcatcacaa aaatcgacgc tcaagtcaga ggtggcgaaa 8940
   cccgacagga ctataaagat accaggcgtt tccccctgga agctccctcg tgcgctctcc 9000
   tgttccgacc ctgccgctta ccggatacct gtccgccttt ctcccttcgg gaagcgtggc 9060
   gctttctcat agctcacgct gtaggtatct cagttcggtg taggtcgttc gctccaagct 9120
   gggctgtgtg cacgaacccc ccgttcagcc cgaccgctgc gccttatccg gtaactatcg 9180
   tcttgagtcc aacccggtaa gacacgactt atcgccactg gcagcagcca ctggtaacag 9240
   gattagcaga gcgaggtatg taggcggtgc tacagagttc ttgaagtggt ggcctaacta 9300
   cggctacact agaaggacag tatttggtat ctgcgctctg ctgaagccag ttaccttcgg 9360
   aaaaagagtt ggtagctctt gatccggcaa acaaaccacc gctggtagcg gtggtttttt 9420
   tgtttgcaag cagcagatta cgcgcagaaa aaaaggatct caagaagatc ctttgatctt 9480
   ttctacgggg tctgacgctc agtggaacga aaactcacgt taagggattt tggtcatgag 9540
   attatcaaaa aggatcttca cctagatcct tttaaattaa aaatgaagtt ttaaatcaat 9600
   ctaaagtata tatgagtaaa cttggtctga cagttaccaa tgcttaatca gtgaggcacc 9660
   tatctcagcg atctgtctat ttcgttcatc catagttgcc tgactccccg tcgtgtagat 9720
   aactacgata cgggagggct taccatctgg ccccagtgct gcaatgatac cgcgagaccc 9780
   acgctcaccg gctccagatt tatcagcaat aaaccagcca gccggaaggg ccgagcgcag 9840
   aagtggtcct gcaactttat ccgcctccat ccagtctatt aattgttgcc gggaagctag 9900
   agtaagtagt tcgccagtta atagtttgcg caacgttgtt gccattgcta caggcatcgt 9960
   ggtgtcacgc tcgtcgtttg gtatggcttc attcagctcc ggttcccaac gatcaaggcg 10020
   agttacatga tcccccatgt tgtgcaaaaa agcggttagc tccttcggtc ctccgatcgt 10080
   tgtcagaagt aagttggccg cagtgttatc actcatggtt atggcagcac tgcataattc 10140
   tcttactgtc atgccatccg taagatgctt ttctgtgact ggtgagtact caaccaagtc 10200
   attctgagaa tagtgtatgc ggcgaccgag ttgctcttgc ccggcgtcaa tacgggataa 10260
   taccgcgcca catagcagaa ctttaaaagt gctcatcatt ggaaaacgtt cttcggggcg 10320
   aaaactctca aggatcttac cgctgttgag atccagttcg atgtaaccca ctcgtgcacc 10380
   caactgatct tcagcatctt ttactttcac cagcgtttct gggtgagcaa aaacaggaag 10440
   gcaaaatgcc gcaaaaaagg gaataagggc gacacggaaa tgttgaatac tcatactctt 10500
   cctttttcaa tattattgaa gcatttatca gggttattgt ctcatgagcg gatacatatt 10560
   tgaatgtatt tagaaaaata aacaaatagg ggttccgcgc acatttcccc gaaaagtgcc 10620
   acctaaattg taagcgttaa tattttgtta aaattcgcgt taaatttttg ttaaatcagc 10680
   tcatttttta accaataggc cgaaatcggc aaaatccctt ataaatcaaa agaatagacc 10740
   gagatagggt tgagtgttgt tccagtttgg aacaagagtc cactattaaa gaacgtggac 10800
   tccaacgtca aagggcgaaa aaccgtctat cagggcgatg gcccactacg tgaaccatca 10860
   ccctaatcaa gttttttggg gtcgaggtgc cgtaaagcac taaatcggaa ccctaaaggg 10920
   agcccccgat ttagagcttg acggggaaag ccaacctggc ttatcgaaat taatacgact 10980
   cactataggg agaccggc 10998
<210> 5
   <211> 12481
   <212> DNA
   <213> Artificial Sequence
<220>
   <223> Description of Artificial Sequence: pONY3.1, EIAV gag/pol expression plasmid
<400> 5
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgaggtcg acggtatcgc ccgaacaggg acctgagagg ggcgcagacc 1140
   ctacctgttg aacctggctg atcgtaggat ccccgggaca gcagaggaga acttacagaa 1200
   gtcttctgga ggtgttcctg gccagaacac aggaggacag gtaagatggg agaccctttg 1260
   acatggagca aggcgctcaa gaagttagag aaggtgacgg tacaagggtc tcagaaatta 1320
   actactggta actgtaattg ggcgctaagt ctagtagact tatttcatga taccaacttt 1380
   gtaaaagaaa aggactggca gctgagggat gtcattccat tgctggaaga tgtaactcag 1440
   acgctgtcag gacaagaaag agaggccttt gaaagaacat ggtgggcaat ttctgctgta 1500
   aagatgggcc tccagattaa taatgtagta gatggaaagg catcattcca gctcctaaga 1560
   gcgaaatatg aaaagaagac tgctaataaa aagcagtctg agccctctga agaatatcca 1620
   atcatgatag atggggctgg aaacagaaat tttagacctc taacacctag aggatatact 1680
   acttgggtga ataccataca gacaaatggt ctattaaatg aagctagtca aaacttattt 1740
   gggatattat cagtagactg tacttctgaa gaaatgaatg catttttgga tgtggtacct 1800
   ggccaggcag gacaaaagca gatattactt gatgcaattg ataagatagc agatgattgg 1860
   gataatagac atccattacc gaatgctcca ctggtggcac caccacaagg gcctattccc 1920
   atgacagcaa ggtttattag aggtttagga gtacctagag aaagacagat ggagcctgct 1980
   tttgatcagt ttaggcagac atatagacaa tggataatag aagccatgtc agaaggcatc 2040
   aaagtgatga ttggaaaacc taaagctcaa aatattaggc aaggagctaa ggaaccttac 2100
   ccagaatttg tagacagact attatcccaa ataaaaagtg agggacatcc acaagagatt 2160
   tcaaaattct tgactgatac actgactatt cagaacgcaa atgaggaatg tagaaatgct 2220
   atgagacatt taagaccaga ggatacatta gaagagaaaa tgtatgcttg cagagacatt 2280
   ggaactacaa aacaaaagat gatgttattg gcaaaagcac ttcagactgg tcttgcgggc 2340
   ccatttaaag gtggagcctt gaaaggaggg ccactaaagg cagcacaaac atgttataac 2400
   tgtgggaagc caggacattt atctagtcaa tgtagagcac ctaaagtctg ttttaaatgt 2460
   aaacagcctg gacatttctc aaagcaatgc agaagtgttc caaaaaacgg gaagcaaggg 2520
   gctcaaggga ggccccagaa acaaactttc ccgatacaac agaagagtca gcacaacaaa 2580
   tctgttgtac aagagactcc tcagactcaa aatctgtacc cagatctgag cgaaataaaa 2640
   aaggaataca atgtcaagga gaaggatcaa gtagaggatc tcaacctgga cagtttgtgg 2700
   gagtaacata taatctagag aaaaggccta ctacaatagt attaattaat gatactccct 2760
   taaatgtact gttagacaca ggagcagata cttcagtgtt gactactgca cattataata 2820
   ggttaaaata tagagggaga aaatatcaag ggacgggaat aataggagtg ggaggaaatg 2880
   tggaaacatt ttctacgcct gtgactataa agaaaaaggg tagacacatt aagacaagaa 2940
   tgctagtggc agatattcca gtgactattt tgggacgaga tattcttcag gacttaggtg 3000
   caaaattggt tttggcacag ctctccaagg aaataaaatt tagaaaaata gagttaaaag 3060
   agggcacaat ggggccaaaa attcctcaat ggccactcac taaggagaaa ctagaagggg 3120
   ccaaagagat agtccaaaga ctattgtcag agggaaaaat atcagaagct agtgacaata 3180
   atccttataa ttcacccata tttgtaataa aaaagaggtc tggcaaatgg aggttattac 3240
   aagatctgag agaattaaac aaaacagtac aagtaggaac ggaaatatcc agaggattgc 3300
   ctcacccggg aggattaatt aaatgtaaac acatgactgt attagatatt ggagatgcat 3360
   atttcactat acccttagat ccagagttta gaccatatac agctttcact attccctcca 3420
   ttaatcatca agaaccagat aaaagatatg tgtggaaatg tttaccacaa ggattcgtgt 3480
   tgagcccata tatatatcag aaaacattac aggaaatttt acaacctttt agggaaagat 3540
   atcctgaagt acaattgtat caatatatgg atgatttgtt catgggaagt aatggttcta 3600
   aaaaacaaca caaagagtta atcatagaat taagggcgat cttactggaa aagggttttg 3660
   agacaccaga tgataaatta caagaagtgc caccttatag ctggctaggt tatcaacttt 3720
   gtcctgaaaa ttggaaagta caaaaaatgc aattagacat ggtaaagaat ccaaccctta 3780
   atgatgtgca aaaattaatg gggaatataa catggatgag ctcagggatc ccagggttga 3840
   cagtaaaaca cattgcagct actactaagg gatgtttaga gttgaatcaa aaagtaattt 3900
   ggacggaaga ggcacaaaaa gagttagaag aaaataatga gaagattaaa aatgctcaag 3960
   ggttacaata ttataatcca gaagaagaaa tgttatgtga ggttgaaatt acaaaaaatt 4020
   atgaggcaac ttatgttata aaacaatcac aaggaatcct atgggcaggt aaaaagatta 4080
   tgaaggctaa taagggatgg tcaacagtaa aaaatttaat gttattgttg caacatgtgg 4140
   caacagaaag tattactaga gtaggaaaat gtccaacgtt taaggtacca tttaccaaag 4200
   agcaagtaat gtgggaaatg caaaaaggat ggtattattc ttggctccca gaaatagtat 4260
   atacacatca agtagttcat gatgattgga gaatgaaatt ggtagaagaa cctacatcag 4320
   gaataacaat atacactgat gggggaaaac aaaatggaga aggaatagca gcttatgtga 4380
   ccagtaatgg gagaactaaa cagaaaaggt taggacctgt cactcatcaa gttgctgaaa 4440
   gaatggcaat acaaatggca ttagaggata ccagagataa acaagtaaat atagtaactg 4500
   atagttatta ttgttggaaa aatattacag aaggattagg tttagaagga ccacaaagtc 4560
   cttggtggcc tataatacaa aatatacgag aaaaagagat agtttatttt gcttgggtac 4620
   ctggtcacaa agggatatat ggtaatcaat tggcagatga agccgcaaaa ataaaagaag 4680
   aaatcatgct agcataccaa ggcacacaaa ttaaagagaa aagagatgaa gatgcagggt 4740
   ttgacttatg tgttccttat gacatcatga tacctgtatc tgacacaaaa atcataccca 4800
   cagatgtaaa aattcaagtt cctcctaata gctttggatg ggtcactggg aaatcatcaa 4860
   tggcaaaaca ggggttatta attaatggag gaataattga tgaaggatat acaggagaaa 4920
   tacaagtgat atgtactaat attggaaaaa gtaatattaa attaatagag ggacaaaaat 4980
   ttgcacaatt aattatacta cagcatcact caaattccag acagccttgg gatgaaaata 5040
   aaatatctca gagaggggat aaaggatttg gaagtacagg agtattctgg gtagaaaata 5100
   ttcaggaagc acaagatgaa catgagaatt ggcatacatc accaaagata ttggcaagaa 5160
   attataagat accattgact gtagcaaaac agataactca agaatgtcct cattgcacta 5220
   agcaaggatc aggacctgca ggttgtgtca tgagatctcc taatcattgg caggcagatt 5280
   gcacacattt ggacaataag ataatattga cttttgtaga gtcaaattca ggatacatac 5340
   atgctacatt attgtcaaaa gaaaatgcat tatgtacttc attggctatt ttagaatggg 5400
   caagattgtt ttcaccaaag tccttacaca cagataacgg cactaatttt gtggcagaac 5460
   cagttgtaaa tttgttgaag ttcctaaaga tagcacatac cacaggaata ccatatcatc 5520
   cagaaagtca gggtattgta gaaagggcaa ataggacctt gaaagagaag attcaaagtc 5580
   atagagacaa cactcaaaca ctggaggcag ctttacaact tgctctcatt acttgtaaca 5640
   aagggaggga aagtatggga ggacagacac catgggaagt atttatcact aatcaagcac 5700
   aagtaataca tgagaaactt ttactacagc aagcacaatc ctccaaaaaa ttttgttttt 5760
   acaaaatccc tggtgaacat gattggaagg gacctactag ggtgctgtgg aagggtgatg 5820
   gtgcagtagt agttaatgat gaaggaaagg gaataattgc tgtaccatta accaggacta 5880
   agttactaat aaaaccaaat tgagtattgt tgcaggaagc aagacccaac taccattgtc 5940
   agctgtgttt cctgaggtct ctaggaattg attacctcga tgcttcatta aggaagaaga 6000
   ataaacaaag actgaaggca atccaacaag gaagacaacc tcaatatttg ttataaggtt 6060
   tgatatatgg gagtatttgg taaaggggta acatggtcag catcgcattc tatgggggaa 6120
   tcccaggggg aatctcaacc cctattaccc aacagtcaga aaaatctaag tgtgaggaga 6180
   acacaatgtt tcaaccttat tgttataata atgacagtaa gaacagcatg gcagaatcga 6240
   aggaagcaag agaccaagaa atgaacctga aagaagaatc taaagaagaa aaaagaagaa 6300
   atgactggtg gaaaataggt atgtttctgt tatgcttagc aggaactact ggaggaatac 6360
   tttggtggta tgaaggactc ccacagcaac attatatagg gttggtggcg atagggggaa 6420
   gattaaacgg atctggccaa tcaaatgcta tagaatgctg ggggttcctc ccggggtgta 6480
   gaccatttca aaattacttc agttatgaga ccaatagaag catgcatatg gataataata 6540
   ctgctacatt attagaagct ttaaccaata taactgctct ataaataaca aaacagaatt 6600
   agaaacatgg aagttagtaa agacttctgg cataactcct ttacctattt cttctgaagc 6660
   taacactgga ctaattagac ataagagaga ttttggtata agtgcaatag tggcagctat 6720
   tgtagccgct actgctattg ctgctagcgc tactatgtct tatgttgctc taactgaggt 6780
   taacaaaata atggaagtac aaaatcatac ttttgaggta gaaaatagta ctctaaatgg 6840
   tatggattta atagaacgac aaataaagat attatatgct atgattcttc aaacacatgc 6900
   agatgttcaa ctgttaaagg aaagacaaca ggtagaggag acatttaatt taattggatg 6960
   tatagaaaga acacatgtat tttgtcatac tggtcatccc tggaatatgt catggggaca 7020
   tttaaatgag tcaacacaat gggatgactg ggtaagcaaa atggaagatt taaatcaaga 7080
   gatactaact acacttcatg gagccaggaa caatttggca caatccatga taacattcaa 7140
   tacaccagat agtatagctc aatttggaaa agacctttgg agtcatattg gaaattggat 7200
   tcctggattg ggagcttcca ttataaaata tatagtgatg tttttgctta tttatttgtt 7260
   actaacctct tcgcctaaga tcctcagggc cctctggaag gtgaccagtg gtgcagggtc 7320
   ctccggcagt cgttacctga agaaaaaatt ccatcacaaa catgcatcgc gagaagacac 7380
   ctgggaccag gcccaacaca acatacacct agcaggcgtg accggtggat caggggacaa 7440
   atactacaag cagaagtact ccaggaacga ctggaatgga gaatcagagg agtacaacag 7500
   gcggccaaag agctgggtga agtcaatcga ggcatttgga gagagctata tttccgagaa 7560
   gaccaaaggg gagatttctc agcctggggc ggctatcaac gagcacaaga acggctctgg 7620
   ggggaacaat cctcaccaag ggtccttaga cctggagatt cgaagcgaag gaggaaacat 7680
   ttatgactgt tgcattaaag cccaagaagg aactctcgct atcccttgct gtggatttcc 7740
   cttatggcta ttttggggac tagtaattat agtaggacgc atagcaggct atggattacg 7800
   tggactcgct gttataataa ggatttgtat tagaggctta aatttgatat ttgaaataat 7860
   cagaaaaatg cttgattata ttggaagagc tttaaatcct ggcacatctc atgtatcaat 7920
   gcctcagtat gtttagaaaa acaagggggg aactgtgggg tttttatgag gggttttata 7980
   aatgattata agagtaaaaa gaaagttgct gatgctctca taaccttgta taacccaaag 8040
   gactagctca tgttgctagg caactaaacc gcaataaccg catttgtgac gcgagttccc 8100
   cattggtgac gcgtggtacc tctagagtcg acccgggcgg ccgcttccct ttagtgaggg 8160
   ttaatgcttc gagcagacat gataagatac attgatgagt ttggacaaac cacaactaga 8220
   atgcagtgaa aaaaatgctt tatttgtgaa atttgtgatg ctattgcttt atttgtaacc 8280
   attataagct gcaataaaca agttaacaac aacaattgca ttcattttat gtttcaggtt 8340
   cagggggaga tgtgggaggt tttttaaagc aagtaaaacc tctacaaatg tggtaaaatc 8400
   cgataaggat cgatccgggc tggcgtaata gcgaagaggc ccgcaccgat cgcccttccc 8460
   aacagttgcg cagcctgaat ggcgaatgga cgcgccctgt agcggcgcat taagcgcggc 8520
   gggtgtggtg gttacgcgca gcgtgaccgc tacacttgcc agcgccctag cgcccgctcc 8580
   tttcgctttc ttcccttcct ttctcgccac gttcgccggc tttccccgtc aagctctaaa 8640
   tcgggggctc cctttagggt tccgatttag agctttacgg cacctcgacc gcaaaaaact 8700
   tgatttgggt gatggttcac gtagtgggcc atcgccctga tagacggttt ttcgcccttt 8760
   gacgttggag tccacgttct ttaatagtgg actcttgttc caaactggaa caacactcaa 8820
   ccctatctcg gtctattctt ttgatttata agggattttg ccgatttcgg cctattggtt 8880
   aaaaaatgag ctgatttaac aaatatttaa cgcgaatttt aacaaaatat taacgtttac 8940
   aatttcgcct gatgcggtat tttctcctta cgcatctgtg cggtatttca caccgcatac 9000
   gcggatctgc gcagcaccat ggcctgaaat aacctctgaa agaggaactt ggttaggtac 9060
   cttctgaggc ggaaagaacc agctgtggaa tgtgtgtcag ttagggtgtg gaaagtcccc 9120
   aggctcccca gcaggcagaa gtatgcaaag catgcatctc aattagtcag caaccaggtg 9180
   tggaaagtcc ccaggctccc cagcaggcag aagtatgcaa agcatgcatc tcaattagtc 9240
   agcaaccata gtcccgcccc taactccgcc catcccgccc ctaactccgc ccagttccgc 9300
   ccattctccg ccccatggct gactaatttt ttttatttat gcagaggccg aggccgcctc 9360
   ggcctctgag ctattccaga agtagtgagg aggctttttt ggaggcctag gcttttgcaa 9420
   aaagcttgat tcttctgaca caacagtctc gaacttaagg ctagagccac catgattgaa 9480
   caagatggat tgcacgcagg ttctccggcc gcttgggtgg agaggctatt cggctatgac 9540
   tgggcacaac agacaatcgg ctgctctgat gccgccgtgt tccggctgtc agcgcagggg 9600
   cgcccggttc tttttgtcaa gaccgacctg tccggtgccc tgaatgaact gcaggacgag 9660
   gcagcgcggc tatcgtggct ggccacgacg ggcgttcctt gcgcagctgt gctcgacgtt 9720
   gtcactgaag cgggaaggga ctggctgcta ttgggcgaag tgccggggca ggatctcctg 9780
   tcatctcacc ttgctcctgc cgagaaagta tccatcatgg ctgatgcaat gcggcggctg 9840
   catacgcttg atccggctac ctgcccattc gaccaccaag cgaaacatcg catcgagcga 9900
   gcacgtactc ggatggaagc cggtcttgtc gatcaggatg atctggacga agagcatcag 9960
   gggctcgcgc cagccgaact gttcgccagg ctcaaggcgc gcatgcccga cggcgaggat 10020
   ctcgtcgtga cccatggcga tgcctgcttg ccgaatatca tggtggaaaa tggccgcttt 10080
   tctggattca tcgactgtgg ccggctgggt gtggcggacc gctatcagga catagcgttg 10140
   gctacccgtg atattgctga agagcttggc ggcgaatggg ctgaccgctt cctcgtgctt 10200
   tacggtatcg ccgctcccga ttcgcagcgc atcgccttct atcgccttct tgacgagttc 10260
   ttctgagcgg gactctgggg ttcgaaatga ccgaccaagc gacgcccaac ctgccatcac 10320
   gatggccgca ataaaatatc tttattttca ttacatctgt gtgttggttt tttgtgtgaa 10380
   tcgatagcga taaggatccg cgtatggtgc actctcagta caatctgctc tgatgccgca 10440
   tagttaagcc agccccgaca cccgccaaca cccgctgacg cgccctgacg ggcttgtctg 10500
   ctcccggcat ccgcttacag acaagctgtg accgtctccg ggagctgcat gtgtcagagg 10560
   ttttcaccgt catcaccgaa acgcgcgaga cgaaagggcc tcgtgatacg cctattttta 10620
   taggttaatg tcatgataat aatggtttct tagacgtcag gtggcacttt tcggggaaat 10680
   gtgcgcggaa cccctatttg tttatttttc taaatacatt caaatatgta tccgctcatg 10740
   agacaataac cctgataaat gcttcaataa tattgaaaaa ggaagagtat gagtattcaa 10800
   catttccgtg tcgcccttat tccctttttt gcggcatttt gccttcctgt ttttgctcac 10860
   ccagaaacgc tggtgaaagt aaaagatgct gaagatcagt tgggtgcacg agtgggttac 10920
   atcgaactgg atctcaacag cggtaagatc cttgagagtt ttcgccccga agaacgtttt 10980
   ccaatgatga gcacttttaa agttctgcta tgtggcgcgg tattatcccg tattgacgcc 11040
   gggcaagagc aactcggtcg ccgcatacac tattctcaga atgacttggt tgagtactca 11100
   ccagtcacag aaaagcatct tacggatggc atgacagtaa gagaattatg cagtgctgcc 11160
   ataaccatga gtgataacac tgcggccaac ttacttctga caacgatcgg aggaccgaag 11220
   gagctaaccg cttttttgca caacatgggg gatcatgtaa ctcgccttga tcgttgggaa 11280
   ccggagctga atgaagccat accaaacgac gagcgtgaca ccacgatgcc tgtagcaatg 11340
   gcaacaacgt tgcgcaaact attaactggc gaactactta ctctagcttc ccggcaacaa 11400
   ttaatagact ggatggaggc ggataaagtt gcaggaccac ttctgcgctc ggcccttccg 11460
   gctggctggt ttattgctga taaatctgga gccggtgagc gtgggtctcg cggtatcatt 11520
   gcagcactgg ggccagatgg taagccctcc cgtatcgtag ttatctacac gacggggagt 11580
   caggcaacta tggatgaacg aaatagacag atcgctgaga taggtgcctc actgattaag 11640
   cattggtaac tgtcagacca agtttactca tatatacttt agattgattt aaaacttcat 11700
   ttttaattta aaaggatcta ggtgaagatc ctttttgata atctcatgac caaaatccct 11760
   taacgtgagt tttcgttcca ctgagcgtca gaccccgtag aaaagatcaa aggatcttct 11820
   tgagatcctt tttttctgcg cgtaatctgc tgcttgcaaa caaaaaaacc accgctacca 11880
   gcggtggttt gtttgccgga tcaagagcta ccaactcttt ttccgaaggt aactggcttc 11940
   agcagagcgc agataccaaa tactgtcctt ctagtgtagc cgtagttagg ccaccacttc 12000
   aagaactctg tagcaccgcc tacatacctc gctctgctaa tcctgttacc agtggctgct 12060
   gccagtggcg ataagtcgtg tcttaccggg ttggactcaa gacgatagtt accggataag 12120
   gcgcagcggt cgggctgaac ggggggttcg tgcacacagc ccagcttgga gcgaacgacc 12180
   tacaccgaac tgagatacct acagcgtgag ctatgagaaa gcgccacgct tcccgaaggg 12240
   agaaaggcgg acaggtatcc ggtaagcggc agggtcggaa caggagagcg cacgagggag 12300
   cttccagggg gaaacgcctg gtatctttat agtcctgtcg ggtttcgcca cctctgactt 12360
   gagcgtcgat ttttgtgatg ctcgtcaggg gggcggagcc tatggaaaaa cgccagcaac 12420
   gcggcctttt tacggttcct ggccttttgc tggccttttg ctcacatggc tcgacagatc 12480
   t 12481
<210> 6
   <211> 6845
   <212> DNA
   <213> Artificial Sequence
<220>
   <223> Description of Artificial Sequence: pRV67, VSV-G expression plasmid
<400> 6
   tcgacctgca ggatatcgaa ttcattgatc ataatcagcc ataccacatt tgtagaggtt 60
   ttacttgctt taaaaaacct cccacacctc cccctgaacc tgaaacataa aatgaatgca 120
   attgttgttg ttaacttgtt tattgcagct tataatggtt acaaataaag caatagcatc 180
   acaaatttca caaataaagc atttttttca ctgcattcta gttgtggttt gtccaaactc 240
   atcaatgtat cttatcatgt ctggatccgt accgagctcg cgtaatcatg tcatagctgt 300
   ttcctgtgtg aaattgttat ccgctcacaa ttccacacaa catacgagcc ggaagcataa 360
   agtgtaaagc ctggggtgcc taatgagtga gctaactaca ttaattgcgt tgcgctcack 420
   gcccgctttc cartcgggaa acctgtcgtg ccagctgcat taatgaatcg gccaacgcgc 480
   ggggagaggc ggtttgcgta ttgggcgctc ttccgcttcc tcgctcactg actcgctgcg 540
   ctcggtcgtt cggctgcggc gagcggtatc agctcactca aaggcggtaa tacggttatc 600
   cacagaatca ggggataacg caggaaagaa catgtgagca aaaggccagc aaaaggccag 660
   gaaccgtaaa aaggccgcgt tgctggcgtt tttccatagg ctccgccccc ctgacgagca 720
   tcacaaaaat cgacgctcaa gtcagaggtg gcgaaacccg acaggactat aaagatacca 780
   ggcgtttccc cctggaagct ccctcgtgcg ctctcctgtt ccgaccctgc cgcttaccgg 840
   atacctgtcc gcctttctcc cttcgggaag cgtggcgctt tctcatagct cacgctgtag 900
   gtatctcagt tcggtgtagg tcgttcgctc caagctgggc tgtgtgcacg aaccccccgt 960
   tcagcccgac cgctgcgcct tatccggtaa ctatcgtctt gagtccaacc cggtaagaca 1020
   cgacttatcg ccactggcag cagccactgg taacaggatt agcagagcga ggtatgtagg 1080
   cggtgctaca gagttcttga agtggtggcc taactacggc tacmctagaa gracagtatt 1140
   tggkatctgs gcttctgytg aagmcagtta ccttcggaaa aagagttggt agctcttgat 1200
   ccggcaaaca aaccaccgct ggtagcggtg gtttttttgt ttgcaagcag cagattacgc 1260
   gcagaaaaaa aggatctcaa gaagatcctt tgatcttttc tacggggtct gacgctcagt 1320
   ggaacgaaaa ctcacgttaa gggattttgg tcatgagatt atcaaaaagg atcttcacct 1380
   agatcctttt aaattaaaaa tgaagtttta aatcaatcta aagtatatat gagtaaactt 1440
   ggtctgacag ttaccaatgc ttaatcagtg aggcacctat ctcagcgatc tgtctatttc 1500
   gttcatccat agttgcctga ctccccgtcg tgtagataac tacgatacgg gagggcttac 1560
   catctggccc cagtgctgca atgataccgc gagacccacg ctcaccggct ccagatttat 1620
   cagcaataaa ccagccagcc ggaagggccg agcgcagaag tggtcctgca actttatccg 1680
   cctccatcca gtctattaat tgttgccggg aagctagagt aagtagttcg ccagttaata 1740
   gtttgcgcaa cgttgttgcc attgctacag gcatcgtggt gtcacgctcg tcgtttggta 1800
   tggcttcatt cagctccggt tcccaacgat caaggcgagt tacatgatcc cccatgttgt 1860
   gcaaaaaagc ggttagctcc ttcggtcctc cgatcgttgt cagaagtaag ttggccgcag 1920
   tgttatcact catggttatg gcagcactgc ataattctct tactgtcatg ccatccgtaa 1980
   gatgcttttc tgtgactggt gagtactcaa ccaagtcatt ctgagaatag tgtatgcggc 2040
   gaccgagttg ctcttgcccg gcgtcaatac gggataatac cgcgccacat agcagaactt 2100
   taaaagtgct catcattgga aaacgttctt cggggcgaaa actctcaagg atcttaccgc 2160
   tgttgagatc cagttcgatg taacccactc gtgcacccaa ctgatcttca gcatctttta 2220
   ctttcaccag cgtttctggg tgagcaaaaa caggaaggca aaatgccgca aaaaagggaa 2280
   taagggcgac acggaaatgt tgaatactca tactcttcct ttttcaataa gcggccgcgg 2340
   ccatgccggc cactagtctc gagttattat tgaagcattt atcagggtta ttgtctcatg 2400
   agcggataca tatttgaatg tatttagaaa aataaacaaa taggggttcc gcgcacattt 2460
   ccccgaaaag tgccacctga cgtctaagaa accattatta tcatgacatt aacctataaa 2520
   aataggcgta tcacgaggcc ctttcgtctc gcgcgtttcg gtgatgacgg tgaaaacctc 2580
   tgacacatgc agctcccgga gacggtcaca gcttgtctgt aagcggatgc cgggagcaga 2640
   caagcccgtc agggcgcgtc agcgggtgtt ggcgggtgtc ggggctggct taactatgcg 2700
   gcatcagagc agattgtact gagagtgcac catatgaaga cgtcgcctcc tcactacttc 2760
   tggaatagct cagaggccga ggcggcctcg gcctctgcat aaataaaaaa aatwaktcas 2820
   ggcgccattc gccattcagg ctgcgcaact gttgggaagg gcgatcggtg cgggcctctt 2880
   cgctattacg ccagctggcg aaagggggat gtgctgcaag gcgattaagt tgggtaacgc 2940
   cagggttttc ccagtcacga cgttgtaaaa cgacggccag tgccatcgtg tcaaaggaca 3000
   gtgactgcag tgaataataa aatgtgtgtt tgtccgaaat acgcgttttg agawttctgt 3060
   cgccgactaa attcatgtcg cgcgatartg gtgtttatcg ccgatagaga tggcgatatt 3120
   ggaaaaatcg atatttgaaa atatggcata ttgaaaatgt cgccgatgtg agtttctgtg 3180
   taactgatat cgccattttt ccaaaagttg atttttgggc atacgcgata tctggcgata 3240
   cgcttatatc gtttacgggg gatggcgata gacgcctttg gtgacttggg cgattctgtg 3300
   tgtcgcaaat atcgcagttt cgatataggt gacagacgat atgaggctat atcgccgata 3360
   gaggcgacat caagctggca catggccaat gcatatcgat ctatacattg aatcaatatt 3420
   ggccattagc catattattc attggttata tagcataaat caatattggc tattggccat 3480
   tgcatacgtt gtatccatat cataatatgt acatttatat tggctcatgt ccaacattac 3540
   cgccatgttg acattgatta ttgactagtt attaatagta atcaattacg gggtcattag 3600
   ttcatagccc atatatggag ttccgcgtta cataacttac ggtaaatggc ccgcctggct 3660
   gaccgcccaa cgacccccgc ccattgacgt caataatgac gtatgttccc atagtaacgc 3720
   caatagggac tttccattga cgtcaatggg tggagtattt acggtaaact gcccacttgg 3780
   cagtacatca agtgtatcat atgccaagta cgccccctat tgacgtcaat gacggtaaat 3840
   ggcccgcctg gcattatgcc cagtacatga ccttatggga ctttcctact tggcagtaca 3900
   tctacgtatt agtcatcgct attaccatgg tgatgcggtt ttggcagtac atcaatgggc 3960
   gtggatagcg gtttgactca cggggatttc caagtctcca ccccattgac gtcaatggga 4020
   gtttgttttg gcaccaaaat caacgggact ttccaaaatg tcgtaacaac tccgccccat 4080
   tgacgcaaat gggcggtagg cgtgtacggt gggaggtcta tataagcaga gctcgtttag 4140
   tgaaccgtca gatcgcctgg agacgccatc cacgctgttt tgacctccat agaagacacc 4200
   gggaccgatc cagcctccgc ggccgggaac ggtgcattgg aacgcggatt ccccgtgcca 4260
   agagtgacgt aagtaccgcc tatagagtct ataggcccac ccccttggct tcttatgcat 4320
   gctatactgt ttttggcttg gggtctatac acccccgctt cctcatgtta taggtgatgg 4380
   tatagcttag cctataggtg tgggttattg accattattg accactcccc tattggtgac 4440
   gatactttcc attactaatc cataacatgg ctctttgcac aactctcttt attggctata 4500
   tgccaataca ctgtccttca gagactgaca cggactctgt atttttacag gatggggtct 4560
   catttattat ttacaaattc acatatacaa caccaccgtc cccagtgccc gcagttttta 4620
   ttaaacataa cgtgggatct ccagcgaatc tcgggtacgt gttccggaca tggggctctt 4680
   ctccggtagc ggsggagytt ctacatccgr rsccttgytc ccatgcctcc asgrmttcat 4740
   gktcgytcgg cagctccttg ctcctaamca gtggaggcca gacttaggca cagcacgatg 4800
   cccaccacca ccagtgtgcc gcacaaggcc gtggcggtag ggtatgtgtc tgaaaatgag 4860
   ctcggggagc gggcttgcac cgctggacgc atttggaaga cttaaggcag cggcagaaga 4920
   agatgcaggc agctgagttg ttgtgttctg ataagagtca gaggtaactc ccgttgcggt 4980
   gctgttaacg gtggagggca gtgtagtctg agcagtactc gttgctgccg cgcgcgccac 5040
   cagacataat agctgacaga ctaacagact gttcctttcc atgggtcttt tctgcagtca 5100
   ccgtccttga cacgaagctt cccgggatag gtacctcgcg agatccctcg aggaggaatt 5160
   ctgacactat gaagtgcctt ttgtacttag cctttttatt cattggggtg aattgcaagt 5220
   tcaccatagt ttttccacac aaccaaaaag gaaactggaa aaatgttcct tctaattacc 5280
   attattgccc gtcaagctca gatttaaatt ggcataatga cttaataggc acagccttac 5340
   aagtcaaaat gcccaagagt cacaaggcta ttcaagcaga cggttggatg tgtcatgctt 5400
   ccaaatgggt cactacttgt gatttccgct ggtatggacc gaagtatata acacattcca 5460
   tccgatcctt cactccatct gtagaacaat gcaaggaaag cattgaacaa acgaaacaag 5520
   gaacttggct gaatccaggc ttccctcctc aaagttgtgg atatgcaact gtgacggatg 5580
   ccgaagcagt gattgtccag gtgactcctc accatgtgct ggttgatgaa tacacaggag 5640
   aatgggttga ttcacagttc atcaacggaa aatgcagcaa ttacatatgc cccactgtcc 5700
   ataactctac aacctggcat tctgactata aggtcaaagg gctatgtgat tctaacctca 5760
   tttccatgga catcaccttc ttctcagagg acggagagct atcatccctg ggaaaggagg 5820
   gcacagggtt cagaagtaac tactttgctt atgaaactgg aggcaaggcc tgcaaaatgc 5880
   aatactgcaa gcattgggga gtcagactcc catcaggtgt ctggttcgag atggctgata 5940
   aggatctctt tgctgcagcc agattccctg aatgcccaga agggtcaagt atctctgctc 6000
   catctcagac ctcagtggat gtaagtctaa ttcaggacgt tgagaggatc ttggattatt 6060
   ccctctgcca agaaacctgg agcaaaatca gagcgggtct tccaatctct ccagtggatc 6120
   tcagctatct tgctcctaaa aacccaggaa ccggtcctgc tttcaccata atcaatgggg 6180
   gcctaaaata ctttgagacc agatacatca gagtcgatat tgctgctcca atcctctcaa 6240
   gaatggtcgg aatgatcagt ggaactacca cagaaaggga actgtgggat gactgggcac 6300
   catatgaaga cgtggaaatt ggacccaatg gagttctgag gaccagttca ggatataagt 6360
   ttcctttata catgattgga catggtatgt tggactccga tcttcatctt agctcaaagg 6420
   ctcaggtgtt cgaacatcct cacattcaag acgctgcttc gcaacttcct gatgatgaga 6480
   gtttattttt tggtgatact gggctatcca aaaatccaat cgagcttgta gaaggttggt 6540
   tcagtagttg gaaaagctct attgcctctt ttttctttat catagggtta atcattggac 6600
   tattcttggt tctccgagtt ggtatccatc tttgcattaa attaaagcac accaagaaaa 6660
   gacagattta tacagacata gagatgaacc gacttggaaa gtaactcaaa tcctgcacaa 6720
   cagattcttc atgtttggac caaatcaact tgtgatacca tgctcaaaga ggcctcaatt 6780
   atatttgagt ttttaatttt tatgaaaaaa aaaaaaaaaa acggaattcc tcgagggatc 6840
   tagag 6845
<210> 7
   <211> 1375
   <212> DNA
   <213> Artificial Sequence
<220>
   <223> Description of Artificial Sequence: RARbeta2 PCR product
<400> 7
   actgccgcgg gccaccatgt ttgactgtat ggatgttctg tcagtgagtc ccgggcagat 60
   cctggatttc tacaccgcga gcccttcctc ctgcatgctg caggaaaagg ctctcaaagc 120
   ctgcctcagt ggattcaccc aggccgaatg gcagcaccgg catactgctc aatccatcga 180
   gacacagagt accagctctg aggagctcgt cccgagccca ccatctccac ttcctcctcc 240
   tcgggtgtac aagccctgct tcgtttgcca ggacaagtca tcgggctacc actatggcgt 300
   cagtgcctgc gaggggtgca agggcttttt ccgcagaagt attcagaaga acatgatcta 360
   cacttgccat cgagataaga actgcgtcat taacaaggtc actaggaacc gatgccagta 420
   ctgccgcctg cagaagtgct ttgaagtggg catgtccaaa gagtctgtta ggaatgacag 480
   gaacaagaaa aagaaggagc cttcaaagca ggaatgcaca gagagctatg agatgacagc 540
   ggagctagac gacctcactg agaagatccg gaaagcccac caggaaacct ttccctcact 600
   ctgccagctg ggtaaataca ccacgaattc cagcgctgac caccgggtcc gattggactt 660
   gggcctctgg gacaaattca gtgagctggc caccaagtgc attattaaga tcgtggagtt 720
   cgccaagcgt ctgccgggct tcacaggtct gaccatcgca gaccagatca ccctgctcaa 780
   agccgcctgc ttggatatct tgattctcag aatttgtacc aggtataccc cagagcaaga 840
   caccatgact ttctctgatg gccttacact aaatcgaact cagatgcaca atgctggctt 900
   cggtcctctg actgaccttg tgttcacctt tgccaaccag ctcctgcctt tggaaatgga 960
   tgacacagaa acaggccttc tcagtgccat ctgtttaatc tgtggagacc gccaggacct 1020
   tgaggaacca acaaaagtag acaagctcca agaaccactg ctggaagcac taaagattta 1080
   cattagaaaa cgacgaccca gcaagcctca catgtttcca aagatcttaa tgaaaatcac 1140
   agatctccgc agcatcagcg cgaaaggtgc cgaacgtgta attaccttga aaatggaaat 1200
   tcctggatca atgccacctc tcattcagga aatgctggag aattctgaag gacatgaacc 1260
   cttgacccca agttcaagtg ggaatatagc agagcacagt cccagcgtgt cccccagctc 1320
   agtggagaac agtggagtca gtcagtcacc actgctgcag tgagcggccg ccagt      1375
<210> 8
   <211> 1399
   <212> DNA
   <213> Artificial Sequence
<220>
   <223> Description of Artificial Sequence: FLAG RARbeta2 PCR product
<400> 8
   actgccgcgg gccaccatgg actacaagga cgacgatgac aagtttgact gtatggatgt 60
   tctgtcagtg agtcccgggc agatcctgga tttctacacc gcgagccctt cctcctgcat 120
   gctgcaggaa aaggctctca aagcctgcct cagtggattc acccaggccg aatggcagca 180
   ccggcatact gctcaatcca tcgagacaca gagtaccagc tctgaggagc tcgtcccgag 240
   cccaccatct ccacttcctc ctcctcgggt gtacaagccc tgcttcgttt gccaggacaa 300
   gtcatcgggc taccactatg gcgtcagtgc ctgcgagggg tgcaagggct ttttccgcag 360
   aagtattcag aagaacatga tctacacttg ccatcgagat aagaactgcg tcattaacaa 420
   ggtcactagg aaccgatgcc agtactgccg cctgcagaag tgctttgaag tgggcatgtc 480
   caaagagtct gttaggaatg acaggaacaa gaaaaagaag gagccttcaa agcaggaatg 540
   cacagagagc tatgagatga cagcggagct agacgacctc actgagaaga tccggaaagc 600
   ccaccaggaa acctttccct cactctgcca gctgggtaaa tacaccacga attccagcgc 660
   tgaccaccgg gtccgattgg acttgggcct ctgggacaaa ttcagtgagc tggccaccaa 720
   gtgcattatt aagatcgtgg agttcgccaa gcgtctgccg ggcttcacag gtctgaccat 780
   cgcagaccag atcaccctgc tcaaagccgc ctgcttggat atcttgattc tcagaatttg 840
   taccaggtat accccagagc aagacaccat gactttctct gatggcctta cactaaatcg 900
   aactcagatg cacaatgctg gcttcggtcc tctgactgac cttgtgttca cctttgccaa 960
   ccagctcctg cctttggaaa tggatgacac agaaacaggc cttctcagtg ccatctgttt 1020
   aatctgtgga gaccgccagg accttgagga accaacaaaa gtagacaagc tccaagaacc 1080
   actgctggaa gcactaaaga tttacattag aaaacgacga cccagcaagc ctcacatgtt 1140
   tccaaagatc ttaatgaaaa tcacagatct ccgcagcatc agcgcgaaag gtgccgaacg 1200
   tgtaattacc ttgaaaatgg aaattcctgg atcaatgcca cctctcattc aggaaatgct 1260
   ggagaattct gaaggacatg aacccttgac cccaagttca agtgggaata tagcagagca 1320
   cagtcccagc gtgtccccca gctcagtgga gaacagtgga gtcagtcagt caccactgct 1380
   gcagtgagcg gccgccagt                                              1399
<210> 9
   <211> 9127
   <212> DNA
   <213> Artificial Sequence
<220>
   <223> Description of Artificial Sequence: pONY-RARbeta2 vector genome plasmid
<400> 9
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagag tcgacgctct 1800
   cattacttgt aacaaaggga gggaaagtat gggaggacag acaccatggg aagtatttat 1860
   cactaatcaa gcacaagtaa tacatgagaa acttttacta cagcaagcac aatcctccaa 1920
   aaaattttgt ttttacaaaa tccctggtga acatggtcga ctctagaact agtggatccc 1980
   ccgggctgca ggagtgggga ggcacgatgg ccgctttggt cgaggcggat ccggccatta 2040
   gccatattat tcattggtta tatagcataa atcaatattg gctattggcc attgcatacg 2100
   ttgtatccat atcataatat gtacatttat attggctcat gtccaacatt accgccatgt 2160
   tgacattgat tattgactag ttattaatag taatcaatta cggggtcatt agttcatagc 2220
   ccatatatgg agttccgcgt tacataactt acggtaaatg gcccgcctgg ctgaccgccc 2280
   aacgaccccc gcccattgac gtcaataatg acgtatgttc ccatagtaac gccaataggg 2340
   actttccatt gacgtcaatg ggtggagtat ttacggtaaa ctgcccactt ggcagtacat 2400
   caagtgtatc atatgccaag tacgccccct attgacgtca atgacggtaa atggcccgcc 2460
   tggcattatg cccagtacat gaccttatgg gactttccta cttggcagta catctacgta 2520
   ttagtcatcg ctattaccat ggtgatgcgg ttttggcagt acatcaatgg gcgtggatag 2580
   cggtttgact cacggggatt tccaagtctc caccccattg acgtcaatgg gagtttgttt 2640
   tggcaccaaa atcaacggga ctttccaaaa tgtcgtaaca actccgcccc attgacgcaa 2700
   atgggcggta ggcatgtacg gtgggaggtc tatataagca gagctcgttt agtgaaccgt 2760
   cagatcgcct ggagacgcca tccacgctgt tttgacctcc atagaagaca ccgggaccga 2820
   tccagcctcc gcgggccacc atgtttgact gtatggatgt tctgtcagtg agtcccgggc 2880
   agatcctgga tttctacacc gcgagccctt cctcctgcat gctgcaggaa aaggctctca 2940
   aagcctgcct cagtggattc acccaggccg aatggcagca ccggcatact gctcaatcca 3000
   tcgagacaca gagtaccagc tctgaggagc tcgtcccgag cccaccatct ccacttcctc 3060
   ctcctcgggt gtacaagccc tgcttcgttt gccaggacaa gtcatcgggc taccactatg 3120
   gcgtcagtgc ctgcgagggg tgcaagggct ttttccgcag aagtattcag aagaacatga 3180
   tctacacttg ccatcgagat aagaactgcg tcattaacaa ggtcactagg aaccgatgcc 3240
   agtactgccg cctgcagaag tgctttgaag tgggcatgtc caaagagtct gttaggaatg 3300
   acaggaacaa gaaaaagaag gagccttcaa agcaggaatg cacagagagc tatgagatga 3360
   cagcggagct agacgacctc actgagaaga tccggaaagc ccaccaggaa acctttccct 3420
   cactctgcca gctgggtaaa tacaccacga attccagcgc tgaccaccgg gtccgattgg 3480
   acttgggcct ctgggacaaa ttcagtgagc tggccaccaa gtgcattatt aagatcgtgg 3540
   agttcgccaa gcgtctgccg ggcttcacag gtctgaccat cgcagaccag atcaccctgc 3600
   tcaaagccgc ctgcttggat atcttgattc tcagaatttg taccaggtat accccagagc 3660
   aagacaccat gactttctct gatggcctta cactaaatcg aactcagatg cacaatgctg 3720
   gcttcggtcc tctgactgac cttgtgttca cctttgccaa ccagctcctg cctttggaaa 3780
   tggatgacac agaaacaggc cttctcagtg ccatctgttt aatctgtgga gaccgccagg 3840
   accttgagga accaacaaaa gtagacaagc tccaagaacc actgctggaa gcactaaaga 3900
   tttacattag aaaacgacga cccagcaagc ctcacatgtt tccaaagatc ttaatgaaaa 3960
   tcacagatct ccgcagcatc agcgcgaaag gtgccgaacg tgtaattacc ttgaaaatgg 4020
   aaattcctgg atcaatgcca cctctcattc aggaaatgct ggagaattct gaaggacatg 4080
   aacccttgac cccaagttca agtgggaata tagcagagca cagtcccagc gtgtccccca 4140
   gctcagtgga gaacagtgga gtcagtcagt caccactgct gcagtgagcg gccgcgactc 4200
   tagagtcgac ctcgaggggg ggcccggacc tactagggtg ctgtggaagg gtgatggtgc 4260
   agtagtagtt aatgatgaag gaaagggaat aattgctgta ccattaacca ggactaagtt 4320
   actaataaaa ccaaattgag tattgttgca ggaagcaaga cccaactacc attgtcagct 4380
   gtgtttcctg acctcaatat ttgttataag gtttgatatg aatcccaggg ggaatctcaa 4440
   cccctattac ccaacagtca gaaaaatcta agtgtgagga gaacacaatg tttcaacctt 4500
   attgttataa taatgacagt aagaacagca tggcagaatc gaaggaagca agagaccaag 4560
   aatgaacctg aaagaagaat ctaaagaaga aaaaagaaga aatgactggt ggaaaatagg 4620
   tatgtttctg ttatgcttag caggaactac tggaggaata ctttggtggt atgaaggact 4680
   cccacagcaa cattatatag ggttggtggc gataggggga agattaaacg gatctggcca 4740
   atcaaatgct atagaatgct ggggttcctt cccggggtgt agaccatttc aaaattactt 4800
   cagttatgag accaatagaa gcatgcatat ggataataat actgctacat tattagaagc 4860
   tttaaccaat ataactgctc tataaataac aaaacagaat tagaaacatg gaagttagta 4920
   aagacttctg gcataactcc tttacctatt tcttctgaag ctaacactgg actaattaga 4980
   cataagagag attttggtat aagtgcaata gtggcagcta ttgtagccgc tactgctatt 5040
   gctgctagcg ctactatgtc ttatgttgct ctaactgagg ttaacaaaat aatggaagta 5100
   caaaatcata cttttgaggt agaaaatagt actctaaatg gtatggattt aatagaacga 5160
   caaataaaga tattatatgc tatgattctt caaacacatg cagatgttca actgttaaag 5220
   gaaagacaac aggtagagga gacatttaat ttaattggat gtatagaaag aacacatgta 5280
   ttttgtcata ctggtcatcc ctggaatatg tcatggggac atttaaatga gtcaacacaa 5340
   tgggatgact gggtaagcaa aatggaagat ttaaatcaag agatactaac tacacttcat 5400
   ggagccagga acaatttggc acaatccatg ataacattca atacaccaga tagtatagct 5460
   caatttggaa aagacctttg gagtcatatt ggaaattgga ttcctggatt gggagcttcc 5520
   attataaaat atatagtgat gtttttgctt atttatttgt tactaacctc ttcgcctaag 5580
   atcctcaggg ccctctggaa ggtgaccagt ggtgcagggt cctccggcag tcgttacctg 5640
   aagaaaaaat tccatcacaa acatgcatcg cgagaagaca cctgggacca ggcccaacac 5700
   aacatacacc tagcaggcgt gaccggtgga tcaggggaca aatactacaa gcagaagtac 5760
   tccaggaacg actggaatgg agaatcagag gagtacaaca ggcggccaaa gagctgggtg 5820
   aagtcaatcg aggcatttgg agagagctat atttccgaga agaccaaagg ggagatttct 5880
   cagcctgggg cggctatcaa cgagcacaag aacggctctg gggggaacaa tcctcaccaa 5940
   gggtccttag acctggagat tcgaagcgaa ggaggaaaca tttatgactg ttgcattaaa 6000
   gcccaagaag gaactctcgc tatcccttgc tgtggatttc ccttatggct attttgggga 6060
   ctagtaatta tagtaggacg catagcaggc tatggattac gtggactcgc tgttataata 6120
   aggatttgta ttagaggctt aaatttgata tttgaaataa tcagaaaaat gcttgattat 6180
   attggaagag ctttaaatcc tggcacatct catgtatcaa tgcctcagta tgtttagaaa 6240
   aacaaggggg gaactgtggg gtttttatga ggggttttat aaatgattat aagagtaaaa 6300
   agaaagttgc tgatgctctc ataaccttgt ataacccaaa ggactagctc atgttgctag 6360
   gcaactaaac cgcaataacc gcatttgtga cgcgagttcc ccattggtga cgcgttaact 6420
   tcctgttttt acagtatata agtgcttgta ttctgacaat tgggcactca gattctgcgg 6480
   tctgagtccc ttctctgctg ggctgaaaag gcctttgtaa taaatataat tctctactca 6540
   gtccctgtct ctagtttgtc tgttcgagat cctacagagc tcatgccttg gcgtaatcat 6600
   ggtcatagct gtttcctgtg tgaaattgtt atccgctcac aattccacac aacatacgag 6660
   ccggaagcat aaagtgtaaa gcctggggtg cctaatgagt gagctaactc acattaattg 6720
   cgttgcgctc actgcccgct ttccagtcgg gaaacctgtc gtgccagctg cattaatgaa 6780
   tcggccaacg cgcggggaga ggcggtttgc gtattgggcg ctcttccgct tcctcgctca 6840
   ctgactcgct gcgctcggtc gttcggctgc ggcgagcggt atcagctcac tcaaaggcgg 6900
   taatacggtt atccacagaa tcaggggata acgcaggaaa gaacatgtga gcaaaaggcc 6960
   agcaaaaggc caggaaccgt aaaaaggccg cgttgctggc gtttttccat aggctccgcc 7020
   cccctgacga gcatcacaaa aatcgacgct caagtcagag gtggcgaaac ccgacaggac 7080
   tataaagata ccaggcgttt ccccctggaa gctccctcgt gcgctctcct gttccgaccc 7140
   tgccgcttac cggatacctg tccgcctttc tcccttcggg aagcgtggcg ctttctcata 7200
   gctcacgctg taggtatctc agttcggtgt aggtcgttcg ctccaagctg ggctgtgtgc 7260
   acgaaccccc cgttcagccc gaccgctgcg ccttatccgg taactatcgt cttgagtcca 7320
   acccggtaag acacgactta tcgccactgg cagcagccac tggtaacagg attagcagag 7380
   cgaggtatgt aggcggtgct acagagttct tgaagtggtg gcctaactac ggctacacta 7440
   gaaggacagt atttggtatc tgcgctctgc tgaagccagt taccttcgga aaaagagttg 7500
   gtagctcttg atccggcaaa caaaccaccg ctggtagcgg tggttttttt gtttgcaagc 7560
   agcagattac gcgcagaaaa aaaggatctc aagaagatcc tttgatcttt tctacggggt 7620
   ctgacgctca gtggaacgaa aactcacgtt aagggatttt ggtcatgaga ttatcaaaaa 7680
   ggatcttcac ctagatcctt ttaaattaaa aatgaagttt taaatcaatc taaagtatat 7740
   atgagtaaac ttggtctgac agttaccaat gcttaatcag tgaggcacct atctcagcga 7800
   tctgtctatt tcgttcatcc atagttgcct gactccccgt cgtgtagata actacgatac 7860
   gggagggctt accatctggc cccagtgctg caatgatacc gcgagaccca cgctcaccgg 7920
   ctccagattt atcagcaata aaccagccag ccggaagggc cgagcgcaga agtggtcctg 7980
   caactttatc cgcctccatc cagtctatta attgttgccg ggaagctaga gtaagtagtt 8040
   cgccagttaa tagtttgcgc aacgttgttg ccattgctac aggcatcgtg gtgtcacgct 8100
   cgtcgtttgg tatggcttca ttcagctccg gttcccaacg atcaaggcga gttacatgat 8160
   cccccatgtt gtgcaaaaaa gcggttagct ccttcggtcc tccgatcgtt gtcagaagta 8220
   agttggccgc agtgttatca ctcatggtta tggcagcact gcataattct cttactgtca 8280
   tgccatccgt aagatgcttt tctgtgactg gtgagtactc aaccaagtca ttctgagaat 8340
   agtgtatgcg gcgaccgagt tgctcttgcc cggcgtcaat acgggataat accgcgccac 8400
   atagcagaac tttaaaagtg ctcatcattg gaaaacgttc ttcggggcga aaactctcaa 8460
   ggatcttacc gctgttgaga tccagttcga tgtaacccac tcgtgcaccc aactgatctt 8520
   cagcatcttt tactttcacc agcgtttctg ggtgagcaaa aacaggaagg caaaatgccg 8580
   caaaaaaggg aataagggcg acacggaaat gttgaatact catactcttc ctttttcaat 8640
   attattgaag catttatcag ggttattgtc tcatgagcgg atacatattt gaatgtattt 8700
   agaaaaataa acaaataggg gttccgcgca catttccccg aaaagtgcca cctaaattgt 8760
   aagcgttaat attttgttaa aattcgcgtt aaatttttgt taaatcagct cattttttaa 8820
   ccaataggcc gaaatcggca aaatccctta taaatcaaaa gaatagaccg agatagggtt 8880
   gagtgttgtt ccagtttgga acaagagtcc actattaaag aacgtggact ccaacgtcaa 8940
   agggcgaaaa accgtctatc agggcgatgg cccactacgt gaaccatcac cctaatcaag 9000
   ttttttgggg tcgaggtgcc gtaaagcact aaatcggaac cctaaaggga gcccccgatt 9060
   tagagcttga cggggaaagc caacctggct tatcgaaatt aatacgactc actataggga 9120
   gaccggc 9127
<210> 10
   <211> 9151
   <212> DNA
   <213> Artificial Sequence
<220>
   <223> Description of Artificial Sequence:
   pONY-FLAG-RARbeta2 vector genome plasmid
<400> 10
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagag tcgacgctct 1800
   cattacttgt aacaaaggga gggaaagtat gggaggacag acaccatggg aagtatttat 1860
   cactaatcaa gcacaagtaa tacatgagaa acttttacta cagcaagcac aatcctccaa 1920
   aaaattttgt ttttacaaaa tccctggtga acatggtcga ctctagaact agtggatccc 1980
   ccgggctgca ggagtgggga ggcacgatgg ccgctttggt cgaggcggat ccggccatta 2040
   gccatattat tcattggtta tatagcataa atcaatattg gctattggcc attgcatacg 2100
   ttgtatccat atcataatat gtacatttat attggctcat gtccaacatt accgccatgt 2160
   tgacattgat tattgactag ttattaatag taatcaatta cggggtcatt agttcatagc 2220
   ccatatatgg agttccgcgt tacataactt acggtaaatg gcccgcctgg ctgaccgccc 2280
   aacgaccccc gcccattgac gtcaataatg acgtatgttc ccatagtaac gccaataggg 2340
   actttccatt gacgtcaatg ggtggagtat ttacggtaaa ctgcccactt ggcagtacat 2400
   caagtgtatc atatgccaag tacgccccct attgacgtca atgacggtaa atggcccgcc 2460
   tggcattatg cccagtacat gaccttatgg gactttccta cttggcagta catctacgta 2520
   ttagtcatcg ctattaccat ggtgatgcgg ttttggcagt acatcaatgg gcgtggatag 2580
   cggtttgact cacggggatt tccaagtctc caccccattg acgtcaatgg gagtttgttt 2640
   tggcaccaaa atcaacggga ctttccaaaa tgtcgtaaca actccgcccc attgacgcaa 2700
   atgggcggta ggcatgtacg gtgggaggtc tatataagca gagctcgttt agtgaaccgt 2760
   cagatcgcct ggagacgcca tccacgctgt tttgacctcc atagaagaca ccgggaccga 2820
   tccagcctcc gcgggccacc atggactaca aggacgacga tgacaagttt gactgtatgg 2880
   atgttctgtc agtgagtccc gggcagatcc tggatttcta caccgcgagc ccttcctcct 2940
   gcatgctgca ggaaaaggct ctcaaagcct gcctcagtgg attcacccag gccgaatggc 3000
   agcaccggca tactgctcaa tccatcgaga cacagagtac cagctctgag gagctcgtcc 3060
   cgagcccacc atctccactt cctcctcctc gggtgtacaa gccctgcttc gtttgccagg 3120
   acaagtcatc gggctaccac tatggcgtca gtgcctgcga ggggtgcaag ggctttttcc 3180
   gcagaagtat tcagaagaac atgatctaca cttgccatcg agataagaac tgcgtcatta 3240
   acaaggtcac taggaaccga tgccagtact gccgcctgca gaagtgcttt gaagtgggca 3300
   tgtccaaaga gtctgttagg aatgacagga acaagaaaaa gaaggagcct tcaaagcagg 3360
   aatgcacaga gagctatgag atgacagcgg agctagacga cctcactgag aagatccgga 3420
   aagcccacca ggaaaccttt ccctcactct gccagctggg taaatacacc acgaattcca 3480
   gcgctgacca ccgggtccga ttggacttgg gcctctggga caaattcagt gagctggcca 3540
   ccaagtgcat tattaagatc gtggagttcg ccaagcgtct gccgggcttc acaggtctga 3600
   ccatcgcaga ccagatcacc ctgctcaaag ccgcctgctt ggatatcttg attctcagaa 3660
   tttgtaccag gtatacccca gagcaagaca ccatgacttt ctctgatggc cttacactaa 3720
   atcgaactca gatgcacaat gctggcttcg gtcctctgac tgaccttgtg ttcacctttg 3780
   ccaaccagct cctgcctttg gaaatggatg acacagaaac aggccttctc agtgccatct 3840
   gtttaatctg tggagaccgc caggaccttg aggaaccaac aaaagtagac aagctccaag 3900
   aaccactgct ggaagcacta aagatttaca ttagaaaacg acgacccagc aagcctcaca 3960
   tgtttccaaa gatcttaatg aaaatcacag atctccgcag catcagcgcg aaaggtgccg 4020
   aacgtgtaat taccttgaaa atggaaattc ctggatcaat gccacctctc attcaggaaa 4080
   tgctggagaa ttctgaagga catgaaccct tgaccccaag ttcaagtggg aatatagcag 4140
   agcacagtcc cagcgtgtcc cccagctcag tggagaacag tggagtcagt cagtcaccac 4200
   tgctgcagtg agcggccgcg actctagagt cgacctcgag ggggggcccg gacctactag 4260
   ggtgctgtgg aagggtgatg gtgcagtagt agttaatgat gaaggaaagg gaataattgc 4320
   tgtaccatta accaggacta agttactaat aaaaccaaat tgagtattgt tgcaggaagc 4380
   aagacccaac taccattgtc agctgtgttt cctgacctca atatttgtta taaggtttga 4440
   tatgaatccc agggggaatc tcaaccccta ttacccaaca gtcagaaaaa tctaagtgtg 4500
   aggagaacac aatgtttcaa ccttattgtt ataataatga cagtaagaac agcatggcag 4560
   aatcgaagga agcaagagac caagaatgaa cctgaaagaa gaatctaaag aagaaaaaag 4620
   aagaaatgac tggtggaaaa taggtatgtt tctgttatgc ttagcaggaa ctactggagg 4680
   aatactttgg tggtatgaag gactcccaca gcaacattat atagggttgg tggcgatagg 4740
   gggaagatta aacggatctg gccaatcaaa tgctatagaa tgctggggtt ccttcccggg 4800
   gtgtagacca tttcaaaatt acttcagtta tgagaccaat agaagcatgc atatggataa 4860
   taatactgct acattattag aagctttaac caatataact gctctataaa taacaaaaca 4920
   gaattagaaa catggaagtt agtaaagact tctggcataa ctcctttacc tatttcttct 4980
   gaagctaaca ctggactaat tagacataag agagattttg gtataagtgc aatagtggca 5040
   gctattgtag ccgctactgc tattgctgct agcgctacta tgtcttatgt tgctctaact 5100
   gaggttaaca aaataatgga agtacaaaat catacttttg aggtagaaaa tagtactcta 5160
   aatggtatgg atttaataga acgacaaata aagatattat atgctatgat tcttcaaaca 5220
   catgcagatg ttcaactgtt aaaggaaaga caacaggtag aggagacatt taatttaatt 5280
   ggatgtatag aaagaacaca tgtattttgt catactggtc atccctggaa tatgtcatgg 5340
   ggacatttaa atgagtcaac acaatgggat gactgggtaa gcaaaatgga agatttaaat 5400
   caagagatac taactacact tcatggagcc aggaacaatt tggcacaatc catgataaca 5460
   ttcaatacac cagatagtat agctcaattt ggaaaagacc tttggagtca tattggaaat 5520
   tggattcctg gattgggagc ttccattata aaatatatag tgatgttttt gcttatttat 5580
   ttgttactaa cctcttcgcc taagatcctc agggccctct ggaaggtgac cagtggtgca 5640
   gggtcctccg gcagtcgtta cctgaagaaa aaattccatc acaaacatgc atcgcgagaa 5700
   gacacctggg accaggccca acacaacata cacctagcag gcgtgaccgg tggatcaggg 5760
   gacaaatact acaagcagaa gtactccagg aacgactgga atggagaatc agaggagtac 5820
   aacaggcggc caaagagctg ggtgaagtca atcgaggcat ttggagagag ctatatttcc 5880
   gagaagacca aaggggagat ttctcagcct ggggcggcta tcaacgagca caagaacggc 5940
   tctgggggga acaatcctca ccaagggtcc ttagacctgg agattcgaag cgaaggagga 6000
   aacatttatg actgttgcat taaagcccaa gaaggaactc tcgctatccc ttgctgtgga 6060
   tttcccttat ggctattttg gggactagta attatagtag gacgcatagc aggctatgga 6120
   ttacgtggac tcgctgttat aataaggatt tgtattagag gcttaaattt gatatttgaa 6180
   ataatcagaa aaatgcttga ttatattgga agagctttaa atcctggcac atctcatgta 6240
   tcaatgcctc agtatgttta gaaaaacaag gggggaactg tggggttttt atgaggggtt 6300
   ttataaatga ttataagagt aaaaagaaag ttgctgatgc tctcataacc ttgtataacc 6360
   caaaggacta gctcatgttg ctaggcaact aaaccgcaat aaccgcattt gtgacgcgag 6420
   ttccccattg gtgacgcgtt aacttcctgt ttttacagta tataagtgct tgtattctga 6480
   caattgggca ctcagattct gcggtctgag tcccttctct gctgggctga aaaggccttt 6540
   gtaataaata taattctcta ctcagtccct gtctctagtt tgtctgttcg agatcctaca 6600
   gagctcatgc cttggcgtaa tcatggtcat agctgtttcc tgtgtgaaat tgttatccgc 6660
   tcacaattcc acacaacata cgagccggaa gcataaagtg taaagcctgg ggtgcctaat 6720
   gagtgagcta actcacatta attgcgttgc gctcactgcc cgctttccag tcgggaaacc 6780
   tgtcgtgcca gctgcattaa tgaatcggcc aacgcgcggg gagaggcggt ttgcgtattg 6840
   ggcgctcttc cgcttcctcg ctcactgact cgctgcgctc ggtcgttcgg ctgcggcgag 6900
   cggtatcagc tcactcaaag gcggtaatac ggttatccac agaatcaggg gataacgcag 6960
   gaaagaacat gtgagcaaaa ggccagcaaa aggccaggaa ccgtaaaaag gccgcgttgc 7020
   tggcgttttt ccataggctc cgcccccctg acgagcatca caaaaatcga cgctcaagtc 7080
   agaggtggcg aaacccgaca ggactataaa gataccaggc gtttccccct ggaagctccc 7140
   tcgtgcgctc tcctgttccg accctgccgc ttaccggata cctgtccgcc tttctccctt 7200
   cgggaagcgt ggcgctttct catagctcac gctgtaggta tctcagttcg gtgtaggtcg 7260
   ttcgctccaa gctgggctgt gtgcacgaac cccccgttca gcccgaccgc tgcgccttat 7320
   ccggtaacta tcgtcttgag tccaacccgg taagacacga cttatcgcca ctggcagcag 7380
   ccactggtaa caggattagc agagcgaggt atgtaggcgg tgctacagag ttcttgaagt 7440
   ggtggcctaa ctacggctac actagaagga cagtatttgg tatctgcgct ctgctgaagc 7500
   cagttacctt cggaaaaaga gttggtagct cttgatccgg caaacaaacc accgctggta 7560
   gcggtggttt ttttgtttgc aagcagcaga ttacgcgcag aaaaaaagga tctcaagaag 7620
   atcctttgat cttttctacg gggtctgacg ctcagtggaa cgaaaactca cgttaaggga 7680
   ttttggtcat gagattatca aaaaggatct tcacctagat ccttttaaat taaaaatgaa 7740
   gttttaaatc aatctaaagt atatatgagt aaacttggtc tgacagttac caatgcttaa 7800
   tcagtgaggc acctatctca gcgatctgtc tatttcgttc atccatagtt gcctgactcc 7860
   ccgtcgtgta gataactacg atacgggagg gcttaccatc tggccccagt gctgcaatga 7920
   taccgcgaga cccacgctca ccggctccag atttatcagc aataaaccag ccagccggaa 7980
   gggccgagcg cagaagtggt cctgcaactt tatccgcctc catccagtct attaattgtt 8040
   gccgggaagc tagagtaagt agttcgccag ttaatagttt gcgcaacgtt gttgccattg 8100
   ctacaggcat cgtggtgtca cgctcgtcgt ttggtatggc ttcattcagc tccggttccc 8160
   aacgatcaag gcgagttaca tgatccccca tgttgtgcaa aaaagcggtt agctccttcg 8220
   gtcctccgat cgttgtcaga agtaagttgg ccgcagtgtt atcactcatg gttatggcag 8280
   cactgcataa ttctcttact gtcatgccat ccgtaagatg cttttctgtg actggtgagt 8340
   actcaaccaa gtcattctga gaatagtgta tgcggcgacc gagttgctct tgcccggcgt 8400
   caatacggga taataccgcg ccacatagca gaactttaaa agtgctcatc attggaaaac 8460
   gttcttcggg gcgaaaactc tcaaggatct taccgctgtt gagatccagt tcgatgtaac 8520
   ccactcgtgc acccaactga tcttcagcat cttttacttt caccagcgtt tctgggtgag 8580
   caaaaacagg aaggcaaaat gccgcaaaaa agggaataag ggcgacacgg aaatgttgaa 8640
   tactcatact cttccttttt caatattatt gaagcattta tcagggttat tgtctcatga 8700
   gcggatacat atttgaatgt atttagaaaa ataaacaaat aggggttccg cgcacatttc 8760
   cccgaaaagt gccacctaaa ttgtaagcgt taatattttg ttaaaattcg cgttaaattt 8820
   ttgttaaatc agctcatttt ttaaccaata ggccgaaatc ggcaaaatcc cttataaatc 8880
   aaaagaatag accgagatag ggttgagtgt tgttccagtt tggaacaaga gtccactatt 8940
   aaagaacgtg gactccaacg tcaaagggcg aaaaaccgtc tatcagggcg atggcccact 9000
   acgtgaacca tcaccctaat caagtttttt ggggtcgagg tgccgtaaag cactaaatcg 9060
   gaaccctaaa gggagccccc gatttagagc ttgacgggga aagccaacct ggcttatcga 9120
   aattaatacg actcactata gggagaccgg c 9151
<210> 11
   <211> 8528
   <212> DNA
   <213> Artificial Sequence
<220>
   <223> Description of Artificial Sequence: pONY8G 5'cPPT POS delCTS EIAV vector genome plasmid
<400> 11
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagag tcgacgctct 1800
   cattacttgt aacaaaggga gggaaagtat gggaggacag acaccatggg aagtatttat 1860
   cactaatcaa gcacaagtaa tacatgagaa acttttacta cagcaagcac aatcctccaa 1920
   aaaattttgt ttttacaaaa tccctggtga acatggtcga ctctagaact agtggatccc 1980
   ccgggctgca ggagtgggga ggcacgatgg ccgctttggt cgaggcggat ccggccatta 2040
   gccatattat tcattggtta tatagcataa atcaatattg gctattggcc attgcatacg 2100
   ttgtatccat atcataatat gtacatttat attggctcat gtccaacatt accgccatgt 2160
   tgacattgat tattgactag ttattaatag taatcaatta cggggtcatt agttcatagc 2220
   ccatatatgg agttccgcgt tacataactt acggtaaatg gcccgcctgg ctgaccgccc 2280
   aacgaccccc gcccattgac gtcaataatg acgtatgttc ccatagtaac gccaataggg 2340
   actttccatt gacgtcaatg ggtggagtat ttacggtaaa ctgcccactt ggcagtacat 2400
   caagtgtatc atatgccaag tacgccccct attgacgtca atgacggtaa atggcccgcc 2460
   tggcattatg cccagtacat gaccttatgg gactttccta cttggcagta catctacgta 2520
   ttagtcatcg ctattaccat ggtgatgcgg ttttggcagt acatcaatgg gcgtggatag 2580
   cggtttgact cacggggatt tccaagtctc caccccattg acgtcaatgg gagtttgttt 2640
   tggcaccaaa atcaacggga ctttccaaaa tgtcgtaaca actccgcccc attgacgcaa 2700
   atgggcggta ggcatgtacg gtgggaggtc tatataagca gagctcgttt agtgaaccgt 2760
   cagatcgcct ggagacgcca tccacgctgt tttgacctcc atagaagaca ccgggaccga 2820
   tccagcctcc gcggccccaa gcttgttggg atccaccggt cgccaccatg gtgagcaagg 2880
   gcgaggagct gttcaccggg gtggtgccca tcctggtcga gctggacggc gacgtaaacg 2940
   gccacaagtt cagcgtgtcc ggcgagggcg agggcgatgc cacctacggc aagctgaccc 3000
   tgaagttcat ctgcaccacc ggcaagctgc ccgtgccctg gcccaccctc gtgaccaccc 3060
   tgacctacgg cgtgcagtgc ttcagccgct accccgacca catgaagcag cacgacttct 3120
   tcaagtccgc catgcccgaa ggctacgtcc aggagcgcac catcttcttc aaggacgacg 3180
   gcaactacaa gacccgcgcc gaggtgaagt tcgagggcga caccctggtg aaccgcatcg 3240
   agctgaaggg catcgacttc aaggaggacg gcaacatcct ggggcacaag ctggagtaca 3300
   actacaacag ccacaacgtc tatatcatgg ccgacaagca gaagaacggc atcaaggtga 3360
   acttcaagat ccgccacaac atcgaggacg gcagcgtgca gctcgccgac cactaccagc 3420
   agaacacccc catcggcgac ggccccgtgc tgctgcccga caaccactac ctgagcaccc 3480
   agtccgccct gagcaaagac cccaacgaga agcgcgatca catggtcctg ctggagttcg 3540
   tgaccgccgc cgggatcact ctcggcatgg acgagctgta caagtaaagc ggccgcgact 3600
   ctagagtcga cctcgagggg gggcccggac ctactagggt gctgtggaag ggtgatggtg 3660
   cagtagtagt taatgatgaa ggaaagggaa taattgctgt accattaacc aggactaagt 3720
   tactaataaa accaaattga gtattgttgc aggaagcaag acccaactac cattgtcagc 3780
   tgtgtttcct gacctcaata tttgttataa ggtttgatat gaatcccagg gggaatctca 3840
   acccctatta cccaacagtc agaaaaatct aagtgtgagg agaacacaat gtttcaacct 3900
   tattgttata ataatgacag taagaacagc atggcagaat cgaaggaagc aagagaccaa 3960
   gaatgaacct gaaagaagaa tctaaagaag aaaaaagaag aaatgactgg tggaaaatag 4020
   gtatgtttct gttatgctta gcaggaacta ctggaggaat actttggtgg tatgaaggac 4080
   tcccacagca acattatata gggttggtgg cgataggggg aagattaaac ggatctggcc 4140
   aatcaaatgc tatagaatgc tggggttcct tcccggggtg tagaccattt caaaattact 4200
   tcagttatga gaccaataga agcatgcata tggataataa tactgctaca ttattagaag 4260
   ctttaaccaa tataactgct ctataaataa caaaacagaa ttagaaacat ggaagttagt 4320
   aaagacttct ggcataactc ctttacctat ttcttctgaa gctaacactg gactaattag 4380
   acataagaga gattttggta taagtgcaat agtggcagct attgtagccg ctactgctat 4440
   tgctgctagc gctactatgt cttatgttgc tctaactgag gttaacaaaa taatggaagt 4500
   acaaaatcat acttttgagg tagaaaatag tactctaaat ggtatggatt taatagaacg 4560
   acaaataaag atattatatg ctatgattct tcaaacacat gcagatgttc aactgttaaa 4620
   ggaaagacaa caggtagagg agacatttaa tttaattgga tgtatagaaa gaacacatgt 4680
   attttgtcat actggtcatc cctggaatat gtcatgggga catttaaatg agtcaacaca 4740
   atgggatgac tgggtaagca aaatggaaga tttaaatcaa gagatactaa ctacacttca 4800
   tggagccagg aacaatttgg cacaatccat gataacattc aatacaccag atagtatagc 4860
   tcaatttgga aaagaccttt ggagtcatat tggaaattgg attcctggat tgggagcttc 4920
   cattataaaa tatatagtga tgtttttgct tatttatttg ttactaacct cttcgcctaa 4980
   gatcctcagg gccctctgga aggtgaccag tggtgcaggg tcctccggca gtcgttacct 5040
   gaagaaaaaa ttccatcaca aacatgcatc gcgagaagac acctgggacc aggcccaaca 5100
   caacatacac ctagcaggcg tgaccggtgg atcaggggac aaatactaca agcagaagta 5160
   ctccaggaac gactggaatg gagaatcaga ggagtacaac aggcggccaa agagctgggt 5220
   gaagtcaatc gaggcatttg gagagagcta tatttccgag aagaccaaag gggagatttc 5280
   tcagcctggg gcggctatca acgagcacaa gaacggctct ggggggaaca atcctcacca 5340
   agggtcctta gacctggaga ttcgaagcga aggaggaaac atttatgact gttgcattaa 5400
   agcccaagaa ggaactctcg ctatcccttg ctgtggattt cccttatggc tattttgggg 5460
   actagtaatt atagtaggac gcatagcagg ctatggatta cgtggactcg ctgttataat 5520
   aaggatttgt attagaggct taaatttgat atttgaaata atcagaaaaa tgcttgatta 5580
   tattggaaga gctttaaatc ctggcacatc tcatgtatca atgcctcagt atgtttagaa 5640
   aaacaagggg ggaactgtgg ggtttttatg aggggtttta taaatgatta taagagtaaa 5700
   aagaaagttg ctgatgctct cataaccttg tataacccaa aggactagct catgttgcta 5760
   ggcaactaaa ccgcaataac cgcatttgtg acgcgagttc cccattggtg acgcgttaac 5820
   ttcctgtttt tacagtatat aagtgcttgt attctgacaa ttgggcactc agattctgcg 5880
   gtctgagtcc cttctctgct gggctgaaaa ggcctttgta ataaatataa ttctctactc 5940
   agtccctgtc tctagtttgt ctgttcgaga tcctacagag ctcatgcctt ggcgtaatca 6000
   tggtcatagc tgtttcctgt gtgaaattgt tatccgctca caattccaca caacatacga 6060
   gccggaagca taaagtgtaa agcctggggt gcctaatgag tgagctaact cacattaatt 6120
   gcgttgcgct cactgcccgc tttccagtcg ggaaacctgt cgtgccagct gcattaatga 6180
   atcggccaac gcgcggggag aggcggtttg cgtattgggc gctcttccgc ttcctcgctc 6240
   actgactcgc tgcgctcggt cgttcggctg cggcgagcgg tatcagctca ctcaaaggcg 6300
   gtaatacggt tatccacaga atcaggggat aacgcaggaa agaacatgtg agcaaaaggc 6360
   cagcaaaagg ccaggaaccg taaaaaggcc gcgttgctgg cgtttttcca taggctccgc 6420
   ccccctgacg agcatcacaa aaatcgacgc tcaagtcaga ggtggcgaaa cccgacagga 6480
   ctataaagat accaggcgtt tccccctgga agctccctcg tgcgctctcc tgttccgacc 6540
   ctgccgctta ccggatacct gtccgccttt ctcccttcgg gaagcgtggc gctttctcat 6600
   agctcacgct gtaggtatct cagttcggtg taggtcgttc gctccaagct gggctgtgtg 6660
   cacgaacccc ccgttcagcc cgaccgctgc gccttatccg gtaactatcg tcttgagtcc 6720
   aacccggtaa gacacgactt atcgccactg gcagcagcca ctggtaacag gattagcaga 6780
   gcgaggtatg taggcggtgc tacagagttc ttgaagtggt ggcctaacta cggctacact 6840
   agaaggacag tatttggtat ctgcgctctg ctgaagccag ttaccttcgg aaaaagagtt 6900
   ggtagctctt gatccggcaa acaaaccacc gctggtagcg gtggtttttt tgtttgcaag 6960
   cagcagatta cgcgcagaaa aaaaggatct caagaagatc ctttgatctt ttctacgggg 7020
   tctgacgctc agtggaacga aaactcacgt taagggattt tggtcatgag attatcaaaa 7080
   aggatcttca cctagatcct tttaaattaa aaatgaagtt ttaaatcaat ctaaagtata 7140
   tatgagtaaa cttggtctga cagttaccaa tgcttaatca gtgaggcacc tatctcagcg 7200
   atctgtctat ttcgttcatc catagttgcc tgactccccg tcgtgtagat aactacgata 7260
   cgggagggct taccatctgg ccccagtgct gcaatgatac cgcgagaccc acgctcaccg 7320
   gctccagatt tatcagcaat aaaccagcca gccggaaggg ccgagcgcag aagtggtcct 7380
   gcaactttat ccgcctccat ccagtctatt aattgttgcc gggaagctag agtaagtagt 7440
   tcgccagtta atagtttgcg caacgttgtt gccattgcta caggcatcgt ggtgtcacgc 7500
   tcgtcgtttg gtatggcttc attcagctcc ggttcccaac gatcaaggcg agttacatga 7560
   tcccccatgt tgtgcaaaaa agcggttagc tccttcggtc ctccgatcgt tgtcagaagt 7620
   aagttggccg cagtgttatc actcatggtt atggcagcac tgcataattc tcttactgtc 7680
   atgccatccg taagatgctt ttctgtgact ggtgagtact caaccaagtc attctgagaa 7740
   tagtgtatgc ggcgaccgag ttgctcttgc ccggcgtcaa tacgggataa taccgcgcca 7800
   catagcagaa ctttaaaagt gctcatcatt ggaaaacgtt cttcggggcg aaaactctca 7860
   aggatcttac cgctgttgag atccagttcg atgtaaccca ctcgtgcacc caactgatct 7920
   tcagcatctt ttactttcac cagcgtttct gggtgagcaa aaacaggaag gcaaaatgcc 7980
   gcaaaaaagg gaataagggc gacacggaaa tgttgaatac tcatactctt cctttttcaa 8040
   tattattgaa gcatttatca gggttattgt ctcatgagcg gatacatatt tgaatgtatt 8100
   tagaaaaata aacaaatagg ggttccgcgc acatttcccc gaaaagtgcc acctaaattg 8160
   taagcgttaa tattttgtta aaattcgcgt taaatttttg ttaaatcagc tcatttttta 8220
   accaataggc cgaaatcggc aaaatccctt ataaatcaaa agaatagacc gagatagggt 8280
   tgagtgttgt tccagtttgg aacaagagtc cactattaaa gaacgtggac tccaacgtca 8340
   aagggcgaaa aaccgtctat cagggcgatg gcccactacg tgaaccatca ccctaatcaa 8400
   gtttttgggg gtcgaggtgc cgtaaagcac taaatcggaa ccctaaaggg agcccccgat 8460
   ttagagcttg acggggaaag ccaacctggc ttatcgaaat taatacgact cactataggg 8520
   agaccggc 8528
<210> 12
   <211> 10112
   <212> DNA
   <213> Artificial Sequence
<220>
   <223> Description of Artificial Sequence: pESYNGP, codon-optimised EIAV gag/pol expression plasmid
<400> 12
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattcgcca ccatgggcga tcccctcacc tggtccaaag ccctgaagaa 1140
   actggaaaaa gtcaccgttc agggtagcca aaagcttacc acaggcaatt gcaactgggc 1200
   attgtccctg gtggatcttt tccacgacac taatttcgtt aaggagaaag attggcaact 1260
   cagagacgtg atccccctct tggaggacgt gacccaaaca ttgtctgggc aggagcgcga 1320
   agctttcgag cgcacctggt gggccatcag cgcagtcaaa atggggctgc aaatcaacaa 1380
   cgtggttgac ggtaaagcta gctttcaact gctccgcgct aagtacgaga agaaaaccgc 1440
   caacaagaaa caatccgaac ctagcgagga gtacccaatt atgatcgacg gcgccggcaa 1500
   taggaacttc cgcccactga ctcccagggg ctataccacc tgggtcaaca ccatccagac 1560
   aaacggactt ttgaacgaag cctcccagaa cctgttcggc atcctgtctg tggactgcac 1620
   ctccgaagaa atgaatgctt ttctcgacgt ggtgccagga caggctggac agaaacagat 1680
   cctgctcgat gccattgaca agatcgccga cgactgggat aatcgccacc ccctgccaaa 1740
   cgcccctctg gtggctcccc cacaggggcc tatccctatg accgctaggt tcattagggg 1800
   actgggggtg ccccgcgaac gccagatgga gccagcattt gaccaattta ggcagaccta 1860
   cagacagtgg atcatcgaag ccatgagcga ggggattaaa gtcatgatcg gaaagcccaa 1920
   ggcacagaac atcaggcagg gggccaagga accataccct gagtttgtcg acaggcttct 1980
   gtcccagatt aaatccgaag gccaccctca ggagatctcc aagttcttga cagacacact 2040
   gactatccaa aatgcaaatg aagagtgcag aaacgccatg aggcacctca gacctgaaga 2100
   taccctggag gagaaaatgt acgcatgtcg cgacattggc actaccaagc aaaagatgat 2160
   gctgctcgcc aaggctctgc aaaccggcct ggctggtcca ttcaaaggag gagcactgaa 2220
   gggaggtcca ttgaaagctg cacaaacatg ttataattgt gggaagccag gacatttatc 2280
   tagtcaatgt agagcaccta aagtctgttt taaatgtaaa cagcctggac atttctcaaa 2340
   gcaatgcaga agtgttccaa aaaacgggaa gcaaggggct caagggaggc cccagaaaca 2400
   aactttcccg atacaacaga agagtcagca caacaaatct gttgtacaag agactcctca 2460
   gactcaaaat ctgtacccag atctgagcga aataaaaaag gaatacaatg tcaaggagaa 2520
   ggatcaagta gaggatctca acctggacag tttgtgggag taacatacaa tctcgagaag 2580
   aggcccacta ccatcgtcct gatcaatgac acccctctta atgtgctgct ggacaccgga 2640
   gccgacacca gcgttctcac tactgctcac tataacagac tgaaatacag aggaaggaaa 2700
   taccagggca caggcatcat cggcgttgga ggcaacgtcg aaaccttttc cactcctgtc 2760
   accatcaaaa agaaggggag acacattaaa accagaatgc tggtcgccga catccccgtc 2820
   accatccttg gcagagacat tctccaggac ctgggcgcta aactcgtgct ggcacaactg 2880
   tctaaggaaa tcaagttccg caagatcgag ctgaaagagg gcacaatggg tccaaaaatc 2940
   ccccagtggc ccctgaccaa agagaagctt gagggcgcta aggaaatcgt gcagcgcctg 3000
   ctttctgagg gcaagattag cgaggccagc gacaataacc cttacaacag ccccatcttt 3060
   gtgattaaga aaaggagcgg caaatggaga ctcctgcagg acctgaggga actcaacaag 3120
   accgtccagg tcggaactga gatctctcgc ggactgcctc accccggcgg cctgattaaa 3180
   tgcaagcaca tgacagtcct tgacattgga gacgcttatt ttaccatccc cctcgatcct 3240
   gaatttcgcc cctatactgc ttttaccatc cccagcatca atcaccagga gcccgataaa 3300
   cgctatgtgt ggaagtgcct cccccaggga tttgtgctta gcccctacat ttaccagaag 3360
   acacttcaag agatcctcca acctttccgc gaaagatacc cagaggttca actctaccaa 3420
   tatatggacg acctgttcat ggggtccaac gggtctaaga agcagcacaa ggaactcatc 3480
   atcgaactga gggcaatcct cctggagaaa ggcttcgaga cacccgacga caagctgcaa 3540
   gaagttcctc catatagctg gctgggctac cagctttgcc ctgaaaactg gaaagtccag 3600
   aagatgcagt tggatatggt caagaaccca acactgaacg acgtccagaa gctcatgggc 3660
   aatattacct ggatgagctc cggaatccct gggcttaccg ttaagcacat tgccgcaact 3720
   acaaaaggat gcctggagtt gaaccagaag gtcatttgga cagaggaagc tcagaaggaa 3780
   ctggaggaga ataatgaaaa gattaagaat gctcaagggc tccaatacta caatcccgaa 3840
   gaagaaatgt tgtgcgaggt cgaaatcact aagaactacg aagccaccta tgtcatcaaa 3900
   cagtcccaag gcatcttgtg ggccggaaag aaaatcatga aggccaacaa aggctggtcc 3960
   accgttaaaa atctgatgct cctgctccag cacgtcgcca ccgagtctat cacccgcgtc 4020
   ggcaagtgcc ccaccttcaa agttcccttc actaaggagc aggtgatgtg ggagatgcaa 4080
   aaaggctggt actactcttg gcttcccgag atcgtctaca cccaccaagt ggtgcacgac 4140
   gactggagaa tgaagcttgt cgaggagccc actagcggaa ttacaatcta taccgacggc 4200
   ggaaagcaaa acggagaggg aatcgctgca tacgtcacat ctaacggccg caccaagcaa 4260
   aagaggctcg gccctgtcac tcaccaggtg gctgagagga tggctatcca gatggccctt 4320
   gaggacacta gagacaagca ggtgaacatt gtgactgaca gctactactg ctggaaaaac 4380
   atcacagagg gccttggcct ggagggaccc cagtctccct ggtggcctat catccagaat 4440
   atccgcgaaa aggaaattgt ctatttcgcc tgggtgcctg gacacaaagg aatttacggc 4500
   aaccaactcg ccgatgaagc cgccaaaatt aaagaggaaa tcatgcttgc ctaccagggc 4560
   acacagatta aggagaagag agacgaggac gctggctttg acctgtgtgt gccatacgac 4620
   atcatgattc ccgttagcga cacaaagatc attccaaccg atgtcaagat ccaggtgcca 4680
   cccaattcat ttggttgggt gaccggaaag tccagcatgg ctaagcaggg tcttctgatt 4740
   aacgggggaa tcattgatga aggatacacc ggcgaaatcc aggtgatctg cacaaatatc 4800
   ggcaaaagca atattaagct tatcgaaggg cagaagttcg ctcaactcat catcctccag 4860
   caccacagca attcaagaca accttgggac gaaaacaaga ttagccagag aggtgacaag 4920
   ggcttcggca gcacaggtgt gttctgggtg gagaacatcc aggaagcaca ggacgagcac 4980
   gagaattggc acacctcccc taagattttg gcccgcaatt acaagatccc actgactgtg 5040
   gctaagcaga tcacacagga atgcccccac tgcaccaaac aaggttctgg ccccgccggc 5100
   tgcgtgatga ggtcccccaa tcactggcag gcagattgca cccacctcga caacaaaatt 5160
   atcctgacct tcgtggagag caattccggc tacatccacg caacactcct ctccaaggaa 5220
   aatgcattgt gcacctccct cgcaattctg gaatgggcca ggctgttctc tccaaaatcc 5280
   ctgcacaccg acaacggcac caactttgtg gctgaacctg tggtgaatct gctgaagttc 5340
   ctgaaaatcg cccacaccac tggcattccc tatcaccctg aaagccaggg cattgtcgag 5400
   agggccaaca gaactctgaa agaaaagatc caatctcaca gagacaatac acagacattg 5460
   gaggccgcac ttcagctcgc ccttatcacc tgcaacaaag gaagagaaag catgggcggc 5520
   cagaccccct gggaggtctt catcactaac caggcccagg tcatccatga aaagctgctc 5580
   ttgcagcagg cccagtcctc caaaaagttc tgcttttata agatccccgg tgagcacgac 5640
   tggaaaggtc ctacaagagt tttgtggaaa ggagacggcg cagttgtggt gaacgatgag 5700
   ggcaagggga tcatcgctgt gcccctgaca cgcaccaagc ttctcatcaa gccaaactga 5760
   acccggggcg gccgcttccc tttagtgagg gttaatgctt cgagcagaca tgataagata 5820
   cattgatgag tttggacaaa ccacaactag aatgcagtga aaaaaatgct ttatttgtga 5880
   aatttgtgat gctattgctt tatttgtaac cattataagc tgcaataaac aagttaacaa 5940
   caacaattgc attcatttta tgtttcaggt tcagggggag atgtgggagg ttttttaaag 6000
   caagtaaaac ctctacaaat gtggtaaaat ccgataagga tcgatccggg ctggcgtaat 6060
   agcgaagagg cccgcaccga tcgcccttcc caacagttgc gcagcctgaa tggcgaatgg 6120
   acgcgccctg tagcggcgca ttaagcgcgg cgggtgtggt ggttacgcgc agcgtgaccg 6180
   ctacacttgc cagcgcccta gcgcccgctc ctttcgcttt cttcccttcc tttctcgcca 6240
   cgttcgccgg ctttccccgt caagctctaa atcgggggct ccctttaggg ttccgattta 6300
   gagctttacg gcacctcgac cgcaaaaaac ttgatttggg tgatggttca cgtagtgggc 6360
   catcgccctg atagacggtt tttcgccctt tgacgttgga gtccacgttc tttaatagtg 6420
   gactcttgtt ccaaactgga acaacactca accctatctc ggtctattct tttgatttat 6480
   aagggatttt gccgatttcg gcctattggt taaaaaatga gctgatttaa caaatattta 6540
   acgcgaattt taacaaaata ttaacgttta caatttcgcc tgatgcggta ttttctcctt 6600
   acgcatctgt gcggtatttc acaccgcata cgcggatctg cgcagcacca tggcctgaaa 6660
   taacctctga aagaggaact tggttaggta ccttctgagg cggaaagaac cagctgtgga 6720
   atgtgtgtca gttagggtgt ggaaagtccc caggctcccc agcaggcaga agtatgcaaa 6780
   gcatgcatct caattagtca gcaaccaggt gtggaaagtc cccaggctcc ccagcaggca 6840
   gaagtatgca aagcatgcat ctcaattagt cagcaaccat agtcccgccc ctaactccgc 6900
   ccatcccgcc cctaactccg cccagttccg cccattctcc gccccatggc tgactaattt 6960
   tttttattta tgcagaggcc gaggccgcct cggcctctga gctattccag aagtagtgag 7020
   gaggcttttt tggaggccta ggcttttgca aaaagcttga ttcttctgac acaacagtct 7080
   cgaacttaag gctagagcca ccatgattga acaagatgga ttgcacgcag gttctccggc 7140
   cgcttgggtg gagaggctat tcggctatga ctgggcacaa cagacaatcg gctgctctga 7200
   tgccgccgtg ttccggctgt cagcgcaggg gcgcccggtt ctttttgtca agaccgacct 7260
   gtccggtgcc ctgaatgaac tgcaggacga ggcagcgcgg ctatcgtggc tggccacgac 7320
   gggcgttcct tgcgcagctg tgctcgacgt tgtcactgaa gcgggaaggg actggctgct 7380
   attgggcgaa gtgccggggc aggatctcct gtcatctcac cttgctcctg ccgagaaagt 7440
   atccatcatg gctgatgcaa tgcggcggct gcatacgctt gatccggcta cctgcccatt 7500
   cgaccaccaa gcgaaacatc gcatcgagcg agcacgtact cggatggaag ccggtcttgt 7560
   cgatcaggat gatctggacg aagagcatca ggggctcgcg ccagccgaac tgttcgccag 7620
   gctcaaggcg cgcatgcccg acggcgagga tctcgtcgtg acccatggcg atgcctgctt 7680
   gccgaatatc atggtggaaa atggccgctt ttctggattc atcgactgtg gccggctggg 7740
   tgtggcggac cgctatcagg acatagcgtt ggctacccgt gatattgctg aagagcttgg 7800
   cggcgaatgg gctgaccgct tcctcgtgct ttacggtatc gccgctcccg attcgcagcg 7860
   catcgccttc tatcgccttc ttgacgagtt cttctgagcg ggactctggg gttcgaaatg 7920
   accgaccaag cgacgcccaa cctgccatca cgatggccgc aataaaatat ctttattttc 7980
   attacatctg tgtgttggtt ttttgtgtga atcgatagcg ataaggatcc gcgtatggtg 8040
   cactctcagt acaatctgct ctgatgccgc atagttaagc cagccccgac acccgccaac 8100
   acccgctgac gcgccctgac gggcttgtct gctcccggca tccgcttaca gacaagctgt 8160
   gaccgtctcc gggagctgca tgtgtcagag gttttcaccg tcatcaccga aacgcgcgag 8220
   acgaaagggc ctcgtgatac gcctattttt ataggttaat gtcatgataa taatggtttc 8280
   ttagacgtca ggtggcactt ttcggggaaa tgtgcgcgga acccctattt gtttattttt 8340
   ctaaatacat tcaaatatgt atccgctcat gagacaataa ccctgataaa tgcttcaata 8400
   atattgaaaa aggaagagta tgagtattca acatttccgt gtcgccctta ttcccttttt 8460
   tgcggcattt tgccttcctg tttttgctca cccagaaacg ctggtgaaag taaaagatgc 8520
   tgaagatcag ttgggtgcac gagtgggtta catcgaactg gatctcaaca gcggtaagat 8580
   ccttgagagt tttcgccccg aagaacgttt tccaatgatg agcactttta aagttctgct 8640
   atgtggcgcg gtattatccc gtattgacgc cgggcaagag caactcggtc gccgcataca 8700
   ctattctcag aatgacttgg ttgagtactc accagtcaca gaaaagcatc ttacggatgg 8760
   catgacagta agagaattat gcagtgctgc cataaccatg agtgataaca ctgcggccaa 8820
   cttacttctg acaacgatcg gaggaccgaa ggagctaacc gcttttttgc acaacatggg 8880
   ggatcatgta actcgccttg atcgttggga accggagctg aatgaagcca taccaaacga 8940
   cgagcgtgac accacgatgc ctgtagcaat ggcaacaacg ttgcgcaaac tattaactgg 9000
   cgaactactt actctagctt cccggcaaca attaatagac tggatggagg cggataaagt 9060
   tgcaggacca cttctgcgct cggcccttcc ggctggctgg tttattgctg ataaatctgg 9120
   agccggtgag cgtgggtctc gcggtatcat tgcagcactg gggccagatg gtaagccctc 9180
   ccgtatcgta gttatctaca cgacggggag tcaggcaact atggatgaac gaaatagaca 9240
   gatcgctgag ataggtgcct cactgattaa gcattggtaa ctgtcagacc aagtttactc 9300
   atatatactt tagattgatt taaaacttca tttttaattt aaaaggatct aggtgaagat 9360
   cctttttgat aatctcatga ccaaaatccc ttaacgtgag ttttcgttcc actgagcgtc 9420
   agaccccgta gaaaagatca aaggatcttc ttgagatcct ttttttctgc gcgtaatctg 9480
   ctgcttgcaa acaaaaaaac caccgctacc agcggtggtt tgtttgccgg atcaagagct 9540
   accaactctt tttccgaagg taactggctt cagcagagcg cagataccaa atactgtcct 9600
   tctagtgtag ccgtagttag gccaccactt caagaactct gtagcaccgc ctacatacct 9660
   cgctctgcta atcctgttac cagtggctgc tgccagtggc gataagtcgt gtcttaccgg 9720
   gttggactca agacgatagt taccggataa ggcgcagcgg tcgggctgaa cggggggttc 9780
   gtgcacacag cccagcttgg agcgaacgac ctacaccgaa ctgagatacc tacagcgtga 9840
   gctatgagaa agcgccacgc ttcccgaagg gagaaaggcg gacaggtatc cggtaagcgg 9900
   cagggtcgga acaggagagc gcacgaggga gcttccaggg ggaaacgcct ggtatcttta 9960
   tagtcctgtc gggtttcgcc acctctgact tgagcgtcga tttttgtgat gctcgtcagg 10020
   ggggcggagc ctatggaaaa acgccagcaa cgcggccttt ttacggttcc tggccttttg 10080
   ctggcctttt gctcacatgg ctcgacagat ct 10112
<210> 13
   <211> 10114
   <212> DNA
   <213> Artificial Sequence
<220>
   <223> Description of Artificial Sequence: pESDSYNGP, codon-optimised EIAV gag/pol expression plasmid
<400> 13
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattccagg taagatgggc gatcccctca cctggtccaa agccctgaag 1140
   aaactggaaa aagtcaccgt tcagggtagc caaaagctta ccacaggcaa ttgcaactgg 1200
   gcattgtccc tggtggatct tttccacgac actaatttcg ttaaggagaa agattggcaa 1260
   ctcagagacg tgatccccct cttggaggac gtgacccaaa cattgtctgg gcaggagcgc 1320
   gaagctttcg agcgcacctg gtgggccatc agcgcagtca aaatggggct gcaaatcaac 1380
   aacgtggttg acggtaaagc tagctttcaa ctgctccgcg ctaagtacga gaagaaaacc 1440
   gccaacaaga aacaatccga acctagcgag gagtacccaa ttatgatcga cggcgccggc 1500
   aataggaact tccgcccact gactcccagg ggctatacca cctgggtcaa caccatccag 1560
   acaaacggac ttttgaacga agcctcccag aacctgttcg gcatcctgtc tgtggactgc 1620
   acctccgaag aaatgaatgc ttttctcgac gtggtgccag gacaggctgg acagaaacag 1680
   atcctgctcg atgccattga caagatcgcc gacgactggg ataatcgcca ccccctgcca 1740
   aacgcccctc tggtggctcc cccacagggg cctatcccta tgaccgctag gttcattagg 1800
   ggactggggg tgccccgcga acgccagatg gagccagcat ttgaccaatt taggcagacc 1860
   tacagacagt ggatcatcga agccatgagc gaggggatta aagtcatgat cggaaagccc 1920
   aaggcacaga acatcaggca gggggccaag gaaccatacc ctgagtttgt cgacaggctt 1980
   ctgtcccaga ttaaatccga aggccaccct caggagatct ccaagttctt gacagacaca 2040
   ctgactatcc aaaatgcaaa tgaagagtgc agaaacgcca tgaggcacct cagacctgaa 2100
   gataccctgg aggagaaaat gtacgcatgt cgcgacattg gcactaccaa gcaaaagatg 2160
   atgctgctcg ccaaggctct gcaaaccggc ctggctggtc cattcaaagg aggagcactg 2220
   aagggaggtc cattgaaagc tgcacaaaca tgttataatt gtgggaagcc aggacattta 2280
   tctagtcaat gtagagcacc taaagtctgt tttaaatgta aacagcctgg acatttctca 2340
   aagcaatgca gaagtgttcc aaaaaacggg aagcaagggg ctcaagggag gccccagaaa 2400
   caaactttcc cgatacaaca gaagagtcag cacaacaaat ctgttgtaca agagactcct 2460
   cagactcaaa atctgtaccc agatctgagc gaaataaaaa aggaatacaa tgtcaaggag 2520
   aaggatcaag tagaggatct caacctggac agtttgtggg agtaacatac aatctcgaga 2580
   agaggcccac taccatcgtc ctgatcaatg acacccctct taatgtgctg ctggacaccg 2640
   gagccgacac cagcgttctc actactgctc actataacag actgaaatac agaggaagga 2700
   aataccaggg cacaggcatc atcggcgttg gaggcaacgt cgaaaccttt tccactcctg 2760
   tcaccatcaa aaagaagggg agacacatta aaaccagaat gctggtcgcc gacatccccg 2820
   tcaccatcct tggcagagac attctccagg acctgggcgc taaactcgtg ctggcacaac 2880
   tgtctaagga aatcaagttc cgcaagatcg agctgaaaga gggcacaatg ggtccaaaaa 2940
   tcccccagtg gcccctgacc aaagagaagc ttgagggcgc taaggaaatc gtgcagcgcc 3000
   tgctttctga gggcaagatt agcgaggcca gcgacaataa cccttacaac agccccatct 3060
   ttgtgattaa gaaaaggagc ggcaaatgga gactcctgca ggacctgagg gaactcaaca 3120
   agaccgtcca ggtcggaact gagatctctc gcggactgcc tcaccccggc ggcctgatta 3180
   aatgcaagca catgacagtc cttgacattg gagacgctta ttttaccatc cccctcgatc 3240
   ctgaatttcg cccctatact gcttttacca tccccagcat caatcaccag gagcccgata 3300
   aacgctatgt gtggaagtgc ctcccccagg gatttgtgct tagcccctac atttaccaga 3360
   agacacttca agagatcctc caacctttcc gcgaaagata cccagaggtt caactctacc 3420
   aatatatgga cgacctgttc atggggtcca acgggtctaa gaagcagcac aaggaactca 3480
   tcatcgaact gagggcaatc ctcctggaga aaggcttcga gacacccgac gacaagctgc 3540
   aagaagttcc tccatatagc tggctgggct accagctttg ccctgaaaac tggaaagtcc 3600
   agaagatgca gttggatatg gtcaagaacc caacactgaa cgacgtccag aagctcatgg 3660
   gcaatattac ctggatgagc tccggaatcc ctgggcttac cgttaagcac attgccgcaa 3720
   ctacaaaagg atgcctggag ttgaaccaga aggtcatttg gacagaggaa gctcagaagg 3780
   aactggagga gaataatgaa aagattaaga atgctcaagg gctccaatac tacaatcccg 3840
   aagaagaaat gttgtgcgag gtcgaaatca ctaagaacta cgaagccacc tatgtcatca 3900
   aacagtccca aggcatcttg tgggccggaa agaaaatcat gaaggccaac aaaggctggt 3960
   ccaccgttaa aaatctgatg ctcctgctcc agcacgtcgc caccgagtct atcacccgcg 4020
   tcggcaagtg ccccaccttc aaagttccct tcactaagga gcaggtgatg tgggagatgc 4080
   aaaaaggctg gtactactct tggcttcccg agatcgtcta cacccaccaa gtggtgcacg 4140
   acgactggag aatgaagctt gtcgaggagc ccactagcgg aattacaatc tataccgacg 4200
   gcggaaagca aaacggagag ggaatcgctg catacgtcac atctaacggc cgcaccaagc 4260
   aaaagaggct cggccctgtc actcaccagg tggctgagag gatggctatc cagatggccc 4320
   ttgaggacac tagagacaag caggtgaaca ttgtgactga cagctactac tgctggaaaa 4380
   acatcacaga gggccttggc ctggagggac cccagtctcc ctggtggcct atcatccaga 4440
   atatccgcga aaaggaaatt gtctatttcg cctgggtgcc tggacacaaa ggaatttacg 4500
   gcaaccaact cgccgatgaa gccgccaaaa ttaaagagga aatcatgctt gcctaccagg 4560
   gcacacagat taaggagaag agagacgagg acgctggctt tgacctgtgt gtgccatacg 4620
   acatcatgat tcccgttagc gacacaaaga tcattccaac cgatgtcaag atccaggtgc 4680
   cacccaattc atttggttgg gtgaccggaa agtccagcat ggctaagcag ggtcttctga 4740
   ttaacggggg aatcattgat gaaggataca ccggcgaaat ccaggtgatc tgcacaaata 4800
   tcggcaaaag caatattaag cttatcgaag ggcagaagtt cgctcaactc atcatcctcc 4860
   agcaccacag caattcaaga caaccttggg acgaaaacaa gattagccag agaggtgaca 4920
   agggcttcgg cagcacaggt gtgttctggg tggagaacat ccaggaagca caggacgagc 4980
   acgagaattg gcacacctcc cctaagattt tggcccgcaa ttacaagatc ccactgactg 5040
   tggctaagca gatcacacag gaatgccccc actgcaccaa acaaggttct ggccccgccg 5100
   gctgcgtgat gaggtccccc aatcactggc aggcagattg cacccacctc gacaacaaaa 5160
   ttatcctgac cttcgtggag agcaattccg gctacatcca cgcaacactc ctctccaagg 5220
   aaaatgcatt gtgcacctcc ctcgcaattc tggaatgggc caggctgttc tctccaaaat 5280
   ccctgcacac cgacaacggc accaactttg tggctgaacc tgtggtgaat ctgctgaagt 5340
   tcctgaaaat cgcccacacc actggcattc cctatcaccc tgaaagccag ggcattgtcg 5400
   agagggccaa cagaactctg aaagaaaaga tccaatctca cagagacaat acacagacat 5460
   tggaggccgc acttcagctc gcccttatca cctgcaacaa aggaagagaa agcatgggcg 5520
   gccagacccc ctgggaggtc ttcatcacta accaggccca ggtcatccat gaaaagctgc 5580
   tcttgcagca ggcccagtcc tccaaaaagt tctgctttta taagatcccc ggtgagcacg 5640
   actggaaagg tcctacaaga gttttgtgga aaggagacgg cgcagttgtg gtgaacgatg 5700
   agggcaaggg gatcatcgct gtgcccctga cacgcaccaa gcttctcatc aagccaaact 5760
   gaacccgggg cggccgcttc cctttagtga gggttaatgc ttcgagcaga catgataaga 5820
   tacattgatg agtttggaca aaccacaact agaatgcagt gaaaaaaatg ctttatttgt 5880
   gaaatttgtg atgctattgc tttatttgta accattataa gctgcaataa acaagttaac 5940
   aacaacaatt gcattcattt tatgtttcag gttcaggggg agatgtggga ggttttttaa 6000
   agcaagtaaa acctctacaa atgtggtaaa atccgataag gatcgatccg ggctggcgta 6060
   atagcgaaga ggcccgcacc gatcgccctt cccaacagtt gcgcagcctg aatggcgaat 6120
   ggacgcgccc tgtagcggcg cattaagcgc ggcgggtgtg gtggttacgc gcagcgtgac 6180
   cgctacactt gccagcgccc tagcgcccgc tcctttcgct ttcttccctt cctttctcgc 6240
   cacgttcgcc ggctttcccc gtcaagctct aaatcggggg ctccctttag ggttccgatt 6300
   tagagcttta cggcacctcg accgcaaaaa acttgatttg ggtgatggtt cacgtagtgg 6360
   gccatcgccc tgatagacgg tttttcgccc tttgacgttg gagtccacgt tctttaatag 6420
   tggactcttg ttccaaactg gaacaacact caaccctatc tcggtctatt cttttgattt 6480
   ataagggatt ttgccgattt cggcctattg gttaaaaaat gagctgattt aacaaatatt 6540
   taacgcgaat tttaacaaaa tattaacgtt tacaatttcg cctgatgcgg tattttctcc 6600
   ttacgcatct gtgcggtatt tcacaccgca tacgcggatc tgcgcagcac catggcctga 6660
   aataacctct gaaagaggaa cttggttagg taccttctga ggcggaaaga accagctgtg 6720
   gaatgtgtgt cagttagggt gtggaaagtc cccaggctcc ccagcaggca gaagtatgca 6780
   aagcatgcat ctcaattagt cagcaaccag gtgtggaaag tccccaggct ccccagcagg 6840
   cagaagtatg caaagcatgc atctcaatta gtcagcaacc atagtcccgc ccctaactcc 6900
   gcccatcccg cccctaactc cgcccagttc cgcccattct ccgccccatg gctgactaat 6960
   ttttttattt tatgcagagg ccgaggccgc ctcggcctct gagctattcc agaagtagtg 7020
   aggaggcttt tttggaggcc taggcttttg caaaaagctt gattcttctg acacaacagt 7080
   ctcgaactta aggctagagc caccatgatt gaacaagatg gattgcacgc aggttctccg 7140
   gccgcttggg tggagaggct attcggctat gactgggcac aacagacaat cggctgctct 7200
   gatgccgccg tgttccggct gtcagcgcag gggcgcccgg ttctttttgt caagaccgac 7260
   ctgtccggtg ccctgaatga actgcaggac gaggcagcgc ggctatcgtg gctggccacg 7320
   acgggcgttc cttgcgcagc tgtgctcgac gttgtcactg aagcgggaag ggactggctg 7380
   ctattgggcg aagtgccggg gcaggatctc ctgtcatctc accttgctcc tgccgagaaa 7440
   gtatccatca tggctgatgc aatgcggcgg ctgcatacgc ttgatccggc tacctgccca 7500
   ttcgaccacc aagcgaaaca tcgcatcgag cgagcacgta ctcggatgga agccggtctt 7560
   gtcgatcagg atgatctgga cgaagagcat caggggctcg cgccagccga actgttcgcc 7620
   aggctcaagg cgcgcatgcc cgacggcgag gatctcgtcg tgacccatgg cgatgcctgc 7680
   ttgccgaata tcatggtgga aaatggccgc ttttctggat tcatcgactg tggccggctg 7740
   ggtgtggcgg accgctatca ggacatagcg ttggctaccc gtgatattgc tgaagagctt 7800
   ggcggcgaat gggctgaccg cttcctcgtg ctttacggta tcgccgctcc cgattcgcag 7860
   cgcatcgcct tctatcgcct tcttgacgag ttcttctgag cgggactctg gggttcgaaa 7920
   tgaccgacca agcgacgccc aacctgccat cacgatggcc gcaataaaat atctttattt 7980
   tcattacatc tgtgtgttgg ttttttgtgt gaatcgatag cgataaggat ccgcgtatgg 8040
   tgcactctca gtacaatctg ctctgatgcc gcatagttaa gccagccccg acacccgcca 8100
   acacccgctg acgcgccctg acgggcttgt ctgctcccgg catccgctta cagacaagct 8160
   gtgaccgtct ccgggagctg catgtgtcag aggttttcac cgtcatcacc gaaacgcgcg 8220
   agacgaaagg gcctcgtgat acgcctattt ttataggtta atgtcatgat aataatggtt 8280
   tcttagacgt caggtggcac ttttcgggga aatgtgcgcg gaacccctat ttgtttattt 8340
   ttctaaatac attcaaatat gtatccgctc atgagacaat aaccctgata aatgcttcaa 8400
   taatattgaa aaaggaagag tatgagtatt caacatttcc gtgtcgccct tattcccttt 8460
   tttgcggcat tttgccttcc tgtttttgct cacccagaaa cgctggtgaa agtaaaagat 8520
   gctgaagatc agttgggtgc acgagtgggt tacatcgaac tggatctcaa cagcggtaag 8580
   atccttgaga gttttcgccc cgaagaacgt tttccaatga tgagcacttt taaagttctg 8640
   ctatgtggcg cggtattatc ccgtattgac gccgggcaag agcaactcgg tcgccgcata 8700
   cactattctc agaatgactt ggttgagtac tcaccagtca cagaaaagca tcttacggat 8760
   ggcatgacag taagagaatt atgcagtgct gccataacca tgagtgataa cactgcggcc 8820
   aacttacttc tgacaacgat cggaggaccg aaggagctaa ccgctttttt gcacaacatg 8880
   ggggatcatg taactcgcct tgatcgttgg gaaccggagc tgaatgaagc cataccaaac 8940
   gacgagcgtg acaccacgat gcctgtagca atggcaacaa cgttgcgcaa actattaact 9000
   ggcgaactac ttactctagc ttcccggcaa caattaatag actggatgga ggcggataaa 9060
   gttgcaggac cacttctgcg ctcggccctt ccggctggct ggtttattgc tgataaatct 9120
   ggagccggtg agcgtgggtc tcgcggtatc attgcagcac tggggccaga tggtaagccc 9180
   tcccgtatcg tagttatcta cacgacgggg agtcaggcaa ctatggatga acgaaataga 9240
   cagatcgctg agataggtgc ctcactgatt aagcattggt aactgtcaga ccaagtttac 9300
   tcatatatac tttagattga tttaaaactt catttttaat ttaaaaggat ctaggtgaag 9360
   atcctttttg ataatctcat gaccaaaatc ccttaacgtg agttttcgtt ccactgagcg 9420
   tcagaccccg tagaaaagat caaaggatct tcttgagatc ctttttttct gcgcgtaatc 9480
   tgctgcttgc aaacaaaaaa accaccgcta ccagcggtgg tttgtttgcc ggatcaagag 9540
   ctaccaactc tttttccgaa ggtaactggc ttcagcagag cgcagatacc aaatactgtc 9600
   cttctagtgt agccgtagtt aggccaccac ttcaagaact ctgtagcacc gcctacatac 9660
   ctcgctctgc taatcctgtt accagtggct gctgccagtg gcgataagtc gtgtcttacc 9720
   gggttggact caagacgata gttaccggat aaggcgcagc ggtcgggctg aacggggggt 9780
   tcgtgcacac agcccagctt ggagcgaacg acctacaccg aactgagata cctacagcgt 9840
   gagctatgag aaagcgccac gcttcccgaa gggagaaagg cggacaggta tccggtaagc 9900
   ggcagggtcg gaacaggaga gcgcacgagg gagcttccag ggggaaacgc ctggtatctt 9960
   tatagtcctg tcgggtttcg ccacctctga cttgagcgtc gatttttgtg atgctcgtca 10020
   ggggggcgga gcctatggaa aaacgccagc aacgcggcct ttttacggtt cctggccttt 10080
   tgctggcctt ttgctcacat ggctcgacag atct                             10114
<210> 14
   <211> 5993
   <212> DNA
   <213> Artificial Sequence
<220>
   <223> Description of Artificial Sequence: pClneoERev, EIAV Rev expression plasmid
<400> 14
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagt aacggccgcc agtgtgctgg aattcggctt atggcagaat cgaaggaagc 1140
   aagagaccaa gaaatgaacc tgaaagaaga atctaaagaa gaaaaaagaa gaaatgactg 1200
   gtggaaaata gatcctcagg gccctctgga aggtgaccag tggtgcaggg tcctccggca 1260
   gtcgttacct gaagaaaaaa ttccatcaca aacatgcatc gcgagaagac acctgggacc 1320
   aggcccaaca caacatacac ctagcaggcg tgaccggtgg atcaggggac aaatactaca 1380
   agcagaagta ctccaggaac gactggaatg gagaatcaga ggagtacaac aggcggccaa 1440
   agagctgggt gaagtcaatc gaggcatttg gagagagcta tatttccgag aagaccaaag 1500
   gggagatttc tcagcctggg gcggctatca acgagcacaa gaacggctct ggggggaaca 1560
   atcctcacca agggtcctta gacctggaga ttcgaagcga aggaggaaac atttatgaag 1620
   ccgaattctg cagatatcca tcacactggc ggccgcttcc ctttagtgag ggttaatgct 1680
   tcgagcagac atgataagat acattgatga gtttggacaa accacaacta gaatgcagtg 1740
   aaaaaaatgc tttatttgtg aaatttgtga tgctattgct ttatttgtaa ccattataag 1800
   ctgcaataaa caagttaaca acaacaattg cattcatttt atgtttcagg ttcaggggga 1860
   gatgtgggag gttttttaaa gcaagtaaaa cctctacaaa tgtggtaaaa tccgataagg 1920
   atcgatccgg gctggcgtaa tagcgaagag gcccgcaccg atcgcccttc ccaacagttg 1980
   cgcagcctga atggcgaatg gacgcgccct gtagcggcgc attaagcgcg gcgggtgtgg 2040
   tggttacgcg cagcgtgacc gctacacttg ccagcgccct agcgcccgct cctttcgctt 2100
   tcttcccttc ctttctcgcc acgttcgccg gctttccccg tcaagctcta aatcgggggc 2160
   tccctttagg gttccgattt agagctttac ggcacctcga ccgcaaaaaa cttgatttgg 2220
   gtgatggttc acgtagtggg ccatcgccct gatagacggt ttttcgccct ttgacgttgg 2280
   agtccacgtt ctttaatagt ggactcttgt tccaaactgg aacaacactc aaccctatct 2340
   cggtctattc ttttgattta taagggattt tgccgatttc ggcctattgg ttaaaaaatg 2400
   agctgattta acaaatattt aacgcgaatt ttaacaaaat attaacgttt acaatttcgc 2460
   ctgatgcggt attttctcct tacgcatctg tgcggtattt cacaccgcat acgcggatct 2520
   gcgcagcacc atggcctgaa ataacctctg aaagaggaac ttggttaggt accttctgag 2580
   gcggaaagaa ccagctgtgg aatgtgtgtc agttagggtg tggaaagtcc ccaggctccc 2640
   cagcaggcag aagtatgcaa agcatgcatc tcaattagtc agcaaccagg tgtggaaagt 2700
   ccccaggctc cccagcaggc agaagtatgc aaagcatgca tctcaattag tcagcaacca 2760
   tagtcccgcc cctaactccg cccatcccgc ccctaactcc gcccagttcc gcccattctc 2820
   cgccccatgg ctgactaatt ttttttattt atgcagaggc cgaggccgcc tcggcctctg 2880
   agctattcca gaagtagtga ggaggctttt ttggaggcct aggcttttgc aaaaagcttg 2940
   attcttctga cacaacagtc tcgaacttaa ggctagagcc accatgattg aacaagatgg 3000
   attgcacgca ggttctccgg ccgcttgggt ggagaggcta ttcggctatg actgggcaca 3060
   acagacaatc ggctgctctg atgccgccgt gttccggctg tcagcgcagg ggcgcccggt 3120
   tctttttgtc aagaccgacc tgtccggtgc cctgaatgaa ctgcaggacg aggcagcgcg 3180
   gctatcgtgg ctggccacga cgggcgttcc ttgcgcagct gtgctcgacg ttgtcactga 3240
   agcgggaagg gactggctgc tattgggcga agtgccgggg caggatctcc tgtcatctca 3300
   ccttgctcct gccgagaaag tatccatcat ggctgatgca atgcggcggc tgcatacgct 3360
   tgatccggct acctgcccat tcgaccacca agcgaaacat cgcatcgagc gagcacgtac 3420
   tcggatggaa gccggtcttg tcgatcagga tgatctggac gaagagcatc aggggctcgc 3480
   gccagccgaa ctgttcgcca ggctcaaggc gcgcatgccc gacggcgagg atctcgtcgt 3540
   gacccatggc gatgcctgct tgccgaatat catggtggaa aatggccgct tttctggatt 3600
   catcgactgt ggccggctgg gtgtggcgga ccgctatcag gacatagcgt tggctacccg 3660
   tgatattgct gaagagcttg gcggcgaatg ggctgaccgc ttcctcgtgc tttacggtat 3720
   cgccgctccc gattcgcagc gcatcgcctt ctatcgcctt cttgacgagt tcttctgagc 3780
   gggactctgg ggttcgaaat gaccgaccaa gcgacgccca acctgccatc acgatggccg 3840
   caataaaata tctttatttt cattacatct gtgtgttggt tttttgtgtg aatcgatagc 3900
   gataaggatc cgcgtatggt gcactctcag tacaatctgc tctgatgccg catagttaag 3960
   ccagccccga cacccgccaa cacccgctga cgcgccctga cgggcttgtc tgctcccggc 4020
   atccgcttac agacaagctg tgaccgtctc cgggagctgc atgtgtcaga ggttttcacc 4080
   gtcatcaccg aaacgcgcga gacgaaaggg cctcgtgata cgcctatttt tataggttaa 4140
   tgtcatgata ataatggttt cttagacgtc aggtggcact tttcggggaa atgtgcgcgg 4200
   aacccctatt tgtttatttt tctaaataca ttcaaatatg tatccgctca tgagacaata 4260
   accctgataa atgcttcaat aatattgaaa aaggaagagt atgagtattc aacatttccg 4320
   tgtcgccctt attccctttt ttgcggcatt ttgccttcct gtttttgctc acccagaaac 4380
   gctggtgaaa gtaaaagatg ctgaagatca gttgggtgca cgagtgggtt acatcgaact 4440
   ggatctcaac agcggtaaga tccttgagag ttttcgcccc gaagaacgtt ttccaatgat 4500
   gagcactttt aaagttctgc tatgtggcgc ggtattatcc cgtattgacg ccgggcaaga 4560
   gcaactcggt cgccgcatac actattctca gaatgacttg gttgagtact caccagtcac 4620
   agaaaagcat cttacggatg gcatgacagt aagagaatta tgcagtgctg ccataaccat 4680
   gagtgataac actgcggcca acttacttct gacaacgatc ggaggaccga aggagctaac 4740
   cgcttttttg cacaacatgg gggatcatgt aactcgcctt gatcgttggg aaccggagct 4800
   gaatgaagcc ataccaaacg acgagcgtga caccacgatg cctgtagcaa tggcaacaac 4860
   gttgcgcaaa ctattaactg gcgaactact tactctagct tcccggcaac aattaataga 4920
   ctggatggag gcggataaag ttgcaggacc acttctgcgc tcggcccttc cggctggctg 4980
   gtttattgct gataaatctg gagccggtga gcgtgggtct cgcggtatca ttgcagcact 5040
   ggggccagat ggtaagccct cccgtatcgt agttatctac acgacgggga gtcaggcaac 5100
   tatggatgaa cgaaatagac agatcgctga gataggtgcc tcactgatta agcattggta 5160
   actgtcagac caagtttact catatatact ttagattgat ttaaaacttc atttttaatt 5220
   taaaaggatc taggtgaaga tcctttttga taatctcatg accaaaatcc cttaacgtga 5280
   gttttcgttc cactgagcgt cagaccccgt agaaaagatc aaaggatctt cttgagatcc 5340
   tttttttctg cgcgtaatct gctgcttgca aacaaaaaaa ccaccgctac cagcggtggt 5400
   ttgtttgccg gatcaagagc taccaactct ttttccgaag gtaactggct tcagcagagc 5460
   gcagatacca aatactgtcc ttctagtgta gccgtagtta ggccaccact tcaagaactc 5520
   tgtagcaccg cctacatacc tcgctctgct aatcctgtta ccagtggctg ctgccagtgg 5580
   cgataagtcg tgtcttaccg ggttggactc aagacgatag ttaccggata aggcgcagcg 5640
   gtcgggctga acggggggtt cgtgcacaca gcccagcttg gagcgaacga cctacaccga 5700
   actgagatac ctacagcgtg agctatgaga aagcgccacg cttcccgaag ggagaaaggc 5760
   ggacaggtat ccggtaagcg gcagggtcgg aacaggagag cgcacgaggg agcttccagg 5820
   gggaaacgcc tggtatcttt atagtcctgt cgggtttcgc cacctctgac ttgagcgtcg 5880
   atttttgtga tgctcgtcag gggggcggag cctatggaaa aacgccagca acgcggcctt 5940
   tttacggttc ctggcctttt gctggccttt tgctcacatg gctcgacaga tct 5993
<210> 15
   <211> 5961
   <212> DNA
   <213> Artificial Sequence
<220>
   <223> Description of Artificial Sequence: pESYNREV,
   codon-optimised EIAV Rev expression plasmid
<400> 15
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgagaatt cgccaccatg gctgagagca aggaggccag ggatcaagag 1140
   atgaacctca aggaagagag caaagaggag aagcgccgca acgactggtg gaagatcgac 1200
   ccacaaggcc ccctggaggg ggaccagtgg tgccgcgtgc tgagacagtc cctgcccgag 1260
   gagaagattc ctagccagac ctgcatcgcc agaagacacc tcggccccgg tcccacccag 1320
   cacacaccct ccagaaggga taggtggatt aggggccaga ttttgcaagc cgaggtcctc 1380
   caagaaaggc tggaatggag aattaggggc gtgcaacaag ccgctaaaga gctgggagag 1440
   gtgaatcgcg gcatctggag ggagctctac ttccgcgagg accagagggg cgatttctcc 1500
   gcatggggag gctaccagag ggcacaagaa aggctgtggg gcgagcagag cagcccccgc 1560
   gtcttgaggc ccggagactc caaaagacgc cgcaaacacc tgtgaagtcg acccgggcgg 1620
   ccgcttccct ttagtgaggg ttaatgcttc gagcagacat gataagatac attgatgagt 1680
   ttggacaaac cacaactaga atgcagtgaa aaaaatgctt tatttgtgaa atttgtgatg 1740
   ctattgcttt atttgtaacc attataagct gcaataaaca agttaacaac aacaattgca 1800
   ttcattttat gtttcaggtt cagggggaga tgtgggaggt tttttaaagc aagtaaaacc 1860
   tctacaaatg tggtaaaatc cgataaggat cgatccgggc tggcgtaata gcgaagaggc 1920
   ccgcaccgat cgcccttccc aacagttgcg cagcctgaat ggcgaatgga cgcgccctgt 1980
   agcggcgcat taagcgcggc gggtgtggtg gttacgcgca gcgtgaccgc tacacttgcc 2040
   agcgccctag cgcccgctcc tttcgctttc ttcccttcct ttctcgccac gttcgccggc 2100
   tttccccgtc aagctctaaa tcgggggctc cctttagggt tccgatttag agctttacgg 2160
   cacctcgacc gcaaaaaact tgatttgggt gatggttcac gtagtgggcc atcgccctga 2220
   tagacggttt ttcgcccttt gacgttggag tccacgttct ttaatagtgg actcttgttc 2280
   caaactggaa caacactcaa ccctatctcg gtctattctt ttgatttata agggattttg 2340
   ccgatttcgg cctattggtt aaaaaatgag ctgatttaac aaatatttaa cgcgaatttt 2400
   aacaaaatat taacgtttac aatttcgcct gatgcggtat tttctcctta cgcatctgtg 2460
   cggtatttca caccgcatac gcggatctgc gcagcaccat ggcctgaaat aacctctgaa 2520
   agaggaactt ggttaggtac cttctgaggc ggaaagaacc agctgtggaa tgtgtgtcag 2580
   ttagggtgtg gaaagtcccc aggctcccca gcaggcagaa gtatgcaaag catgcatctc 2640
   aattagtcag caaccaggtg tggaaagtcc ccaggctccc cagcaggcag aagtatgcaa 2700
   agcatgcatc tcaattagtc agcaaccata gtcccgcccc taactccgcc catcccgccc 2760
   ctaactccgc ccagttccgc ccattctccg ccccatggct gactaatttt ttttatttat 2820
   gcagaggccg aggccgcctc ggcctctgag ctattccaga agtagtgagg aggctttttt 2880
   ggaggcctag gcttttgcaa aaagcttgat tcttctgaca caacagtctc gaacttaagg 2940
   ctagagccac catgattgaa caagatggat tgcacgcagg ttctccggcc gcttgggtgg 3000
   agaggctatt cggctatgac tgggcacaac agacaatcgg ctgctctgat gccgccgtgt 3060
   tccggctgtc agcgcagggg cgcccggttc tttttgtcaa gaccgacctg tccggtgccc 3120
   tgaatgaact gcaggacgag gcagcgcggc tatcgtggct ggccacgacg ggcgttcctt 3180
   gcgcagctgt gctcgacgtt gtcactgaag cgggaaggga ctggctgcta ttgggcgaag 3240
   tgccggggca ggatctcctg tcatctcacc ttgctcctgc cgagaaagta tccatcatgg 3300
   ctgatgcaat gcggcggctg catacgcttg atccggctac ctgcccattc gaccaccaag 3360
   cgaaacatcg catcgagcga gcacgtactc ggatggaagc cggtcttgtc gatcaggatg 3420
   atctggacga agagcatcag gggctcgcgc cagccgaact gttcgccagg ctcaaggcgc 3480
   gcatgcccga cggcgaggat ctcgtcgtga cccatggcga tgcctgcttg ccgaatatca 3540
   tggtggaaaa tggccgcttt tctggattca tcgactgtgg ccggctgggt gtggcggacc 3600
   gctatcagga catagcgttg gctacccgtg atattgctga agagcttggc ggcgaatggg 3660
   ctgaccgctt cctcgtgctt tacggtatcg ccgctcccga ttcgcagcgc atcgccttct 3720
   atcgccttct tgacgagttc ttctgagcgg gactctgggg ttcgaaatga ccgaccaagc 3780
   gacgcccaac ctgccatcac gatggccgca ataaaatatc tttattttca ttacatctgt 3840
   gtgttggttt tttgtgtgaa tcgatagcga taaggatccg cgtatggtgc actctcagta 3900
   caatctgctc tgatgccgca tagttaagcc agccccgaca cccgccaaca cccgctgacg 3960
   cgccctgacg ggcttgtctg ctcccggcat ccgcttacag acaagctgtg accgtctccg 4020
   ggagctgcat gtgtcagagg ttttcaccgt catcaccgaa acgcgcgaga cgaaagggcc 4080
   tcgtgatacg cctattttta taggttaatg tcatgataat aatggtttct tagacgtcag 4140
   gtggcacttt tcggggaaat gtgcgcggaa cccctatttg tttatttttc taaatacatt 4200
   caaatatgta tccgctcatg agacaataac cctgataaat gcttcaataa tattgaaaaa 4260
   ggaagagtat gagtattcaa catttccgtg tcgcccttat tccctttttt gcggcatttt 4320
   gccttcctgt ttttgctcac ccagaaacgc tggtgaaagt aaaagatgct gaagatcagt 4380
   tgggtgcacg agtgggttac atcgaactgg atctcaacag cggtaagatc cttgagagtt 4440
   ttcgccccga agaacgtttt ccaatgatga gcacttttaa agttctgcta tgtggcgcgg 4500
   tattatcccg tattgacgcc gggcaagagc aactcggtcg ccgcatacac tattctcaga 4560
   atgacttggt tgagtactca ccagtcacag aaaagcatct tacggatggc atgacagtaa 4620
   gagaattatg cagtgctgcc ataaccatga gtgataacac tgcggccaac ttacttctga 4680
   caacgatcgg aggaccgaag gagctaaccg cttttttgca caacatgggg gatcatgtaa 4740
   ctcgccttga tcgttgggaa ccggagctga atgaagccat accaaacgac gagcgtgaca 4800
   ccacgatgcc tgtagcaatg gcaacaacgt tgcgcaaact attaactggc gaactactta 4860
   ctctagcttc ccggcaacaa ttaatagact ggatggaggc ggataaagtt gcaggaccac 4920
   ttctgcgctc ggcccttccg gctggctggt ttattgctga taaatctgga gccggtgagc 4980
   gtgggtctcg cggtatcatt gcagcactgg ggccagatgg taagccctcc cgtatcgtag 5040
   ttatctacac gacggggagt caggcaacta tggatgaacg aaatagacag atcgctgaga 5100
   taggtgcctc actgattaag cattggtaac tgtcagacca agtttactca tatatacttt 5160
   agattgattt aaaacttcat ttttaattta aaaggatcta ggtgaagatc ctttttgata 5220
   atctcatgac caaaatccct taacgtgagt tttcgttcca ctgagcgtca gaccccgtag 5280
   aaaagatcaa aggatcttct tgagatcctt tttttctgcg cgtaatctgc tgcttgcaaa 5340
   caaaaaaacc accgctacca gcggtggttt gtttgccgga tcaagagcta ccaactcttt 5400
   ttccgaaggt aactggcttc agcagagcgc agataccaaa tactgtcctt ctagtgtagc 5460
   cgtagttagg ccaccacttc aagaactctg tagcaccgcc tacatacctc gctctgctaa 5520
   tcctgttacc agtggctgct gccagtggcg ataagtcgtg tcttaccggg ttggactcaa 5580
   gacgatagtt accggataag gcgcagcggt cgggctgaac ggggggttcg tgcacacagc 5640
   ccagcttgga gcgaacgacc tacaccgaac tgagatacct acagcgtgag ctatgagaaa 5700
   gcgccacgct tcccgaaggg agaaaggcgg acaggtatcc ggtaagcggc agggtcggaa 5760
   caggagagcg cacgagggag cttccagggg gaaacgcctg gtatctttat agtcctgtcg 5820
   ggtttcgcca cctctgactt gagcgtcgat ttttgtgatg ctcgtcaggg gggcggagcc 5880
   tatggaaaaa cgccagcaac gcggcctttt tacggttcct ggccttttgc tggccttttg 5940
   ctcacatggc tcgacagatc t 5961
<210> 16
   <211> 42
   <212> DNA
   <213> Artificial Sequence
<220>
   <223> Description of Artificial Sequence: Primer
<400> 16
   ggctagagaa ttccaggtaa gatgggcgat cccctcacct gg 42
<210> 17
   <211> 22
   <212> DNA
   <213> Artificial Sequence
<220>
   <223> Description of Artificial Sequence: Primer
<400> 17
   ttgggtactc ctcgctaggt tc 22
<210> 18
   <211> 21
   <212> DNA
   <213> Artificial Sequence
<220>
   <223> Description of Artificial Sequence: Sequence flanking the EIAV gag/pol ORF
<400> 18
   tctagagaat tcgccaccat g 21
<210> 19
   <211> 18
   <212> RNA
   <213> Artificial Sequence
<220>
   <223> Description of Artificial Sequence: Sequence flanking the EIAV gag/pol ORF
<400> 19
   ugaacccggg gcggccgc 18
<210> 20
   <211> 42
   <212> DNA
   <213> Artificial Sequence
<220>
   <223> Description of Artificial Sequence: Primer
<400> 20
   cagtacccgc gggccaccat gtttgactgt atggatgttc tg 42
<210> 21
   <211> 37
   <212> DNA
   <213> Artificial Sequence
<220>
   <223> Description of Artificial Sequence: Primer
<400> 21
   cagtacctgc agatcattgc acgagtggtg actgact 37
<210> 22
   <211> 38
   <212> DNA
   <213> Artificial Sequence
<220>
   <223> Description of Artificial Sequence: Primer
<400> 22
   caggttattc tagagtcgac gctctcatta cttgtaac 38
<210> 23
   <211> 41
   <212> DNA
   <213> Artificial Sequence
<220>
   <223> Description of Artificial Sequence: Primer
<400> 23
   cgaatgcgtt ctagagtcga ccatgttcac cagggatttt g 41
<210> 24
   <211> 23
   <212> DNA
   <213> Artificial Sequence
<220>
   <223> Description of Artificial Sequence: Primer
<400> 24
   cacctagcag gcgtgaccgg tgg 23
<210> 25
   <211> 37
   <212> DNA
   <213> Artificial Sequence
<220>
   <223> Description of Artificial Sequence: Primer
<400> 25
   cctaccaatt gtataaaacc cctcataaaa accccac 37
<210> 26
   <211> 24
   <212> DNA
   <213> Artificial Sequence
<220>
   <223> Description of Artificial Sequence: Primer
<400> 26
   cacaggtcaa acctcctagg aatg 24
<210> 27
   <211> 21
   <212> DNA
   <213> Artificial Sequence
<220>
   <223> Description of Artificial Sequence: Primer
<400> 27
   tcctgctcaa cttcctgtcg a 21
<210> 28
   <211> 19
   <212> DNA
   <213> Artificial Sequence
<220>
   <223> Description of Artificial Sequence: Probe
<400> 28
   cgagacgcta ccatggcta 19
<210> 29
   <211> 29
   <212> DNA
   <213> Artificial Sequence
<220>
   <223> Description of Artificial Sequence: Primer
<400> 29
   accagtagtt aatttctgag acccttgta 29
<210> 30
   <211> 21
   <212> DNA
   <213> Artificial Sequence
<220>
   <223> Description of Artificial Sequence: Primer
<400> 30
   attgggagac cctttgacat t 21
<210> 31
   <211> 30
   <212> DNA
   <213> Artificial Sequence
<220>
   <223> Description of Artificial Sequence: Probe
<400> 31
   caccttctct aacttcttga gcgccttgct 30
<210> 32
   <211> 42
   <212> DNA
   <213> Artificial Sequence
<220>
   <223> Description of Artificial Sequence: Primer
<400> 32
   actgccgcgg gccaccatgt ttgactgtat ggatgttctg tc 42
<210> 33
   <211> 66
   <212> DNA
   <213> Artificial Sequence
<220>
   <223> Description of Artificial Sequence: Primer
<400> 33
   actgccgcgg gccaccatgg actacaagga cgacgatgac aagtttgact gtatggatgt 60
   tctgtc 66
<210> 34
   <211> 29
   <212> DNA
   <213> Artificial Sequence
<220>
   <223> Description of Artificial Sequence: Primer
<400> 34
   actggcggcc gctcactgca gcagtggtg 29
<210> 35
   <211> 38
   <212> DNA
   <213> Artificial Sequence
<220>
   <223> Description of Artificial Sequence: Primer
<400> 35
   caggttattc tagagtcgac gctctcatta cttgtaac 38
<210> 36
   <211> 41
   <212> DNA
   <213> Artificial Sequence
<220>
   <223> Description of Artificial Sequence: Primer
<400> 36
   cgaatgcgtt ctagagtcga ccatgttcac cagggatttt g 41
<210> 37
   <211> 19
   <212> DNA
   <213> Artificial Sequence
<220>
   <223> Description of Artificial Sequence: Primer
<400> 37
   tgtagctctc tgagcactc 19
<210> 38
   <211> 19
   <212> DNA
   <213> Artificial Sequence
<220>
   <223> Description of Artificial Sequence: Primer
<400> 38
   tacgccttct tctttcccc 19
<210> 39
   <211> 21
   <212> DNA
   <213> Artificial Sequence
<220>
   <223> Description of Artificial Sequence: Primer
<400> 39
   cttttataac cagaaccggg c 21
<210> 40
   <211> 21
   <212> DNA
   <213> Artificial Sequence
<220>
   <223> Description of Artificial Sequence: Primer
<400> 40
   caagtagaag ccaggaaagt c 21
<210> 41
   <211> 21
   <212> DNA
   <213> Artificial Sequence
<220>
   <223> Description of Artificial Sequence: Primer
<400> 41
   ctaagaagac ccacacttct g 21
<210> 42
   <211> 19
   <212> DNA
   <213> Artificial Sequence
<220>
   <223> Description of Artificial Sequence: Primer
<400> 42
   aagtgaggtg aaaactggg 19
<210> 43
   <211> 18
   <212> DNA
   <213> Artificial Sequence
<220>
   <223> Description of Artificial Sequence: Primer
<400> 43
   ttcacagcct ggcataac 18
<210> 44
   <211> 19
   <212> DNA
   <213> Artificial Sequence
<220>
   <223> Description of Artificial Sequence: Primer
<400> 44
   gagaaggaag tgagccatc 19
<210> 45
   <211> 21
   <212> DNA
   <213> Artificial Sequence
<220>
   <223> Description of Artificial Sequence: Primer
<400> 45
   tctctgtgca ttcctgcttt g 21
<210> 46
   <211> 20
   <212> DNA
   <213> Artificial Sequence
<220>
   <223> Description of Artificial Sequence: Primer
<400> 46
   tggacacatg actcactacc 20
<210> 47
   <211> 20
   <212> DNA
   <213> Artificial Sequence
<220>
   <223> Description of Artificial Sequence: Primer
<400> 47
   atgttctgtc agtgagtccc 20
<210> 48
   <211> 19
   <212> DNA
   <213> Artificial Sequence
<220>
   <223> Description of Artificial Sequence: Primer
<400> 48
   gcatgtcaga ggacaactg 19
<210> 49
   <211> 18
   <212> DNA
   <213> Artificial Sequence
<220>
   <223> Description of Artificial Sequence: Primer
<400> 49
   agcctggaaa atgccatc 18
<210> 50
   <211> 22
   <212> DNA
   <213> Artificial Sequence
<220>
   <223> Description of Artificial Sequence: Primer
<400> 50
   ttacagcttc cttggacatg cc 22
<210> 51
   <211> 20
   <212> DNA
   <213> Artificial Sequence
<220>
   <223> Description of Artificial Sequence: Primer
<400> 51
   agatgctgag ccctagcttc 20
<210> 52
   <211> 20
   <212> DNA
   <213> Artificial Sequence
<220>
   <223> Description of Artificial Sequence: Primer
<400> 52
   ttactacgca gagccactgg 20
<210> 53
   <211> 19
   <212> DNA
   <213> Artificial Sequence
<220>
   <223> Description of Artificial Sequence: Primer
<400> 53
   ggaagatgga agagggaac 19
<210> 54
   <211> 19
   <212> DNA
   <213> Artificial Sequence
<220>
   <223> Description of Artificial Sequence: Primer
<400> 54
   caaatttact gggggttgg 19
<210> 55
   <211> 20
   <212> DNA
   <213> Artificial Sequence
<220>
   <223> Description of Artificial Sequence: Primer
<400> 55
   ggctggattt tggattgaag 20
<210> 56
   <211> 22
   <212> DNA
   <213> Artificial Sequence
<220>
   <223> Description of Artificial Sequence: Primer
<400> 56
   ttctgtcctc tcactacctt gg 22
<210> 57
   <211> 20
   <212> DNA
   <213> Artificial Sequence
<220>
   <223> Description of Artificial Sequence: Primer
<400> 57
   cattaccgcg agtcactaac 20
<210> 58
   <211> 20
   <212> DNA
   <213> Artificial Sequence
<220>
   <223> Description of Artificial Sequence: Primer
<400> 58
   cgtagacaaa atggtgaagg 20
<210> 59
   <211> 20
   <212> DNA
   <213> Artificial Sequence
<220>
   <223> Description of Artificial Sequence: Primer
<400> 59
   gactccacga catactcagc 20
<210> 60
   <211> 18
   <212> DNA
   <213> Artificial Sequence
<220>
   <223> Description of Artificial Sequence: Primer
<400> 60
   gcttcttcat tgacccac 18
<210> 61
   <211> 20
   <212> DNA
   <213> Artificial Sequence
<220>
   <223> Description of Artificial Sequence: Primer
<400> 61
   cttcaccgtc aggtctttac 20
<210> 62
   <211> 20
   <212> DNA
   <213> Artificial Sequence
<220>
   <223> Description of Artificial Sequence: Primer
<400> 62
   gcaaggaccg gaatgagaac 20
<210> 63
   <211> 21
   <212> DNA
   <213> Artificial Sequence
<220>
   <223> Description of Artificial Sequence: Primer
<400> 63
   tctaggggca gctcagaaaa g 21
<210> 64
   <211> 21
   <212> DNA
   <213> Artificial Sequence
<220>
   <223> Description of Artificial Sequence: Primer
<400> 64
   agaataaagg ggtagtgaag g 21
<210> 65
   <211> 18
   <212> DNA
   <213> Artificial Sequence
<220>
   <223> Description of Artificial Sequence: Primer
<400> 65
   catcaatgtc cccacttg 18
<210> 66
   <211> 20
   <212> DNA
   <213> Artificial Sequence
<220>
   <223> Description of Artificial Sequence: Primer
<400> 66
   tgccagtagt agccacgaag 20
<210> 67
   <211> 19
   <212> DNA
   <213> Artificial Sequence
<220>
   <223> Description of Artificial Sequence: Primer
<400> 67
   tgagcagttc attccaccc 19
<210> 68
   <211> 10998
   <212> DNA
   <213> Artificial Sequence
<220>
   <223> Description of Artificial Sequence: pONY8.OZ
<400> 68
   agatcttgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaattgatat ttgaaaatat ggcatattga aaatgtcgcc gatgtgagtt tctgtgtaac 180
   tgatatcgcc atttttccaa aagtgatttt tgggcatacg cgatatctgg cgatagcgct 240
   tatatcgttt acgggggatg gcgatagacg actttggtga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggcc 420
   attagccata ttattcattg gttatatagc ataaatcaat attggctatt ggccattgca 480
   tacgttgtat ccatatcgta atatgtacat ttatattggc tcatgtccaa cattaccgcc 540
   atgttgacat tgattattga ctagttatta atagtaatca attacggggt cattagttca 600
   tagcccatat atggagttcc gcgttacata acttacggta aatggcccgc ctggctgacc 660
   gcccaacgac ccccgcccat tgacgtcaat aatgacgtat gttcccatag taacgccaat 720
   agggactttc cattgacgtc aatgggtgga gtatttacgg taaactgccc acttggcagt 780
   acatcaagtg tatcatatgc caagtccgcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aacaactgcg atcgcccgcc 1080
   ccgttgacgc aaatgggcgg taggcgtgta cggtgggagg tctatataag cagagctcgt 1140
   ttagtgaacc gggcactcag attctgcggt ctgagtccct tctctgctgg gctgaaaagg 1200
   cctttgtaat aaatataatt ctctactcag tccctgtctc tagtttgtct gttcgagatc 1260
   ctacagttgg cgcccgaaca gggacctgag aggggcgcag accctacctg ttgaacctgg 1320
   ctgatcgtag gatccccggg acagcagagg agaacttaca gaagtcttct ggaggtgttc 1380
   ctggccagaa cacaggagga caggtaagat tgggagaccc tttgacattg gagcaaggcg 1440
   ctcaagaagt tagagaaggt gacggtacaa gggtctcaga aattaactac tggtaactgt 1500
   aattgggcgc taagtctagt agacttattt catgatacca actttgtaaa agaaaaggac 1560
   tggcagctga gggatgtcat tccattgctg gaagatgtaa ctcagacgct gtcaggacaa 1620
   gaaagagagg cctttgaaag aacatggtgg gcaatttctg ctgtaaagat gggcctccag 1680
   attaataatg tagtagatgg aaaggcatca ttccagctcc taagagcgaa atatgaaaag 1740
   aagactgcta ataaaaagca gtctgagccc tctgaagaat atctctagaa ctagtggatc 1800
   ccccgggctg caggagtggg gaggcacgat ggccgctttg gtcgaggcgg atccggccat 1860
   tagccatatt attcattggt tatatagcat aaatcaatat tggctattgg ccattgcata 1920
   cgttgtatcc atatcataat atgtacattt atattggctc atgtccaaca ttaccgccat 1980
   gttgacattg attattgact agttattaat agtaatcaat tacggggtca ttagttcata 2040
   gcccatatat ggagttccgc gttacataac ttacggtaaa tggcccgcct ggctgaccgc 2100
   ccaacgaccc ccgcccattg acgtcaataa tgacgtatgt tcccatagta acgccaatag 2160
   ggactttcca ttgacgtcaa tgggtggagt atttacggta aactgcccac ttggcagtac 2220
   atcaagtgta tcatatgcca agtacgcccc ctattgacgt caatgacggt aaatggcccg 2280
   cctggcatta tgcccagtac atgaccttat gggactttcc tacttggcag tacatctacg 2340
   tattagtcat cgctattacc atggtgatgc ggttttggca gtacatcaat gggcgtggat 2400
   agcggtttga ctcacgggga tttccaagtc tccaccccat tgacgtcaat gggagtttgt 2460
   tttggcacca aaatcaacgg gactttccaa aatgtcgtaa caactccgcc ccattgacgc 2520
   aaatgggcgg taggcatgta cggtgggagg tctatataag cagagctcgt ttagtgaacc 2580
   gtcagatcgc ctggagacgc catccacgct gttttgacct ccatagaaga caccgggacc 2640
   gatccagcct ccgcggcccc aagcttcagc tgctcgagga tctgcggatc cggggaattc 2700
   cccagtctca ggatccacca tgggggatcc cgtcgtttta caacgtcgtg actgggaaaa 2760
   ccctggcgtt acccaactta atcgccttgc agcacatccc cctttcgcca gctggcgtaa 2820
   tagcgaagag gcccgcaccg atcgcccttc ccaacagttg cgcagcctga atggcgaatg 2880
   gcgctttgcc tggtttccgg caccagaagc ggtgccggaa agctggctgg agtgcgatct 2940
   tcctgaggcc gatactgtcg tcgtcccctc aaactggcag atgcacggtt acgatgcgcc 3000
   catctacacc aacgtaacct atcccattac ggtcaatccg ccgtttgttc ccacggagaa 3060
   tccgacgggt tgttactcgc tcacatttaa tgttgatgaa agctggctac aggaaggcca 3120
   gacgcgaatt atttttgatg gcgttaactc ggcgtttcat ctgtggtgca acgggcgctg 3180
   ggtcggttac ggccaggaca gtcgtttgcc gtctgaattt gacctgagcg catttttacg 3240
   cgccggagaa aaccgcctcg cggtgatggt gctgcgttgg agtgacggca gttatctgga 3300
   agatcaggat atgtggcgga tgagcggcat tttccgtgac gtctcgttgc tgcataaacc 3360
   gactacacaa atcagcgatt tccatgttgc cactcgcttt aatgatgatt tcagccgcgc 3420
   tgtactggag gctgaagttc agatgtgcgg cgagttgcgt gactacctac gggtaacagt 3480
   ttctttatgg cagggtgaaa cgcaggtcgc cagcggcacc gcgcctttcg gcggtgaaat 3540
   tatcgatgag cgtggtggtt atgccgatcg cgtcacacta cgtctgaacg tcgaaaaccc 3600
   gaaactgtgg agcgccgaaa tcccgaatct ctatcgtgcg gtggttgaac tgcacaccgc 3660
   cgacggcacg ctgattgaag cagaagcctg cgatgtcggt ttccgcgagg tgcggattga 3720
   aaatggtctg ctgctgctga acggcaagcc gttgctgatt cgaggcgtta accgtcacga 3780
   gcatcatcct ctgcatggtc aggtcatgga tgagcagacg atggtgcagg atatcctgct 3840
   gatgaagcag aacaacttta acgccgtgcg ctgttcgcat tatccgaacc atccgctgtg 3900
   gtacacgctg tgcgaccgct acggcctgta tgtggtggat gaagccaata ttgaaaccca 3960
   cggcatggtg ccaatgaatc gtctgaccga tgatccgcgc tggctaccgg cgatgagcga 4020
   acgcgtaacg cgaatggtgc agcgcgatcg taatcacccg agtgtgatca tctggtcgct 4080
   ggggaatgaa tcaggccacg gcgctaatca cgacgcgctg tatcgctgga tcaaatctgt 4140
   cgatccttcc cgcccggtgc agtatgaagg cggcggagcc gacaccacgg ccaccgatat 4200
   tatttgcccg atgtacgcgc gcgtggatga agaccagccc ttcccggctg tgccgaaatg 4260
   gtccatcaaa aaatggcttt cgctacctgg agagacgcgc ccgctgatcc tttgcgaata 4320
   cgcccacgcg atgggtaaca gtcttggcgg tttcgctaaa tactggcagg cgtttcgtca 4380
   gtatccccgt ttacagggcg gcttcgtctg ggactgggtg gatcagtcgc tgattaaata 4440
   tgatgaaaac ggcaacccgt ggtcggctta cggcggtgat tttggcgata cgccgaacga 4500
   tcgccagttc tgtatgaacg gtctggtctt tgccgaccgc acgccgcatc cagcgctgac 4560
   ggaagcaaaa caccagcagc agtttttcca gttccgttta tccgggcaaa ccatcgaagt 4620
   gaccagcgaa tacctgttcc gtcatagcga taacgagctc ctgcactgga tggtggcgct 4680
   ggatggtaag ccgctggcaa gcggtgaagt gcctctggat gtcgctccac aaggtaaaca 4740
   gttgattgaa ctgcctgaac taccgcagcc ggagagcgcc gggcaactct ggctcacagt 4800
   acgcgtagtg caaccgaacg cgaccgcatg gtcagaagcc gggcacatca gcgcctggca 4860
   gcagtggcgt ctggcggaaa acctcagtgt gacgctcccc gccgcgtccc acgccatccc 4920
   gcatctgacc accagcgaaa tggatttttg catcgagctg ggtaataagc gttggcaatt 4980
   taaccgccag tcaggctttc tttcacagat gtggattggc gataaaaaac aactgctgac 5040
   gccgctgcgc gatcagttca cccgtgcacc gctggataac gacattggcg taagtgaagc 5100
   gacccgcatt gaccctaacg cctgggtcga acgctggaag gcggcgggcc attaccaggc 5160
   cgaagcagcg ttgttgcagt gcacggcaga tacacttgct gatgcggtgc tgattacgac 5220
   cgctcacgcg tggcagcatc aggggaaaac cttatttatc agccggaaaa cctaccggat 5280
   tgatggtagt ggtcaaatgg cgattaccgt tgatgttgaa gtggcgagcg atacaccgca 5340
   tccggcgcgg attggcctga actgccagct ggcgcaggta gcagagcggg taaactggct 5400
   cggattaggg ccgcaagaaa actatcccga ccgccttact gccgcctgtt ttgaccgctg 5460
   ggatctgcca ttgtcagaca tgtatacccc gtacgtcttc ccgagcgaaa acggtctgcg 5520
   ctgcgggacg cgcgaattga attatggccc acaccagtgg cgcggcgact tccagttcaa 5580
   catcagccgc tacagtcaac agcaactgat ggaaaccagc catcgccatc tgctgcacgc 5640
   ggaagaaggc acatggctga atatcgacgg tttccatatg gggattggtg gcgacgactc 5700
   ctggagcccg tcagtatcgg cggaattcca gctgagcgcc ggtcgctacc attaccagtt 5760
   ggtctggtgt caaaaataat aataaccggg caggggggat ccgcagatcc ggctgtggaa 5820
   tgtgtgtcag ttagggtgtg gaaagtcccc aggctcccca gcaggcagaa gtatgcaaag 5880
   catgcctgca ggaattcgat atcaagctta tcgataccgt cgacctcgag ggggggcccg 5940
   gtacccagct tttgttccct ttagtgaggg ttaattgcgc gggaagtatt tatcactaat 6000
   caagcacaag taatacatga gaaactttta ctacagcaag cacaatcctc caaaaaattt 6060
   tgtttttaca aaatccctgg tgaacatgat tggaagggac ctactagggt gctgtggaag 6120
   ggtgatggtg cagtagtagt taatgatgaa ggaaagggaa taattgctgt accattaacc 6180
   aggactaagt tactaataaa accaaattga gtattgttgc aggaagcaag acccaactac 6240
   cattgtcagc tgtgtttcct gacctcaata tttgttataa ggtttgatat gaatcccagg 6300
   gggaatctca acccctatta cccaacagtc agaaaaatct aagtgtgagg agaacacaat 6360
   gtttcaacct tattgttata ataatgacag taagaacagc atggcagaat cgaaggaagc 6420
   aagagaccaa gaatgaacct gaaagaagaa tctaaagaag aaaaaagaag aaatgactgg 6480
   tggaaaatag gtatgtttct gttatgctta gcaggaacta ctggaggaat actttggtgg 6540
   tatgaaggac tcccacagca acattatata gggttggtgg cgataggggg aagattaaac 6600
   ggatctggcc aatcaaatgc tatagaatgc tggggttcct tcccggggtg tagaccattt 6660
   caaaattact tcagttatga gaccaataga agcatgcata tggataataa tactgctaca 6720
   ttattagaag ctttaaccaa tataactgct ctataaataa caaaacagaa ttagaaacat 6780
   ggaagttagt aaagacttct ggcataactc ctttacctat ttcttctgaa gctaacactg 6840
   gactaattag acataagaga gattttggta taagtgcaat agtggcagct attgtagccg 6900
   ctactgctat tgctgctagc gctactatgt cttatgttgc tctaactgag gttaacaaaa 6960
   taatggaagt acaaaatcat acttttgagg tagaaaatag tactctaaat ggtatggatt 7020
   taatagaacg acaaataaag atattatatg ctatgattct tcaaacacat gcagatgttc 7080
   aactgttaaa ggaaagacaa caggtagagg agacatttaa tttaattgga tgtatagaaa 7140
   gaacacatgt attttgtcat actggtcatc cctggaatat gtcatgggga catttaaatg 7200
   agtcaacaca atgggatgac tgggtaagca aaatggaaga tttaaatcaa gagatactaa 7260
   ctacacttca tggagccagg aacaatttgg cacaatccat gataacattc aatacaccag 7320
   atagtatagc tcaatttgga aaagaccttt ggagtcatat tggaaattgg attcctggat 7380
   tgggagcttc cattataaaa tatatagtga tgtttttgct tatttatttg ttactaacct 7440
   cttcgcctaa gatcctcagg gccctctgga aggtgaccag tggtgcaggg tcctccggca 7500
   gtcgttacct gaagaaaaaa ttccatcaca aacatgcatc gcgagaagac acctgggacc 7560
   aggcccaaca caacatacac ctagcaggcg tgaccggtgg atcaggggac aaatactaca 7620
   agcagaagta ctccaggaac gactggaatg gagaatcaga ggagtacaac aggcggccaa 7680
   agagctgggt gaagtcaatc gaggcatttg gagagagcta tatttccgag aagaccaaag 7740
   gggagatttc tcagcctggg gcggctatca acgagcacaa gaacggctct gggggggaca 7800
   atcctcacca agggtcctta gacctggaga ttcgaagcga aggaggaaac atttatgact 7860
   gttgcattaa agcccaagaa ggaactctcg ctatcccttg ctgtggattt cccttatggc 7920
   tattttgggg actagtaatt atagtaggac gcatagcagg ctatggatta cgtggactcg 7980
   ctgttataat aaggatttgt attagaggct taaatttgat atttgaaata atcagaaaaa 8040
   tgcttgatta tattggaaga gctttaaatc ctggcacatc tcatgtatca atgcctcagt 8100
   atgtttagaa aaacaagggg ggaactgtgg ggtttttatg aggggtttta taaatgatta 8160
   taagagtaaa aagaaagttg ctgatgctct cataaccttg tataacccaa aggactagct 8220
   catgttgcta ggcaactaaa ccgcaataac cgcatttgtg acgcgagttc cccattggtg 8280
   acgcgttaac ttcctgtttt tacagtatat aagtgcttgt attctgacaa ttgggcactc 8340
   agattctgcg gtctgagtcc cttctctgct gggctgaaaa ggcctttgta ataaatataa 8400
   ttctctactc agtccctgtc tctagtttgt ctgttcgaga tcctacagag ctcatgcctt 8460
   ggcgtaatca tggtcatagc tgtttcctgt gtgaaattgt tatccgctca caattccaca 8520
   caacatacga gccggaagca taaagtgtaa agcctggggt gcctaatgag tgagctaact 8580
   cacattaatt gcgttgcgct cactgcccgc tttccagtcg ggaaacctgt cgtgccagct 8640
   gcattaatga atcggccaac gcgcggggag aggcggtttg cgtattgggc gctcttccgc 8700
   ttcctcgctc actgactcgc tgcgctcggt cgttcggctg cggcgagcgg tatcagctca 8760
   ctcaaaggcg gtaatacggt tatccacaga atcaggggat aacgcaggaa agaacatgtg 8820
   agcaaaaggc cagcaaaagg ccaggaaccg taaaaaggcc gcgttgctgg cgtttttcca 8880
   taggctccgc ccccctgacg agcatcacaa aaatcgacgc tcaagtcaga ggtggcgaaa 8940
   cccgacagga ctataaagat accaggcgtt tccccctgga agctccctcg tgcgctctcc 9000
   tgttccgacc ctgccgctta ccggatacct gtccgccttt ctcccttcgg gaagcgtggc 9060
   gctttctcat agctcacgct gtaggtatct cagttcggtg taggtcgttc gctccaagct 9120
   gggctgtgtg cacgaacccc ccgttcagcc cgaccgctgc gccttatccg gtaactatcg 9180
   tcttgagtcc aacccggtaa gacacgactt atcgccactg gcagcagcca ctggtaacag 9240
   gattagcaga gcgaggtatg taggcggtgc tacagagttc ttgaagtggt ggcctaacta 9300
   cggctacact agaaggacag tatttggtat ctgcgctctg ctgaagccag ttaccttcgg 9360
   aaaaagagtt ggtagctctt gatccggcaa acaaaccacc gctggtagcg gtggtttttt 9420
   tgtttgcaag cagcagatta cgcgcagaaa aaaaggatct caagaagatc ctttgatctt 9480
   ttctacgggg tctgacgctc agtggaacga aaactcacgt taagggattt tggtcatgag 9540
   attatcaaaa aggatcttca cctagatcct tttaaattaa aaatgaagtt ttaaatcaat 9600
   ctaaagtata tatgagtaaa cttggtctga cagttaccaa tgcttaatca gtgaggcacc 9660
   tatctcagcg atctgtctat ttcgttcatc catagttgcc tgactccccg tcgtgtagat 9720
   aactacgata cgggagggct taccatctgg ccccagtgct gcaatgatac cgcgagaccc 9780
   acgctcaccg gctccagatt tatcagcaat aaaccagcca gccggaaggg ccgagcgcag 9840
   aagtggtcct gcaactttat ccgcctccat ccagtctatt aattgttgcc gggaagctag 9900
   agtaagtagt tcgccagtta atagtttgcg caacgttgtt gccattgcta caggcatcgt 9960
   ggtgtcacgc tcgtcgtttg gtatggcttc attcagctcc ggttcccaac gatcaaggcg 10020
   agttacatga tcccccatgt tgtgcaaaaa agcggttagc tccttcggtc ctccgatcgt 10080
   tgtcagaagt aagttggccg cagtgttatc actcatggtt atggcagcac tgcataattc 10140
   tcttactgtc atgccatccg taagatgctt ttctgtgact ggtgagtact caaccaagtc 10200
   attctgagaa tagtgtatgc ggcgaccgag ttgctcttgc ccggcgtcaa tacgggataa 10260
   taccgcgcca catagcagaa ctttaaaagt gctcatcatt ggaaaacgtt cttcggggcg 10320
   aaaactctca aggatcttac cgctgttgag atccagttcg atgtaaccca ctcgtgcacc 10380
   caactgatct tcagcatctt ttactttcac cagcgtttct gggtgagcaa aaacaggaag 10440
   gcaaaatgcc gcaaaaaagg gaataagggc gacacggaaa tgttgaatac tcatactctt 10500
   cctttttcaa tattattgaa gcatttatca gggttattgt ctcatgagcg gatacatatt 10560
   tgaatgtatt tagaaaaata aacaaatagg ggttccgcgc acatttcccc gaaaagtgcc 10620
   acctaaattg taagcgttaa tattttgtta aaattcgcgt taaatttttg ttaaatcagc 10680
   tcatttttta accaataggc cgaaatcggc aaaatccctt ataaatcaaa agaatagacc 10740
   gagatagggt tgagtgttgt tccagtttgg aacaagagtc cactattaaa gaacgtggac 10800
   tccaacgtca aagggcgaaa aaccgtctat cagggcgatg gcccactacg tgaaccatca 10860
   ccctaatcaa gttttttggg gtcgaggtgc cgtaaagcac taaatcggaa ccctaaaggg 10920
   agcccccgat ttagagcttg acggggaaag ccaacctggc ttatcgaaat taatacgact 10980
   cactataggg agaccggc 10998
<210> 69
   <211> 12481
   <212> DNA
   <213> Artificial Sequence
<220>
   <223> Description of Artificial Sequence: pONY3.1
<400> 69
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaaggt agccttgcag aagttggtcg tgaggcactg ggcaggtaag 900
   tatcaaggtt acaagacagg tttaaggaga ccaatagaaa ctgggcttgt cgagacagag 960
   aagactcttg cgtttctgat aggcacctat tggtcttact gacatccact ttgcctttct 1020
   ctccacaggt gtccactccc agttcaatta cagctcttaa ggctagagta cttaatacga 1080
   ctcactatag gctagcctcg aggtcgacgg tatcgcccga acagggacct gagaggggcg 1140
   cagaccctac ctgttgaacc tggctgatcg taggatcccc gggacagcag aggagaactt 1200
   acagaagtct tctggaggtg ttcctggcca gaacacagga ggacaggtaa gatgggagac 1260
   cctttgacat ggagcaaggc gctcaagaag ttagagaagg tgacggtaca agggtctcag 1320
   aaattaacta ctggtaactg taattgggcg ctaagtctag tagacttatt tcatgatacc 1380
   aactttgtaa aagaaaagga ctggcagctg agggatgtca ttccattgct ggaagatgta 1440
   actcagacgc tgtcaggaca agaaagagag gcctttgaaa gaacatggtg ggcaatttct 1500
   gctgtaaaga tgggcctcca gattaataat gtagtagatg gaaaggcatc attccagctc 1560
   ctaagagcga aatatgaaaa gaagactgct aataaaaagc agtctgagcc ctctgaagaa 1620
   tatccaatca tgatagatgg ggctggaaac agaaatttta gacctctaac acctagagga 1680
   tatactactt gggtgaatac catacagaca aatggtctat taaatgaagc tagtcaaaac 1740
   ttatttggga tattatcagt agactgtact tctgaagaaa tgaatgcatt tttggatgtg 1800
   gtacctggcc aggcaggaca aaagcagata ttacttgatg caattgataa gatagcagat 1860
   gattgggata atagacatcc attaccgaat gctccactgg tggcaccacc acaagggcct 1920
   attcccatga cagcaaggtt tattagaggt ttaggagtac ctagagaaag acagatggag 1980
   cctgcttttg atcagtttag gcagacatat agacaatgga taatagaagc catgtcagaa 2040
   ggcatcaaag tgatgattgg aaaacctaaa gctcaaaata ttaggcaagg agctaaggaa 2100
   ccttacccag aatttgtaga cagactatta tcccaaataa aaagtgaggg acatccacaa 2160
   gagatttcaa aattcttgac tgatacactg actattcaga acgcaaatga ggaatgtaga 2220
   aatgctatga gacatttaag accagaggat acattagaag agaaaatgta tgcttgcaga 2280
   gacattggaa ctacaaaaca aaagatgatg ttattggcaa aagcacttca gactggtctt 2340
   gcgggcccat ttaaaggtgg agccttgaaa ggagggccac taaaggcagc acaaacatgt 2400
   tataactgtg ggaagccagg acatttatct agtcaatgta gagcacctaa agtctgtttt 2460
   aaatgtaaac agcctggaca tttctcaaag caatgcagaa gtgttccaaa aaacgggaag 2520
   caaggggctc aagggaggcc ccagaaacaa actttcccga tacaacagaa gagtcagcac 2580
   aacaaatctg ttgtacaaga gactcctcag actcaaaatc tgtacccaga tctgagcgaa 2640
   ataaaaaagg aatacaatgt caaggagaag gatcaagtag aggatctcaa cctggacagt 2700
   ttgtgggagt aacatataat ctagagaaaa ggcctactac aatagtatta attaatgata 2760
   ctcccttaaa tgtactgtta gacacaggag cagatacttc agtgttgact actgcacatt 2820
   ataataggtt aaaatataga gggagaaaat atcaagggac gggaataata ggagtgggag 2880
   gaaatgtgga aacattttct acgcctgtga ctataaagaa aaagggtaga cacattaaga 2940
   caagaatgct agtggcagat attccagtga ctattttggg acgagatatt cttcaggact 3000
   taggtgcaaa attggttttg gcacagctct ccaaggaaat aaaatttaga aaaatagagt 3060
   taaaagaggg cacaatgggg ccaaaaattc ctcaatggcc actcactaag gagaaactag 3120
   aaggggccaa agagatagtc caaagactat tgtcagaggg aaaaatatca gaagctagtg 3180
   acaataatcc ttataattca cccatatttg taataaaaaa gaggtctggc aaatggaggt 3240
   tattacaaga tctgagagaa ttaaacaaaa cagtacaagt aggaacggaa atatccagag 3300
   gattgcctca cccgggagga ttaattaaat gtaaacacat gactgtatta gatattggag 3360
   atgcatattt cactataccc ttagatccag agtttagacc atatacagct ttcactattc 3420
   cctccattaa tcatcaagaa ccagataaaa gatatgtgtg gaaatgttta ccacaaggat 3480
   tcgtgttgag cccatatata tatcagaaaa cattacagga aattttacaa ccttttaggg 3540
   aaagatatcc tgaagtacaa ttgtatcaat atatggatga tttgttcatg ggaagtaatg 3600
   gttctaaaaa acaacacaaa gagttaatca tagaattaag ggcgatctta ctggaaaagg 3660
   gttttgagac accagatgat aaattacaag aagtgccacc ttatagctgg ctaggttatc 3720
   aactttgtcc tgaaaattgg aaagtacaaa aaatgcaatt agacatggta aagaatccaa 3780
   cccttaatga tgtgcaaaaa ttaatgggga atataacatg gatgagctca gggatcccag 3840
   ggttgacagt aaaacacatt gcagctacta ctaagggatg tttagagttg aatcaaaaag 3900
   taatttggac ggaagaggca caaaaagagt tagaagaaaa taatgagaag attaaaaatg 3960
   ctcaagggtt acaatattat aatccagaag aagaaatgtt atgtgaggtt gaaattacaa 4020
   aaaattatga ggcaacttat gttataaaac aatcacaagg aatcctatgg gcaggtaaaa 4080
   agattatgaa ggctaataag ggatggtcaa cagtaaaaaa tttaatgtta ttgttgcaac 4140
   atgtggcaac agaaagtatt actagagtag gaaaatgtcc aacgtttaag gtaccattta 4200
   ccaaagagca agtaatgtgg gaaatgcaaa aaggatggta ttattcttgg ctcccagaaa 4260
   tagtatatac acatcaagta gttcatgatg attggagaat gaaattggta gaagaaccta 4320
   catcaggaat aacaatatac actgatgggg gaaaacaaaa tggagaagga atagcagctt 4380
   atgtgaccag taatgggaga actaaacaga aaaggttagg acctgtcact catcaagttg 4440
   ctgaaagaat ggcaatacaa atggcattag aggataccag agataaacaa gtaaatatag 4500
   taactgatag ttattattgt tggaaaaata ttacagaagg attaggttta gaaggaccac 4560
   aaagtccttg gtggcctata atacaaaata tacgagaaaa agagatagtt tattttgctt 4620
   gggtacctgg tcacaaaggg atatatggta atcaattggc agatgaagcc gcaaaaataa 4680
   aagaagaaat catgctagca taccaaggca cacaaattaa agagaaaaga gatgaagatg 4740
   cagggtttga cttatgtgtt ccttatgaca tcatgatacc tgtatctgac acaaaaatca 4800
   tacccacaga tgtaaaaatt caagttcctc ctaatagctt tggatgggtc actgggaaat 4860
   catcaatggc aaaacagggg ttattaatta atggaggaat aattgatgaa ggatatacag 4920
   gagaaataca agtgatatgt actaatattg gaaaaagtaa tattaaatta atagagggac 4980
   aaaaatttgc acaattaatt atactacagc atcactcaaa ttccagacag ccttgggatg 5040
   aaaataaaat atctcagaga ggggataaag gatttggaag tacaggagta ttctgggtag 5100
   aaaatattca ggaagcacaa gatgaacatg agaattggca tacatcacca aagatattgg 5160
   caagaaatta taagatacca ttgactgtag caaaacagat aactcaagaa tgtcctcatt 5220
   gcactaagca aggatcagga cctgcaggtt gtgtcatgag atctcctaat cattggcagg 5280
   cagattgcac acatttggac aataagataa tattgacttt tgtagagtca aattcaggat 5340
   acatacatgc tacattattg tcaaaagaaa atgcattatg tacttcattg gctattttag 5400
   aatgggcaag attgttttca ccaaagtcct tacacacaga taacggcact aattttgtgg 5460
   cagaaccagt tgtaaatttg ttgaagttcc taaagatagc acataccaca ggaataccat 5520
   atcatccaga aagtcagggt attgtagaaa gggcaaatag gaccttgaaa gagaagattc 5580
   aaagtcatag agacaacact caaacactgg aggcagcttt acaacttgct ctcattactt 5640
   gtaacaaagg gagggaaagt atgggaggac agacaccatg ggaagtattt atcactaatc 5700
   aagcacaagt aatacatgag aaacttttac tacagcaagc acaatcctcc aaaaaatttt 5760
   gtttttacaa aatccctggt gaacatgatt ggaagggacc tactagggtg ctgtggaagg 5820
   gtgatggtgc agtagtagtt aatgatgaag gaaagggaat aattgctgta ccattaacca 5880
   ggactaagtt actaataaaa ccaaattgag tattgttgca ggaagcaaga cccaactacc 5940
   attgtcagct gtgtttcctg aggtctctag gaattgatta cctcgatgct tcattaagga 6000
   agaagaataa acaaagactg aaggcaatcc aacaaggaag acaacctcaa tatttgttat 6060
   aaggtttgat atatgggagt atttggtaaa ggggtaacat ggtcagcatc gcattctatg 6120
   ggggaatccc agggggaatc tcaaccccta ttacccaaca gtcagaaaaa tctaagtgtg 6180
   aggagaacac aatgtttcaa ccttattgtt ataataatga cagtaagaac agcatggcag 6240
   aatcgaagga agcaagagac caagaaatga acctgaaaga agaatctaaa gaagaaaaaa 6300
   gaagaaatga ctggtggaaa ataggtatgt ttctgttatg cttagcagga actactggag 6360
   gaatactttg gtggtatgaa ggactcccac agcaacatta tatagggttg gtggcgatag 6420
   ggggaagatt aaacggatct ggccaatcaa atgctataga atgctggggt tccttcccgg 6480
   ggtgtagacc atttcaaaat tacttcagtt atgagaccaa tagaagcatg catatggata 6540
   ataatactgc tacattatta gaagctttaa ccaatataac tgctctataa ataacaaaac 6600
   agaattagaa acatggaagt tagtaaagac ttctggcata actcctttac ctatttcttc 6660
   tgaagctaac actggactaa ttagacataa gagagatttt ggtataagtg caatagtggc 6720
   agctattgta gccgctactg ctattgctgc tagcgctact atgtcttatg ttgctctaac 6780
   tgaggttaac aaaataatgg aagtacaaaa tcatactttt gaggtagaaa atagtactct 6840
   aaatggtatg gatttaatag aacgacaaat aaagatatta tatgctatga ttcttcaaac 6900
   acatgcagat gttcaactgt taaaggaaag acaacaggta gaggagacat ttaatttaat 6960
   tggatgtata gaaagaacac atgtattttg tcatactggt catccctgga atatgtcatg 7020
   gggacattta aatgagtcaa cacaatggga tgactgggta agcaaaatgg aagatttaaa 7080
   tcaagagata ctaactacac ttcatggagc caggaacaat ttggcacaat ccatgataac 7140
   attcaataca ccagatagta tagctcaatt tggaaaagac ctttggagtc atattggaaa 7200
   ttggattcct ggattgggag cttccattat aaaatatata gtgatgtttt tgcttattta 7260
   tttgttacta acctcttcgc ctaagatcct cagggccctc tggaaggtga ccagtggtgc 7320
   agggtcctcc ggcagtcgtt acctgaagaa aaaattccat cacaaacatg catcgcgaga 7380
   agacacctgg gaccaggccc aacacaacat acacctagca ggcgtgaccg gtggatcagg 7440
   ggacaaatac tacaagcaga agtactccag gaacgactgg aatggagaat cagaggagta 7500
   caacaggcgg ccaaagagct gggtgaagtc aatcgaggca tttggagaga gctatatttc 7560
   cgagaagacc aaaggggaga tttctcagcc tggggcggct atcaacgagc acaagaacgg 7620
   ctctgggggg aacaatcctc accaagggtc cttagacctg gagattcgaa gcgaaggagg 7680
   aaacatttat gactgttgca ttaaagccca agaaggaact ctcgctatcc cttgctgtgg 7740
   atttccctta tggctatttt ggggactagt aattatagta ggacgcatag caggctatgg 7800
   attacgtgga ctcgctgtta taataaggat ttgtattaga ggcttaaatt tgatatttga 7860
   aataatcaga aaaatgcttg attatattgg aagagcttta aatcctggca catctcatgt 7920
   atcaatgcct cagtatgttt agaaaaacaa ggggggaact gtggggtttt tatgaggggt 7980
   tttataaatg attataagag taaaaagaaa gttgctgatg ctctcataac cttgtataac 8040
   ccaaaggact agctcatgtt gctaggcaac taaaccgcaa taaccgcatt tgtgacgcga 8100
   gttccccatt ggtgacgcgt ggtacctcta gagtcgaccc gggcggccgc ttccctttag 8160
   tgagggttaa tgcttcgagc agacatgata agatacattg atgagtttgg acaaaccaca 8220
   actagaatgc agtgaaaaaa atgctttatt tgtgaaattt gtgatgctat tgctttattt 8280
   gtaaccatta taagctgcaa taaacaagtt aacaacaaca attgcattca ttttatgttt 8340
   caggttcagg gggagatgtg ggaggttttt taaagcaagt aaaacctcta caaatgtggt 8400
   aaaatccgat aaggatcgat ccgggctggc gtaatagcga agaggcccgc accgatcgcc 8460
   cttcccaaca gttgcgcagc ctgaatggcg aatggacgcg ccctgtagcg gcgcattaag 8520
   cgcggcgggt gtggtggtta cgcgcagcgt gaccgctaca cttgccagcg ccctagcgcc 8580
   cgctcctttc gctttcttcc cttcctttct cgccacgttc gccggctttc cccgtcaagc 8640
   tctaaatcgg gggctccctt tagggttccg atttagagct ttacggcacc tcgaccgcaa 8700
   aaaacttgat ttgggtgatg gttcacgtag tgggccatcg ccctgataga cggtttttcg 8760
   ccctttgacg ttggagtcca cgttctttaa tagtggactc ttgttccaaa ctggaacaac 8820
   actcaaccct atctcggtct attcttttga tttataaggg attttgccga tttcggccta 8880
   ttggttaaaa aatgagctga tttaacaaat atttaacgcg aattttaaca aaatattaac 8940
   gtttacaatt tcgcctgatg cggtattttc tccttacgca tctgtgcggt atttcacacc 9000
   gcatacgcgg atctgcgcag caccatggcc tgaaataacc tctgaaagag gaacttggtt 9060
   aggtaccttc tgaggcggaa agaaccagct gtggaatgtg tgtcagttag ggtgtggaaa 9120
   gtccccaggc tccccagcag gcagaagtat gcaaagcatg catctcaatt agtcagcaac 9180
   caggtgtgga aagtccccag gctccccagc aggcagaagt atgcaaagca tgcatctcaa 9240
   ttagtcagca accatagtcc cgcccctaac tccgcccatc ccgcccctaa ctccgcccag 9300
   ttccgcccat tctccgcccc atggctgact aatttttttt atttatgcag aggccgaggc 9360
   cgcctcggcc tctgagctat tccagaagta gtgaggaggc ttttttggag gcctaggctt 9420
   ttgcaaaaag cttgattctt ctgacacaac agtctcgaac ttaaggctag agccaccatg 9480
   attgaacaag atggattgca cgcaggttct ccggccgctt gggtggagag gctattcggc 9540
   tatgactggg cacaacagac aatcggctgc tctgatgccg ccgtgttccg gctgtcagcg 9600
   caggggcgcc cggttctttt tgtcaagacc gacctgtccg gtgccctgaa tgaactgcag 9660
   gacgaggcag cgcggctatc gtggctggcc acgacgggcg ttccttgcgc agctgtgctc 9720
   gacgttgtca ctgaagcggg aagggactgg ctgctattgg gcgaagtgcc ggggcaggat 9780
   ctcctgtcat ctcaccttgc tcctgccgag aaagtatcca tcatggctga tgcaatgcgg 9840
   cggctgcata cgcttgatcc ggctacctgc ccattcgacc accaagcgaa acatcgcatc 9900
   gagcgagcac gtactcggat ggaagccggt cttgtcgatc aggatgatct ggacgaagag 9960
   catcaggggc tcgcgccagc cgaactgttc gccaggctca aggcgcgcat gcccgacggc 10020
   gaggatctcg tcgtgaccca tggcgatgcc tgcttgccga atatcatggt ggaaaatggc 10080
   cgcttttctg gattcatcga ctgtggccgg ctgggtgtgg cggaccgcta tcaggacata 10140
   gcgttggcta cccgtgatat tgctgaagag cttggcggcg aatgggctga ccgcttcctc 10200
   gtgctttacg gtatcgccgc tcccgattcg cagcgcatcg ccttctatcg ccttcttgac 10260
   gagttcttct gagcgggact ctggggttcg aaatgaccga ccaagcgacg cccaacctgc 10320
   catcacgatg gccgcaataa aatatcttta ttttcattac atctgtgtgt tggttttttg 10380
   tgtgaatcga tagcgataag gatccgcgta tggtgcactc tcagtacaat ctgctctgat 10440
   gccgcatagt taagccagcc ccgacacccg ccaacacccg ctgacgcgcc ctgacgggct 10500
   tgtctgctcc cggcatccgc ttacagacaa gctgtgaccg tctccgggag ctgcatgtgt 10560
   cagaggtttt caccgtcatc accgaaacgc gcgagacgaa agggcctcgt gatacgccta 10620
   tttttatagg ttaatgtcat gataataatg gtttcttaga cgtcaggtgg cacttttcgg 10680
   ggaaatgtgc gcggaacccc tatttgttta tttttctaaa tacattcaaa tatgtatccg 10740
   ctcatgagac aataaccctg ataaatgctt caataatatt gaaaaaggaa gagtatgagt 10800
   attcaacatt tccgtgtcgc ccttattccc ttttttgcgg cattttgcct tcctgttttt 10860
   gctcacccag aaacgctggt gaaagtaaaa gatgctgaag atcagttggg tgcacgagtg 10920
   ggttacatcg aactggatct caacagcggt aagatccttg agagttttcg ccccgaagaa 10980
   cgttttccaa tgatgagcac ttttaaagtt ctgctatgtg gcgcggtatt atcccgtatt 11040
   gacgccgggc aagagcaact cggtcgccgc atacactatt ctcagaatga cttggttgag 11100
   tactcaccag tcacagaaaa gcatcttacg gatggcatga cagtaagaga attatgcagt 11160
   gctgccataa ccatgagtga taacactgcg gccaacttac ttctgacaac gatcggagga 11220
   ccgaaggagc taaccgcttt tttgcacaac atgggggatc atgtaactcg ccttgatcgt 11280
   tgggaaccgg agctgaatga agccatacca aacgacgagc gtgacaccac gatgcctgta 11340
   gcaatggcaa caacgttgcg caaactatta actggcgaac tacttactct agcttcccgg 11400
   caacaattaa tagactggat ggaggcggat aaagttgcag gaccacttct gcgctcggcc 11460
   cttccggctg gctggtttat tgctgataaa tctggagccg gtgagcgtgg gtctcgcggt 11520
   atcattgcag cactggggcc agatggtaag ccctcccgta tcgtagttat ctacacgacg 11580
   gggagtcagg caactatgga tgaacgaaat agacagatcg ctgagatagg tgcctcactg 11640
   attaagcatt ggtaactgtc agaccaagtt tactcatata tactttagat tgatttaaaa 11700
   cttcattttt aatttaaaag gatctaggtg aagatccttt ttgataatct catgaccaaa 11760
   atcccttaac gtgagttttc gttccactga gcgtcagacc ccgtagaaaa gatcaaagga 11820
   tcttcttgag atcctttttt tctgcgcgta atctgctgct tgcaaacaaa aaaaccaccg 11880
   ctaccagcgg tggtttgttt gccggatcaa gagctaccaa ctctttttcc gaaggtaact 11940
   ggcttcagca gagcgcagat accaaatact gtccttctag tgtagccgta gttaggccac 12000
   cacttcaaga actctgtagc accgcctaca tacctcgctc tgctaatcct gttaccagtg 12060
   gctgctgcca gtggcgataa gtcgtgtctt accgggttgg actcaagacg atagttaccg 12120
   gataaggcgc agcggtcggg ctgaacgggg ggttcgtgca cacagcccag cttggagcga 12180
   acgacctaca ccgaactgag atacctacag cgtgagctat gagaaagcgc cacgcttccc 12240
   gaagggagaa aggcggacag gtatccggta agcggcaggg tcggaacagg agagcgcacg 12300
   agggagcttc cagggggaaa cgcctggtat ctttatagtc ctgtcgggtt tcgccacctc 12360
   tgacttgagc gtcgattttt gtgatgctcg tcaggggggc ggagcctatg gaaaaacgcc 12420
   agcaacgcgg cctttttacg gttcctggcc ttttgctggc cttttgctca catggctcga 12480
   c 12481
<210> 70
   <211> 10112
   <212> DNA
   <213> Artificial Sequence
<220>
   <223> Description of Artificial Sequence: pEsynGP
<400> 70
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattcgcca ccatgggcga tcccctcacc tggtccaaag ccctgaagaa 1140
   actggaaaaa gtcaccgttc agggtagcca aaagcttacc acaggcaatt gcaactgggc 1200
   attgtccctg gtggatcttt tccacgacac taatttcgtt aaggagaaag attggcaact 1260
   cagagacgtg atccccctct tggaggacgt gacccaaaca ttgtctgggc aggagcgcga 1320
   agctttcgag cgcacctggt gggccatcag cgcagtcaaa atggggctgc aaatcaacaa 1380
   cgtggttgac ggtaaagcta gctttcaact gctccgcgct aagtacgaga agaaaaccgc 1440
   caacaagaaa caatccgaac ctagcgagga gtacccaatt atgatcgacg gcgccggcaa 1500
   taggaacttc cgcccactga ctcccagggg ctataccacc tgggtcaaca ccatccagac 1560
   aaacggactt ttgaacgaag cctcccagaa cctgttcggc atcctgtctg tggactgcac 1620
   ctccgaagaa atgaatgctt ttctcgacgt ggtgccagga caggctggac agaaacagat 1680
   cctgctcgat gccattgaca agatcgccga cgactgggat aatcgccacc ccctgccaaa 1740
   cgcccctctg gtggctcccc cacaggggcc tatccctatg accgctaggt tcattagggg 1800
   actgggggtg ccccgcgaac gccagatgga gccagcattt gaccaattta ggcagaccta 1860
   cagacagtgg atcatcgaag ccatgagcga ggggattaaa gtcatgatcg gaaagcccaa 1920
   ggcacagaac atcaggcagg gggccaagga accataccct gagtttgtcg acaggcttct 1980
   gtcccagatt aaatccgaag gccaccctca ggagatctcc aagttcttga cagacacact 2040
   gactatccaa aatgcaaatg aagagtgcag aaacgccatg aggcacctca gacctgaaga 2100
   taccctggag gagaaaatgt acgcatgtcg cgacattggc actaccaagc aaaagatgat 2160
   gctgctcgcc aaggctctgc aaaccggcct ggctggtcca ttcaaaggag gagcactgaa 2220
   gggaggtcca ttgaaagctg cacaaacatg ttataattgt gggaagccag gacatttatc 2280
   tagtcaatgt agagcaccta aagtctgttt taaatgtaaa cagcctggac atttctcaaa 2340
   gcaatgcaga agtgttccaa aaaacgggaa gcaaggggct caagggaggc cccagaaaca 2400
   aactttcccg atacaacaga agagtcagca caacaaatct gttgtacaag agactcctca 2460
   gactcaaaat ctgtacccag atctgagcga aataaaaaag gaatacaatg tcaaggagaa 2520
   ggatcaagta gaggatctca acctggacag tttgtgggag taacatacaa tctcgagaag 2580
   aggcccacta ccatcgtcct gatcaatgac acccctctta atgtgctgct ggacaccgga 2640
   gccgacacca gcgttctcac tactgctcac tataacagac tgaaatacag aggaaggaaa 2700
   taccagggca caggcatcat cggcgttgga ggcaacgtcg aaaccttttc cactcctgtc 2760
   accatcaaaa agaaggggag acacattaaa accagaatgc tggtcgccga catccccgtc 2820
   accatccttg gcagagacat tctccaggac ctgggcgcta aactcgtgct ggcacaactg 2880
   tctaaggaaa tcaagttccg caagatcgag ctgaaagagg gcacaatggg tccaaaaatc 2940
   ccccagtggc ccctgaccaa agagaagctt gagggcgcta aggaaatcgt gcagcgcctg 3000
   ctttctgagg gcaagattag cgaggccagc gacaataacc cttacaacag ccccatcttt 3060
   gtgattaaga aaaggagcgg caaatggaga ctcctgcagg acctgaggga actcaacaag 3120
   accgtccagg tcggaactga gatctctcgc ggactgcctc accccggcgg cctgattaaa 3180
   tgcaagcaca tgacagtcct tgacattgga gacgcttatt ttaccatccc cctcgatcct 3240
   gaatttcgcc cctatactgc ttttaccatc cccagcatca atcaccagga gcccgataaa 3300
   cgctatgtgt ggaagtgcct cccccaggga tttgtgctta gcccctacat ttaccagaag 3360
   acacttcaag agatcctcca acctttccgc gaaagatacc cagaggttca actctaccaa 3420
   tatatggacg acctgttcat ggggtccaac gggtctaaga agcagcacaa ggaactcatc 3480
   atcgaactga gggcaatcct cctggagaaa ggcttcgaga cacccgacga caagctgcaa 3540
   gaagttcctc catatagctg gctgggctac cagctttgcc ctgaaaactg gaaagtccag 3600
   aagatgcagt tggatatggt caagaaccca acactgaacg acgtccagaa gctcatgggc 3660
   aatattacct ggatgagctc cggaatccct gggcttaccg ttaagcacat tgccgcaact 3720
   acaaaaggat gcctggagtt gaaccagaag gtcatttgga cagaggaagc tcagaaggaa 3780
   ctggaggaga ataatgaaaa gattaagaat gctcaagggc tccaatacta caatcccgaa 3840
   gaagaaatgt tgtgcgaggt cgaaatcact aagaactacg aagccaccta tgtcatcaaa 3900
   cagtcccaag gcatcttgtg ggccggaaag aaaatcatga aggccaacaa aggctggtcc 3960
   accgttaaaa atctgatgct cctgctccag cacgtcgcca ccgagtctat cacccgcgtc 4020
   ggcaagtgcc ccaccttcaa agttcccttc actaaggagc aggtgatgtg ggagatgcaa 4080
   aaaggctggt actactcttg gcttcccgag atcgtctaca cccaccaagt ggtgcacgac 4140
   gactggagaa tgaagcttgt cgaggagccc actagcggaa ttacaatcta taccgacggc 4200
   ggaaagcaaa acggagaggg aatcgctgca tacgtcacat ctaacggccg caccaagcaa 4260
   aagaggctcg gccctgtcac tcaccaggtg gctgagagga tggctatcca gatggccctt 4320
   gaggacacta gagacaagca ggtgaacatt gtgactgaca gctactactg ctggaaaaac 4380
   atcacagagg gccttggcct ggagggaccc cagtctccct ggtggcctat catccagaat 4440
   atccgcgaaa aggaaattgt ctatttcgcc tgggtgcctg gacacaaagg aatttacggc 4500
   aaccaactcg ccgatgaagc cgccaaaatt aaagaggaaa tcatgcttgc ctaccagggc 4560
   acacagatta aggagaagag agacgaggac gctggctttg acctgtgtgt gccatacgac 4620
   atcatgattc ccgttagcga cacaaagatc attccaaccg atgtcaagat ccaggtgcca 4680
   cccaattcat ttggttgggt gaccggaaag tccagcatgg ctaagcaggg tcttctgatt 4740
   aacgggggaa tcattgatga aggatacacc ggcgaaatcc aggtgatctg cacaaatatc 4800
   ggcaaaagca atattaagct tatcgaaggg cagaagttcg ctcaactcat catcctccag 4860
   caccacagca attcaagaca accttgggac gaaaacaaga ttagccagag aggtgacaag 4920
   ggcttcggca gcacaggtgt gttctgggtg gagaacatcc aggaagcaca ggacgagcac 4980
   gagaattggc acacctcccc taagattttg gcccgcaatt acaagatccc actgactgtg 5040
   gctaagcaga tcacacagga atgcccccac tgcaccaaac aaggttctgg ccccgccggc 5100
   tgcgtgatga ggtcccccaa tcactggcag gcagattgca cccacctcga caacaaaatt 5160
   atcctgacct tcgtggagag caattccggc tacatccacg caacactcct ctccaaggaa 5220
   aatgcattgt gcacctccct cgcaattctg gaatgggcca ggctgttctc tccaaaatcc 5280
   ctgcacaccg acaacggcac caactttgtg gctgaacctg tggtgaatct gctgaagttc 5340
   ctgaaaatcg cccacaccac tggcattccc tatcaccctg aaagccaggg cattgtcgag 5400
   agggccaaca gaactctgaa agaaaagatc caatctcaca gagacaatac acagacattg 5460
   gaggccgcac ttcagctcgc ccttatcacc tgcaacaaag gaagagaaag catgggcggc 5520
   cagaccccct gggaggtctt catcactaac caggcccagg tcatccatga aaagctgctc 5580
   ttgcagcagg cccagtcctc caaaaagttc tgcttttata agatccccgg tgagcacgac 5640
   tggaaaggtc ctacaagagt tttgtggaaa ggagacggcg cagttgtggt gaacgatgag 5700
   ggcaagggga tcatcgctgt gcccctgaca cgcaccaagc ttctcatcaa gccaaactga 5760
   acccggggcg gccgcttccc tttagtgagg gttaatgctt cgagcagaca tgataagata 5820
   cattgatgag tttggacaaa ccacaactag aatgcagtga aaaaaatgct ttatttgtga 5880
   aatttgtgat gctattgctt tatttgtaac cattataagc tgcaataaac aagttaacaa 5940
   caacaattgc attcatttta tgtttcaggt tcagggggag atgtgggagg ttttttaaag 6000
   caagtaaaac ctctacaaat gtggtaaaat ccgataagga tcgatccggg ctggcgtaat 6060
   agcgaagagg cccgcaccga tcgcccttcc caacagttgc gcagcctgaa tggcgaatgg 6120
   acgcgccctg tagcggcgca ttaagcgcgg cgggtgtggt ggttacgcgc agcgtgaccg 6180
   ctacacttgc cagcgcccta gcgcccgctc ctttcgcttt cttcccttcc tttctcgcca 6240
   cgttcgccgg ctttccccgt caagctctaa atcgggggct ccctttaggg ttccgattta 6300
   gagctttacg gcacctcgac cgcaaaaaac ttgatttggg tgatggttca cgtagtgggc 6360
   catcgccctg atagacggtt tttcgccctt tgacgttgga gtccacgttc tttaatagtg 6420
   gactcttgtt ccaaactgga acaacactca accctatctc ggtctattct tttgatttat 6480
   aagggatttt gccgatttcg gcctattggt taaaaaatga gctgatttaa caaatattta 6540
   acgcgaattt taacaaaata ttaacgttta caatttcgcc tgatgcggta ttttctcctt 6600
   acgcatctgt gcggtatttc acaccgcata cgcggatctg cgcagcacca tggcctgaaa 6660
   taacctctga aagaggaact tggttaggta ccttctgagg cggaaagaac cagctgtgga 6720
   atgtgtgtca gttagggtgt ggaaagtccc caggctcccc agcaggcaga agtatgcaaa 6780
   gcatgcatct caattagtca gcaaccaggt gtggaaagtc cccaggctcc ccagcaggca 6840
   gaagtatgca aagcatgcat ctcaattagt cagcaaccat agtcccgccc ctaactccgc 6900
   ccatcccgcc cctaactccg cccagttccg cccattctcc gccccatggc tgactaattt 6960
   tttttattta tgcagaggcc gaggccgcct cggcctctga gctattccag aagtagtgag 7020
   gaggcttttt tggaggccta ggcttttgca aaaagcttga ttcttctgac acaacagtct 7080
   cgaacttaag gctagagcca ccatgattga acaagatgga ttgcacgcag gttctccggc 7140
   cgcttgggtg gagaggctat tcggctatga ctgggcacaa cagacaatcg gctgctctga 7200
   tgccgccgtg ttccggctgt cagcgcaggg gcgcccggtt ctttttgtca agaccgacct 7260
   gtccggtgcc ctgaatgaac tgcaggacga ggcagcgcgg ctatcgtggc tggccacgac 7320
   gggcgttcct tgcgcagctg tgctcgacgt tgtcactgaa gcgggaaggg actggctgct 7380
   attgggcgaa gtgccggggc aggatctcct gtcatctcac cttgctcctg ccgagaaagt 7440
   atccatcatg gctgatgcaa tgcggcggct gcatacgctt gatccggcta cctgcccatt 7500
   cgaccaccaa gcgaaacatc gcatcgagcg agcacgtact cggatggaag ccggtcttgt 7560
   cgatcaggat gatctggacg aagagcatca ggggctcgcg ccagccgaac tgttcgccag 7620
   gctcaaggcg cgcatgcccg acggcgagga tctcgtcgtg acccatggcg atgcctgctt 7680
   gccgaatatc atggtggaaa atggccgctt ttctggattc atcgactgtg gccggctggg 7740
   tgtggcggac cgctatcagg acatagcgtt ggctacccgt gatattgctg aagagcttgg 7800
   cggcgaatgg gctgaccgct tcctcgtgct ttacggtatc gccgctcccg attcgcagcg 7860
   catcgccttc tatcgccttc ttgacgagtt cttctgagcg ggactctggg gttcgaaatg 7920
   accgaccaag cgacgcccaa cctgccatca cgatggccgc aataaaatat ctttattttc 7980
   attacatctg tgtgttggtt ttttgtgtga atcgatagcg ataaggatcc gcgtatggtg 8040
   cactctcagt acaatctgct ctgatgccgc atagttaagc cagccccgac acccgccaac 8100
   acccgctgac gcgccctgac gggcttgtct gctcccggca tccgcttaca gacaagctgt 8160
   gaccgtctcc gggagctgca tgtgtcagag gttttcaccg tcatcaccga aacgcgcgag 8220
   acgaaagggc ctcgtgatac gcctattttt ataggttaat gtcatgataa taatggtttc 8280
   ttagacgtca ggtggcactt ttcggggaaa tgtgcgcgga acccctattt gtttattttt 8340
   ctaaatacat tcaaatatgt atccgctcat gagacaataa ccctgataaa tgcttcaata 8400
   atattgaaaa aggaagagta tgagtattca acatttccgt gtcgccctta ttcccttttt 8460
   tgcggcattt tgccttcctg tttttgctca cccagaaacg ctggtgaaag taaaagatgc 8520
   tgaagatcag ttgggtgcac gagtgggtta catcgaactg gatctcaaca gcggtaagat 8580
   ccttgagagt tttcgccccg aagaacgttt tccaatgatg agcactttta aagttctgct 8640
   atgtggcgcg gtattatccc gtattgacgc cgggcaagag caactcggtc gccgcataca 8700
   ctattctcag aatgacttgg ttgagtactc accagtcaca gaaaagcatc ttacggatgg 8760
   catgacagta agagaattat gcagtgctgc cataaccatg agtgataaca ctgcggccaa 8820
   cttacttctg acaacgatcg gaggaccgaa ggagctaacc gcttttttgc acaacatggg 8880
   ggatcatgta actcgccttg atcgttggga accggagctg aatgaagcca taccaaacga 8940
   cgagcgtgac accacgatgc ctgtagcaat ggcaacaacg ttgcgcaaac tattaactgg 9000
   cgaactactt actctagctt cccggcaaca attaatagac tggatggagg cggataaagt 9060
   tgcaggacca cttctgcgct cggcccttcc ggctggctgg tttattgctg ataaatctgg 9120
   agccggtgag cgtgggtctc gcggtatcat tgcagcactg gggccagatg gtaagccctc 9180
   ccgtatcgta gttatctaca cgacggggag tcaggcaact atggatgaac gaaatagaca 9240
   gatcgctgag ataggtgcct cactgattaa gcattggtaa ctgtcagacc aagtttactc 9300
   atatatactt tagattgatt taaaacttca tttttaattt aaaaggatct aggtgaagat 9360
   cctttttgat aatctcatga ccaaaatccc ttaacgtgag ttttcgttcc actgagcgtc 9420
   agaccccgta gaaaagatca aaggatcttc ttgagatcct ttttttctgc gcgtaatctg 9480
   ctgcttgcaa acaaaaaaac caccgctacc agcggtggtt tgtttgccgg atcaagagct 9540
   accaactctt tttccgaagg taactggctt cagcagagcg cagataccaa atactgtcct 9600
   tctagtgtag ccgtagttag gccaccactt caagaactct gtagcaccgc ctacatacct 9660
   cgctctgcta atcctgttac cagtggctgc tgccagtggc gataagtcgt gtcttaccgg 9720
   gttggactca agacgatagt taccggataa ggcgcagcgg tcgggctgaa cggggggttc 9780
   gtgcacacag cccagcttgg agcgaacgac ctacaccgaa ctgagatacc tacagcgtga 9840
   gctatgagaa agcgccacgc ttcccgaagg gagaaaggcg gacaggtatc cggtaagcgg 9900
   cagggtcgga acaggagagc gcacgaggga gcttccaggg ggaaacgcct ggtatcttta 9960
   tagtcctgtc gggtttcgcc acctctgact tgagcgtcga tttttgtgat gctcgtcagg 10020
   ggggcggagc ctatggaaaa acgccagcaa cgcggccttt ttacggttcc tggccttttg 10080
   ctggcctttt gctcacatgg ctcgacagat ct 10112
<210> 71
   <211> 10114
   <212> DNA
   <213> Artificial Sequence
<220>
   <223> Description of Artificial Sequence: pESDSYNGP
<400> 71
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctaga gaattccagg taagatgggc gatcccctca cctggtccaa agccctgaag 1140
   aaactggaaa aagtcaccgt tcagggtagc caaaagctta ccacaggcaa ttgcaactgg 1200
   gcattgtccc tggtggatct tttccacgac actaatttcg ttaaggagaa agattggcaa 1260
   ctcagagacg tgatccccct cttggaggac gtgacccaaa cattgtctgg gcaggagcgc 1320
   gaagctttcg agcgcacctg gtgggccatc agcgcagtca aaatggggct gcaaatcaac 1380
   aacgtggttg acggtaaagc tagctttcaa ctgctccgcg ctaagtacga gaagaaaacc 1440
   gccaacaaga aacaatccga acctagcgag gagtacccaa ttatgatcga cggcgccggc 1500
   aataggaact tccgcccact gactcccagg ggctatacca cctgggtcaa caccatccag 1560
   acaaacggac ttttgaacga agcctcccag aacctgttcg gcatcctgtc tgtggactgc 1620
   acctccgaag aaatgaatgc ttttctcgac gtggtgccag gacaggctgg acagaaacag 1680
   atcctgctcg atgccattga caagatcgcc gacgactggg ataatcgcca ccccctgcca 1740
   aacgcccctc tggtggctcc cccacagggg cctatcccta tgaccgctag gttcattagg 1800
   ggactggggg tgccccgcga acgccagatg gagccagcat ttgaccaatt taggcagacc 1860
   tacagacagt ggatcatcga agccatgagc gaggggatta aagtcatgat cggaaagccc 1920
   aaggcacaga acatcaggca gggggccaag gaaccatacc ctgagtttgt cgacaggctt 1980
   ctgtcccaga ttaaatccga aggccaccct caggagatct ccaagttctt gacagacaca 2040
   ctgactatcc aaaatgcaaa tgaagagtgc agaaacgcca tgaggcacct cagacctgaa 2100
   gataccctgg aggagaaaat gtacgcatgt cgcgacattg gcactaccaa gcaaaagatg 2160
   atgctgctcg ccaaggctct gcaaaccggc ctggctggtc cattcaaagg aggagcactg 2220
   aagggaggtc cattgaaagc tgcacaaaca tgttataatt gtgggaagcc aggacattta 2280
   tctagtcaat gtagagcacc taaagtctgt tttaaatgta aacagcctgg acatttctca 2340
   aagcaatgca gaagtgttcc aaaaaacggg aagcaagggg ctcaagggag gccccagaaa 2400
   caaactttcc cgatacaaca gaagagtcag cacaacaaat ctgttgtaca agagactcct 2460
   cagactcaaa atctgtaccc agatctgagc gaaataaaaa aggaatacaa tgtcaaggag 2520
   aaggatcaag tagaggatct caacctggac agtttgtggg agtaacatac aatctcgaga 2580
   agaggcccac taccatcgtc ctgatcaatg acacccctct taatgtgctg ctggacaccg 2640
   gagccgacac cagcgttctc actactgctc actataacag actgaaatac agaggaagga 2700
   aataccaggg cacaggcatc atcggcgttg gaggcaacgt cgaaaccttt tccactcctg 2760
   tcaccatcaa aaagaagggg agacacatta aaaccagaat gctggtcgcc gacatccccg 2820
   tcaccatcct tggcagagac attctccagg acctgggcgc taaactcgtg ctggcacaac 2880
   tgtctaagga aatcaagttc cgcaagatcg agctgaaaga gggcacaatg ggtccaaaaa 2940
   tcccccagtg gcccctgacc aaagagaagc ttgagggcgc taaggaaatc gtgcagcgcc 3000
   tgctttctga gggcaagatt agcgaggcca gcgacaataa cccttacaac agccccatct 3060
   ttgtgattaa gaaaaggagc ggcaaatgga gactcctgca ggacctgagg gaactcaaca 3120
   agaccgtcca ggtcggaact gagatctctc gcggactgcc tcaccccggc ggcctgatta 3180
   aatgcaagca catgacagtc cttgacattg gagacgctta ttttaccatc cccctcgatc 3240
   ctgaatttcg cccctatact gcttttacca tccccagcat caatcaccag gagcccgata 3300
   aacgctatgt gtggaagtgc ctcccccagg gatttgtgct tagcccctac atttaccaga 3360
   agacacttca agagatcctc caacctttcc gcgaaagata cccagaggtt caactctacc 3420
   aatatatgga cgacctgttc atggggtcca acgggtctaa gaagcagcac aaggaactca 3480
   tcatcgaact gagggcaatc ctcctggaga aaggcttcga gacacccgac gacaagctgc 3540
   aagaagttcc tccatatagc tggctgggct accagctttg ccctgaaaac tggaaagtcc 3600
   agaagatgca gttggatatg gtcaagaacc caacactgaa cgacgtccag aagctcatgg 3660
   gcaatattac ctggatgagc tccggaatcc ctgggcttac cgttaagcac attgccgcaa 3720
   ctacaaaagg atgcctggag ttgaaccaga aggtcatttg gacagaggaa gctcagaagg 3780
   aactggagga gaataatgaa aagattaaga atgctcaagg gctccaatac tacaatcccg 3840
   aagaagaaat gttgtgcgag gtcgaaatca ctaagaacta cgaagccacc tatgtcatca 3900
   aacagtccca aggcatcttg tgggccggaa agaaaatcat gaaggccaac aaaggctggt 3960
   ccaccgttaa aaatctgatg ctcctgctcc agcacgtcgc caccgagtct atcacccgcg 4020
   tcggcaagtg ccccaccttc aaagttccct tcactaagga gcaggtgatg tgggagatgc 4080
   aaaaaggctg gtactactct tggcttcccg agatcgtcta cacccaccaa gtggtgcacg 4140
   acgactggag aatgaagctt gtcgaggagc ccactagcgg aattacaatc tataccgacg 4200
   gcggaaagca aaacggagag ggaatcgctg catacgtcac atctaacggc cgcaccaagc 4260
   aaaagaggct cggccctgtc actcaccagg tggctgagag gatggctatc cagatggccc 4320
   ttgaggacac tagagacaag caggtgaaca ttgtgactga cagctactac tgctggaaaa 4380
   acatcacaga gggccttggc ctggagggac cccagtctcc ctggtggcct atcatccaga 4440
   atatccgcga aaaggaaatt gtctatttcg cctgggtgcc tggacacaaa ggaatttacg 4500
   gcaaccaact cgccgatgaa gccgccaaaa ttaaagagga aatcatgctt gcctaccagg 4560
   gcacacagat taaggagaag agagacgagg acgctggctt tgacctgtgt gtgccatacg 4620
   acatcatgat tcccgttagc gacacaaaga tcattccaac cgatgtcaag atccaggtgc 4680
   cacccaattc atttggttgg gtgaccggaa agtccagcat ggctaagcag ggtcttctga 4740
   ttaacggggg aatcattgat gaaggataca ccggcgaaat ccaggtgatc tgcacaaata 4800
   tcggcaaaag caatattaag cttatcgaag ggcagaagtt cgctcaactc atcatcctcc 4860
   agcaccacag caattcaaga caaccttggg acgaaaacaa gattagccag agaggtgaca 4920
   agggcttcgg cagcacaggt gtgttctggg tggagaacat ccaggaagca caggacgagc 4980
   acgagaattg gcacacctcc cctaagattt tggcccgcaa ttacaagatc ccactgactg 5040
   tggctaagca gatcacacag gaatgccccc actgcaccaa acaaggttct ggccccgccg 5100
   gctgcgtgat gaggtccccc aatcactggc aggcagattg cacccacctc gacaacaaaa 5160
   ttatcctgac cttcgtggag agcaattccg gctacatcca cgcaacactc ctctccaagg 5220
   aaaatgcatt gtgcacctcc ctcgcaattc tggaatgggc caggctgttc tctccaaaat 5280
   ccctgcacac cgacaacggc accaactttg tggctgaacc tgtggtgaat ctgctgaagt 5340
   tcctgaaaat cgcccacacc actggcattc cctatcaccc tgaaagccag ggcattgtcg 5400
   agagggccaa cagaactctg aaagaaaaga tccaatctca cagagacaat acacagacat 5460
   tggaggccgc acttcagctc gcccttatca cctgcaacaa aggaagagaa agcatgggcg 5520
   gccagacccc ctgggaggtc ttcatcacta accaggccca ggtcatccat gaaaagctgc 5580
   tcttgcagca ggcccagtcc tccaaaaagt tctgctttta taagatcccc ggtgagcacg 5640
   actggaaagg tcctacaaga gttttgtgga aaggagacgg cgcagttgtg gtgaacgatg 5700
   agggcaaggg gatcatcgct gtgcccctga cacgcaccaa gcttctcatc aagccaaact 5760
   gaacccgggg cggccgcttc cctttagtga gggttaatgc ttcgagcaga catgataaga 5820
   tacattgatg agtttggaca aaccacaact agaatgcagt gaaaaaaatg ctttatttgt 5880
   gaaatttgtg atgctattgc tttatttgta accattataa gctgcaataa acaagttaac 5940
   aacaacaatt gcattcattt tatgtttcag gttcaggggg agatgtggga ggttttttaa 6000
   agcaagtaaa acctctacaa atgtggtaaa atccgataag gatcgatccg ggctggcgta 6060
   atagcgaaga ggcccgcacc gatcgccctt cccaacagtt gcgcagcctg aatggcgaat 6120
   ggacgcgccc tgtagcggcg cattaagcgc ggcgggtgtg gtggttacgc gcagcgtgac 6180
   cgctacactt gccagcgccc tagcgcccgc tcctttcgct ttcttccctt cctttctcgc 6240
   cacgttcgcc ggctttcccc gtcaagctct aaatcggggg ctccctttag ggttccgatt 6300
   tagagcttta cggcacctcg accgcaaaaa acttgatttg ggtgatggtt cacgtagtgg 6360
   gccatcgccc tgatagacgg tttttcgccc tttgacgttg gagtccacgt tctttaatag 6420
   tggactcttg ttccaaactg gaacaacact caaccctatc tcggtctatt cttttgattt 6480
   ataagggatt ttgccgattt cggcctattg gttaaaaaat gagctgattt aacaaatatt 6540
   taacgcgaat tttaacaaaa tattaacgtt tacaatttcg cctgatgcgg tattttctcc 6600
   ttacgcatct gtgcggtatt tcacaccgca tacgcggatc tgcgcagcac catggcctga 6660
   aataacctct gaaagaggaa cttggttagg taccttctga ggcggaaaga accagctgtg 6720
   gaatgtgtgt cagttagggt gtggaaagtc cccaggctcc ccagcaggca gaagtatgca 6780
   aagcatgcat ctcaattagt cagcaaccag gtgtggaaag tccccaggct ccccagcagg 6840
   cagaagtatg caaagcatgc atctcaatta gtcagcaacc atagtcccgc ccctaactcc 6900
   gcccatcccg cccctaactc cgcccagttc cgcccattct ccgccccatg gctgactaat 6960
   ttttttattt tatgcagagg ccgaggccgc ctcggcctct gagctattcc agaagtagtg 7020
   aggaggcttt tttggaggcc taggcttttg caaaaagctt gattcttctg acacaacagt 7080
   ctcgaactta aggctagagc caccatgatt gaacaagatg gattgcacgc aggttctccg 7140
   gccgcttggg tggagaggct attcggctat gactgggcac aacagacaat cggctgctct 7200
   gatgccgccg tgttccggct gtcagcgcag gggcgcccgg ttctttttgt caagaccgac 7260
   ctgtccggtg ccctgaatga actgcaggac gaggcagcgc ggctatcgtg gctggccacg 7320
   acgggcgttc cttgcgcagc tgtgctcgac gttgtcactg aagcgggaag ggactggctg 7380
   ctattgggcg aagtgccggg gcaggatctc ctgtcatctc accttgctcc tgccgagaaa 7440
   gtatccatca tggctgatgc aatgcggcgg ctgcatacgc ttgatccggc tacctgccca 7500
   ttcgaccacc aagcgaaaca tcgcatcgag cgagcacgta ctcggatgga agccggtctt 7560
   gtcgatcagg atgatctgga cgaagagcat caggggctcg cgccagccga actgttcgcc 7620
   aggctcaagg cgcgcatgcc cgacggcgag gatctcgtcg tgacccatgg cgatgcctgc 7680
   ttgccgaata tcatggtgga aaatggccgc ttttctggat tcatcgactg tggccggctg 7740
   ggtgtggcgg accgctatca ggacatagcg ttggctaccc gtgatattgc tgaagagctt 7800
   ggcggcgaat gggctgaccg cttcctcgtg ctttacggta tcgccgctcc cgattcgcag 7860
   cgcatcgcct tctatcgcct tcttgacgag ttcttctgag cgggactctg gggttcgaaa 7920
   tgaccgacca agcgacgccc aacctgccat cacgatggcc gcaataaaat atctttattt 7980
   tcattacatc tgtgtgttgg ttttttgtgt gaatcgatag cgataaggat ccgcgtatgg 8040
   tgcactctca gtacaatctg ctctgatgcc gcatagttaa gccagccccg acacccgcca 8100
   acacccgctg acgcgccctg acgggcttgt ctgctcccgg catccgctta cagacaagct 8160
   gtgaccgtct ccgggagctg catgtgtcag aggttttcac cgtcatcacc gaaacgcgcg 8220
   agacgaaagg gcctcgtgat acgcctattt ttataggtta atgtcatgat aataatggtt 8280
   tcttagacgt caggtggcac ttttcgggga aatgtgcgcg gaacccctat ttgtttattt 8340
   ttctaaatac attcaaatat gtatccgctc atgagacaat aaccctgata aatgcttcaa 8400
   taatattgaa aaaggaagag tatgagtatt caacatttcc gtgtcgccct tattcccttt 8460
   tttgcggcat tttgccttcc tgtttttgct cacccagaaa cgctggtgaa agtaaaagat 8520
   gctgaagatc agttgggtgc acgagtgggt tacatcgaac tggatctcaa cagcggtaag 8580
   atccttgaga gttttcgccc cgaagaacgt tttccaatga tgagcacttt taaagttctg 8640
   ctatgtggcg cggtattatc ccgtattgac gccgggcaag agcaactcgg tcgccgcata 8700
   cactattctc agaatgactt ggttgagtac tcaccagtca cagaaaagca tcttacggat 8760
   ggcatgacag taagagaatt atgcagtgct gccataacca tgagtgataa cactgcggcc 8820
   aacttacttc tgacaacgat cggaggaccg aaggagctaa ccgctttttt gcacaacatg 8880
   ggggatcatg taactcgcct tgatcgttgg gaaccggagc tgaatgaagc cataccaaac 8940
   gacgagcgtg acaccacgat gcctgtagca atggcaacaa cgttgcgcaa actattaact 9000
   ggcgaactac ttactctagc ttcccggcaa caattaatag actggatgga ggcggataaa 9060
   gttgcaggac cacttctgcg ctcggccctt ccggctggct ggtttattgc tgataaatct 9120
   ggagccggtg agcgtgggtc tcgcggtatc attgcagcac tggggccaga tggtaagccc 9180
   tcccgtatcg tagttatcta cacgacgggg agtcaggcaa ctatggatga acgaaataga 9240
   cagatcgctg agataggtgc ctcactgatt aagcattggt aactgtcaga ccaagtttac 9300
   tcatatatac tttagattga tttaaaactt catttttaat ttaaaaggat ctaggtgaag 9360
   atcctttttg ataatctcat gaccaaaatc ccttaacgtg agttttcgtt ccactgagcg 9420
   tcagaccccg tagaaaagat caaaggatct tcttgagatc ctttttttct gcgcgtaatc 9480
   tgctgcttgc aaacaaaaaa accaccgcta ccagcggtgg tttgtttgcc ggatcaagag 9540
   ctaccaactc tttttccgaa ggtaactggc ttcagcagag cgcagatacc aaatactgtc 9600
   cttctagtgt agccgtagtt aggccaccac ttcaagaact ctgtagcacc gcctacatac 9660
   ctcgctctgc taatcctgtt accagtggct gctgccagtg gcgataagtc gtgtcttacc 9720
   gggttggact caagacgata gttaccggat aaggcgcagc ggtcgggctg aacggggggt 9780
   tcgtgcacac agcccagctt ggagcgaacg acctacaccg aactgagata cctacagcgt 9840
   gagctatgag aaagcgccac gcttcccgaa gggagaaagg cggacaggta tccggtaagc 9900
   ggcagggtcg gaacaggaga gcgcacgagg gagcttccag ggggaaacgc ctggtatctt 9960
   tatagtcctg tcgggtttcg ccacctctga cttgagcgtc gatttttgtg atgctcgtca 10020
   ggggggcgga gcctatggaa aaacgccagc aacgcggcct ttttacggtt cctggccttt 10080
   tgctggcctt ttgctcacat ggctcgacag atct                            10114
<210> 72
   <211> 11131
   <212> DNA
   <213> Artificial Sequence
<220>
   <223> Description of Artificial Sequence: pONY4.0Z
<400> 72
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc aacctggctt atcgaaatta atacgactca 360
   ctatagggag accggcagat cttgaataat aaaatgtgtg tttgtccgaa atacgcgttt 420
   tgagatttct gtcgccgact aaattcatgt cgcgcgatag tggtgtttat cgccgataga 480
   gatggcgata ttggaaaaat tgatatttga aaatatggca tattgaaaat gtcgccgatg 540
   tgagtttctg tgtaactgat atcgccattt ttccaaaagt gatttttggg catacgcgat 600
   atctggcgat agcgcttata tcgtttacgg gggatggcga tagacgactt tggtgacttg 660
   ggcgattctg tgtgtcgcaa atatcgcagt ttcgatatag gtgacagacg atatgaggct 720
   atatcgccga tagaggcgac atcaagctgg cacatggcca atgcatatcg atctatacat 780
   tgaatcaata ttggccatta gccatattat tcattggtta tatagcataa atcaatattg 840
   gctattggcc attgcatacg ttgtatccat atcgtaatat gtacatttat attggctcat 900
   gtccaacatt accgccatgt tgacattgat tattgactag ttattaatag taatcaatta 960
   cggggtcatt agttcatagc ccatatatgg agttccgcgt tacataactt acggtaaatg 1020
   gcccgcctgg ctgaccgccc aacgaccccc gcccattgac gtcaataatg acgtatgttc 1080
   ccatagtaac gccaataggg actttccatt gacgtcaatg ggtggagtat ttacggtaaa 1140
   ctgcccactt ggcagtacat caagtgtatc atatgccaag tccgccccct attgacgtca 1200
   atgacggtaa atggcccgcc tggcattatg cccagtacat gaccttacgg gactttccta 1260
   cttggcagta catctacgta ttagtcatcg ctattaccat ggtgatgcgg ttttggcagt 1320
   acaccaatgg gcgtggatag cggtttgact cacggggatt tccaagtctc caccccattg 1380
   acgtcaatgg gagtttgttt tggcaccaaa atcaacggga ctttccaaaa tgtcgtaaca 1440
   actgcgatcg cccgccccgt tgacgcaaat gggcggtagg cgtgtacggt gggaggtcta 1500
   tataagcaga gctcgtttag tgaaccgggc actcagattc tgcggtctga gtcccttctc 1560
   tgctgggctg aaaaggcctt tgtaataaat ataattctct actcagtccc tgtctctagt 1620
   ttgtctgttc gagatcctac agttggcgcc cgaacaggga cctgagaggg gcgcagaccc 1680
   tacctgttga acctggctga tcgtaggatc cccgggacag cagaggagaa cttacagaag 1740
   tcttctggag gtgttcctgg ccagaacaca ggaggacagg taagatggga gaccctttga 1800
   catggagcaa ggcgctcaag aagttagaga aggtgacggt acaagggtct cagaaattaa 1860
   ctactggtaa ctgtaattgg gcgctaagtc tagtagactt atttcatgat accaactttg 1920
   taaaagaaaa ggactggcag ctgagggatg tcattccatt gctggaagat gtaactcaga 1980
   cgctgtcagg acaagaaaga gaggcctttg aaagaacatg gtgggcaatt tctgctgtaa 2040
   agatgggcct ccagattaat aatgtagtag atggaaaggc atcattccag ctcctaagag 2100
   cgaaatatga aaagaagact gctaataaaa agcagtctga gccctctgaa gaatatctct 2160
   agaactagtg gatcccccgg gctgcaggag tggggaggca cgatggccgc tttggtcgag 2220
   gcggatccgg ccattagcca tattattcat tggttatata gcataaatca atattggcta 2280
   ttggccattg catacgttgt atccatatca taatatgtac atttatattg gctcatgtcc 2340
   aacattaccg ccatgttgac attgattatt gactagttat taatagtaat caattacggg 2400
   gtcattagtt catagcccat atatggagtt ccgcgttaca taacttacgg taaatggccc 2460
   gcctggctga ccgcccaacg acccccgccc attgacgtca ataatgacgt atgttcccat 2520
   agtaacgcca atagggactt tccattgacg tcaatgggtg gagtatttac ggtaaactgc 2580
   ccacttggca gtacatcaag tgtatcatat gccaagtacg ccccctattg acgtcaatga 2640
   cggtaaatgg cccgcctggc attatgccca gtacatgacc ttatgggact ttcctacttg 2700
   gcagtacatc tacgtattag tcatcgctat taccatggtg atgcggtttt ggcagtacat 2760
   caatgggcgt ggatagcggt ttgactcacg gggatttcca agtctccacc ccattgacgt 2820
   caatgggagt ttgttttggc accaaaatca acgggacttt ccaaaatgtc gtaacaactc 2880
   cgccccattg acgcaaatgg gcggtaggca tgtacggtgg gaggtctata taagcagagc 2940
   tcgtttagtg aaccgtcaga tcgcctggag acgccatcca cgctgttttg acctccatag 3000
   aagacaccgg gaccgatcca gcctccgcgg ccccaagctt cagctgctcg aggatctgcg 3060
   gatccgggga attccccagt ctcaggatcc accatggggg atcccgtcgt tttacaacgt 3120
   cgtgactggg aaaaccctgg cgttacccaa cttaatcgcc ttgcagcaca tccccctttc 3180
   gccagctggc gtaatagcga agaggcccgc accgatcgcc cttcccaaca gttgcgcagc 3240
   ctgaatggcg aatggcgctt tgcctggttt ccggcaccag aagcggtgcc ggaaagctgg 3300
   ctggagtgcg atcttcctga ggccgatact gtcgtcgtcc cctcaaactg gcagatgcac 3360
   ggttacgatg cgcccatcta caccaacgta acctatccca ttacggtcaa tccgccgttt 3420
   gttcccacgg agaatccgac gggttgttac tcgctcacat ttaatgttga tgaaagctgg 3480
   ctacaggaag gccagacgcg aattattttt gatggcgtta actcggcgtt tcatctgtgg 3540
   tgcaacgggc gctgggtcgg ttacggccag gacagtcgtt tgccgtctga atttgacctg 3600
   agcgcatttt tacgcgccgg agaaaaccgc ctcgcggtga tggtgctgcg ttggagtgac 3660
   ggcagttatc tggaagatca ggatatgtgg cggatgagcg gcattttccg tgacgtctcg 3720
   ttgctgcata aaccgactac acaaatcagc gatttccatg ttgccactcg ctttaatgat 3780
   gatttcagcc gcgctgtact ggaggctgaa gttcagatgt gcggcgagtt gcgtgactac 3840
   ctacgggtaa cagtttcttt atggcagggt gaaacgcagg tcgccagcgg caccgcgcct 3900
   ttcggcggtg aaattatcga tgagcgtggt ggttatgccg atcgcgtcac actacgtctg 3960
   aacgtcgaaa acccgaaact gtggagcgcc gaaatcccga atctctatcg tgcggtggtt 4020
   gaactgcaca ccgccgacgg cacgctgatt gaagcagaag cctgcgatgt cggtttccgc 4080
   gaggtgcgga ttgaaaatgg tctgctgctg ctgaacggca agccgttgct gattcgaggc 4140
   gttaaccgtc acgagcatca tcctctgcat ggtcaggtca tggatgagca gacgatggtg 4200
   caggatatcc tgctgatgaa gcagaacaac tttaacgccg tgcgctgttc gcattatccg 4260
   aaccatccgc tgtggtacac gctgtgcgac cgctacggcc tgtatgtggt ggatgaagcc 4320
   aatattgaaa cccacggcat ggtgccaatg aatcgtctga ccgatgatcc gcgctggcta 4380
   ccggcgatga gcgaacgcgt aacgcgaatg gtgcagcgcg atcgtaatca cccgagtgtg 4440
   atcatctggt cgctggggaa tgaatcaggc cacggcgcta atcacgacgc gctgtatcgc 4500
   tggatcaaat ctgtcgatcc ttcccgcccg gtgcagtatg aaggcggcgg agccgacacc 4560
   acggccaccg atattatttg cccgatgtac gcgcgcgtgg atgaagacca gcccttcccg 4620
   gctgtgccga aatggtccat caaaaaatgg ctttcgctac ctggagagac gcgcccgctg 4680
   atcctttgcg aatacgccca cgcgatgggt aacagtcttg gcggtttcgc taaatactgg 4740
   caggcgtttc gtcagtatcc ccgtttacag ggcggcttcg tctgggactg ggtggatcag 4800
   tcgctgatta aatatgatga aaacggcaac ccgtggtcgg cttacggcgg tgattttggc 4860
   gatacgccga acgatcgcca gttctgtatg aacggtctgg tctttgccga ccgcacgccg 4920
   catccagcgc tgacggaagc aaaacaccag cagcagtttt tccagttccg tttatccggg 4980
   caaaccatcg aagtgaccag cgaatacctg ttccgtcata gcgataacga gctcctgcac 5040
   tggatggtgg cgctggatgg taagccgctg gcaagcggtg aagtgcctct ggatgtcgct 5100
   ccacaaggta aacagttgat tgaactgcct gaactaccgc agccggagag cgccgggcaa 5160
   ctctggctca cagtacgcgt agtgcaaccg aacgcgaccg catggtcaga agccgggcac 5220
   atcagcgcct ggcagcagtg gcgtctggcg gaaaacctca gtgtgacgct ccccgccgcg 5280
   tcccacgcca tcccgcatct gaccaccagc gaaatggatt tttgcatcga gctgggtaat 5340
   aagcgttggc aatttaaccg ccagtcaggc tttctttcac agatgtggat tggcgataaa 5400
   aaacaactgc tgacgccgct gcgcgatcag ttcacccgtg caccgctgga taacgacatt 5460
   ggcgtaagtg aagcgacccg cattgaccct aacgcctggg tcgaacgctg gaaggcggcg 5520
   ggccattacc aggccgaagc agcgttgttg cagtgcacgg cagatacact tgctgatgcg 5580
   gtgctgatta cgaccgctca cgcgtggcag catcagggga aaaccttatt tatcagccgg 5640
   aaaacctacc ggattgatgg tagtggtcaa atggcgatta ccgttgatgt tgaagtggcg 5700
   agcgatacac cgcatccggc gcggattggc ctgaactgcc agctggcgca ggtagcagag 5760
   cgggtaaact ggctcggatt agggccgcaa gaaaactatc ccgaccgcct tactgccgcc 5820
   tgttttgacc gctgggatct gccattgtca gacatgtata ccccgtacgt cttcccgagc 5880
   gaaaacggtc tgcgctgcgg gacgcgcgaa ttgaattatg gcccacacca gtggcgcggc 5940
   gacttccagt tcaacatcag ccgctacagt caacagcaac tgatggaaac cagccatcgc 6000
   catctgctgc acgcggaaga aggcacatgg ctgaatatcg acggtttcca tatggggatt 6060
   ggtggcgacg actcctggag cccgtcagta tcggcggaat tccagctgag cgccggtcgc 6120
   taccattacc agttggtctg gtgtcaaaaa taataataac cgggcagggg ggatccgcag 6180
   atccggctgt ggaatgtgtg tcagttaggg tgtggaaagt ccccaggctc cccagcaggc 6240
   agaagtatgc aaagcatgcc tgcaggaatt cgatatcaag cttatcgata ccgtcgacct 6300
   cgaggggggg cccggtaccc agcttttgtt ccctttagtg agggttaatt gcgcgggaag 6360
   tatttatcac taatcaagca caagtaatac atgagaaact tttactacag caagcacaat 6420
   cctccaaaaa attttgtttt tacaaaatcc ctggtgaaca tgattggaag ggacctacta 6480
   gggtgctgtg gaagggtgat ggtgcagtag tagttaatga tgaaggaaag ggaataattg 6540
   ctgtaccatt aaccaggact aagttactaa taaaaccaaa ttgagtattg ttgcaggaag 6600
   caagacccaa ctaccattgt cagctgtgtt tcctgaggtc tctaggaatt gattacctcg 6660
   atgcttcatt aaggaagaag aataaacaaa gactgaaggc aatccaacaa ggaagacaac 6720
   ctcaatattt gttataaggt ttgatatatg ggagtatttg gtaaaggggt aacatggtca 6780
   gcatcgcatt ctatggggga atcccagggg gaatctcaac ccctattacc caacagtcag 6840
   aaaaatctaa gtgtgaggag aacacaatgt ttcaacctta ttgttataat aatgacagta 6900
   agaacagcat ggcagaatcg aaggaagcaa gagaccaaga aatgaacctg aaagaagaat 6960
   ctaaagaaga aaaaagaaga aatgactggt ggaaaatagg tatgtttctg ttatgcttag 7020
   caggaactac tggaggaata ctttggtggt atgaaggact cccacagcaa cattatatag 7080
   ggttggtggc gataggggga agattaaacg gatctggcca atcaaatgct atagaatgct 7140
   ggggttcctt cccggggtgt agaccatttc aaaattactt cagttatgag accaatagaa 7200
   gcatgcatat ggataataat actgctacat tattagaagc tttaaccaat ataactgctc 7260
   tataaataac aaaacagaat tagaaacatg gaagttagta aagacttctg gcataactcc 7320
   tttacctatt tcttctgaag ctaacactgg actaattaga cataagagag attttggtat 7380
   aagtgcaata gtggcagcta ttgtagccgc tactgctatt gctgctagcg ctactatgtc 7440
   ttatgttgct ctaactgagg ttaacaaaat aatggaagta caaaatcata cttttgaggt 7500
   agaaaatagt actctaaatg gtatggattt aatagaacga caaataaaga tattatatgc 7560
   tatgattctt caaacacatg cagatgttca actgttaaag gaaagacaac aggtagagga 7620
   gacatttaat ttaattggat gtatagaaag aacacatgta ttttgtcata ctggtcatcc 7680
   ctggaatatg tcatggggac atttaaatga gtcaacacaa tgggatgact gggtaagcaa 7740
   aatggaagat ttaaatcaag agatactaac tacacttcat ggagccagga acaatttggc 7800
   acaatccatg ataacattca atacaccaga tagtatagct caatttggaa aagacctttg 7860
   gagtcatatt ggaaattgga ttcctggatt gggagcttcc attataaaat atatagtgat 7920
   gtttttgctt atttatttgt tactaacctc ttcgcctaag atcctcaggg ccctctggaa 7980
   ggtgaccagt ggtgcagggt cctccggcag tcgttacctg aagaaaaaat tccatcacaa 8040
   acatgcatcg cgagaagaca cctgggacca ggcccaacac aacatacacc tagcaggcgt 8100
   gaccggtgga tcaggggaca aatactacaa gcagaagtac tccaggaacg actggaatgg 8160
   agaatcagag gagtacaaca ggcggccaaa gagctgggtg aagtcaatcg aggcatttgg 8220
   agagagctat atttccgaga agaccaaagg ggagatttct cagcctgggg cggctatcaa 8280
   cgagcacaag aacggctctg gggggaacaa tcctcaccaa gggtccttag acctggagat 8340
   tcgaagcgaa ggaggaaaca tttatgactg ttgcattaaa gcccaagaag gaactctcgc 8400
   tatcccttgc tgtggatttc ccttatggct attttgggga ctagtaatta tagtaggacg 8460
   catagcaggc tatggattac gtggactcgc tgttataata aggatttgta ttagaggctt 8520
   aaatttgata tttgaaataa tcagaaaaat gcttgattat attggaagag ctttaaatcc 8580
   tggcacatct catgtatcaa tgcctcagta tgtttagaaa aacaaggggg gaactgtggg 8640
   gtttttatga ggggttttat aaatgattat aagagtaaaa agaaagttgc tgatgctctc 8700
   ataaccttgt ataacccaaa ggactagctc atgttgctag gcaactaaac cgcaataacc 8760
   gcatttgtga cgcgagttcc ccattggtga cgcgttaact tcctgttttt acagtatata 8820
   agtgcttgta ttctgacaat tgggcactca gattctgcgg tctgagtccc ttctctgctg 8880
   ggctgaaaag gcctttgtaa taaatataat tctctactca gtccctgtct ctagtttgtc 8940
   tgttcgagat cctacagagc tcatgccttg gcgtaatcat ggtcatagct gtttcctgtg 9000
   tgaaattgtt atccgctcac aattccacac aacatacgag ccggaagcat aaagtgtaaa 9060
   gcctggggtg cctaatgagt gagctaactc acattaattg cgttgcgctc actgcccgct 9120
   ttccagtcgg gaaacctgtc gtgccagctg cattaatgaa tcggccaacg cgcggggaga 9180
   ggcggtttgc gtattgggcg ctcttccgct tcctcgctca ctgactcgct gcgctcggtc 9240
   gttcggctgc ggcgagcggt atcagctcac tcaaaggcgg taatacggtt atccacagaa 9300
   tcaggggata acgcaggaaa gaacatgtga gcaaaaggcc agcaaaaggc caggaaccgt 9360
   aaaaaggccg cgttgctggc gtttttccat aggctccgcc cccctgacga gcatcacaaa 9420
   aatcgacgct caagtcagag gtggcgaaac ccgacaggac tataaagata ccaggcgttt 9480
   ccccctggaa gctccctcgt gcgctctcct gttccgaccc tgccgcttac cggatacctg 9540
   tccgcctttc tcccttcggg aagcgtggcg ctttctcata gctcacgctg taggtatctc 9600
   agttcggtgt aggtcgttcg ctccaagctg ggctgtgtgc acgaaccccc cgttcagccc 9660
   gaccgctgcg ccttatccgg taactatcgt cttgagtcca acccggtaag acacgactta 9720
   tcgccactgg cagcagccac tggtaacagg attagcagag cgaggtatgt aggcggtgct 9780
   acagagttct tgaagtggtg gcctaactac ggctacacta gaaggacagt atttggtatc 9840
   tgcgctctgc tgaagccagt taccttcgga aaaagagttg gtagctcttg atccggcaaa 9900
   caaaccaccg ctggtagcgg tggttttttt gtttgcaagc agcagattac gcgcagaaaa 9960
   aaaggatctc aagaagatcc tttgatcttt tctacggggt ctgacgctca gtggaacgaa 10020
   aactcacgtt aagggatttt ggtcatgaga ttatcaaaaa ggatcttcac ctagatcctt 10080
   ttaaattaaa aatgaagttt taaatcaatc taaagtatat atgagtaaac ttggtctgac 10140
   agttaccaat gcttaatcag tgaggcacct atctcagcga tctgtctatt tcgttcatcc 10200
   atagttgcct gactccccgt cgtgtagata actacgatac gggagggctt accatctggc 10260
   cccagtgctg caatgatacc gcgagaccca cgctcaccgg ctccagattt atcagcaata 10320
   aaccagccag ccggaagggc cgagcgcaga agtggtcctg caactttatc cgcctccatc 10380
   cagtctatta attgttgccg ggaagctaga gtaagtagtt cgccagttaa tagtttgcgc 10440
   aacgttgttg ccattgctac aggcatcgtg gtgtcacgct cgtcgtttgg tatggcttca 10500
   ttcagctccg gttcccaacg atcaaggcga gttacatgat cccccatgtt gtgcaaaaaa 10560
   gcggttagct ccttcggtcc tccgatcgtt gtcagaagta agttggccgc agtgttatca 10620
   ctcatggtta tggcagcact gcataattct cttactgtca tgccatccgt aagatgcttt 10680
   tctgtgactg gtgagtactc aaccaagtca ttctgagaat agtgtatgcg gcgaccgagt 10740
   tgctcttgcc cggcgtcaat acgggataat accgcgccac atagcagaac tttaaaagtg 10800
   ctcatcattg gaaaacgttc ttcggggcga aaactctcaa ggatcttacc gctgttgaga 10860
   tccagttcga tgtaacccac tcgtgcaccc aactgatctt cagcatcttt tactttcacc 10920
   agcgtttctg ggtgagcaaa aacaggaagg caaaatgccg caaaaaaggg aataagggcg 10980
   acacggaaat gttgaatact catactcttc ctttttcaat attattgaag catttatcag 11040
   ggttattgtc tcatgagcgg atacatattt gaatgtattt agaaaaataa acaaataggg 11100
   gttccgcgca catttccccg aaaagtgcca c 11131
<210> 73
   <211> 517
   <212> DNA
   <213> Artificial Sequence
<220>
   <223> Description of Artificial Sequence: Codon optimised EIAV REV
<400> 73
   gaattcgcca ccatggctga gagcaaggag gccagggatc aagagatgaa cctcaaggaa 60
   gagagcaaag aggagaagcg ccgcaacgac tggtggaaga tcgacccaca aggccccctg 120
   gagggggacc agtggtgccg cgtgctgaga cagtccctgc ccgaggagaa gattcctagc 180
   cagacctgca tcgccagaag acacctcggc cccggtccca cccagcacac accctccaga 240
   agggataggt ggattagggg ccagattttg caagccgagg tcctccaaga aaggctggaa 300
   tggagaatta ggggcgtgca acaagccgct aaagagctgg gagaggtgaa tcgcggcatc 360
   tggagggagc tctacttccg cgaggaccag aggggcgatt tctccgcatg gggaggctac 420
   cagagggcac aagaaaggct gtggggcgag cagagcagcc cccgcgtctt gaggcccgga 480
   gactccaaaa gacgccgcaa acacctgtga agtcgac                          517